(19) Europäisches Patentamt
European Patent Office
Office européen des brevets

(11) EP 4 678 652 A1

(12) EUROPEAN PATENT APPLICATION
published in accordance with Art. 153(4) EPC

(43) Date of publication:
**14.01.2026 Bulletin 2026/03**

(21) Application number: **24792752.8**

(22) Date of filing: **19.04.2024**

(51) International Patent Classification (IPC):
***C07K 7/52*** $^{(2006.01)}$ ***C07K 1/02*** $^{(2006.01)}$
***C07K 1/30*** $^{(2006.01)}$ ***C07K 1/34*** $^{(2006.01)}$
***C07K 7/06*** $^{(2006.01)}$ ***C07K 7/64*** $^{(2006.01)}$

(52) Cooperative Patent Classification (CPC):
**C07K 1/02; C07K 1/30; C07K 1/34; C07K 7/06; C07K 7/52; C07K 7/64**

(86) International application number:
**PCT/JP2024/015529**

(87) International publication number:
**WO 2024/219480 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.04.2023 JP 2023069189**

(71) Applicant: **CHUGAI SEIYAKU KABUSHIKI KAISHA**
**Tokyo 115-8543 (JP)**

(72) Inventors:
- **HORIBE Takahiro**
  **Yokohama City, Kanagawa 244-8602 (JP)**
- **SHIINA Junichi**
  **Yokohama City, Kanagawa 244-8602 (JP)**
- **WANG Yinli**
  **Yokohama City, Kanagawa 244-8602 (JP)**
- **INOMATA Yuuki**
  **Yokohama City, Kanagawa 244-8602 (JP)**

(74) Representative: **Vossius & Partner**
**Patentanwälte Rechtsanwälte mbB**
**Siebertstrasse 3**
**81675 München (DE)**

# (54) METHOD FOR PRODUCING CYCLIC PEPTIDE COMPOUND

(57) As a cyclization method in the production of a cyclic peptide compound having a double bond cross-linked between amino acids, there is provided a cyclization method by means of a cyclization position that can reduce the amount produced of a cyclic dimer, which is a by-product. There is also provided, as a method for producing a cyclization precursor peptide compound, an efficient production method in which three fragment peptides are each synthesized and liquid phase fragment coupling is performed for synthesis. Furthermore, there are provided a crystal of a cyclic peptide compound and a method for producing the crystal by crystallization.

*Fig.1*

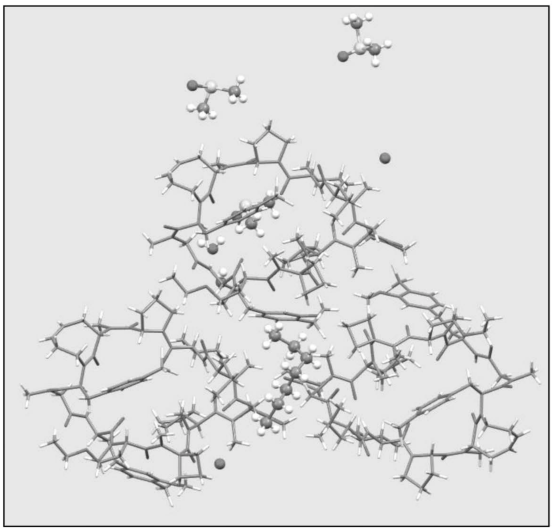

EP 4 678 652 A1

## Description

[Technical Field]

The present invention relates to a method for producing a cyclic peptide compound, in particular, a method for producing a cyclic peptide compound having selective inhibitory action on KRAS over HRAS and NRAS.

[Background Art]

RAS is a protein belonging to the small GTPase family, and KRAS, NRAS, and HRAS are known. RAS is defined as being in an activated or an inactivated state depending on its binding state to GDP or GTP. It is activated by the exchange reaction from GDP to GTP by GEFs (guanine nucleotide exchange factors) and inactivated by the hydrolysis reaction of GTP by GAPs (GTPase-activating proteins) (Non Patent Literature 1). Activated RAS induces cell proliferation, survival, and differentiation by activating various downstream signals in the MAPK pathway, PI3K/Akt pathway, RAL pathway, and others, and the constitutive activation of RAS plays an important role in the development and progression of cancer. In cancer, it is known that the RAS-RAF-MEK-ERK pathway is activated by the activation of upstream signals of RAS, constitutive activation of RAS, and/or activating mutations of RAS (Non Patent Literature 2). These activating mutations of RAS have been found in numerous cancer types. G12, G13, and Q61 are known as hot spots of RAS mutation, and G12 is frequently found mutated in KRAS and Q61 in NRAS. These mutations are also known to be associated with the prognosis of patients (Non Patent Literature 3).

In this regard, a cyclic peptide compound represented by the following formula (1), which has selectivity in RAS, specifically selective inhibitory action on KRAS over HRAS and NRAS (hereinafter, also referred to as cyclic peptide compound (1)), has been reported (Patent Literature 1).

[Formula 1]

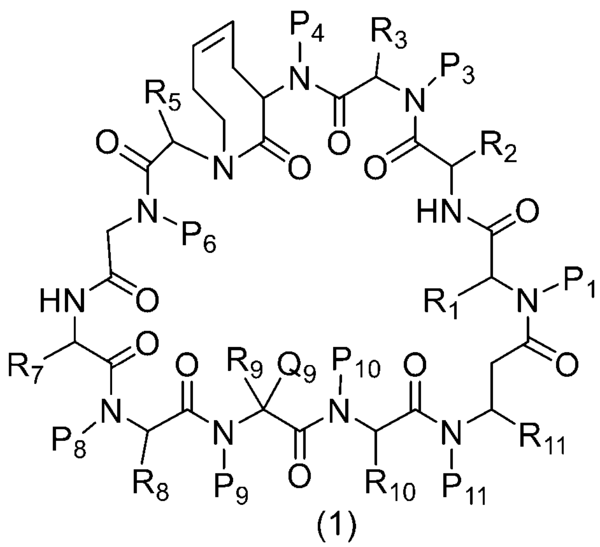

(1)

[Citation List]

[Patent Literature]

[Patent Literature 1]
International Publication No. WO 2022/234853

[Non Patent Literature]

[Non Patent Literature 1]
Nat. Rev. Drug Discov. 2014 Nov; 13(11): 828-851.

[Non Patent Literature 2]
Nat. Rev. Drug Discov. 2014 Dec; 13(12): 928-942.
[Non Patent Literature 3]
Nat. Rev. Drug Discov. 2016 Nov; 15(11): 771-785.

[Summary of Invention]

[Technical Problem]

As far as the present inventors are aware, there are no reported examples as to which position of the cyclization precursor peptide compound is preferred for cyclization in the production of the cyclic peptide compound (1), which has a double bond crosslinked between amino acids. Patent Literature 1 describes a method for producing the cyclic peptide compound (1) by a cyclization reaction at the cyclization position A. However, as described in Examples 1-26 below, in the production method described in Patent Literature 1, a cyclic dimer is found as a by-product in addition to the target cyclic peptide compound (1), and the ratio between the cyclic peptide compound (1) and the cyclic dimer is 75:25, which is low in selectivity.

[Formula 2]

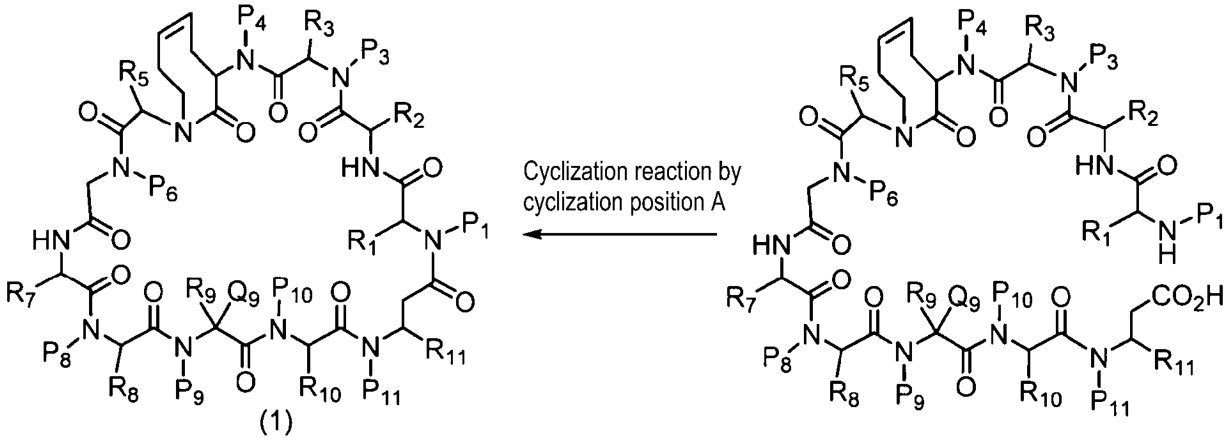


(1)

In addition, the production of the cyclization precursor peptide compound described in Patent Literature 1 is performed by sequential linkage of amino acids or some tripeptides by the Fmoc method and synthesis on a solid phase. Since solid phase synthesis requires excessive amounts of amino acids and reagents and a large amount of organic solvents for washing in each step, it has been desirable to avoid solid phase synthesis whenever possible for mass production. Also, as far as the present inventors are aware, there are no reported examples as to crystals of the cyclic peptide compound (1).

The present invention has been made in view of such situations, and an object thereof is to provide an efficient method for producing the cyclic peptide compound (1) having a double bond crosslinked between amino acids.

Another object of the present invention is to provide a crystal with excellent stability of the cyclic peptide compound (1).

[Solution to Problem]

In view of the above circumstances, the present inventors have intensively studied and finally completed the present invention. That is, in one aspect, the present invention provides, as a cyclization method in the production of the cyclic peptide compound (1) having a double bond crosslinked between amino acids, a cyclization method by means of a cyclization position that can reduce the amount of a cyclic dimer, which is a by-product. In one aspect, the present invention also provides, as a method for producing the cyclization precursor peptide compound, an efficient production method in which three fragment peptides are each synthesized and liquid phase fragment coupling is performed for synthesis. In one aspect, the present invention further provides a crystal of the cyclic peptide compound (1) and a method for producing the crystal by crystallization.

In one non-limiting specific aspect, the present invention encompasses the following.

[A1] A method for producing a cyclic peptide compound represented by formula (1), or a salt thereof, or a solvate

thereof, the method comprising a step of reacting an N-terminal amino acid residue of a peptide compound represented by formula (2) or (3) with a C-terminal amino acid residue of the peptide compound in a solvent for cyclization (cyclization step):

[Formula 3]

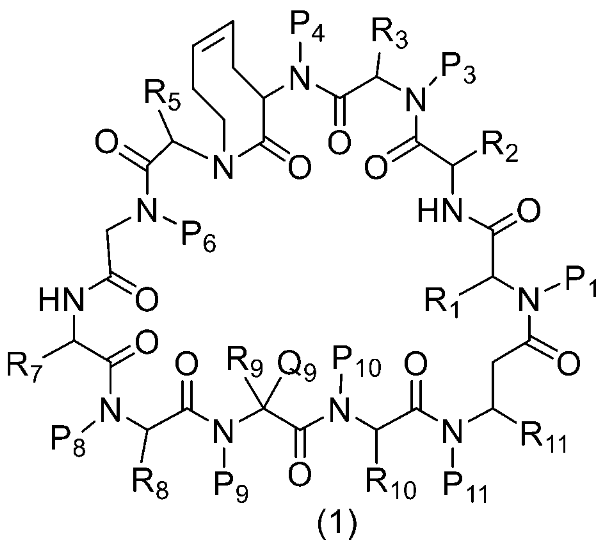

(1)

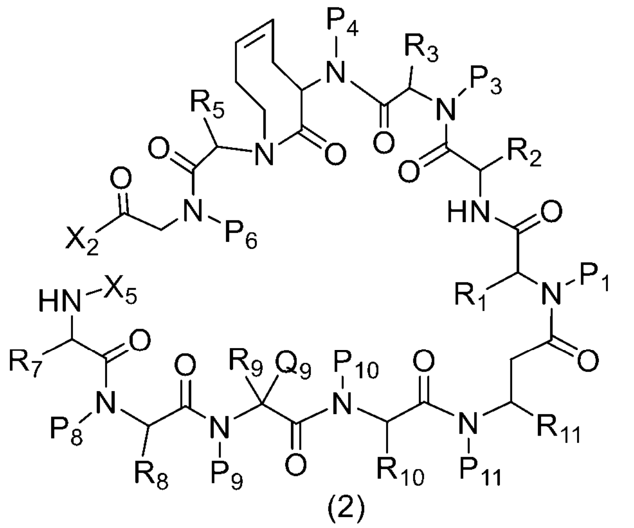

(2)

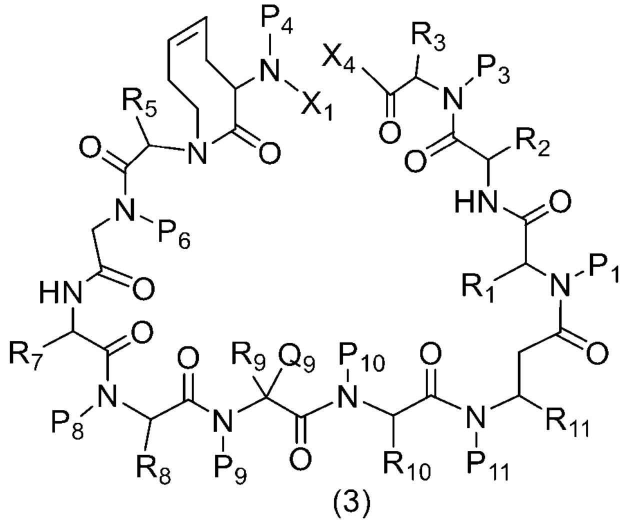

(3)

wherein $R_1$ is a $C_1$-$C_6$ alkyl;

$P_1$ is a $C_1$-$C_6$ alkyl;
$R_2$ is a $C_1$-$C_6$ alkyl;
$R_3$ is hydrogen, or $R_3$ forms a 4- to 7-membered saturated heterocyclic ring together with $P_3$, a carbon atom to which $R_3$ is bonded, and a nitrogen atom to which $P_3$ is bonded;
$P_3$ is a $C_1$-$C_6$ alkyl or a $C_3$-$C_8$ cycloalkyl, or $P_3$ forms a 4- to 7-membered saturated heterocyclic ring together with $R_3$, a carbon atom to which $R_3$ is bonded, and a nitrogen atom to which $P_3$ is bonded;
$P_4$ is a $C_1$-$C_6$ alkyl;
$R_5$ is benzyl optionally substituted with one or more groups selected from the group consisting of a $C_1$-$C_6$ alkyl, a $C_1$-$C_6$ haloalkyl, and a $C_3$-$C_8$ cycloalkyl;
$P_6$ is a $C_1$-$C_6$ alkyl;
$R_7$ is phenethyl optionally substituted with one or more groups selected from the group consisting of a halogen, a $C_1$-$C_6$ haloalkyl, and a $C_1$-$C_6$ alkoxy;
$R_8$ forms a 4- to 7-membered saturated heterocyclic ring together with $P_8$, a carbon atom to which $R_8$ is bonded, and a nitrogen atom to which $P_8$ is bonded, the 4- to 7-membered saturated heterocyclic ring optionally substituted with a $C_1$-$C_6$ alkoxy;
$R_9$ forms a 3- to 8-membered alicyclic ring together with $Q_9$ and a carbon atom to which $R_9$ and $Q_9$ are bonded, the 3- to 8-membered alicyclic ring optionally substituted with one or more $C_1$-$C_6$ alkyls;
$P_9$ is hydrogen or a $C_1$-$C_6$ alkyl;
$R_{10}$ is a $C_1$-$C_6$ alkyl or a $C_3$-$C_8$ cycloalkyl;
$P_{10}$ is a $C_1$-$C_6$ alkyl;
$R_{11}$ is a di-$C_1$-$C_6$ alkylaminocarbonyl or a 4- to 8-membered cyclic aminocarbonyl;

$P_{11}$ is a $C_1$-$C_6$ alkyl;
$X_1$ and $X_5$ are each independently hydrogen or a protecting group for an amino group; and
$X_2$ and $X_4$ are each independently a halogen, a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

[A2] A method for producing a cyclic peptide compound represented by formula (1), or a salt thereof, or a solvate thereof, the method comprising:

(a) a step of providing peptide compounds represented by formulae (4) to (6), or salts thereof, or solvates of the peptide compounds or salts;
(b) a step of reacting N-terminal amino acid residues of the peptide compounds represented by formulae (4) to (6) with C-terminal amino acid residues of the peptide compounds in a solvent for linkage (linking step); and
(c) a step of reacting an N-terminal amino acid residue of a peptide compound obtained in the step (b) with a C-terminal amino acid residue of the peptide compound in a solvent for cyclization (cyclization step):

[Formula 4]

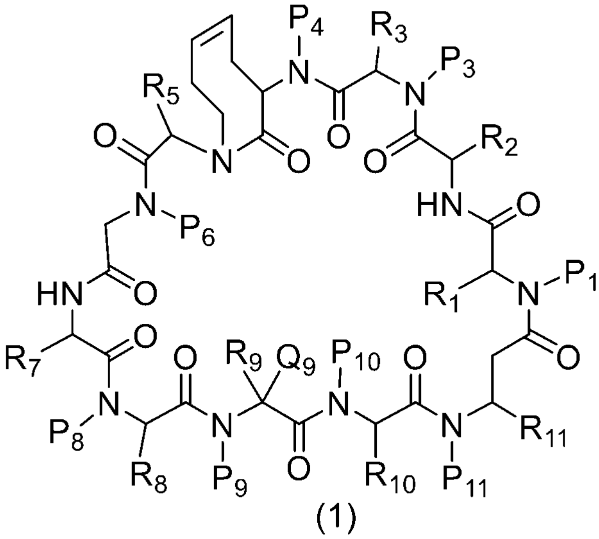

(1)

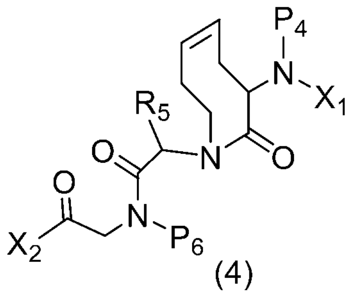

(4)

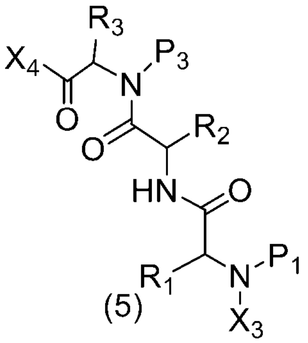

(5)

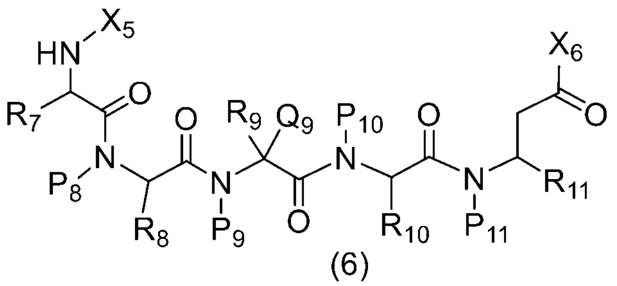

(6)

wherein $R_1$ is a $C_1$-$C_6$ alkyl;

$P_1$ is a $C_1$-$C_6$ alkyl;
$R_2$ is a $C_1$-$C_6$ alkyl;
$R_3$ is hydrogen, or $R_3$ forms a 4- to 7-membered saturated heterocyclic ring together with $P_3$, a carbon atom to which $R_3$ is bonded, and a nitrogen atom to which $P_3$ is bonded;
$P_3$ is a $C_1$-$C_6$ alkyl or a $C_3$-$C_8$ cycloalkyl, or $P_3$ forms a 4- to 7-membered saturated heterocyclic ring together with $R_3$, a carbon atom to which $R_3$ is bonded, and a nitrogen atom to which $P_3$ is bonded;
$P_4$ is a $C_1$-$C_6$ alkyl;
$R_5$ is benzyl optionally substituted with one or more groups selected from the group consisting of a $C_1$-$C_6$ alkyl, a $C_1$-$C_6$ haloalkyl, and a $C_3$-$C_8$ cycloalkyl;
$P_6$ is a $C_1$-$C_6$ alkyl;
$R_7$ is phenethyl optionally substituted with one or more groups selected from the group consisting of a halogen, a $C_1$-$C_6$ haloalkyl, and a $C_1$-$C_6$ alkoxy;
$R_8$ forms a 4- to 7-membered saturated heterocyclic ring together with $P_8$, a carbon atom to which $R_8$ is bonded, and a nitrogen atom to which $P_8$ is bonded, the 4- to 7-membered saturated heterocyclic ring optionally substituted with a $C_1$-$C_6$ alkoxy;
$R_9$ forms a 3- to 8-membered alicyclic ring together with $Q_9$ and a carbon atom to which $R_9$ and $Q_9$ are bonded, the 3- to 8-membered alicyclic ring optionally substituted with one or more $C_1$-$C_6$ alkyls;

$P_9$ is hydrogen or a $C_1$-$C_6$ alkyl;
$R_{10}$ is a $C_1$-$C_6$ alkyl or a $C_3$-$C_8$ cycloalkyl;
$P_{10}$ is a $C_1$-$C_6$ alkyl;
$R_{11}$ is a di-$C_1$-$C_6$ alkylaminocarbonyl or a 4- to 8-membered cyclic aminocarbonyl;
$P_{11}$ is a $C_1$-$C_6$ alkyl;
$X_1$, $X_3$, and $X_5$ are each independently hydrogen or a protecting group for an amino group; and
$X_2$, $X_4$, and $X_6$ are each independently a halogen, a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

[A3] The method according to [A2], comprising, in the step (b),

(b-1) a step of reacting an N-terminal amino acid residue of the peptide compound represented by formula (5) with a C-terminal amino acid residue of the peptide compound represented by formula (6) in a solvent for linkage, thereby converting them into a peptide compound represented by formula (7) (linking step):

[Formula 5]

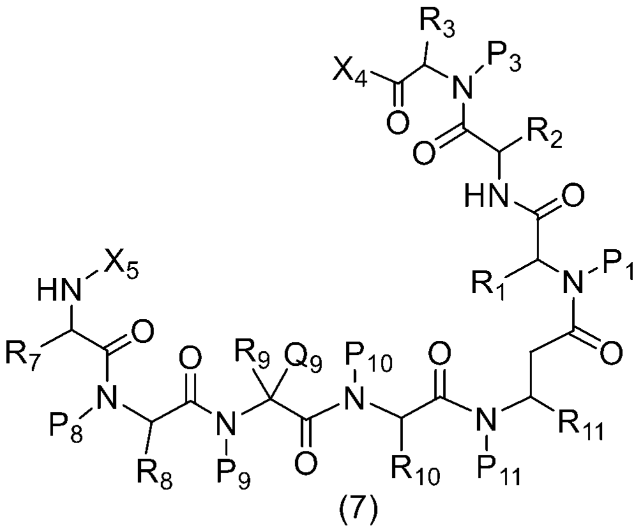

(7)

wherein $R_1$, $R_2$, $R_3$, $R_7$, $R_8$, $R_9$, $R_{10}$, $R_{11}$, $P_1$, $P_3$, $P_8$, $P_9$, $P_{10}$, $P_{11}$, $Q_9$, $X_4$, and $X_5$ are the same as in [A2].

[A4] The method according to [A3], further comprising, in the step (b),

(b-2) a step of reacting an N-terminal amino acid residue of the peptide compound represented by formula (4) with a C-terminal amino acid residue of the peptide compound represented by formula (7) in a solvent for linkage, thereby converting them into a peptide compound represented by formula (2) (linking step), and
in the step (c),
(c-1) a step of reacting an N-terminal amino acid residue of the peptide compound represented by formula (2) with a C-terminal amino acid residue of the peptide compound in a solvent for cyclization (cyclization step).

[A5] The method according to [A3], further comprising, in the step (b),

(b-3) a step of reacting an N-terminal amino acid residue of the peptide compound represented by formula (7) with a C-terminal amino acid residue of the peptide compound represented by formula (4) in a solvent for linkage, thereby converting them into a compound represented by formula (3) (linking step), and
in the step (c),
(c-2) a step of reacting an N-terminal amino acid residue of the peptide compound represented by formula (3) with a C-terminal amino acid residue of the peptide compound in a solvent for cyclization (cyclization step).

[A6] The method according to any of [A1] to [A5], wherein the linkage of an N-terminal amino acid residue of a peptide compound with a C-terminal amino acid residue of a peptide compound is linkage of an amino group of the N-terminal

amino acid residue with a carboxyl group of the C-terminal amino acid residue.

[A7] The method according to any of [A1] to [A6], wherein the linkage of an N-terminal amino acid residue of a peptide compound with a C-terminal amino acid residue of a peptide compound is linkage by means of an amide bond of an amino group of the N-terminal amino acid residue with a carboxyl group of the C-terminal amino acid residue.

[A8] The method according to any of [A1] to [A7], wherein the solvent in the cyclization step includes one or more selected from the group consisting of a nitrile-based solvent, a halogen-based solvent, an ether-based solvent, an amide-based solvent, an ester-based solvent, and a carbonate-based solvent.

[A9] The method according to [A8],

wherein the nitrile-based solvent is one or more selected from the group consisting of acetonitrile and propionitrile,

wherein the halogen-based solvent is one or more selected from the group consisting of dichloromethane, chloroform, and 1,2-dichloroethane,

wherein the ether-based solvent is one or more selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, t-butyl methyl ether, diglyme, triglyme, anisole, and tetraglyme,

wherein the amide-based solvent is one or more selected from the group consisting of DMF, NMP, DMA, NEP, NBP, and formamide,

wherein the ester-based solvent is one or more selected from the group consisting of methyl acetate, ethyl acetate, methyl propionate, butyl acetate, propyl acetate, isopropyl acetate, isobutyl acetate, pentyl acetate, and $\gamma$-valerolactone, and

wherein the carbonate-based solvent is one or more selected from the group consisting of dimethyl carbonate, diethyl carbonate, and dibutyl carbonate.

[A10] The method according to [A8], wherein the solvent in the cyclization step is one or more selected from the group consisting of acetonitrile, dimethyl carbonate, 2-methyltetrahydropyran, 4-methyltetrahydropyran, tetrahydrofuran, ethyl acetate, isopropyl acetate, dichloromethane, DMF, and anisole.

[A11] The method according to [A8], wherein the solvent in the cyclization step is one or more selected from the group consisting of acetonitrile, 2-methyltetrahydrofuran, ethyl acetate, and dichloromethane.

[A12] The method according to [A8], wherein the solvent in the cyclization step is acetonitrile, 2-methyltetrahydrofuran, or ethyl acetate.

[A13] The method according to any of [A1] to [A12], wherein the cyclization step is performed in the presence of a condensation reagent.

[A14] The method according to [A13], wherein the condensation reagent in the cyclization step is one or more selected from the group consisting of HATU, COMU, DMT-MM, PyOxim, PyBOP, HCTU, T3P, EDCI, BEP, and PyClop.

[A15] The method according to [A13], wherein the condensation reagent in the cyclization step is one selected from the group consisting of HATU, COMU, PyOxim, PyBOP, HCTU, and T3P.

[A16] The method according to [A13], wherein the condensation reagent in the cyclization step is HATU.

[A17] The method according to [A13], wherein the condensation reagent in the cyclization step is COMU.

[A18] The method according to [A13], wherein the condensation reagent in the cyclization step is HATU and the solvent in the cyclization step is acetonitrile.

[A19] The method according to [A13], wherein the condensation reagent in the cyclization step is HATU and the solvent in the cyclization step is 2-methyltetrahydrofuran.

[A20] The method according to [A13], wherein the condensation reagent in the cyclization step is COMU and the solvent in the cyclization step is acetonitrile.

[A21] The method according to [A13], wherein the condensation reagent in the cyclization step is COMU and the solvent in the cyclization step is 2-methyltetrahydrofuran.

[A22] The method according to any of [A1] to [A21], wherein the cyclization step is performed in the presence of a base.

[A23] The method according to [A22], wherein the base in the cyclization step is an organic base.

[A24] The method according to [A22], wherein the base in the cyclization step is an organic base comprising a tertiary amine.

[A25] The method according to [A22], wherein the base in the cyclization step is one or more selected from the group consisting of 2,2,6,6-tetramethylpiperidine, N-methylmorpholine, N,N-diisopropylethylamine (DIPEA), 2,4,6-collidine, 2,6-lutidine, pyridine, 1,8-diazabicyclo[5.4.0]-7-undecene (DBU), 2,3,6,7-tetrahydro-1H,5H-9-azabenzo[ij]quinolidine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-ethylmorpholine, and p-dimethylaminopyridine (DMAP).

[A26] The method according to [A22], wherein the base in the cyclization step is one or more selected from the group

consisting of 2,2,6,6-tetramethylpiperidine, N-methylmorpholine, N,N-diisopropylethylamine (DIPEA), 2,4,6-collidine, 2,6-lutidine, and pyridine.

[A27] The method according to [A22], wherein the condensation reagent in the cyclization step is HATU, the solvent in the cyclization step is acetonitrile, and the base in the cyclization step is N,N-diisopropylethylamine (DIPEA).

[A28] The method according to [A22], wherein the condensation reagent in the cyclization step is HATU, the solvent in the cyclization step is 2-MeTHF, and the base in the cyclization step is N,N-diisopropylethylamine (DIPEA).

[A29] The method according to [A22], wherein the condensation reagent in the cyclization step is COMU, the solvent in the cyclization step is acetonitrile, and the base in the cyclization step is 2,6-lutidine.

[A30] The method according to [A22], wherein the condensation reagent in the cyclization step is COMU, the solvent in the cyclization step is 2-methyltetrahydrofuran, and the base in the cyclization step is 2,6-lutidine.

[A31] The method according to any of [A1] to [A30], wherein the cyclization step is performed by a liquid phase method.

[A32] The method according to any of [A1] to [A31], wherein in the cyclization step, the peptide compound and the base are mixed in a mixed solution obtained by mixing the solvent and condensation reagent in the cyclization step.

[A33] The method according to any of [A1] to [A32], wherein a content of the total by-products generated in the cyclization step is less than 20%, less than 15%, less than 10%, less than 5%, or less than 3%, as determined by UVarea value at 220 nm by HPLC analysis, based on the total amount of products.

[A34] The method according to any of [A1] to [A33], wherein a content of each of by-products generated in the cyclization step is less than 15%, less than 10%, less than 5%, less than 3%, less than 1%, or an undetectable amount, as determined by UVarea value at 220 nm by HPLC analysis, based on the total amount of products.

[A35] The method according to any of [A1] to [A34], wherein a content of each of by-products generated in the cyclization step is less than 15%, less than 10%, less than 5%, less than 3%, less than 1%, or an undetectable amount, as determined by UVarea value at 220 nm by HPLC analysis, based on the total amount of products, and the by-products comprise an epimer and/or a cyclic dimer.

[A36] The method according to any of [A1] to [A35], wherein by-products generated in the cyclization step comprise an epimer, and a content of the epimer is less than 10%, less than 7.5%, less than 5%, less than 2.5%, or less than 1%, as determined by UVarea value at 220 nm by HPLC analysis, based on the total amount of products.

[A37] The method according to any of [A1] to [A36], wherein by-products generated in the cyclization step comprise a cyclic dimer, and a content of the cyclic dimer is less than 15%, less than 10%, less than 5%, less than 2.5%, or less than 1%, as determined by UVarea value at 220 nm by HPLC analysis, based on the total amount of products.

[A38] The method according to any of [A1] to [A37], wherein the solvent in the linking step includes one or more selected from the group consisting of a nitrile-based solvent, a halogen-based solvent, an ether-based solvent, an amide-based solvent, an ester-based solvent, and a carbonate-based solvent.

[A39] The method according to [A38],

wherein the nitrile-based solvent is one or more selected from the group consisting of acetonitrile and propionitrile,

wherein the halogen-based solvent is one or more selected from the group consisting of dichloromethane, chloroform and 1,2-dichloroethane,

wherein the ether-based solvent is one or more selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, t-butyl methyl ether, diglyme, triglyme, anisole and tetraglyme,

wherein the amide-based solvent is one or more selected from the group consisting of DMF, NMP, DMA, NEP, NBP and formamide,

wherein the ester-based solvent is one or more selected from the group consisting of methyl acetate, ethyl acetate, methyl propionate, butyl acetate, propyl acetate, isopropyl acetate, isobutyl acetate, pentyl acetate and γ-valerolactone, and

wherein the carbonate-based solvent is one or more selected from the group consisting of dimethyl carbonate, diethyl carbonate and dibutyl carbonate.

[A40] The method according to [A38], wherein the solvent in the linking step is one or more selected from the group consisting of acetonitrile, dimethyl carbonate, 2-methyltetrahydrofuran, 4-methyltetrahydropyran, THF, ethyl acetate, isopropyl acetate, DMF, and anisole.

[A41] The method according to [A38], wherein the solvent in the linking step is a mixed solvent of one or more selected from the group consisting of acetonitrile, dimethyl carbonate, 2-methyltetrahydrofuran, 4-methyltetrahydropyran, THF, ethyl acetate, isopropyl acetate, and anisole with DMF.

[A42] The method according to [A38], wherein the solvent in the linking step is a mixed solvent of acetonitrile, 2-methyltetrahydrofuran, and DMF.

[A43] The method according to [A38], wherein the solvent in the linking step is a mixed solvent of 2-methyltetrahydrofuran and DMF.

[A44] The method according to any of [A1] to [A43], wherein the linking step is performed in the presence of a condensation reagent.

[A45] The method according to [A44], wherein the condensation reagent in the linking step is one or more selected from the group consisting of HATU, COMU, DMT-MM, PyOxim, PyBOP, HCTU, T3P, EDCI, BEP, and PyClop.

[A46] The method according to [A44], wherein the condensation reagent in the linking step is one selected from the group consisting of HATU, COMU, PyOxim, PyBOP, HCTU, and T3P.

[A47] The method according to [A44], wherein the condensation reagent in the linking step is one selected from the group consisting of HATU and COMU.

[A48] The method according to [A44], wherein the condensation reagent in the linking step is HATU.

[A49] The method according to [A44], wherein the condensation reagent in the linking step is COMU.

[A50] The method according to [A44], wherein the condensation reagent in the linking step is HATU and the solvent in the linking step is acetonitrile or 2-MeTHF.

[A51] The method according to [A44], wherein the condensation reagent in the linking step is HATU and the solvent in the linking step is a mixed solvent of acetonitrile, 2-MeTHF, and DMF.

[A52] The method according to [A44], wherein the condensation reagent in the linking step is COMU and the solvent in the linking step is acetonitrile or 2-MeTHF.

[A53] The method according to [A44], wherein the condensation reagent in the linking step is COMU and the solvent in the linking step is a mixed solvent of acetonitrile, 2-MeTHF, and DMF.

[A54] The method according to any of [A1] to [A53], wherein the linking step is performed in the presence of a base.

[A55] The method according to [A54], wherein the base in the linking step is an organic base.

[A56] The method according to [A54], wherein the base in the linking step is an organic base comprising a tertiary amine.

[A57] The method according to [A54], wherein the base in the linking step is one or more selected from the group consisting of 2,2,6,6-tetramethylpiperidine, N-methylmorpholine, N,N-diisopropylethylamine (DIPEA), 2,4,6-collidine, 2,6-lutidine, pyridine, 1,8-diazabicyclo[5.4.0]-7-undecene (DBU), 2,3,6,7-tetrahydro-1H,5H-9-azabenzo[ij]quinolidine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-ethylmorpholine, and p-dimethylaminopyridine (DMAP).

[A58] The method according to [A54], wherein the base in the linking step is one or more selected from the group consisting of 2,2,6,6-tetramethylpiperidine, N-methylmorpholine, N,N-diisopropylethylamine (DIPEA), 2,4,6-collidine, 2,6-lutidine, and pyridine.

[A59] The method according to [A54], wherein the condensation reagent in the linking step is HATU, the solvent in the linking step is acetonitrile or 2-MeTHF, and the base in the linking step is N,N-diisopropylethylamine (DIPEA).

[A60] The method according to [A54], wherein the condensation reagent in the linking step is HATU, the solvent in the linking step is a mixed solvent of acetonitrile, 2-methyltetrahydrofuran, and DMF, and the base in the linking step is N,N-diisopropylethylamine (DIPEA).

[A61] The method according to [A54], wherein the condensation reagent in the linking step is COMU, the solvent in the linking step is acetonitrile or 2-methyltetrahydrofuran, and the base in the linking step is N-methylmorpholine or 2,6-lutidine.

[A62] The method according to [A54], wherein the condensation reagent in the linking step is COMU, the solvent in the linking step is a mixed solvent of acetonitrile, 2-methyltetrahydrofuran, and DMF, and the base in the linking step is N-methylmorpholine or 2,6-lutidine.

[A62-1] The method according to [A54], wherein the condensation reagent in the linking step is HATU, the solvent in the linking step is a mixed solvent of acetonitrile and 2-methyltetrahydrofuran, and the base in the linking step is N-methylmorpholine.

[A62-2] The method according to [A54], wherein the condensation reagent in the linking step is HATU, the solvent in the linking step is acetonitrile, and the base in the linking step is N-methylmorpholine.

[A63] The method according to any of [A1] to [A62], wherein the linking step is performed by a liquid phase method.

[A64] The method according to any of [A1] to [A63], wherein column chromatography is used for isolation and/or purification of the cyclic peptide compound, or a salt thereof, or a solvate thereof.

[A65] The method according to any of [A1] to [A63], wherein column chromatography is not used for isolation and/or purification of the cyclic peptide compound, or a salt thereof, or a solvate thereof.

[A66] The method according to any of [A1] to [A65], further comprising a step of isolating and/or purifying the cyclic peptide compound, or a salt thereof, or a solvate thereof by crystallization to obtain a crystal of the cyclic peptide compound, or a salt thereof, or a solvate thereof.

[A67] The method according to any of [A1] to [A66], wherein $R_1$ is a $C_3$-$C_4$ alkyl.

[A67-1] The method according to any of [A1] to [A66], wherein $R_1$ is n-propyl.

[A67-2] The method according to any of [A1] to [A66], wherein $R_1$ is 2-methylpropyl.
[A68] The method according to any of [A1] to [A67], wherein $P_1$ is a $C_1$-$C_4$ alkyl.
[A68-1] The method according to any of [A1] to [A67], wherein $P_1$ is methyl.
[A69] The method according to any of [A1] to [A68], wherein $R_2$ is a $C_3$-$C_4$ alkyl.
[A69-1] The method according to any of [A1] to [A68], wherein $R_2$ is 1-methylpropyl.
[A70] The method according to any of [A1] to [A69], wherein $R_3$ is hydrogen, or $R_3$ forms a 5-membered saturated heterocyclic ring together with $P_3$, a carbon atom to which $R_3$ is bonded, and a nitrogen atom to which $P_3$ is bonded.
[A70-1] The method according to any of [A1] to [A69], wherein $R_3$ is hydrogen.
[A70-2] The method according to any of [A1] to [A69], wherein $R_3$ forms a 5-membered saturated heterocyclic ring together with $P_3$, a carbon atom to which $R_3$ is bonded, and a nitrogen atom to which $P_3$ is bonded.
[A71] The method according to any of [A1] to [A70], wherein $P_3$ is a $C_1$-$C_4$ alkyl, or $P_3$ forms a 5-membered saturated heterocyclic ring together with $R_3$, a carbon atom to which $R_3$ is bonded, and a nitrogen atom to which $P_3$ is bonded.
[A71-1] The method according to any of [A 1] to [A70], wherein $P_3$ is methyl.
[A71-2] The method according to any of [A1] to [A70], wherein $P_3$ forms a 5-membered saturated heterocyclic ring together with $R_3$, a carbon atom to which $R_3$ is bonded, and a nitrogen atom to which $P_3$ is bonded.
[A72] The method according to any of [A1] to [A71], wherein $P_4$ is a $C_1$-$C_4$ alkyl.
[A72-1] The method according to any of [A1] to [A71], wherein $P_4$ is methyl.
[A73] The method according to any of [A1] to [A72], wherein $R_5$ is benzyl optionally substituted with a $C_1$-$C_4$ haloalkyl.
[A73-1] The method according to any of [A1] to [A72], wherein $R_5$ is 4-trifluoromethylbenzyl.
[A74] The method according to any of [A1] to [A73], wherein $P_6$ is a $C_1$-$C_4$ alkyl.
[A74-1] The method according to any of [A1] to [A73], wherein $P_6$ is methyl.
[A75] The method according to any of [A1] to [A74], wherein $R_7$ is phenethyl optionally substituted with one or more groups selected from the group consisting of a halogen, trifluoromethyl, and methoxy.
[A75-1] The method according to any of [A1] to [A74], wherein $R_7$ is 3-methoxy-4-trifluoromethylphenethyl.
[A75-2] The method according to any of [A1] to [A74], wherein $R_7$ is 3,5-difluoro-4-trifluoromethylphenethyl.
[A76] The method according to any of [A1] to [A75], wherein $R_8$ forms a 5-membered saturated heterocyclic ring together with $P_8$, a carbon atom to which $R_8$ is bonded, and a nitrogen atom to which $P_8$ is bonded, the 5-membered saturated heterocyclic ring substituted with a $C_1$-$C_4$ alkyl.
[A76-1] The method according to any of [A1] to [A75], wherein $R_8$ forms a 5-membered saturated heterocyclic ring together with $P_8$, a carbon atom to which $R_8$ is bonded, and a nitrogen atom to which $P_8$ is bonded, the 5-membered saturated heterocyclic ring substituted with ethoxy.
[A77] The method according to any of [A1] to [A76], wherein $R_9$ forms a 4- to 6-membered alicyclic ring together with $Q_9$ and a carbon atom to which $R_9$ and $Q_9$ are bonded.
[A77-1] The method according to any of [A1] to [A76], wherein $R_9$ forms a 4-membered alicyclic ring together with $Q_9$ and a carbon atom to which $R_9$ and $Q_9$ are bonded.
[A77-2] The method according to any of [A1] to [A76], wherein $R_9$ forms a 5-membered alicyclic ring together with $Q_9$ and a carbon atom to which $R_9$ and $Q_9$ are bonded.
[A78] The method according to any of [A1] to [A77], wherein $P_9$ is hydrogen or a $C_1$-$C_4$ alkyl.
[A78-1] The method according to any of [A1] to [A77], wherein $P_9$ is hydrogen.
[A78-2] The method according to any of [A1] to [A77], wherein $P_9$ is methyl.
[A79] The method according to any of [A1] to [A78], wherein $R_{10}$ is a $C_4$-$C_6$ cycloalkyl.
[A79-1] The method according to any of [A1] to [A78], wherein $R_{10}$ is cyclopentyl.
[A80] The method according to any of [A1] to [A79], wherein $P_{10}$ is a $C_1$-$C_4$ alkyl.
[A80-1] The method according to any of [A1] to [A79], wherein $P_{10}$ is methyl.
[A81] The method according to any of [A1] to [A80], wherein $R_{11}$ is a di-$C_1$-$C_4$ alkylaminocarbonyl or a 5- to 6-membered cyclic aminocarbonyl.
[A81-1] The method according to any of [A 1] to [A80], wherein $R_{11}$ is dimethylaminocarbonyl.
[A82] The method according to any of [A1] to [A81], wherein $P_{11}$ is a $C_1$-$C_4$ alkyl.
[A82-1] The method according to any of [A1] to [A81], wherein $P_{11}$ is methyl.
[A83] The method according to any of [A1] to [A82], wherein $X_1$, $X_3$, and $X_5$ are each independently one selected from the group consisting of hydrogen, a carbamate-based protecting group, an acyl-based protecting group, a sulfonamide-based protecting group, and a silyl-based protecting group.
[A84] The method according to [A83], wherein the carbamate-based protecting group is one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group.
[A85] The method according to [A83], wherein the acyl-based protecting group is one selected from the group consisting of a trifluoroacetyl group, an acetyl group, and a benzoyl group.
[A86] The method according to [A83], wherein the sulfonamide-based protecting group is one selected from the group

consisting of a 2-nitrobenzenesulfonyl group, a 4-nitrobenzenesulfonyl group, and a 2,4-dinitrobenzenesulfonyl group.

[A87] The method according to [A83], wherein the silyl-based protecting group is one selected from the group consisting of a TMS group, a TBDMS group, a TES group, a TIPS group, and a TBDPS group.

[A88] The method according to any of [A1] to [A87], wherein $X_1$ is hydrogen or a carbamate-based protecting group.

[A88-1] The method according to any of [A1] to [A87], wherein $X_1$ is hydrogen.

[A88-2] The method according to any of [A1] to [A87], wherein $X_1$ is an Fmoc group.

[A89] The method according to any of [A1] to [A88], wherein $X_3$ is hydrogen or a carbamate-based protecting group.

[A89-1] The method according to any of [A1] to [A88], wherein $X_3$ is hydrogen.

[A89-2] The method according to any of [A1] to [A88], wherein $X_3$ is a Cbz group.

[A90] The method according to any of [A1] to [A89], wherein $X_5$ is hydrogen or a carbamate-based protecting group.

[A90-1] The method according to any of [A1] to [A89], wherein $X_5$ is hydrogen.

[A90-2] The method according to any of [A1] to [A89], wherein $X_5$ is a Cbz group.

[A91] The method according to any of [A1] to [A90], wherein $X_2$, $X_4$, and $X_6$ are each independently a halogen, a hydroxy group, an optionally substituted $C_1$-$C_6$ alkoxy, an optionally substituted $C_6$-$C_{10}$ aryloxy, an optionally substituted $C_7$-$C_{14}$ aralkoxy, an optionally substituted 4-to 8-membered cyclic aminooxy, or a group represented by -$OSiR_xR_yR_z$, wherein $R_x$, $R_y$, and $R_z$ are each independently a $C_1$-$C_6$ alkyl or a $C_6$-$C_{10}$ aryl.

[A91-1] The method according to any of [A1] to [A90], wherein the halogen is chlorine or bromine.

[A91-2] The method according to any of [A1] to [A90], wherein the optionally substituted alkoxy is t-butoxy, methoxy, ethoxy, or isopropoxy.

[A91-3] The method according to any of [A1] to [A90], wherein the optionally substituted aryloxy is pentafluorophenyloxy or nitrophenyloxy.

[A91-4] The method according to any of [A1] to [A90], wherein the optionally substituted aralkoxy is optionally substituted benzyloxy.

[A91-5] The method according to any of [A1] to [A90], wherein the optionally substituted cyclic aminooxy is N-hydroxysucciniminooxy.

[A91-6] The method according to any of [A1] to [A90], wherein the group represented by - $OSiR_xR_yR_z$ is trimethylsilyloxy, triethylsilyloxy, triisopropylsilyloxy, triphenylsilyloxy, tri-t-butylsilyloxy, di-t-butylisobutylsilyloxy, or tris(triethylsilyl)silyloxy.

[A92] The method according to any of [A1] to [A91], wherein $X_2$ is a hydroxy group, t-butoxy, or benzyloxy.

[A92-1] The method according to any of [A1] to [A91], wherein $X_2$ is a hydroxy group.

[A92-2] The method according to any of [A1] to [A91], wherein $X_2$ is t-butoxy.

[A93] The method according to any of [A1] to [A92], wherein $X_4$ is a hydroxy group, t-butoxy, or benzyloxy.

[A93-1] The method according to any of [A1] to [A92], wherein $X_4$ is a hydroxy group.

[A93-2] The method according to any of [A1] to [A92], wherein $X_4$ is t-butoxy.

[A94] The method according to any of [A1] to [A93], wherein $X_6$ is a hydroxy group, t-butoxy, or benzyloxy.

[A94-1] The method according to any of [A1] to [A93], wherein $X_6$ is a hydroxy group.

[A94-2] The method according to any of [A1] to [A93], wherein $X_6$ is t-butoxy.

[A95] The method according to any of [A1] to [A94], wherein the cyclic peptide compound, or a salt thereof, or a solvate thereof is a solvate of the cyclic peptide compound.

[A95-1] The method according to [A95], wherein the solvate of the cyclic peptide compound is a hydrate of the cyclic peptide compound.

[A96] The method according to any of [A1] to [A95], wherein the cyclic peptide compound is represented by formula (1a):

[Formula 6]

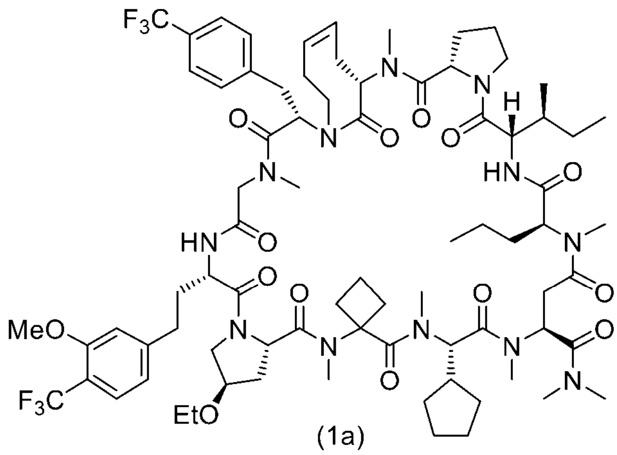

(1a)

.

[A97] The method according to any of [A1] to [A95], wherein the cyclic peptide compound is a crystal of a cyclic peptide compound represented by formula (1a):

[Formula 7]

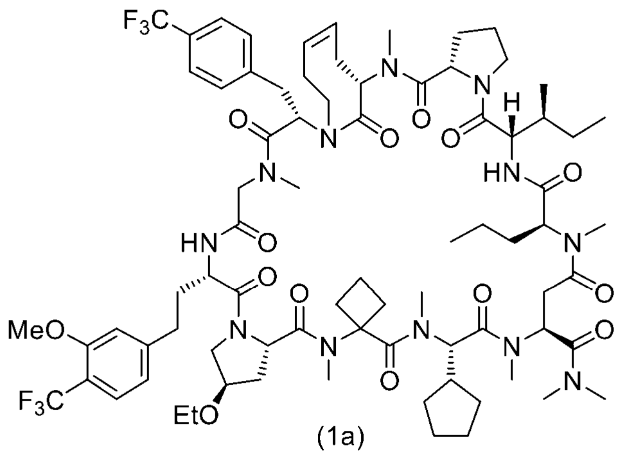

(1a)

.

[A97-1] The method according to [A97], wherein the crystal of a cyclic peptide compound is a non-solvate crystal or a solvate crystal.
[A97-2] The method according to [A97], wherein the crystal of a cyclic peptide compound is a solvate crystal.
[A97-3] The method according to [A97-2], wherein the solvate crystal of a cyclic peptide compound is a hydrate crystal.
[B1] A method for producing a cyclic peptide compound represented by formula (1a), or a salt thereof, or a solvate thereof, the method comprising a step of reacting an N-terminal amino acid residue of a peptide compound represented by formula (2a) or (3a) with a C-terminal amino acid residue of the peptide compound in a solvent for cyclization (cyclization step):

[Formula 8]

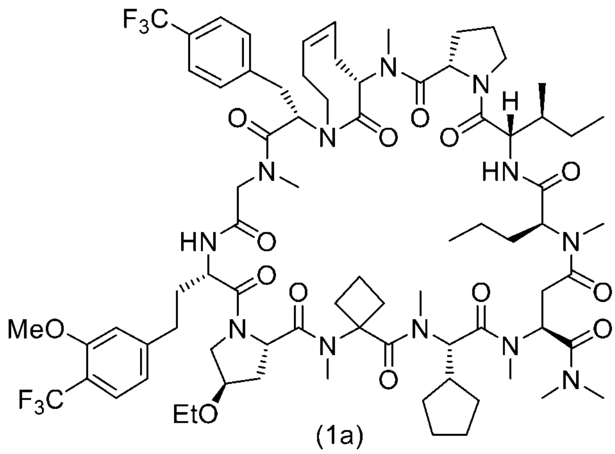

(1a)

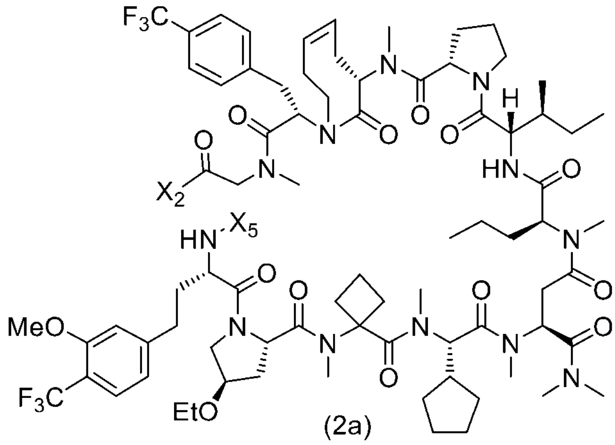

(2a)

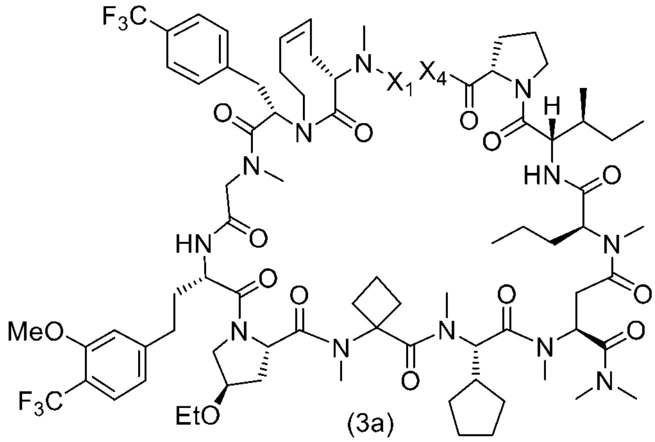

(3a)

wherein $X_1$ and $X_5$ are each independently hydrogen or a protecting group for an amino group; and $X_2$ and $X_4$ are each independently a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

[B2] A method for producing a cyclic peptide compound represented by formula (1a), or a salt thereof, or a solvate thereof, the method comprising:

(a) a step of providing peptide compounds represented by formulae (4a) to (6a), or salts thereof, or solvates of the peptide compounds or salts;

(b) a step of reacting N-terminal amino acid residues of the peptide compounds represented by formulae (4a) to

(6a) with C-terminal amino acid residues of the peptide compounds in a solvent for linkage (linking step); and (c) a step of reacting an N-terminal amino acid residue of a peptide compound obtained in the step (b) with a C-terminal amino acid residue of the peptide compound in a solvent for cyclization (cyclization step):

[Formula 9]

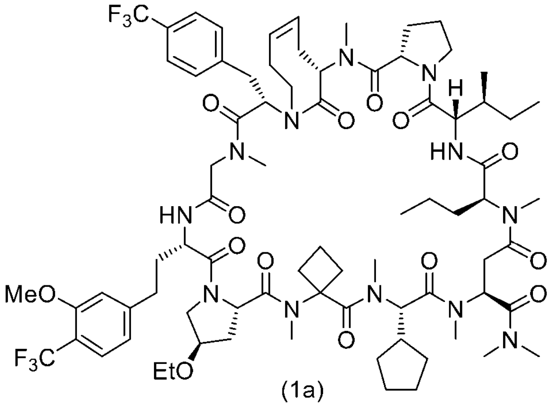

(1a)

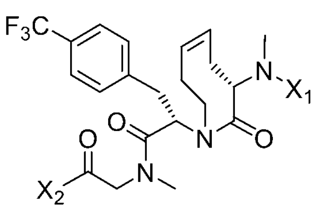

(4a)

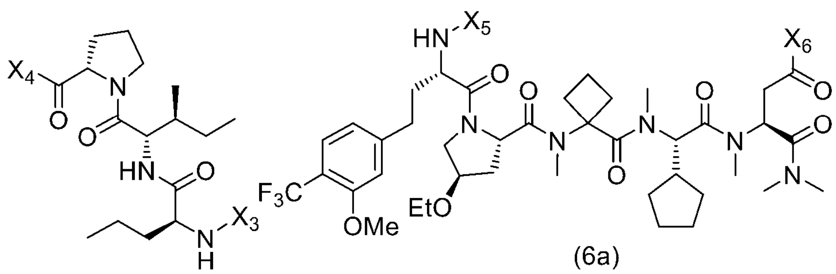

(5a) (6a)

wherein $X_1$, $X_3$, and $X_5$ are each independently hydrogen or a protecting group for an amino group; and

$X_2$, $X_4$, and $X_6$ are each independently a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

[B3] The method according to [B2], comprising, in the step (b),

(b-1) a step of reacting an N-terminal amino acid residue of the peptide compound represented by formula (5a) with a C-terminal amino acid residue of the peptide compound represented by formula (6a) in a solvent for linkage, thereby converting them into a peptide compound represented by formula (7a) (linking step):

[Formula 10]

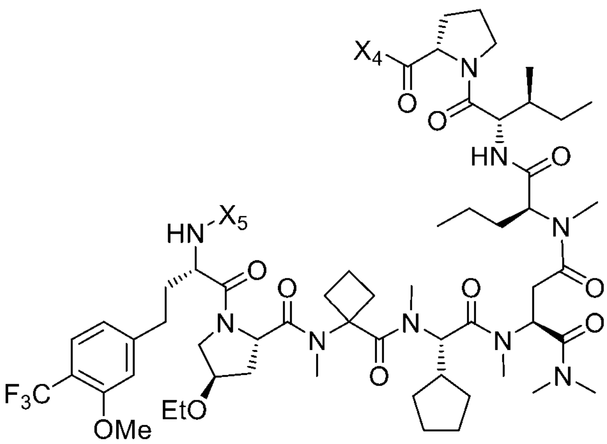

(7a)

wherein $X_4$ and $X_5$ are the same as in [B2].

[B4] The method according to [B3], further comprising, in the step (b),

(b-2) a step of reacting an N-terminal amino acid residue of the peptide compound represented by formula (4a) with a C-terminal amino acid residue of the peptide compound represented by formula (7a) in a solvent for linkage, thereby converting them into a peptide compound represented by formula (2a) (linking step), and
in the step (c),
(c-1) a step of reacting an N-terminal amino acid residue of the peptide compound represented by formula (2a) with a C-terminal amino acid residue of the peptide compound in a solvent for cyclization (cyclization step).

[B5] The method according to [B3], further comprising, in the step (b),

(b-3) a step of reacting an N-terminal amino acid residue of the peptide compound represented by formula (7a) with a C-terminal amino acid residue of the peptide compound represented by formula (4a) in a solvent for linkage, thereby converting them into a peptide compound represented by formula (3a) (linking step), and
in the step (c),
(c-2) a step of reacting an N-terminal amino acid residue of the peptide compound represented by formula (3a) with a C-terminal amino acid residue of the peptide compound in a solvent for cyclization (cyclization step).

[B6] The method according to any of [B1] to [B5], wherein the solvent in the cyclization step is the solvent according to any of [A8] to [A12].
[B7] The method according to any of [B1] to [B6], wherein the cyclization step is performed in the presence of a condensation reagent.
[B8] The method according to [B7], wherein the condensation reagent in the cyclization step is the condensation reagent according to any of [A14] to [A17].
[B9] The method according to [B7], wherein the solvent and condensation reagent in the cyclization step is the solvent and condensation reagent according to any of [A18] to [A21].
[B10] The method according to any of [B1] to [B9], wherein the cyclization step is performed in the presence of a base.
[B11] The method according to [B10], wherein the base in the cyclization step is the base according to any of [A24] to [A26].
[B12] The method according to [B10], wherein the solvent, condensation reagent, and base in the cyclization step is the solvent, condensation reagent, and base according to any of [A27] to [A30].
[B13] The method according to any of [B1] to [B12], wherein the cyclization step is performed by a liquid phase method.
[B14] The method according to any of [B1] to [B13], wherein in the cyclization step, the peptide compound and the base are mixed in a mixed solution obtained by mixing the solvent and condensation reagent in the cyclization step.
[B15] The method according to any of [B1] to [B14], wherein a content of the total by-products generated in the cyclization step is less than 20%, less than 15%, less than 10%, less than 5%, or less than 3%, as determined by UVarea value at 220 nm by HPLC analysis, based on the total amount of products.
[B16] The method according to any of [B1] to [B15], wherein a content of each of by-products generated in the

cyclization step is less than 15%, less than 10%, less than 5%, less than 3%, less than 1%, or an undetectable amount, as determined by UVarea value at 220 nm by HPLC analysis, based on the total amount of products.

[B17] The method according to any of [B1] to [B16], wherein a content of each of by-products generated in the cyclization step is less than 15%, less than 10%, less than 5%, less than 3%, less than 1%, or an undetectable amount, as determined by UVarea value at 220 nm by HPLC analysis, based on the total amount of products, and the by-products comprise an epimer and/or a cyclic dimer.

[B18] The method according to any of [B1] to [B17], wherein by-products generated in the cyclization step comprise an epimer, and a content of the epimer is less than 10%, less than 7.5%, less than 5%, less than 2.5%, or less than 1%, as determined by UVarea value at 220 nm by HPLC analysis, based on the total amount of products.

[B19] The method according to any of [B1] to [B18], wherein by-products generated in the cyclization step comprise a cyclic dimer, and a content of the cyclic dimer is less than 15%, less than 10%, less than 5%, less than 2.5%, or less than 1%, as determined by UVarea value at 220 nm by HPLC analysis, based on the total amount of products.

[B20] The method according to any of [B1] to [B19], wherein the solvent in the linking step is the solvent according to any of [A38] to [A43].

[B21] The method according to any of [B1] to [B20], wherein the linking step is performed in the presence of a condensation reagent.

[B22] The method according to [B21], wherein the condensation reagent in the linking step is the condensation reagent according to any of [A45] to [A49].

[B23] The method according to [B21], wherein the solvent and condensation reagent in the linking step is the solvent and condensation reagent according to any of [A50] to [A53].

[B24] The method according to any of [B1] to [B23], wherein the linking step is performed in the presence of a base.

[B25] The method according to [B24], wherein the base in the linking step is the base according to any of [A55] to [A59].

[B26] The method according to [B24], wherein the solvent, condensation reagent, and base in the linking step is the solvent, condensation reagent, and base according to any of [A60] to [A62].

[B27] The method according to any of [B1] to [B26], wherein the linking step is performed by a liquid phase method.

[B28] The method according to any of [B1] to [B27], wherein column chromatography is used for isolation and/or purification of the cyclic peptide compound, or a salt thereof, or a solvate thereof.

[B29] The method according to any of [B1] to [B27], wherein column chromatography is not used for isolation and/or purification of the cyclic peptide compound, or a salt thereof, or a solvate thereof.

[B30] The method according to any of [B1] to [B29], further comprising a step of isolating and/or purifying the cyclic peptide compound, or a salt thereof, or a solvate thereof by crystallization to obtain a crystal of the cyclic peptide compound, or a salt thereof, or a solvate thereof.

[C1] A compound represented by formula (4a) or a salt thereof:

[Formula 11]

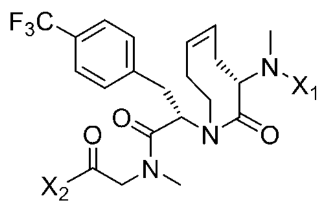

(4a)

wherein $X_1$ is hydrogen or a protecting group for an amino group; and

$X_2$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

[C2] The compound or a salt thereof according to [C1], wherein $X_1$ is hydrogen.

[C3] The compound or a salt thereof according to [C1], wherein $X_1$ is a Fmoc group.

[C4] The compound or a salt thereof according to any of [C1] to [C3], wherein $X_2$ is t-butoxy.

[C5] tert-Butyl 2-[methyl-[(2S)-2-[(4Z,7S)-7-(methylamino)-8-oxo-2,3,6,7-tetrahydroazocin-1-yl]-3-[4-(trifluoromethyl)phenyl]propanoyl]amino]acetate (compound 9).

[C6] A compound represented by formula (5a) or a salt thereof:

[Formula 12]

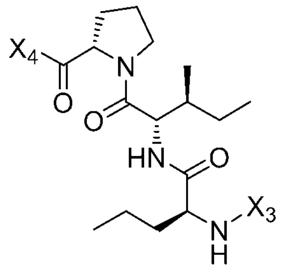

(5a)

wherein $X_3$ is hydrogen or a protecting group for an amino group; and
$X_4$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.
[C7] The compound or a salt thereof according to [C6], wherein $X_3$ is hydrogen.
[C8] The compound or a salt thereof according to [C6], wherein $X_3$ is a Fmoc group or a Cbz group.
[C9] The compound or a salt thereof according to any of [C6] to [C8], wherein $X_4$ is t-butoxy.
[C10] tert-Butyl (2S)-1-[(2S,3S)-3-methyl-2-[[(2S)-2-(methylamino)pentanoyl]amino]pentanoyl]pyrrolidine-2-acetate (compound 13).
[C11] A compound represented by formula (6a) or a salt thereof:

[Formula 13]

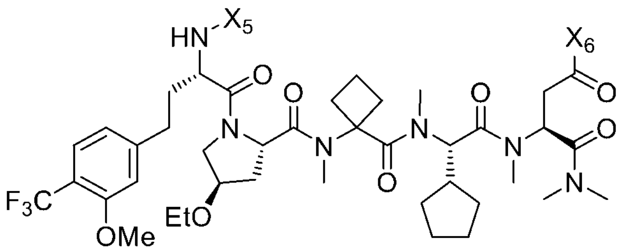

(6a)

wherein $X_5$ is hydrogen or a protecting group for an amino group; and
$X_6$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.
[C12] The compound or a salt thereof according to [C11], wherein $X_5$ is hydrogen.
[C13] The compound or a salt thereof according to [C11], wherein $X_5$ is a Cbz group.
[C14] The compound or a salt thereof according to any of [C11] to [C13], wherein $X_6$ is a hydroxy group or t-butoxy.
[C15] (3S)-3-[[(2S)-2-[[1-[[(2S,4R)-1-[(2S)-2-(Benzyloxycarbonylamino)-4-[3-methoxy-4-(trifluoromethyl)phenyl]butanoyl]-4-ethoxy-pyrrolidine-2-carbonyl]-methylamino]cyclobutanecarbonyl]-methyl-amino]-2-cyclopentyl-acetyl]-methyl-amino]-4-(dimethylamino)-4-oxo-butyric acid (compound 20).
[C16] A compound represented by formula (7a) or a salt thereof:

[Formula 14]

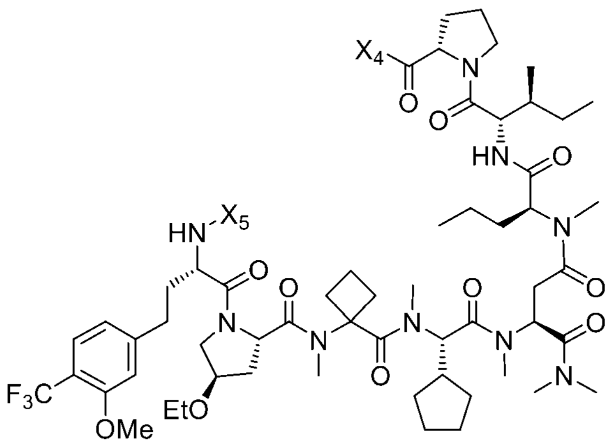

(7a)

wherein $X_5$ is hydrogen or a protecting group for an amino group; and

$X_4$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

[C17] The compound or a salt thereof according to [C16], wherein $X_5$ is hydrogen.

[C18] The compound or a salt thereof according to [C16], wherein $X_5$ is a Cbz group.

[C19] The compound or a salt thereof according to any of [C16] to [C18], wherein $X_6$ is a hydroxy group or t-butoxy.

[C20] (2S)-1-[(2S,3S)-2-[[(2S)-2-[[(3S)-3-[[(2S)-2-[[1-[[(2S,4R)-1-[(2S)-2-(Benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentylacetyl]-methyl-amino]-4-(dimethylamino)-4-oxo-butanoyl]-methylamino]pentanoyl]amino]-3-methyl-pentanoyl]pyrrolidine-2-carboxylic acid (compound 22).

[C20-1] tert-Butyl (2S)-1-[(2S,3S)-2-[[(2S)-2-[[(3S)-3-[[(2S)-2-[[1-[[(2S,4R)-1-[(2S)-2-amino-4-[3-methoxy-4-(trifluoromethyl)phenyl]butanoyl]-4-ethoxy-pyrrolidine-2-carbonyl]-methylamino]cyclobutanecarbonyl]-methyl-amino]-2-cyclopentyl-acetyl]-methyl-amino]-4-(dimethylamino)-4-oxo-butanoyl]-methyl-amino]pentanoyl]amino]-3-methyl-pentanoyl]pyrrolidine-2-carboxylate (compound 37).

[C21] A compound represented by formula (2a) or a salt thereof:

[Formula 15]

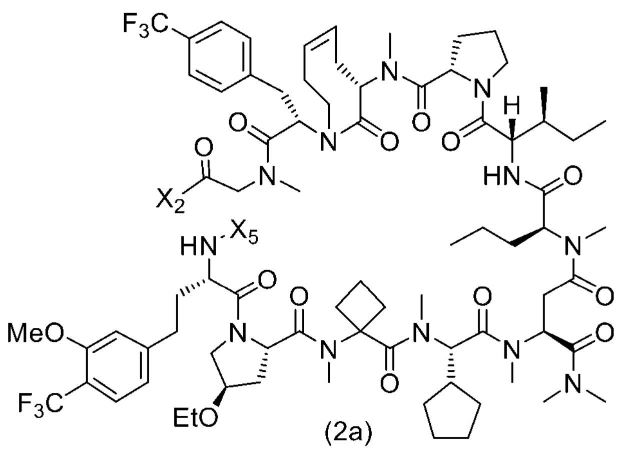

(2a)

wherein $X_5$ is hydrogen or a protecting group for an amino group; and

$X_2$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

[C22] The compound or a salt thereof according to [C21], wherein $X_5$ is hydrogen.

[C23] The compound or a salt thereof according to [C21], wherein $X_5$ is a Cbz group.

[C24] The compound or a salt thereof according to any of [C21] to [C23], wherein $X_2$ is a hydroxy group or t-butoxy.

[C25] 2-[[(2S)-2-[(4Z,7S)-7-[[(2S)-1-[(2S,3S)-2-[[(2S)-2-[[(3S)-3-[[(2S)-2-[[1-[[(2S,4R)-1-[(2S)-2-Amino-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acety1]-methylamino]-4-(dimethylamino)-4-oxo-butanoyl]-methyl-amino]pentanoyl]amino]-3-methyl-pentanoyl]pyrrolidine-2-carbonyl]-methyl-amino]-8-oxo-2,3,6,7-tetrahydroazocin-1-yl]-3-[4-(trifluoromethyl)phenyl]propanoyl]-methyl-amino]acetic acid (compound 24).

[C26] A compound represented by formula (3a) or a salt thereof:

[Formula 16]

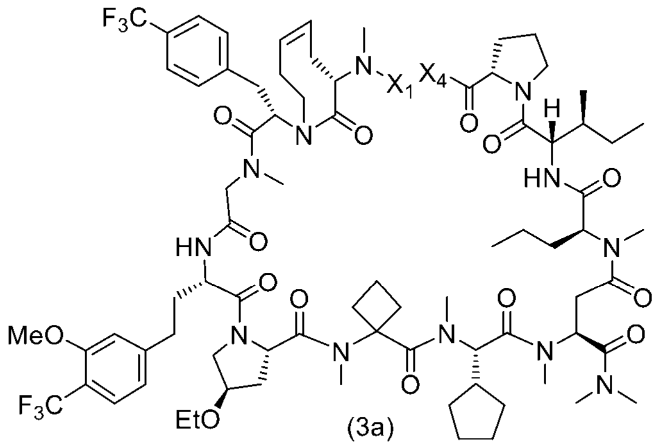

(3a)

wherein $X_1$ is hydrogen or a protecting group for an amino group; and

$X_4$ is a hydroxy group, an optionally substituted alkoxy, an optionally substituted aryloxy, an optionally substituted aralkoxy, an optionally substituted cyclic aminooxy or a group represented by $-OSiR_xR_yR_z$, wherein $R_x$, $R_y$, and $R_z$ are each independently an alkyl or an aryl.

[C27] The compound or a salt thereof according to [C26], wherein $X_1$ is hydrogen.

[C28] The compound or a salt thereof according to [C26], wherein $X_1$ is a Fmoc group.

[C29] The compound or a salt thereof according to any of [C26] to [C28], wherein $X_4$ is a hydroxy group or t-butoxy.

[C30] (S)-2-[(S)-3-[(S)-2-cyclopentyl-2-[1-[(2S,4R)-4-ethoxy-1-[(S)-4-[3-methoxy-4-(trifluoromethyl)phenyl]-2-[(2-[(S)-N-methyl-2-[(R,Z)-3-(methylamino)-2-oxo-3,4,7,8-tetrahydroazocin-1(2H)-yl]-3-[4-(trifluoromethyl)phenyl]propanamido]acetamido)butanoyl]-N-methylpyrrolidine-2-carboxyamido]-N-methylcyclobutane-1-carboxyamido]-N-methylacetamido]-4-(dimethylamino)-N-methyl-4-oxobutanamido]pentanoyl]-L-isoleucyl-L-proline (compound 40).

[C31] A method for producing the compound according to any of [C1] to [C30], wherein solid phase synthesis is not used.

[C32] A method for producing a cyclic peptide compound represented by formula (1a), wherein solid phase synthesis is not used.

[D1] A crystal of a cyclic peptide compound represented by formula (1a), or a salt thereof, or a solvate thereof:

[Formula 17]

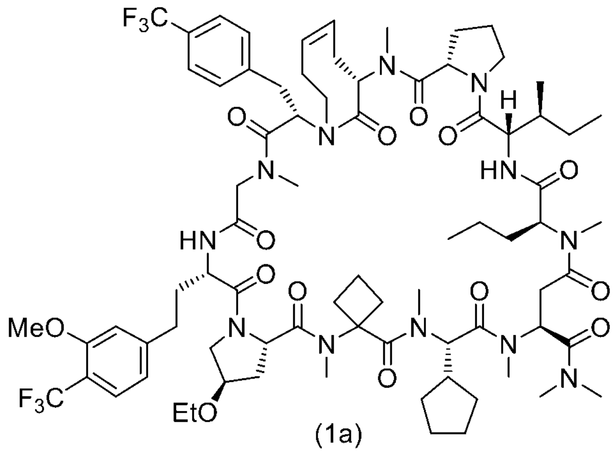

(1a)

[D2] The crystal according to [D1], wherein the crystal is selected from the group consisting of a non-solvate crystal of a cyclic peptide compound, a solvate crystal of a cyclic peptide compound, a non-solvate crystal of a salt of a cyclic peptide compound, and a solvate crystal of a salt of a cyclic peptide compound.
[D3] The crystal according to [D2], wherein the crystal is a solvate crystal of a cyclic peptide compound.
[D4] The crystal according to [D3], wherein the solvate crystal is a hydrate crystal of a cyclic peptide compound.
[D5] The crystal according to [D4], wherein the hydrate crystal is a form A crystal including at least 7 peaks selected from the group consisting of 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°) as diffraction angles (2θ values) by powder X-ray diffraction.
[D5-1] The crystal according to [D4], wherein the hydrate crystal is a form A crystal including at least 8 peaks selected from the group consisting of 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°) as diffraction angles (2θ values) by powder X-ray diffraction.
[D5-2] The crystal according to [D4], wherein the hydrate crystal is a form A crystal including peaks of 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°) as diffraction angles (2θ values) by powder X-ray diffraction.
[D5-3] The form A crystal according to any of [D5] to [D5-2], wherein the diffraction angles (2θ values) are diffraction angles (2θ values) of a hydrate crystal stored at a relative humidity of 10% or more for 15 minutes or longer.
[D6] The crystal according to [D4], wherein the hydrate crystal is a form B crystal including at least 7 peaks selected from the group consisting of 4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°) as diffraction angles (2θ values) by powder X-ray diffraction.
[D6-1] The crystal according to [D4], wherein the hydrate crystal is a form B crystal including at least 8 peaks selected from the group consisting of 4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°) as diffraction angles (2θ values) by powder X-ray diffraction.
[D6-2] The crystal according to [D4], wherein the hydrate crystal is a form B crystal including peaks of 4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°) as diffraction angles (2θ values) by powder X-ray diffraction.
[D6-3] The form B crystal according to any of [D6] to [D6-2], wherein the diffraction angles (2θ values) are diffraction angles (2θ values) of a hydrate crystal stored at a relative humidity of 30% or more for 15 minutes or longer.
[D7] The crystal according to [D3], wherein the solvate crystal is a form F crystal including at least 7 peaks selected from the group consisting of 6.99°, 8.49°, 9.49°, 9.88°, 10.21°, 11.81°, 12.32°, 12.75°, 13.17°, 13.94°, 14.92°, 15.20°, 15.64°, 16.78°, 17.01°, and 17.47° (±0.2°) as diffraction angles (2θ values) by powder X-ray diffraction.
[D7-1] The crystal according to [D3], wherein the solvate crystal is a form F crystal including at least 8 peaks selected from the group consisting of 6.99°, 8.49°, 9.49°, 9.88°, 10.21°, 11.81°, 12.32°, 12.75°, 13.17°, 13.94°, 14.92°, 15.20°, 15.64°, 16.78°, 17.01°, and 17.47° (±0.2°) as diffraction angles (2θ values) by powder X-ray diffraction.
[D7-2] The crystal according to [D3], wherein the solvate crystal is a form F crystal including peaks of 6.99°, 8.49°, 9.49°, 9.88°, 10.21°, 11.81°, 12.32°, 12.75°, 13.17°, 13.94°, 14.92°, 15.20°, 15.64°, 16.78°, 17.01°, and 17.47° (±0.2°) as diffraction angles (2θ values) by powder X-ray diffraction.
[D7-3] The crystal according to any of [D7] to [D7-2], wherein the solvate crystal is an acetone/heptane/water solvate.
[D8] The crystal according to [D4], wherein the hydrate crystal is a form J crystal including at least 7 peaks selected from the group consisting of 6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°) as diffraction angles (2θ values) by powder X-ray diffraction.
[D8-1] The crystal according to [D4], wherein the hydrate crystal is a form J crystal including at least 8 peaks selected

from the group consisting of 6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°) as diffraction angles (2θ values) by powder X-ray diffraction.

[D8-2] The crystal according to [D4], wherein the hydrate crystal is a form J crystal including peaks of 6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°) as diffraction angles (2θ values) by powder X-ray diffraction.

[D8-3] The form J crystal according to any of [D8] to [D8-2], wherein the diffraction angles (2θ values) are diffraction angles (2θ values) of a hydrate crystal stored at a relative humidity of less than 10% for 15 minutes or longer.

[D9] The crystal according to [D4], wherein the hydrate crystal is a form Y crystal including at least 7 peaks selected from the group consisting of 5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°) as diffraction angles (2θ values) by powder X-ray diffraction.

[D9-1] The crystal according to [D4], wherein the hydrate crystal is a form Y crystal including at least 8 peaks selected from the group consisting of 5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°) as diffraction angles (2θ values) by powder X-ray diffraction.

[D9-2] The crystal according to [D4], wherein the hydrate crystal is a form Y crystal including peaks of 5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°) as diffraction angles (2θ values) by powder X-ray diffraction.

[D9-3] The form Y crystal according to any of [D9] to [D9-2], wherein the diffraction angles (2θ values) are diffraction angles (2θ values) of a hydrate crystal stored at a relative humidity of less than 30% for 15 minutes or longer.

[D10] The crystal according to [D3], wherein the hydrate crystal is a form K crystal including at least 7 peaks selected from the group consisting of 7.49°, 7.91°, 8.14°, 9.11°, 9.33°, 11.04°, 11.71°, 12.52°, 13.21°, 13.70°, 14.82°, 15.13°, 15.52°, 15.68°, 17.22°, and 17.51° (±0.2°) as diffraction angles (2θ values) by powder X-ray diffraction.

[D10-1] The crystal according to [D3], wherein the hydrate crystal is a form K crystal including at least 8 peaks selected from the group consisting of 7.49°, 7.91°, 8.14°, 9.11°, 9.33°, 11.04°, 11.71°, 12.52°, 13.21°, 13.70°, 14.82°, 15.13°, 15.52°, 15.68°, 17.22°, and 17.51° (±0.2°) as diffraction angles (2θ values) by powder X-ray diffraction.

[D10-2] The crystal according to [D3], wherein the solvate crystal is a form K crystal including peaks of 7.49°, 7.91°, 8.14°, 9.11°, 9.33°, 11.04°, 11.71°, 12.52°, 13.21°, 13.70°, 14.82°, 15.13°, 15.52°, 15.68°, 17.22°, and 17.51° (±0.2°) as diffraction angles (2θ values) by powder X-ray diffraction.

[D11] The crystal according to [D3], wherein the solvate crystal is a form G dimethyl sulfoxide/heptane/water solvate crystal with the structure of single crystal X-ray analysis shown in Figure 1.

[D11-1] The crystal according to [D3], wherein the solvate crystal is a form E 2-propanol/heptane/water solvate crystal with the structure of single crystal X-ray analysis shown in Figure 3.

[D11-2] The crystal according to [D3], wherein the solvate crystal is a form H ethanol/water solvate crystal with the structure of single crystal X-ray analysis shown in Figure 8.

[D11-3] The crystal according to [D3], wherein the solvate crystal is a form D 1,4-dioxane/water solvate crystal with the structure of single crystal X-ray analysis shown in Figure 26.

[D11-4] The crystal according to [D3], wherein the solvate crystal is a form L dimethyl sulfoxide/water solvate crystal with the structure of single crystal X-ray analysis shown in Figure 28.

[D11-5] The crystal according to [D3], wherein the solvate crystal is a form M propylene glycol/water solvate crystal with the structure of single crystal X-ray analysis shown in Figure 31.

[D11-6] The crystal according to [D3], wherein the solvate crystal is a form M propylene glycol/water solvate crystal having the diffraction angles by powder X-ray diffraction shown in Figure 29.

[D11-7] The crystal according to [D3], wherein the solvate crystal is a form M propylene glycol/water solvate crystal exhibiting the thermogravimetry-differential thermal analysis data shown in Figure 30(A).

[D11-8] The crystal according to [D3], wherein the solvate crystal is a form N propylene glycol solvate crystal having the diffraction angles by powder X-ray diffraction shown in Figure 29.

[D11-9] The crystal according to [D3], wherein the solvate crystal is a form N propylene glycol solvate crystal exhibiting the thermogravimetry-differential thermal analysis data shown in Figure 30(B).

[D12] A method for producing the crystal of a cyclic peptide compound according to any of [D1] to [D10-2], the method comprising: a step of dissolving the cyclic peptide compound in a polar organic solvent in an amount that allows the cyclic peptide compound to dissolve therein to obtain a solution; and a step of adding a hydrocarbon-based solvent or water to the solution to obtain a crystal of the cyclic peptide compound.

[D12-1] The method according to [D12], wherein the raw material cyclic peptide compound has a purity of 85% or more.

[D13] A method for producing the crystal of a cyclic peptide compound according to any of [D1] to [D10-2], the method comprising: a step of adding a mixed solution of a hydrocarbon-based solvent and a polar organic solvent or a mixed solution of water and a polar organic solvent to the cyclic peptide compound in an amorphous state to obtain a crystal of the cyclic peptide compound.

[D14] The method according to any of [D12] to [D13-1], wherein the polar organic solvent is one or more selected from the group consisting of DMSO, acetone, 2-butanone, methanol, ethanol, 1-propanol, 2-propanol, propylene glycol, 1,4-dioxane, and ethyl acetate.
[D15] The method according to [D14], wherein the polar organic solvent is acetone.
[D15-1] The method according to [D14], wherein the polar organic solvent is ethanol.
[D16] The method according to any of [D12] to [D13-1], wherein the hydrocarbon-based solvent is one or more selected from the group consisting of heptane, hexane, pentane, toluene, and xylene.
[D17] The method according to [D16], wherein the hydrocarbon-based solvent is heptane.
[D18] A method for producing the crystal of a cyclic peptide compound according to any of [D1] to [D10-2], the method comprising: a step of dissolving the cyclic peptide compound in an amorphous state in DMSO to obtain a solution; a step of freeze-drying the solution to obtain a freeze-dried product of the cyclic peptide compound; and a step of adding a mixed solution of water and a polar organic solvent to the freeze-dried product to obtain a crystal of the cyclic peptide compound.
[D19] The method according to [D18], wherein the polar organic solvent is one or more selected from the group consisting of DMSO, acetone, 2-butanone, methanol, ethanol, 1-propanol, 2-propanol, 1,4-dioxane, and propylene glycol.
[D20] The method according to [D19], wherein the polar organic solvent is acetone.
[D21] The method according to any of [D12] to [D20], further comprising, after the step of obtaining a crystal of the cyclic peptide compound, a step of filtering the crystal.
[D22] The method according to any of [D12] to [D21], further comprising, after the step of obtaining a crystal of the cyclic peptide compound, a step of drying the crystal.
[D23] The method according to any of [D12] to [D22], wherein the crystal of a cyclic peptide compound is a solvate crystal.
[D24] The method according to [D23], wherein the solvate crystal of a cyclic peptide compound is a hydrate crystal.
[D25] The method according to [D21] or [D22], wherein the crystal of a cyclic peptide compound is formed as a solvate crystal in a solvent and obtained as a hydrate crystal after the filtrating step and/or the drying step.
[D26] A composition comprising a cyclic peptide compound represented by formula (1a), or a salt thereof, or a solvate thereof, wherein the compound contains a cyclic dimer of formula (1a), which is an impurity, at a proportion of 1.5 w/w% or less:

[Formula 18]

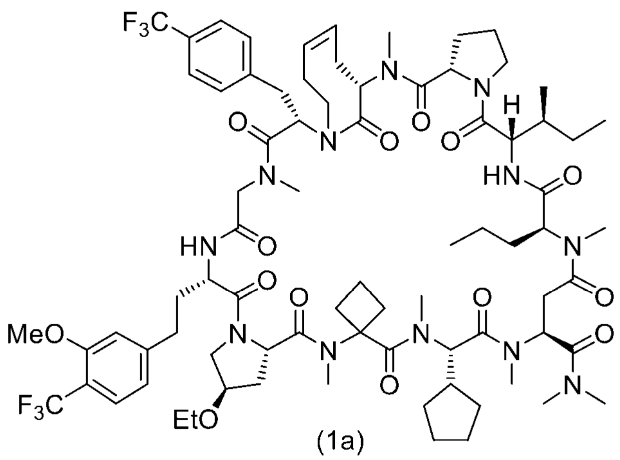

(1a)

[D27] A composition comprising a cyclic peptide compound represented by formula (1a), or a salt thereof, or a solvate thereof, wherein the compound contains a cyclic dimer of formula (1a), which is an impurity, at a proportion of 0.001 w/w% or more:

[Formula 19]

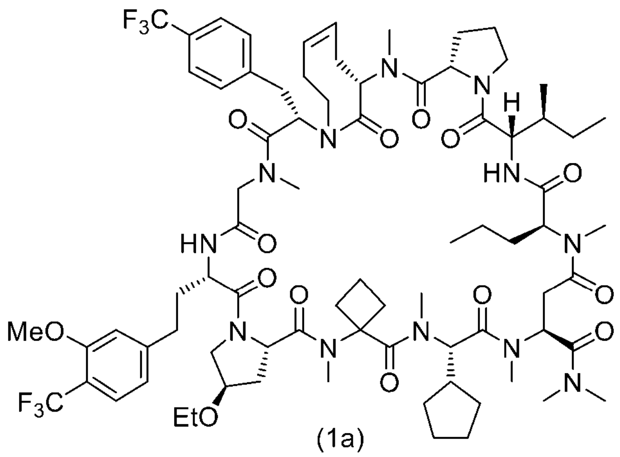

(1a)

[D28] A composition comprising a cyclic peptide compound represented by formula (1a), or a salt thereof, or a solvate thereof, wherein the compound contains acetone at a proportion of 2.0 w/w% or less:

[Formula 20]

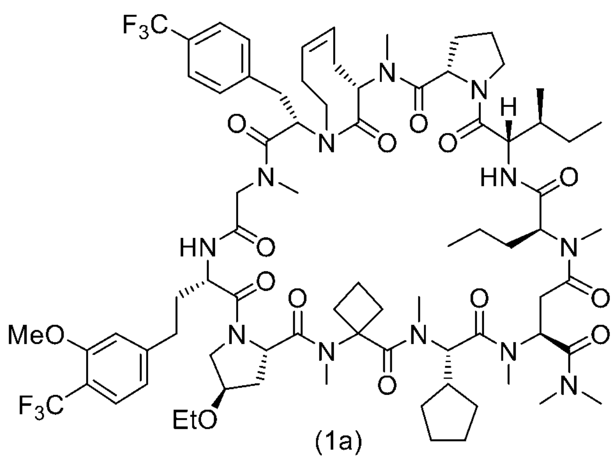

(1a)

[D29] A composition comprising a cyclic peptide compound represented by formula (1a), or a salt thereof, or a solvate thereof, wherein the compound contains acetone at a proportion of 0.001 w/w% or more:

[Formula 21]

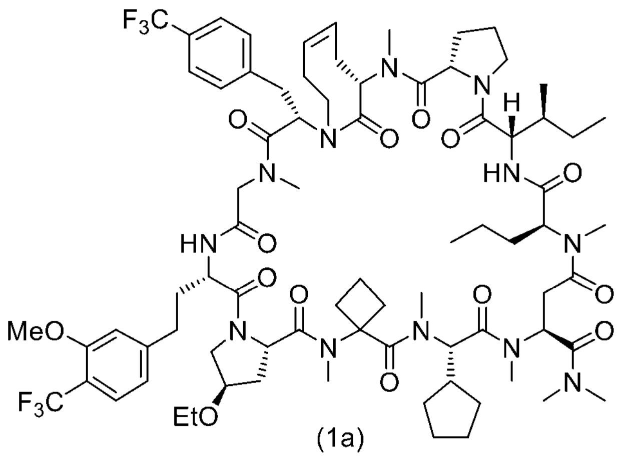

(1a)

In the above numberings, the number cited in the dependent item includes the branch number of the number, unless otherwise mentioned. For example, the [A67] cited in the dependent item shows that not only [A67] but also its branch number [A67-1] are included. The same applies to other numberings.

[Advantageous Effects of Invention]

According to the present invention, a cyclic peptide compound, or a salt thereof, or a solvate thereof can be efficiently produced while suppressing the production of a cyclic dimer, which is a by-product. The production method of the present invention enables reducing costs in production of the peptide compound and also reducing the environmental loads, and therefore, the production method of the present invention is particularly useful for synthesis of the peptide on a large scale.

[Brief Description of Drawings]

[Figure 1] Figure 1 shows the crystal structure of the crystal (form G) obtained in Example 3-1. Compound 1 is drawn with the Capped Stick model and the others are drawn with the Ball-and-Stick model.

[Figure 2] Figure 2 shows the results of powder X-ray diffraction measurement of the crystal (form A) obtained in Example 3-2. The vertical axis represents a diffraction intensity, and the horizontal axis represents a diffraction angle 2θ (°).

[Figure 3] Figure 3 shows the crystal structure of the crystal obtained in Example 3-3. Compound 1 is drawn with the Capped Stick model, and the others are drawn with the Ball-and-Stick model.

[Figure 4] Figure 4 shows the results of powder X-ray diffraction measurement of the crystal (form E) obtained in Example 3-3. The vertical axis represents a diffraction intensity, and the horizontal axis represents a diffraction angle 2θ (°).

[Figure 5] Figure 5 shows the results of powder X-ray diffraction measurement of the crystal (form B) obtained in Example 3-4. The vertical axis represents a diffraction intensity, and the horizontal axis represents a diffraction angle 2θ (°).

[Figure 6] Figure 6 shows the results of powder X-ray diffraction measurement of the crystal (form B) obtained in Example 3-5. The vertical axis represents a diffraction intensity, and the horizontal axis represents a diffraction angle 2θ (°).

[Figure 7] Figure 7 shows the crystal structure of the crystal (form H) obtained in Example 3-6. Compound 1 is drawn with the Capped Stick model and the others are drawn with the Ball-and-Stick model.

[Figure 8] Figure 8 shows the results of powder X-ray diffraction measurement of the crystal (form B) obtained in Example 3-6. The vertical axis represents a diffraction intensity, and the horizontal axis represents a diffraction angle 2θ (°).

[Figure 9] Figure 9 shows the crystal structure of the crystal (form C) obtained in Example 3-7. Compound 1 is drawn with the Capped Stick model, and the others are drawn with the Ball-and-Stick model.

[Figure 10] Figure 10 shows the results of powder X-ray diffraction measurement of the crystal (form B) obtained in

Example 3-7. The vertical axis represents a diffraction intensity, and the horizontal axis represents a diffraction angle 2θ (°).

[Figure 11] Figure 11 shows the results of powder X-ray diffraction measurement of the crystal (form A) obtained in Example 3-8. The vertical axis represents a diffraction intensity, and the horizontal axis represents a diffraction angle 2θ (°).

[Figure 12] Figure 12 shows the results of thermogravimetry-differential thermal analysis of the crystal (form A) obtained in Example 3-8. The horizontal axis represents a temperature (°C), and the right vertical axis represents a weight change (%) of the sample in thermogravimetric analysis. The left vertical axis represents a heat flow observed in differential thermal analysis.

[Figure 13] Figure 13 shows the results of $^1$H-NMR measurement of the crystal (form A) obtained in Example 3-8. The vertical axis represents a signal intensity, and the horizontal axis represents a chemical shift δ (ppm).

[Figure 14] Figure 14 shows the results of powder X-ray diffraction measurement of the crystal (form A) obtained in Example 3-9. The vertical axis represents a diffraction intensity, and the horizontal axis represents a diffraction angle 2θ (°).

[Figure 15] Figure 15 shows the results of thermogravimetry-differential thermal analysis of the crystal (form A) obtained in Example 3-9. The horizontal axis represents a temperature (°C) and the right vertical axis represents a weight change (%) of the sample in thermogravimetric analysis. The left vertical axis represents a heat flow observed in differential thermal analysis.

[Figure 16] Figure 16 shows the results of $^1$H-NMR measurement of the crystal (form A) obtained in Example 3-9. The vertical axis represents a signal intensity, and the horizontal axis represents a chemical shift δ (ppm).

[Figure 17] Figure 17 shows the results of powder X-ray diffraction measurement of the crystal (form F) obtained in Example 3-10. The vertical axis represents a diffraction intensity, and the horizontal axis represents a diffraction angle 2θ (°).

[Figure 18] Figure 18 shows the crystal structure of the crystal (form F) obtained in Example 3-11. Compound 1 is drawn with the Capped Stick model and the others are drawn with the Ball-and-Stick model.

[Figure 19] Figure 19 shows the results of powder X-ray diffraction measurement of the crystal (form A) obtained in Example 3-12. The vertical axis represents a diffraction intensity, and the horizontal axis represents a diffraction angle 2θ (°).

[Figure 20] Figure 20 shows the results of thermogravimetry-differential thermal analysis of the crystal (form A) obtained in Example 3-12. The horizontal axis represents a temperature (°C), and the right vertical axis represents a weight change (%) of the sample in thermogravimetric analysis. The left vertical axis represents a heat flow observed in differential thermal analysis.

[Figure 21] Figure 21 shows the results of powder X-ray diffraction measurement of the crystal obtained in Example 3-12 at a relative humidity of (A) 0% (form J), (B) 10% (form A), (C) 20% (form A), (D) 50% (form A), and (E) 90% (form A). The vertical axis represents a diffraction intensity, and the horizontal axis represents a diffraction angle 2θ (°). The peaks around 4.89° and 6.65° in the figure are peaks originating from the measurement equipment.

[Figure 22] Figure 22 shows the results of powder X-ray diffraction measurement of the crystal (form B) obtained in Example 3-13. The vertical axis represents a diffraction intensity, and the horizontal axis represents a diffraction angle 2θ (°).

[Figure 23] Figure 23 shows the results of thermogravimetry-differential thermal analysis of the crystal (form B) obtained in Example 3-13. The horizontal axis represents a temperature (°C), and the right vertical axis represents a weight change (%) of the sample in thermogravimetric analysis. The left vertical axis represents a heat flow observed in differential thermal analysis.

[Figure 24] Figure 24 shows the results of powder X-ray diffraction measurement of the crystal obtained in Example 3-13 at a relative humidity of (A) 0% (form Y), (B) 30% (form B), (C) 50% (form B), and (D) 90% (form B). The vertical axis represents a diffraction intensity, and the horizontal axis represents a diffraction angle 2θ (°). The peak around 6.63° in the figure is a peak originating from the measurement equipment.

[Figure 25] Figure 25 shows the crystal structure of the crystal (form B) obtained in Example 3-14. Compound 1 is drawn with the Capped Stick model and the others are drawn with the Ball-and-Stick model.

[Figure 26] Figure 26 shows the crystal structure of the crystal (form D) obtained in Example 3-15. Compound 1 is drawn with the Capped Stick model and the others are drawn with the Ball-and-Stick model.

[Figure 27] Figure 27 shows the crystal structure of the crystal (form L) obtained in Example 3-16. Compound 1 is drawn with the Capped Stick model and the others are drawn with the Ball-and-Stick model.

[Figure 28] Figure 28 shows the results of powder X-ray diffraction measurement of the crystal (form L) obtained in Example 3-16 (A: wet powder, B: dry powder). The vertical axis represents a diffraction intensity, and the horizontal axis represents a diffraction angle 2θ (°).

[Figure 29] Figure 29 shows the results of powder X-ray diffraction measurement of the crystal obtained in Example 3-17 (A: form M, B: form N). The vertical axis represents a diffraction intensity, and the horizontal axis represents a

diffraction angle 2θ (°).

[Figure 30] Figure 30 shows the results of thermogravimetry-differential thermal analysis of the crystal (form N) obtained in Example 3-17. The horizontal axis represents a temperature (°C), and the right vertical axis represents a weight change (%) of the sample in thermogravimetric analysis. The left vertical axis represents a heat flow observed in differential thermal analysis.

[Figure 31] Figure 31 shows the crystal structure of the crystal (form M) obtained in Example 3-18. Compound 1 is drawn with the Capped Stick model and the others are drawn with the Ball-and-Stick model.

[Figure 32] Figure 32 shows the results of powder X-ray diffraction measurement of the crystal (form K) obtained in Example 3-19. The vertical axis represents a diffraction intensity, and the horizontal axis represents a diffraction angle 2θ (°).

[Figure 33] Figure 33 shows the results of thermogravimetry-differential thermal analysis of the crystal (form K) obtained in Example 3-19. The horizontal axis represents a temperature (°C), and the right vertical axis represents a weight change (%) of the sample in thermogravimetric analysis. The left vertical axis represents a heat flow observed in differential thermal analysis.

[Figure 34] Figure 34 shows the results of $^1$H-NMR measurement of the crystal (form K) obtained in Example 3-19. The vertical axis represents a signal intensity and the horizontal axis represents a chemical shift δ (ppm).

[Figure 35] Figure 35 shows the results of powder X-ray diffraction measurement of the crystals obtained in (A) Example 5-1, (B) Example 5-2, (C) Example 5-3, (D) Example 5-4, (E) Example 5-5, (F) Example 5-6, (G) Example 5-7, (H) Example 5-8, and (I) Example 5-9. The vertical axis represents a diffraction intensity, and the horizontal axis represents a diffraction angle 2θ (°).

[Figure 36] Figure 36 shows the results of powder X-ray diffraction measurement of the crystal (form B) obtained in Example 3-22. The vertical axis represents a diffraction intensity, and the horizontal axis represents a diffraction angle 2θ (°).

[Description of Embodiments]

Abbreviations

The abbreviations used herein are listed below.

2-MeTHF: 2-methyltetrahydrofuran
20% Pip/DMF: N,N-dimethylformamide solution containing 20% piperidine
EtOAc: ethyl acetate
Alloc: allyloxycarbonyl
BEP: 2-bromo-1-ethyl pyridinium tetrafluoroborate
BHT: 2,6-di-tert-butyl-4-methylphenol
Boc: t-butoxycarbonyl
Cbz: benzyloxycarbonyl
COMU: (1-cyano-2-ethoxy-2-oxoethylideneaminoxy)dimethylamino-morpholino-carbenium hexafluorophosphate
CPME: cyclopentyl methyl ether
CSA: 10-camphorsulfonic acid
DCM: dichloromethane
DEPBT: diethyl 3,4-dihydro-4-oxo-1,2,3-benzotriazin-3-yl phosphate
DIPEA: N,N-diisopropylethylamine
DMA: N,N-dimethylacetamide
DMAP: 4-dimethylaminopyridine
DMF: N,N-dimethylformamide
DMSO: dimethyl sulfoxide
DMT-MM: 4-(4,6-dimethoxy-1,3,5-triazin-2-yl)-4-methylmorpholinium chloride
EDCI: 1-ethyl-3-(3-dimethylaminopropyl)carbodiimide
FDPP: pentafluorophenyl diphenyl phosphinate
Fmoc or FMOC: 9-fluorenylmethyloxycarbonyl
HATU: O-(7-azabenzotriazol-1-yl)-N,N,N',N'-tetramethyluronium hexafluorophosphate
HMDS: 1,1,1,3,3,3-hexamethyldisilazane
HOAt: 1-hydroxy-7-azabenzotriazole
HOBt: 1-hydroxybenzotriazole
IPAc: isopropyl acetate
MeCN: acetonitrile

MTBE: methyl tert-butyl ether
MTHP: 4-methyltetrahydropyran
NMM: 4-methylmorpholine
NMP: N-methylpyrrolidone
PyBOP: 1H-benzotriazol-1-yloxytripyrrolidinophosphonium hexafluorophosphate
PyClop: chlorotripyrrolidino phosphonium hexafluorophosphate
PyOxim: (ethylcyano(hydroxyimino)acetat-O2)-tri-(1-pyrrolidinyl)-phosphonium hexafluorophosphate
T3P: propylphosphonic anhydride
TBAF: tetrabutylammonium fluoride
Teoc: 2-(trimethylsilyl)ethoxycarbonyl
TFA: trifluoroacetic acid
THF: tetrahydrofuran
TMSOTf: trimethylsilyl trifluoromethanesulfonate
Troc: 2,2,2-trichloroethoxycarbonyl
PDA: photodiode array
FA: formic acid
qNMR: quantitative nuclear magnetic resonance
UPLC: Ultra-Performance Liquid Chromatography (trademark of the Waters Corporation)
HPLC: high-performance liquid chromatograph
Et: ethyl
DBU: 1,8-diazabicyclo[5.4.0]undec-7-ene
ESI: electrospray ionization
Bu: butyl
Me: methyl
oxyma: ethyl cyano(hydroxyimino)acetate
DIC: N,N'-diisopropylcarbodiimide
TFE: 2,2,2-trifluoroethanol
IPA: 2-propanol
NMI: 1-methylimidazole
TCFH: chloro-N,N,N',N'-tetramethylformamidinium hexafluorophosphate
NMR: nuclear magnetic resonance
TMS: tetramethylsilane

Definition of functional groups and the like (All terms and phrases herein are used as commonly understood in the art. Examples are given below, but they are not limited thereto.)

The term "halogen" as used herein refers to, for example, F, Cl, Br, or I.

The term "alkyl" as used herein is a monovalent group derived from an aliphatic hydrocarbon by removing any one hydrogen atom, and has a subset of hydrocarbyl or hydrocarbon group structures that do not contain a hetero atom (which is an atom other than carbon and hydrogen atoms) or an unsaturated carbon-carbon bond, and contain hydrogen and carbon atoms in the backbone. The alkyl includes not only a linear form but a branched form. The alkyl is specifically an alkyl having 1 to 20 carbon atoms ($C_1$ to $C_{20}$; hereinafter, "$C_p$ to $C_q$" means that the number of carbon atoms is p to q), preferably a $C_1$ to $C_{10}$ alkyl, and more preferably a $C_1$ to $C_6$ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The term "alkenyl" as used herein is a monovalent group having at least one double bond (two adjacent sp2 carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. Preferred examples of the alkenyl include a $C_2$-$C_{10}$ alkenyl, and more preferred examples thereof include a $C_2$-$C_6$ alkenyl. Specific examples of the alkenyl include vinyl, allyl, 1-propenyl, 2-propenyl, 1-butenyl, 2-butenyl (which includes cis and trans), 3-butenyl, pentenyl, 3-methyl-2-butenyl, and hexenyl.

The term "alkynyl" as used herein is a monovalent group having at least one triple bond (two adjacent sp carbon atoms). The alkynyl includes not only a linear form but a branched form. Preferred examples of the alkynyl include a $C_2$-$C_{10}$ alkynyl, and more preferably a $C_2$-$C_6$ alkynyl. Specific examples of the alkynyl include ethynyl, 1-propynyl, propargyl, 3-butynyl, pentynyl, and hexynyl.

The term "cycloalkyl" as used herein means a saturated or partially saturated cyclic monovalent aliphatic

hydrocarbon group and includes a monocyclic ring, a bicyclo ring, and a spiro ring. The cycloalkyl is preferably a $C_3$-$C_8$ cycloalkyl, more preferably a $C_3$-$C_7$ cycloalkyl, and still more preferably a $C_3$-$C_6$ cycloalkyl. Specific examples of the cycloalkyl include cyclopropyl, cyclobutyl, cyclopentyl, cyclohexyl, cycloheptyl, cyclooctyl, bicyclo[2.2.1]heptyl, and spiro [3.3]heptyl.

The term "aryl" as used herein means a monovalent aromatic hydrocarbon ring, that is, an aromatic hydrocarbon ring group. Preferred examples of the aryl include a $C_6$-$C_{10}$ aryl. Specific examples of the aryl include phenyl and naphthyl (such as 1-naphthyl and 2-naphthyl).

The term "heteroaryl" as used herein means an aromatic cyclic monovalent group containing a carbon atom as well as 1 to 5 heteroatoms, that is, an aromatic heterocyclic group. The ring may be a monocyclic ring or a condensed ring with another ring, and may be partially saturated. The number of atoms constituting the ring of heteroaryl is preferably 5 to 10 (5- to 10-membered heteroaryl), and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, and imidazopyridyl, pyrazolopyridyl, imidazopyridyl, triazolopyridyl, pyrrolopyrazinyl, and flopyridyl.

The "aralkyl (arylalkyl)" as used herein means a group in which one or more hydrogen atoms of the "alkyl" as defined herein are substituted with the "aryl" as defined herein. As the aralkyl, a $C_7$-$C_{14}$ aralkyl is preferred, and a $C_7$-$C_{10}$ aralkyl is more preferred. Specific examples of the aralkyl include benzyl, phenethyl, and 3-phenylpropyl.

The "heteroarylalkyl" as used herein means a group in which one or more hydrogen atoms of the "alkyl" as defined herein are substituted with the "heteroaryl" as defined herein. As the heteroarylalkyl, a 5- to 10-membered heteroaryl-$C_1$-$C_6$ alkyl is preferred, and a 5- to 10-membered heteroaryl-$C_1$-$C_2$ alkyl is more preferred. Specific examples of the heteroarylalkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl) ethyl, and 2-(5-benzofuranyl)ethyl.

The term "alkoxy" as used herein means an oxy group to which the "alkyl" as defined herein is bonded. As the alkoxy, a $C_1$-$C_6$ alkoxy is preferred, and a $C_1$-$C_4$ alkoxy is more preferred. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The term "alkoxyalkyl" as used herein means a group in which one or more hydrogen atoms of the "alkyl" as defined herein are substituted with the "alkoxy" as defined herein. As the alkoxyalkyl, a $C_1$-$C_6$ alkoxy-$C_1$-$C_6$ alkyl is preferred, and a $C_1$-$C_6$ alkoxy-$C_1$-$C_2$ alkyl is more preferred. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

The term "aryloxy" as used herein means an oxy group to which the "aryl" as defined herein is bonded. As the aryloxy, a $C_6$-$C_{10}$ aryloxy is preferred. Specific examples of the aryloxy include phenoxy, 1-naphthyloxy, and 2-naphthyloxy.

The term "aralkoxy" as used herein means an oxy group to which the "aralkyl" as defined herein is bonded. As the aralkoxy, a $C_7$-$C_{14}$ aralkoxy is preferred, and a $C_7$-$C_{10}$ aralkoxy is more preferred. Specific examples of the aralkoxy include benzyloxy, phenethyloxy, and 3-phenylpropoxy.

The term "amino" as used herein means $-NH_2$ in the narrow sense and means -NRR' in the broad sense. In this context, R and R' are each independently selected from hydrogen, alkyl, alkenyl, alkynyl, cycloalkyl, heterocyclyl, aryl, or heteroaryl, or R and R' mean a group in which they form a ring together with the nitrogen atom to which they are bonded. Preferred examples of the amino include $-NH_2$, a mono-$C_1$-$C_6$ alkylamino, a di-$C_1$-$C_6$ alkylamino, and a 4- to 8-membered cyclic amino.

The term "monoalkylamino" as used herein means a group in which R is hydrogen and R' is the "alkyl" as defined herein, in the "amino" as defined herein. Preferred examples of the monoalkylamino include a mono-$C_1$-$C_6$ alkylamino. Specific examples of the monoalkylamino include methylamino, ethylamino, n-propylamino, i-propylamino, n-butylamino, s-butylamino, and t-butylamino.

The term "dialkylamino" as used herein means a group in which R and R' are independently the "alkyl" as defined herein, in the "amino" as defined herein. Preferred examples of the dialkylamino include a di-$C_1$-$C_6$ alkylamino. Specific examples of the dialkylamino include dimethylamino and diethylamino.

The term "cyclic amino" as used herein means a group in which R and R' form a ring together with the nitrogen atom to which they are bonded, in the "amino" as defined herein. Preferred examples of the cyclic amino include a 4- to 8-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

The term "cyclic aminooxy" as used herein means an oxy group to which the "cyclic amino" as defined herein is bonded. Preferred examples of the cyclic aminooxy include a 4- to 8-membered cyclic aminooxy. Specific examples of the cyclic aminooxy include 1-azetidyloxy, 1-pyrrolidyloxy, 1-piperidyloxy, 1-piperazyloxy, 4-morpholinyloxy, 3-oxazolidyloxy,

1,1-dioxidothiomorpholinyl-4-yloxy, and 3-oxa-8-azabicyclo[3.2.1]octan-8-yloxy.

The term "aminocarbonyl" as used herein means a carbonyl group to which the "amino" as defined herein is bonded. Preferred examples of the aminocarbonyl include $-CONH_2$, a mono-$C_1$-$C_6$ alkylaminocarbonyl, a mono-$C_3$-$C_6$ cycloalkylaminocarbonyl, a di-$C_1$-$C_6$ alkylaminocarbonyl, and a 4- to 8-membered cyclic aminocarbonyl. Specific examples of the aminocarbonyl include $-CONH_2$, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octan-8-ylcarbonyl.

The "haloalkyl" as used herein means a group in which one or more hydrogen atoms of the "alkyl" as defined herein are substituted with halogen. As the haloalkyl, a halo-$C_1$-$C_6$ alkyl is preferred, and a fluoro-$C_1$-$C_6$ alkyl is more preferred. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

The term "alicyclic ring" as used herein means a nonaromatic hydrocarbon ring. The alicyclic ring may have an unsaturated bond in the ring. Also, a carbon atom constituting the ring may be oxidized to form carbonyl. The alicyclic ring may be a monocycle (referred to as monocyclic alicyclic ring herein), or may form a fused ring with a saturated alicyclic ring such as a cyclopentane ring and a cyclohexane ring or an aromatic hydrocarbon ring such as a benzene ring and a naphthalene ring. As the alicyclic ring, a 3- to 10-membered alicyclic ring is preferred, and a 3- to 8-membered alicyclic ring is more preferred. Specific examples of the alicyclic ring include a cyclopropane ring, a cyclobutane ring, a cyclopentane ring, a cyclohexane ring, a cycloheptane ring, a cyclooctane ring, and a bicyclo[2.2.1]heptane ring.

The term "saturated heterocyclic ring" as used herein means a nonaromatic heterocyclic ring containing preferably 1 to 5, more preferably 1 to 3, heteroatoms among the atoms constituting the ring, and having no unsaturated bonds in the ring. The saturated heterocyclic ring has no double or triple bonds in the ring. The saturated heterocyclic ring may be a monocycle, or may form a fused ring or spiro ring with a saturated alicyclic ring such as a cyclopentane ring and a cyclohexane ring or a saturated heterocyclic ring such as a tetrahydropyran ring, a dioxane ring, and a pyrrolidine ring. As the saturated heterocyclic ring, a 4- to 10-membered saturated heterocyclic ring is preferred, a 4- to 7-membered saturated heterocyclic ring is more preferred, and a 5-membered saturated heterocyclic ring is still more preferred. Specific examples of the saturated heterocyclic ring include an azetidine ring, an oxoazetidine ring, an oxetane ring, a tetrahydrofuran ring, a tetrahydropyran ring, a morpholine ring, a thiomorpholine ring, a pyrrolidine ring, a 2-oxopyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, an indoline ring, an azepane ring, a dioxepane ring, and a 5,9-dioxaspiro[3.5]nonane ring.

Examples of the "protecting group for an amino group" as used herein include a carbamate-based protecting group, an acyl-based protecting group, a sulfonamide-based protecting group, and a silyl-based protecting group. Specific examples of the carbamate-based protecting group include a 9-fluorenylmethyloxycarbonyl group (Fmoc group), a benzyloxycarbonyl group (Cbz group), a 2,2,2-trichloroethoxycarbonyl group (Troc group), an allyloxycarbonyl group (Alloc group), a 2-(trimethylsilyl)ethoxycarbonyl group (Teoc group), a triisopropylsilyloxycarbonyl group (TSoc group), a di-t-butylisobutylsilyloxycarbonyl group (BIBSoc group), a di-i-propyl-t-butylsilyloxycarbonyl group (IPCSoc group), a benzyl-di-t-butylsilyloxycarbonyl group (BBSoc group), a di-t-butylcyclohexylsilyloxycarbonyl group (CHBSoc group), a di-t-butyloctadecylsilyloxycarbonyl group (CDBSoc group), and a t-butoxycarbonyl group (Boc group). Specific examples of the acyl-based protecting group include a trifluoroacetyl group, an acetyl group, and a benzoyl group. Specific examples of the sulfonamide-based protecting group include a 2-nitrobenzenesulfonyl group, a 4-nitrobenzenesulfonyl group, and a 2,4-dinitrobenzenesulfonyl group. Specific examples of the silyl-based protecting group include a trimethylsilyl group (TMS group), a t-butyldimethylsilyl group (TBDMS group), a triethylsilyl group (TES group), a triisopropylsilyl group (TIPS group), and a t-butyldiphenylsilyl group (TBDPS group).

The term "optionally substituted" as used herein means that a group may be substituted with any substituent.

The term "optionally protected" as used herein means that a group may be protected by any protecting group.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

The term "peptide compound" as used herein means one in which 2 or more amino acid residues are linked by an amide bond. Peptides having an ester bond in part of the main chain, such as depsipeptide, are also included in the term "peptide compound" herein. The number of amino acid residues contained in the peptide according to the present disclosure is not particularly limited, but preferably 5 to 30 residues, more preferably 8 to 15 residues, and still more preferably 9 to 13 residues. The peptide compound according to the present disclosure preferably contain at least 3 N-substituted amino acids, more preferably at least 5, and still more preferably at least 6. These N-substituted amino acids may be present continuously or discontinuously in the peptide compound. The peptide compound according to the present disclosure may be linear or cyclic, and is preferably a cyclic peptide compound.

The term "cyclic peptide compound" as used herein refers to a peptide compound having a cyclic structure composed of 4 or more amino acid residues. The cyclic structure of the cyclic peptide compound may contain a bond other than an amide bond, and it may contain, for example, a bond selected from the group consisting of a C-O-C bond, a C(O)-O bond, and a C(S)-O bond via an oxygen atom, a C(O)-S bond, a C(S)-S bond, a C-S-S-C bond, a C-S-C bond, a C-S(O)-C bond, and a C-S(O2)-C bond via a sulfur atom, a C-N-C bond, a C=N-C bond, a N-C(O)-N bond, a N-C(S)N bond, and a C(S)-N bond via a nitrogen atom, and a C-C bond. The cyclic peptide compound may have an amino acid or chain peptide structure that is not contained in the cyclic structure, in addition to the cyclic structure. It may also have a structure other than amino acids and chain peptide structures.

The term "cyclization" of a peptide compound means formation of a cyclic portion cyclic structure containing 4 or more amino acid residues. The number of amino acids contained in the cyclic portion of the cyclic peptide compound is not particularly limited herein, but examples thereof include 4 to 20 residues, 5 to 15 residues, and 6 to 13 residues. The method for converting a linear peptide compound to a cyclic peptide compound can be carried out by performing an intramolecular bond formation reaction by the method described in Comprehensive Organic Transformations, A Guide to Functional Group Preparations, 3rd Edition (authored by R. C. Larock), March's Advanced Organic Chemistry: Reactions, Mechanisms, and Structure, 7th Edition (authored by M. B. Smith and J. March), or the like. It is also possible to further perform a functional group conversion reaction after the bond formation reaction. Examples of the bond formation reaction include a C(O)-N bond formed from a carboxylic acid and an amine, a C-O-C bond, a C(O)-O bond, and a C(S)-O bond via an oxygen atom, a C(O)-S bond, a C(S)-S bond, a C-S-S-C bond, a C-S-C bond, a C-S(O)-C bond, and a C-S(O2)-C bond via a sulfur atom, and a C-N-C bond, a C=N-C bond, a N-C(O)-N bond, a N-C(S)N bond, and a C(S)-N bond via a nitrogen atom. Further examples thereof include a C-C bond formation reaction catalyzed by a transition metal, such as the Suzuki reaction, the Heck reaction, and the Sonogashira reaction. Examples of the functional group conversion reaction that is further performed after the bond formation reaction include an oxidation reaction or a reduction reaction. Specific examples thereof include a reaction in which a sulfur atom is oxidized and converted to a sulfoxide group or a sulfone group. Other examples thereof include a reduction reaction in which, among carbon-carbon bonds, a triple bond or a double bond is reduced and converted to a double bond or a single bond. Two amino acids may be bonded at the main chain of the amino acid to form a closed ring structure by a peptide bond, or a covalent bond may be formed between two amino acids via, for example, a bond between side chains or between a side chain and the main chain of the two amino acids.

The term "amino acid" as used herein includes a natural amino acid and a non-natural amino acid. The term "amino acid" as used herein may mean an amino acid residue. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, and Pro. Examples of the non-natural amino acid include, but are not particularly limited to, a β-amino acid, a D-type amino acid, a N-substituted amino acid, an α,α-disubstituted amino acid, an amino acid having a side chain different from that of natural amino acids, and a hydroxycarboxylic acid. As used herein, the amino acid may have any configuration. The selection of a side chain of the amino acid is not particularly limited, and the side chain is freely selected from, in addition to a hydrogen atom, for example, an alkyl group, an alkenyl group, an alkynyl group, an aryl group, a heteroaryl group, an aralkyl group, a heteroarylalkyl group, a cycloalkyl group, a spiro-bonded cycloalkyl group. Each of the side chains may have a substituent. The substituent is also not limited, and one or two or more substituents may be freely selected independently from any substituents including, for example, a halogen atom, an oxygen atom, a nitrogen atom, a sulfur atom, a boron atom, a silicon atom, or a phosphorus atom. That is, examples of the side chain include an alkyl group, an alkoxy group, an alkoxyalkyl group, an alkenyl group, an alkynyl group, an aryl group, a heteroaryl group, an aralkyl group and a cycloalkyl group which may be substituted, or oxo, aminocarbonyl, and a halogen atom. In one non-limiting embodiment, the amino acid as used herein may be a compound having a carboxyl group and an amino group in the same molecule (even in this case, proline, hydroxyproline, azetidine-2-carboxylic acid, and the like in which a nitrogen atom of the amino group and any atom of the side chain together forms a ring are also included in the amino acid).

As used herein, the "amino acid residue" constituting a peptide compound is sometimes simply referred to as "amino acid".

The term "N-terminal amino acid residue" as used herein means an amino acid residue located at the N-terminus of a peptide. The term "C-terminal amino acid residue" as used herein means an amino acid residue located at the C-terminus of a peptide.

As used herein, the term "number of amino acids (amino acid number)" or "number of amino acid residues (amino acid residue number)" refers to the number of amino acid residues (amino acid units) constituting a peptide compound, and means the number of amino acid units generated upon cleavage of amide bonds, ester bonds, and bonds of cyclized portions that link the amino acids.

The "amino acid" as used herein constituting a peptide compound includes all isotopes corresponding to each. The isotope of the "amino acid" is a form in which at least one atom is replaced with an atom having the same atomic number (proton number) and a different mass number (total number of protons and neutrons) at an abundance ratio different from the natural abundance ratio. Examples of the isotope included in the "amino acid" forming a peptide

compound herein include a hydrogen atom, a carbon atom, a nitrogen atom, an oxygen atom, a phosphorus atom, a sulfur atom, a fluorine atom, a chlorine atom, and the like, and they include $^{2}H$, $^{3}H$; $^{13}C$, $^{14}C$; $^{15}N$; $^{17}O$, $^{18}O$; $^{31}P$, $^{32}P$; $^{35}S$; $^{18}F$; $^{36}Cl$; and the like, respectively. For the compounds as used herein, all the compounds containing any proportions of radioactive or nonradioactive isotopic elements are encompassed within the scope of the present invention.

Examples of the substituent containing a halogen atom herein include an alkyl group, a cycloalkyl group, an alkenyl group, an alkynyl group, an aryl group, a heteroaryl group, and an aralkyl group having halogen as a substituent and more specifically include a fluoroalkyl, a difluoroalkyl, and a trifluoroalkyl.

Examples of the substituent containing an O atom include hydroxy (-OH), oxy (-OR), carbonyl (-C=O-R), carboxy ($-CO_2H$), oxycarbonyl (-C=O-OR), carbonyloxy (-O-C=O-R), thiocarbonyl (-C=O-SR), carbonylthio (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxyl (-C(=O)-SH) and carboxylcarbonyl ($-C(=O)-CO_2H$) groups.

Examples of the oxy (-OR) include an alkoxy, a cycloalkoxy, an alkenyloxy, an alkynyloxy, an aryloxy, a heteroaryloxy, and an aralkyloxy. The alkoxy is preferably a $C_1$-$C_4$ alkoxy or a $C_1$-$C_2$ alkoxy, particularly preferably methoxy or ethoxy.

Examples of the carbonyl (-C=O-R) include formyl (-C=O-H), an alkylcarbonyl, a cycloalkylcarbonyl, an alkenylcarbonyl, an alkynylcarbonyl, an arylcarbonyl, a heteroarylcarbonyl, and an aralkylcarbonyl.

Examples of the oxycarbonyl (-C=O-OR) include an alkyloxycarbonyl, a cycloalkyloxycarbonyl, an alkenyloxycarbonyl, an alkynyloxycarbonyl, an aryloxycarbonyl, a heteroaryloxycarbonyl, and an aralkyloxycarbonyl.

Examples of the carbonyloxy (-O-C=O-R) include an alkylcarbonyloxy, a cycloalkylcarbonyloxy, an alkenylcarbonyloxy, an alkynylcarbonyloxy, an arylcarbonyloxy, a heteroarylcarbonyloxy, and an aralkylcarbonyloxy.

Examples of the thiocarbonyl (-C=O-SR) include an alkylthiocarbonyl, a cycloalkylthiocarbonyl, an alkenylthiocarbonyl, an alkynylthiocarbonyl, an arylthiocarbonyl, a heteroarylthiocarbonyl, and an aralkylthiocarbonyl.

Examples of the carbonylthio (-S-C=O-R) include an alkylcarbonylthio, a cycloalkylcarbonylthio, an alkenylcarbonylthio, an alkynylcarbonylthio, an arylcarbonylthio, a heteroarylcarbonylthio, and an aralkylcarbonylthio.

Examples of the aminocarbonyl (-C=O-NHR) include an alkylaminocarbonyl (for example, a $C_1$-$C_6$ or $C_1$-$C_4$ alkylaminocarbonyl, and in particular, ethylaminocarbonyl and methylaminocarbonyl), a cycloalkylaminocarbonyl, an alkenylaminocarbonyl, an alkynylaminocarbonyl, an arylaminocarbonyl, a heteroarylaminocarbonyl, and an aralkylaminocarbonyl. Examples thereof additionally include groups in which the H atom bonded to the N atom in -C=O-NHR is further replaced with an alkyl, a cycloalkyl, an alkenyl, an alkynyl, an aryl, a heteroaryl, or an aralkyl.

Examples of the carbonylamino (-NH-C=O-R) include an alkylcarbonylamino, a cycloalkylcarbonylamino, an alkenylcarbonylamino, an alkynylcarbonylamino, an arylcarbonylamino, a heteroarylcarbonylamino, and an aralkylcarbonylamino. Examples thereof additionally include groups in which the H atom bonded to the N atom in -NH-C=O-R is further replaced with an alkyl, a cycloalkyl, an alkenyl, an alkynyl, an aryl, a heteroaryl, or an aralkyl.

Examples of the oxycarbonylamino (-NH-C=O-OR) include an alkoxycarbonylamino, a cycloalkoxycarbonylamino, an alkenyloxycarbonylamino, an alkynyloxycarbonylamino, an aryloxycarbonylamino, a heteroaryloxycarbonylamino, and an aralkyloxycarbonylamino. Examples thereof additionally include groups in which the H atom bonded to the N atom in -NH-C=O-OR is further replaced with an alkyl, a cycloalkyl, an alkenyl, an alkynyl, an aryl, a heteroaryl, or an aralkyl.

Examples of the sulfonylamino ($-NH-SO_2-R$) include an alkylsulfonylamino, a cycloalkylsulfonylamino, an alkenylsulfonylamino, an alkynylsulfonylamino, an arylsulfonylamino, a heteroarylsulfonylamino, and an aralkylsulfonylamino. Examples thereof additionally include groups in which the H atom bonded to the N atom in $-NH-SO_2-R$ is further replaced with an alkyl, a cycloalkyl, an alkenyl, an alkynyl, an aryl, a heteroaryl, or an aralkyl.

Examples of the aminosulfonyl ($-SO_2-NHR$) include an alkylaminosulfonyl, a cycloalkylaminosulfonyl, an alkenylaminosulfonyl, an alkynylaminosulfonyl, an arylaminosulfonyl, a heteroarylaminosulfonyl, and an aralkyl amino sulfonyl. Examples thereof additionally include groups in which the H atom bonded to the N atom in $-SO_2-NHR$ is further replaced with an alkyl, a cycloalkyl, an alkenyl, an alkynyl, an aryl, a heteroaryl, or an aralkyl.

Examples of the sulfamoylamino ($-NH-SO_2-NHR$) include an alkylsulfamoylamino, a cycloalkylsulfamoylamino, an alkenylsulfamoylamino, an alkynylsulfamoylamino, an arylsulfamoylamino, a heteroarylsulfamoylamino, and an aralkylsulfamoylamino. Furthermore, the two H atoms bonded to the N atoms in $-NH-SO_2-NHR$ may be replaced with substituents each independently selected from the group consisting of an alkyl, a cycloalkyl, an alkenyl, an alkynyl, an aryl, a heteroaryl, and an aralkyl, and these two substituents may form a ring.

Examples of the substituent containing a S atom include groups such as thiol (-SH), thio (-S-R), sulfinyl (-S=O-R), sulfonyl ($-SO_2-R$), and sulfo ($-SO_3H$).

Examples of the thio (-S-R) that can be selected include an alkylthio, a cycloalkylthio, an alkenylthio, an alkynylthio, an arylthio, a heteroarylthio, and an aralkylthio.

Examples of the sulfonyl ($-SO_2-R$) include an alkylsulfonyl, a cycloalkylsulfonyl, an alkenylsulfonyl, an alkynylsulfonyl, an arylsulfonyl, a heteroarylsulfonyl, and an aralkylsulfonyl.

Examples of the substituent containing a N atom include groups such as azide (-$N_3$; also referred to as "azide group"), cyano (-CN), primary amino (-$NH_2$), secondary amino (-NH-R; also referred to as mono-substituted amino), tertiary amino (-NR(R'); also referred to as di-substituted amino), amidino (-C(=NH)-$NH_2$), substituted amidino (-C(=NR)-NR'R"), guanidino (-NH-C(=NH)-$NH_2$), substituted guanidino (-NR-C(=NR")-NR'R"), aminocarbonylamino (-NR-CO-NR'R"), pyridyl, piperidino, morpholino, and azetidinyl.

Examples of the secondary amino (-NH-R: mono-substituted amino) include an alkylamino, a cycloalkylamino, an alkenylamino, an alkynylamino, an arylamino, a heteroarylamino, and an aralkylamino.

Examples of the tertiary amino (-NR(R'): di-substituted amino) include an amino group having any two substituents each independently selected from an alkyl, a cycloalkyl, an alkenyl, an alkynyl, an aryl, a heteroaryl, and an aralkyl, for example, an alkyl(aralkyl)amino. These two substituents may form a ring. Specific examples thereof include a dialkylamino, particularly, a $C_1$-$C_6$ dialkylamino, a $C_1$-$C_4$ dialkylamino, dimethylamino, and diethylamino. The term "$C_p$-$C_q$ dialkylamino group" as used herein refers to a group in which an amino group is substituted by two $C_p$-$C_q$ alkyl groups. The $C_p$-$C_q$ alkyl groups may be the same or different.

Examples of the substituted amidino (-C(=NR)-NR'R") include groups in which three substituents R, R', and R" on the N atoms are each independently selected from an alkyl, a cycloalkyl, an alkenyl, an alkynyl, an aryl, a heteroaryl, and an aralkyl, for example, an alkyl(aralkyl)(aryl)amidino.

Examples of the substituted guanidino (-NR-C(=NR‴)-NR'R") include groups in which R, R', R", and R‴ are each independently selected from an alkyl, a cycloalkyl, an alkenyl, an alkynyl, an aryl, a heteroaryl, and an aralkyl, and groups in which these substituents form a ring.

Examples of the aminocarbonylamino (-NR-CO-NR'R") include groups in which R, R', and R" are each independently selected from a hydrogen atom, an alkyl, a cycloalkyl, an alkenyl, an alkynyl, an aryl, a heteroaryl, and an aralkyl, and groups in which these substituents form a ring.

The compound according to the present invention may be a salt thereof, preferably a chemically acceptable salt thereof. The compound according to the present invention, or a salt thereof may be a solvate thereof, preferably a chemically acceptable solvate thereof. Examples of the salt of the compound according to the present invention include: hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or base that can be used in the production of a medicament. In the present invention, the solvate of the compound is one in which the compound and a solvent together form a molecular aggregate, and is not particularly limited as long as it is a solvate formed by a solvent. When the solvent is water, the solvate is called a hydrate. The solvate of the compound according to the present invention is preferably a hydrate, and specific examples of such a hydrate include a mono- to decahydrate, preferably a mono- to pentahydrate, and still more preferably a mono- to trihydrate. The solvate of the compound according to the present invention includes not only solvates with a single solvent such as water, alcohol (such as methanol, ethanol, 1-propanol, and 2-propanol), or dimethylformamide, but also solvates with a plurality of solvents.

When the compound according to the present invention is obtained as a free form, the compound can be conventionally transformed into the state of a hydrate or solvate thereof. When the compound according to the present invention is obtained as a free form, the compound can be conventionally transformed into the state of a salt that may be formed by the compound, or a hydrate or solvate thereof. Examples thereof include a hydrate and an ethanolate of the cyclic peptide compound represented by formula (1a) or a salt thereof. Specific examples thereof include, but are not limited to, a hemihydrate, a monohydrate, a dihydrate, a trihydrate, a tetrahydrate, a pentahydrate, a hexahydrate, a heptahydrate, an octahydrate, a nonahydrate, a decahydrate, or a monoethanolate of the cyclic peptide compound represented by formula (1a); a hemihydrate, a monohydrate, a dihydrate, a trihydrate, a tetrahydrate, a pentahydrate, a hexahydrate, a heptahydrate, an octahydrate, a nonahydrate, a decahydrate, or a monoethanolate of a sodium salt of the compound represented by formula (1a); or a hydrate or an ethanolate of a hydrochloride of the cyclic peptide compound represented by formula (1a). The hydrate or solvate may be produced in a crystalline form or non-crystalline form. In the case of the crystalline form, the hydrate or solvate may have crystalline polymorphs. As for the method for producing the hydrate or solvate, the hydrate or solvate can be obtained by a conventional method, for example, by adding a solvent such as ethanol and/or water to the cyclic peptide compound represented by formula (1a) or the peptide compound described herein, followed by stirring, cooling, concentrating, and/or drying.

[Formula 22]

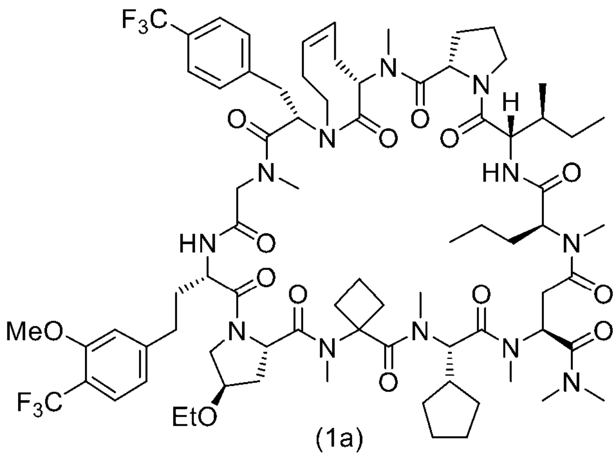

(1a)

When the compound according to the present invention is obtained as a salt, a hydrate, or a solvate of the compound, the compound can be conventionally transformed into a free form thereof.

The term "solvent A/water solvate crystal" as used herein refers to a crystal in which solvent A molecules and water molecules are contained in the crystal lattice of the compound. The term "solvent A/solvent B/water solvate crystal" as used herein refers to a crystal in which solvent A molecules, solvent B molecules, and water molecules are contained in the crystal lattice of the compound. Specifically, for example, the term "acetone/heptane/water solvate crystal" refers to a crystal in which acetone, heptane, and water are contained in the crystal lattice of the compound.

The meaning of the term "and/or" as used herein includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.

The term "epimer" as used herein means a compound in which the configuration of a side chain bonded to the α-carbon of an amino acid residue constituting a cyclic peptide compound is sterically inverted (epimer). The "epimer" includes a cyclic peptide compound in which the α-carbon of the C-terminal amino acid residue of a linear peptide compound is sterically inverted when the linear peptide compound is cyclized to produce the cyclic peptide compound. The epimer in the total products including the cyclic peptide compound produced by the method of the present invention can be determined by, for example, UVarea value at 210 nm or 220 nm by HPLC analysis.

The term "cyclic dimer" as used herein means a compound in which peptide compounds, which are the raw material of the cyclic peptide compound, are linearly bonded to each other and then further cyclized. The cyclic dimer in the total products including the cyclic peptide compound produced by the method of the present invention can be determined by, for example, UVarea value at 210 nm or 220 nm by HPLC analysis.

Method for producing cyclic peptide compound

In an aspect, the present invention relates to a method for producing a cyclic peptide compound represented by formula (1), or a salt thereof, or a solvate thereof. The method includes a step of reacting an N-terminal amino acid residue of a peptide compound represented by formula (2) or (3) with a C-terminal amino acid residue of the peptide compound in a solvent for cyclization (cyclization step) (hereinafter, also referred to as "aspect 1").

[Formula 23]

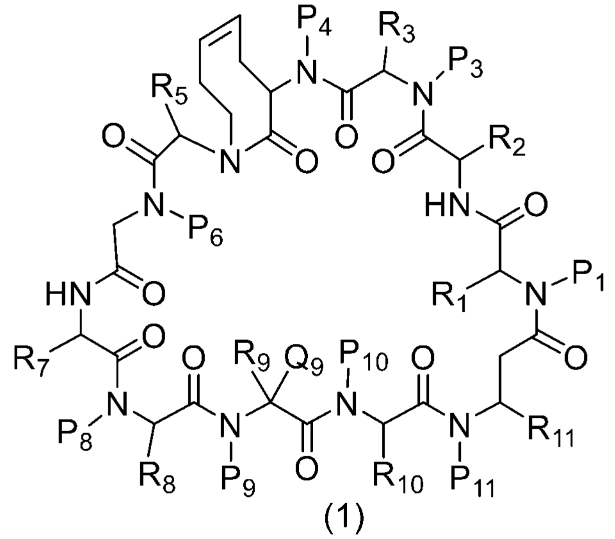

(1)

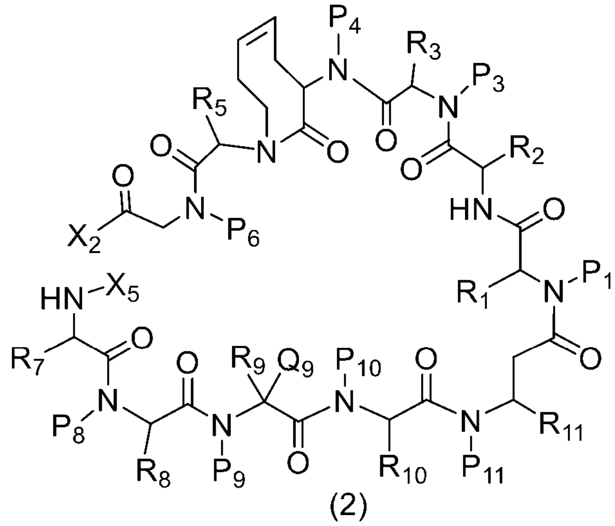

(2)

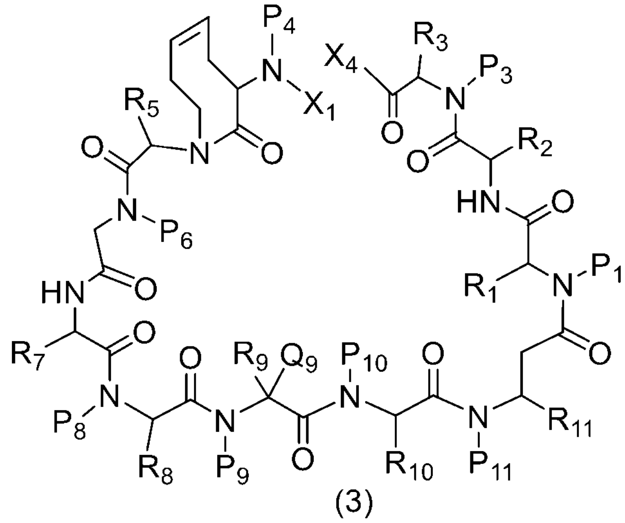

(3)

In an aspect, the present invention relates to a method for producing a cyclic peptide compound represented by formula (1), or a salt thereof, or a solvate thereof. The method includes

(a) a step of providing peptide compounds represented by formulae (4) to (6), or salts thereof, or solvates of the peptide compounds or salts,

(b) a step of reacting N-terminal amino acid residues of the peptide compounds represented by formulae (4) to (6) with C-terminal amino acid residues of the peptide compounds in a solvent for linkage (linking step), and

(c) a step of reacting an N-terminal amino acid residue of a peptide compound obtained in the step (b) with a C-terminal amino acid residue of the peptide compound in a solvent for cyclization (cyclization step) (hereinafter, also referred to as "aspect 2").

[Formula 24]

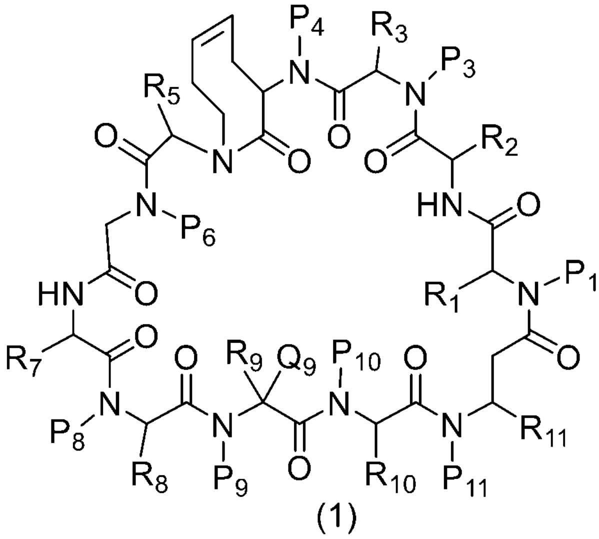

(1)

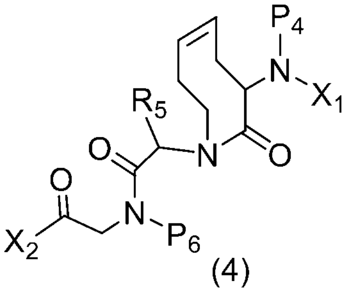

(4)

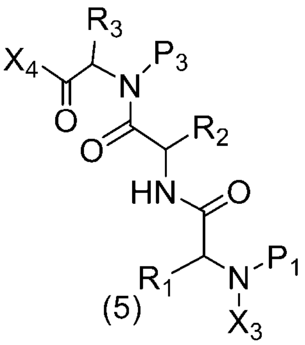

(5)

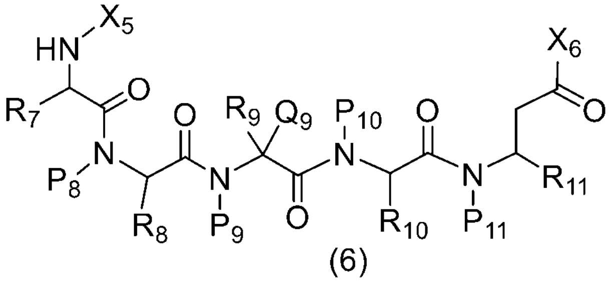

(6)

In aspect 2, the step (b) may include (b-1) a step of reacting an N-terminal amino acid residue of the peptide compound represented by formula (5) with a C-terminal amino acid residue of the peptide compound represented by formula (6) in a solvent for linkage, thereby converting them into a peptide compound represented by formula (7) (linking step).

[Formula 25]

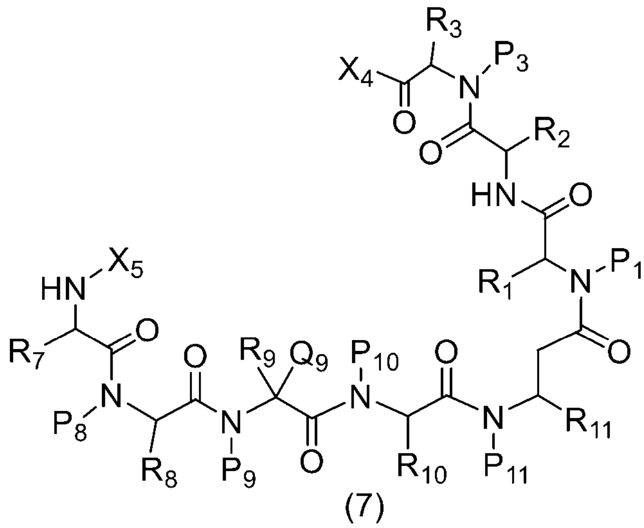

(7)

In aspect 2, the step (b) may include, in addition to the step (b-1),

(b-2) a step of reacting an N-terminal amino acid residue of the peptide compound represented by formula (4) with a C-terminal amino acid residue of the peptide compound represented by formula (7) in a solvent for linkage, thereby converting them into a peptide compound represented by formula (2) (linking step), and
the step (c) may include
(c-1) a step of reacting an N-terminal amino acid residue of the peptide compound represented by formula (2) with a C-terminal amino acid residue of the peptide compound in a solvent for cyclization (cyclization step).

In aspect 2, the step (b) may include, in addition to the step (b-1),

(b-3) a step of reacting an N-terminal amino acid residue of the peptide compound represented by formula (7) with a C-terminal amino acid residue of the peptide compound represented by formula (4) in a solvent for linkage, thereby converting them into a compound represented by formula (3) (linking step), and
the step (c) may include
(c-2) a step of reacting an N-terminal amino acid residue of the peptide compound represented by formula (3) with a C-terminal amino acid residue of the peptide compound in a solvent for cyclization (cyclization step).

In aspects 1 and 2, the linkage of an N-terminal amino acid residue of a peptide compound with a C-terminal amino acid residue of a peptide compound is preferably linkage of an amino group of the N-terminal amino acid residue with a carboxyl group of the C-terminal amino acid residue, and the linkage of an N-terminal amino acid residue of a peptide compound with a C-terminal amino acid residue of a peptide compound is more preferably linkage by means of an amide bond of an amino group of the N-terminal amino acid residue with a carboxyl group of the C-terminal amino acid residue.
In an aspect, the solvent in the cyclization step preferably includes one or more selected from the group consisting of a nitrile-based solvent, a halogen-based solvent, an ether-based solvent, an amide-based solvent, an ester-based solvent, and a carbonate-based solvent. Specific examples of the nitrile-based solvent include acetonitrile and propionitrile. Specific examples of the halogen-based solvent include dichloromethane, chloroform, and 1,2-dichloroethane. Specific examples of the ether-based solvent include diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, t-butyl methyl ether, diglyme, triglyme, anisole, and tetraglyme. Specific examples of the amide-based solvent include DMF, NMP, DMA, NEP, NBP, and formamide. Specific examples of the ester-based solvent include methyl acetate, ethyl acetate, methyl propionate, butyl acetate, propyl acetate, isopropyl acetate, isobutyl acetate, pentyl acetate, and γ-valerolactone. Specific examples of the carbonate-based solvent include dimethyl carbonate, diethyl carbonate, and dibutyl carbonate. The solvent in the cyclization step is preferably one or more selected from the group consisting of acetonitrile, dimethyl carbonate, 2-methyltetrahydrofuran, 4-methyltetrahydropyran, tetrahydrofuran, ethyl acetate, isopropyl acetate, dichloromethane, DMF, and anisole, more preferably one or more selected from the group consisting of acetonitrile, 2-methyltetrahydrofuran, ethyl acetate, and dichloromethane, and still more preferably acetonitrile, 2-methyltetrahydrofuran, or ethyl acetate.
In an aspect, the cyclization step can be performed by stirring the reaction mixture in a solvent in the presence or absence of a condensation reagent, in the presence or absence of a base, at a temperature between -20°C and near the

boiling point of the solvent, preferably between -20°C and 100°C, preferably between -5°C and 60°C, for 10 minutes to 48 hours.

The condensation reagent and base used in the cyclization step, as well as their amounts to be used, are not particularly limited, but a condensation reagent and a base commonly used in peptide synthesis, as well as amount to be used, are preferred (see, for example, Peptide Coupling Reagents, More than a Letter Soup (Chem. Rev. 2011, 111, 6557-6602.)). When a condensation reagent is not used in the cyclization step, the carboxyl group may be converted to an active ester in advance.

Specific examples of the condensation reagent in the cyclization step include N,N'-dicyclohexylcarbodiimide (DCC), N,N'-diisopropylcarbodiimide (DIC), 1-ethyl-3-(3-dimethylaminopropyl)carbodiimide hydrochloride (EDCI HCl), 1-hydroxy-1H-benzotriazole (HOBt), 1-hydroxy-7-azabenzotriazole (HOAt), 2-cyano-2-(hydroxyimino)ethyl acetate (oxyma), 3,4-dihydro-3-hydroxy-4-oxo-1,2,3-benzotriazine (HOOBt or HODhbt), N-hydroxy-5-norbomene-2,3-dicarboximide (HONB), 2,3,4,5,6-pentafluorophenol (HOPfp), N-hydroxysuccinimide (HOSu), 6-chloro-1-hydroxy-1H-benzotriazole (Cl-HOBt), O-(1H-benzotriazol-1-yl)-N,N,N',N'-tetramethyluronium hexafluorophosphate (HBTU), O-(7-aza-1H-benzotriazol-1-yl)-N,N,N',N'-tetramethyluronium hexafluorophosphate (HATU), N-[1-(cyano-2-ethoxy-2-oxoethylideneaminooxy)dimethylamino(morpholino)]uronium hexafluorophosphate (COMU), O-[(ethoxycarbonyl)cyanomethyleneamino]-N,N,N',N'-tetramethyluronium hexafluorophosphate (HOTU), O-(1H-benzotriazol-1-yl)-N,N,N',N'-tetramethyluronium tetrafluoroborate (TBTU), O-(7-azabenzotriazol-1-yl)-N,N,N',N'-tetramethyluronium tetrafluoroborate (TATU), [ethylcyano(hydroxyimino)acetato-$O^2$]tri-1-pyrrolidinylphosphonium hexafluorophosphate (PyOxim), 2-bromo-1-ethyl pyridinium tetrafluoroborate (BEP), 1H-benzotriazole-1-yloxy-tri(pyrrolidino)phosphonium hexafluorophosphate (PyBOP), 1H-benzotriazole-1-yloxy-tris (dimethylamino)phosphonium hexafluorophosphate (BOP), bromotri(pyrrolidino) phosphonium hexafluorophosphate (PyBroP), chlorotri(pyrrolidino)phosphonium hexafluorophosphate (PyCloP), (7-azabenzotriazol-1-yloxy)tripyrrolidino phosphonium hexafluorophosphate (PyAOP), bromotris(dimethylamino)phosphonium hexafluorophosphate (Brop), 3-(diethoxyphosphoryloxy)-1,2,3-benzotriazin-4(3H)-one (DEPBT), N,N,N',N'-tetramethyl-O-(N-succinimidyl)uronium tetrafluoroborate (TSTU), N,N,N',N'-tetramethyl-O-(N-succinimidyl)uronium hexafluorophosphate (HSTU), O-(3,4-dihydro-4-oxo-1,2,3-benzotriazin-3-yl)-N,N,N',N'-tetramethyluronium tetrafluoroborate (TDBTU), tetramethylthiuronium S-(1-oxide-2-pyridyl)-N,N,N',N'-tetrafluoroborate (TOTT), O-(2-oxo-1(2H)pyridyl)-N,N,N',N'-tetramethyluronium tetrafluoroborate (TPTU), N,N' -carbonyl diimidazole (CDI), 1,1'-carbonyl-di-(1,2,4-triazole) (CDT), 4-(4,6-dimethoxy-1,3,5-triazin-2-yl)-4-methylmorpholinium chloride (DMT-MM), and propylphosphonic anhydride (T3P). Among them, from the viewpoint of suppressing by-products, the condensation reagent in the cyclization step is preferably one or more selected from the group consisting of HATU, COMU, DMT-MM, PyOxim, PyBOP, HCTU, T3P, EDCI, BEP, and PyClop, more preferably one selected from the group consisting of HATU, COMU, PyOxim, PyBOP, HCTU, and T3P, and still more preferably HATU or COMU. In addition, the combination of the solvent and the condensation reagent is preferably HATU and acetonitrile or 2-methyltetrahydrofuran; or COMU and acetonitrile or 2-methyltetrahydrofuran, since by-products can be even further suppressed.

As the base in the cyclization step, organic bases are suitably used, and particularly, organic bases containing tertiary amines are preferred. Specific examples of such a base include 2,2,6,6-tetramethylpiperidine, N-methylmorpholine, N,N-diisopropylethylamine (DIPEA), 2,4,6-collidine, 2,6-lutidine, pyridine, 1,8-diazabicyclo[5.4.0]-7-undecene (DBU), 2,3,6,7-tetrahydro-1H,5H-9-azabenzo[ij]quinolidine, 1,4-diazabicyclo[2.2.2]octane (DABCO), 1,5-diazabicyclo [4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-ethylmorpholine, and p-dimethylaminopyridine (DMAP). Among them, from the viewpoint of suppressing by-products, the base in the cyclization step is preferably one or more selected from the group consisting of 2,2,6,6-tetramethylpiperidine, N-methylmorpholine, N,N-diisopropylethylamine (DIPEA), 2,4,6-collidine, 2,6-lutidine, and pyridine, and preferably N,N-diisopropylethylamine (DIPEA) or 2,6-lutidine. In addition, the combination of the solvent, the condensation reagent, and the base is preferably HATU, acetonitrile, and N,N-diisopropylethylamine (DIPEA); HATU, 2-methyltetrahydrofuran, and N,N-diisopropylethylamine (DIPEA); COMU, acetonitrile, and 2,6-lutidine; or COMU, 2-methyltetrahydrofuran, and 2,6-lutidine, since by-products can be even further suppressed.

In an aspect, the cyclization step is performed by a liquid phase method.

In an aspect, the cyclization step is performed by mixing the peptide compound and optionally the base in a mixed solution obtained by mixing the solvent and the condensation reagent. Such an operation may be herein referred to as "inverse dropwise addition". Inverse dropwise addition of the peptide compound and the base over a long period of time, such as several hours to several days, preferably 1 to 24 hours, and more preferably 1 to 10 hours, can suppress generation of by-products without using a large amount of solvent for dilution.

The cyclic peptide compounds produced by the method of the present invention are of high purity with a low content of by-products (such as epimer and cyclic dimer) as described below.

In an aspect, a content of the total by-products generated in the cyclization step is less than 20%, less than 15%, less than 10%, less than 5%, or less than 3%, as determined by UVarea value at 220 nm by HPLC analysis, based on the

total amount of products.

In an aspect, a content of each of by-products generated in the cyclization step is less than 15%, less than 10%, less than 5%, less than 3%, less than 1%, or an undetectable amount, as determined by UVarea value at 220 nm by HPLC analysis, based on the total amount of products.

In an aspect, a content of each of by-products generated in the cyclization step is less than 15%, less than 10%, less than 5%, less than 3%, less than 1%, or an undetectable amount, as determined by UVarea value at 220 nm by HPLC analysis, based on the total amount of products, and the by-products comprise an epimer and/or a cyclic dimer.

In an aspect, by-products generated in the cyclization step comprise an epimer, and a content of the epimer is less than 10%, less than 7.5%, less than 5%, less than 2.5%, or less than 1%, as determined by UVarea value at 220 nm by HPLC analysis, based on the total amount of products.

In an aspect, by-products generated in the cyclization step comprise a cyclic dimer, and a content of the cyclic dimer is less than 15%, less than 10%, less than 5%, less than 2.5%, or less than 1%, as determined by UVarea value at 220 nm by HPLC analysis, based on the total amount of products.

In an aspect, the solvent in the linking step preferably includes one or more selected from the group consisting of a nitrile-based solvent, a halogen-based solvent, an ether-based solvent, an amide-based solvent, an ester-based solvent, and a carbonate-based solvent. Examples of the nitrile-based solvent, halogen-based solvent, ether-based solvent, amide-based solvent, ester-based solvent, and carbonate-based solvent include those exemplified as the solvent in the cyclization step described above. The solvent in the linking step is preferably one or more selected from the group consisting of acetonitrile, dimethyl carbonate, 2-methyltetrahydrofuran, 4-methyltetrahydropyran, tetrahydrofuran, ethyl acetate, isopropyl acetate, DMF, and anisole, more preferably a mixed solvent of one or more selected from the group consisting of acetonitrile, dimethyl carbonate, 2-methyltetrahydrofuran, 4-methyltetrahydropyran, tetrahydrofuran, ethyl acetate, isopropyl acetate, and anisole with DMF, still more preferably a mixed solvent of acetonitrile, 2-methyltetrahydrofuran, and DMF, and particularly preferably a mixed solvent of 2-methyltetrahydrofuran and DMF.

In an aspect, the linking step can be performed by stirring the reaction composition in a solvent in the presence or absence of a condensation reagent, in the presence or absence of a base, at a temperature between -20°C and near the boiling point of the solvent, preferably between -20°C and 100°C, preferably between -5°C and 60°C, for 10 minutes to 48 hours.

The condensation reagent and base used in the linking step, as well as their amounts to be used, are not particularly limited, but a condensation reagent and a base commonly used in peptide synthesis, as well as amount to be used, are preferred (see, for example, Peptide Coupling Reagents, More than a Letter Soup (Chem. Rev. 2011, 111, 6557-6602.)). When a condensation reagent is not used in the linking step, the carboxyl group may be converted to an active ester in advance.

Examples of the condensation reagent in the linking step include those exemplified as the condensation reagent in the cyclization step described above. Among them, from the viewpoint of suppressing by-products, the condensation reagent in the linking step is preferably one or more selected from the group consisting of HATU, COMU, DMT-MM, PyOxim, PyBOP, HCTU, T3P, EDCI, BEP, and PyClop, more preferably one selected from the group consisting of HATU, COMU, PyOxim, PyBOP, HCTU, and T3P, and still more preferably HATU or COMU. In addition, the combination of the solvent and the condensation reagent is preferably HATU and acetonitrile or 2-methyltetrahydrofuran; HATU and a mixed solvent of acetonitrile, 2-methyltetrahydrofuran, and DMF; COMU and acetonitrile or 2- methyltetrahydrofuran; or COMU and a mixed solvent of acetonitrile, 2-methyltetrahydrofuran, and DMF, since by-products can be even further suppressed.

Examples of the base in the linking step include those exemplified as the base in the cyclization step described above. Among them, from the viewpoint of suppressing by-products, the base in the linking step is preferably one or more selected from the group consisting of 2,2,6,6-tetramethylpiperidine, N-methylmorpholine, N,N-diisopropylethylamine (DIPEA), 2,4,6-collidine, 2,6-lutidine, pyridine, 1,8-diazabicyclo[5.4.0]-7-undecene (DBU), 2,3,6,7-tetrahydro-1H,5H-9-azabenzo[ij]quinolidine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino) naphthalene (TMGN), 2-tert-butyl-1,1,3,3-tetramethylguanidine (BTMG), triethylamine (TEA), trimethylamine, 1-methylpiperidine, N,N'-dimethylpiperazine, N-ethylmorpholine, and p-dimethylaminopyridine (DMAP), and preferably one or more selected from the group consisting of 2,2,6,6-tetramethylpiperidine, N-methylmorpholine, N,N-diisopropylethylamine (DIPEA), 2,4,6-collidine, 2,6-lutidine, and pyridine. In addition, the combination of the solvent, the condensation reagent, and the base is preferably HATU, acetonitrile or 2-methyltetrahydrofuran, and N,N-diisopropylethylamine (DIPEA); HATU, a mixed solvent of acetonitrile, 2-methyltetrahydrofuran, and DMF, and N,N-diisopropylethylamine (DIPEA); COMU, acetonitrile or 2- methyltetrahydrofuran, and N-methylmorpholine or 2,6-lutidine; or COMU, a mixed solvent of acetonitrile, 2-methyltetrahydrofuran, and DMF, and N-methylmorpholine or 2,6-lutidine, since by-products can be even further suppressed.

In an aspect, the linking step is performed by a liquid phase method.

In an aspect, the method of the present invention further includes a step of providing peptide compounds represented by formulae (4) to (6), or salts thereof, or solvates thereof. The peptide compounds represented by formulae

(4) to (6) can be produced by, for example, the following general production methods.

General production method for peptide compound represented by formula (4) (peptide compound (4))

A general production method for the peptide compound (4) will be shown below. In the following scheme, $Pg_4$ and $Pg_5$ represent protecting groups for an amino group, $Xg_6$ represents an oxygen atom and a protecting group bonded thereto, $R_5$ represents a side chain of an amino acid, and $P_4$ and $P_6$ represent substituents of a nitrogen atom.

The peptide compound (4) can be produced using the following method.

[Formula 26]

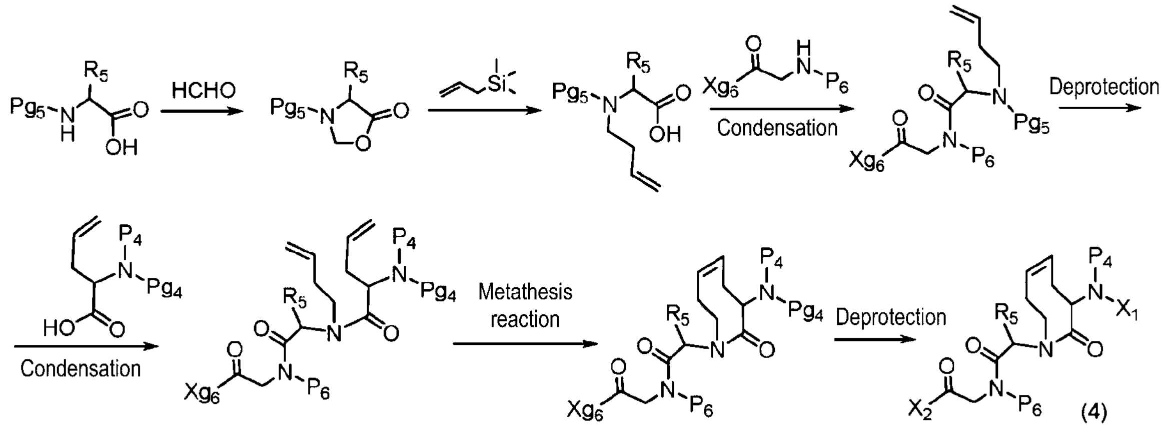


(4)

$X_1$ = H、$X_2$ = $Xg_6$
or
$X_1$ = $Pg_4$、$X_2$ = OH

By allowing an aldehyde to act on a protected amino acid in accordance with the method of Freidinger et al. (J. Org. Chem., 1983, 48(1), 77-81), an oxazolidinone form with a cyclic protecting group introduced can be obtained. Next, by performing a ring-opening reaction using a silicon compound having an olefin in accordance with the method of Nguyen et al. (Synthesis, 2009, 12, 1991), an alkyl group having an olefin can be introduced onto the nitrogen atom. Next, by condensing an amino acid whose C-terminus has been protected, the amino acid can be elongated on the C-terminus side. For the condensation reaction, the condensation reagent and base used in the linking step described above can be used. For example, various methods are possible as the activator of the carboxyl group, such as the combination of DIC and Oxyma, the combination of DIC and HOAt, the combination of HATU and DIPEA, or via a mixed acid anhydride or an acid halide. Then, after deprotecting the protecting group for the amino group, the protected amino acid having an olefin as a side chain can be elongated. Next, through a metathesis reaction, the intramolecular olefins can be cyclized. In the metathesis reaction, dichloro(2-isopropoxybenzylidene)(tricyclohexylphosphine)ruthenium(II): CAS No. 203714-71-0, dichloro(3-phenyl-1H-inden-1-ylidene)bis(tricyclohexylphosphine)ruthenium(II): CAS No. 250220-36-1, dichloro(benzylidene)bis(tricyclohexylphosphine)ruthenium(II): CAS No. 172222-30-9, [1,3-bis(2,4,6-trimethylphenyl)-2-imidazolidinylidene]dichloro(3-phenyl-1H-inden-1-ylidene)(tricyclohexylphosphine)ruthenium(II): CAS No. 536724-67-1, [1,3-bis(2,4,6-trimethylphenyl)-2-imidazolidinylidene]dichloro(benzylidene)(tricyclohexylphosphine)ruthenium(II): CAS No. 246047-72-3, [1,3-bis-(2,4,6-trimethylphenyl)-2-imidazolidinylidene]dichloro(2-isopropoxybenzylidene)ruthenium(II): CAS No. 301224-40-8, [1,3-bis-(2-tolyl)-2-imidazolidinylidene]dichloro(2-isopropoxybenzylidene)ruthenium(II): CAS No. 927429-61-6, [1,3-bis(2,4,6-trimethylphenyl)-2-imidazolidinylidene]dichloro[(2-isopropoxy)(5-trifluoroacetamido)benzylidene]ruthenium(II): CAS No. 1025728-56-6, [1,3-bis(2,6-diisopropylphenyl)-2-imidazolidinylidene]dichloro[5-(isobutoxycarbonylamido)-2-isopropoxybenzylidene]ruthenium(II): CAS No. 1212009-05-6, or the like can be used as the catalyst. Next, the C-terminal protecting group can be deprotected to produce the peptide compound (4) with the C-terminus unprotected, or the N-terminal protecting group can be deprotected to produce the peptide compound (4) with the N-terminus unprotected.

The peptide compound (4) can also be produced using the following method.

[Formula 27]

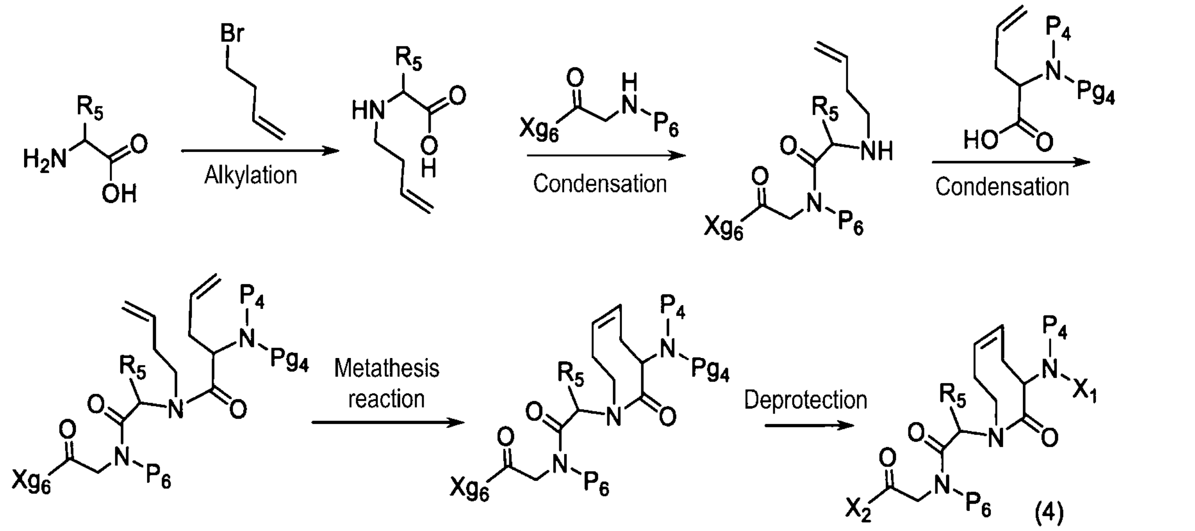


(4)

$X_1$ = H、$X_2$ = $Xg_6$
or
$X_1$ = $Pg_4$、$X_2$ = OH

By allowing an alkylating agent having an olefin to act on an amino acid in the presence of a base, an alkyl group having an olefin can be introduced to the nitrogen atom. Next, by condensing an amino acid whose C-terminus has been protected, the amino acid can be elongated on the C-terminus side. For the condensation reaction, the condensation reagent and the base used in the linking step described above can be used. For example, various methods are possible as the activator of the carboxyl group, such as the combination of DIC and Oxyma, the combination of DIC and HOAt, the combination of HATU and DIPEA, or via a mixed acid anhydride or an acid halide. Subsequently, after deprotecting the protecting group for the amino group, the protected amino acid having an olefin as a side chain can be elongated. Next, through a metathesis reaction, the intramolecular olefins can be cyclized. The catalyst used in the metathesis reaction is the same as described above. Next, the C-terminal protecting group can be deprotected to produce the peptide compound (4) with the C-terminus unprotected, or the N-terminal protecting group can be deprotected to produce the peptide compound (4) with the N-terminus unprotected.

General production method for peptide compound represented by formula (5) (peptide compound (5))

A general production method for the peptide compound (5) will be shown below. In the following scheme, $Pg_1$ and $Pg_2$ represent protecting groups for an amino group, $Xg_3$ represents an oxygen atom and a protecting group bonded thereto, $R_1$, $R_2$, and $R_3$ represent side chains of an amino acid, and $P_1$ and $P_3$ represent substituents of a nitrogen atom.

The peptide compound (5) can be produced using the following method.

[Formula 28]

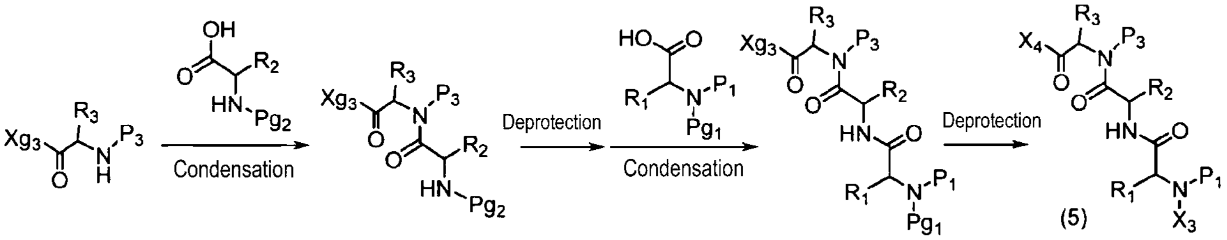


(5)

$X_3$ = H、$X_4$ = $Xg_3$
or
$X_3$ = $Pg_1$、$X_4$ = OH

By condensing a protected amino acid to an amino acid whose C-terminus has been protected, the amino acid can be elongated on the N-terminus side. For the condensation reaction, the condensation reagent and the base used in the linking step above can be used. For example, various methods are possible as the activator of the carboxyl group, such

as the combination of DIC and Oxyma, the combination of DIC and HOAt, the combination of HATU and DIPEA, or via a mixed acid anhydride or an acid halide. Then, after deprotecting the protecting group for the amino group, a protected amino acid can be condensed, thereby synthesizing a fragment composed of 3 amino acids. Next, the C-terminal protecting group can be deprotected to produce the peptide compound (5) with the C-terminus unprotected, or the N-terminal protecting group can be deprotected to produce the peptide compound (5) with the N-terminus unprotected.

The peptide compound (5) can also be produced by solid phase synthesis. In that case, $Xg_3$ in the above formula is an amino acid bonded to the solid phase carrier via an oxygen atom and a linker bonded thereto, to which a protected amino acid can be condensed, thereby elongating the amino acid on the N-terminal side. For the condensation reaction, the condensation reagent and base used in the linking step above can be used. For example, various methods are possible as the activator of the carboxyl group, such as the combination of DIC and Oxyma, the combination of DIC and HOAt, the combination of HATU and DIPEA, or via a mixed acid anhydride or an acid halide. Then, by sequentially performing deprotection of the protecting group for the amino group and condensation of a protected amino acid, a fragment composed of 3 amino acids can be synthesized. Next, by cutting out from the solid phase, the peptide compound (5) with the C-terminal unprotected can be produced.

General production method for peptide compound represented by formula (6) (peptide compound (6))

A general production method for the peptide compound (6) will be shown below. In the following scheme, $Pg_7$, $Pg_8$, $Pg_9$, and $Pg_{10}$ represent protecting groups for an amino group, $Xg_{11}$ represents an oxygen atom and a protecting group bonded thereto, $R_7$, $R_8$, $R_9$, $Q_9$, $R_{10}$, and $R_{11}$ represent side chains of an amino acid, and $P_8$, $P_9$, $P_{10}$, and $P_{11}$ represent substituents of a nitrogen atom.

The peptide compound (6) can be produced using the following method.

[Formula 29]

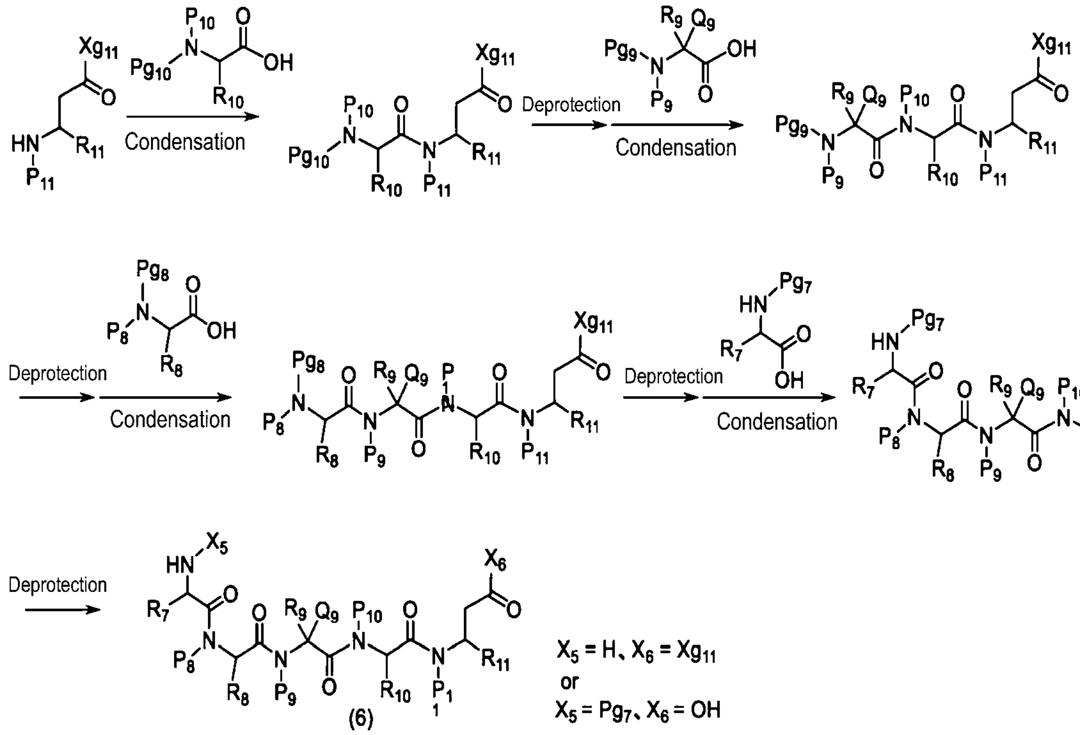

(6)

By condensing a protected amino acid to an amino acid having a β-amino acid skeleton with the carboxy group protected, the amino acid can be elongated on the N-terminus side. For the condensation reaction, the condensation reagent and the base used in the linking step described above can be used. For example, a variety of methods are possible as the activator of the carboxyl group, such as the combination of DIC and Oxyma, the combination of DIC and HOAt, the combination of HATU and DIPEA, or via a mixed acid anhydride or an acid halide. Then, by sequentially performing deprotection of the protecting group for the amino group and condensation of a protected amino acid, a fragment composed of 5 amino acids can be synthesized. Next, the C-terminal protecting group can be deprotected to produce the peptide compound (6) with the C-terminus unprotected, or the N-terminal protecting group can be deprotected to produce

the peptide compound (6) with the N-terminus unprotected.

The peptide compound (6) can also be produced by solid phase synthesis. In that case, $Xg_{11}$ in the above formula is an amino acid having a β-amino acid skeleton bonded to the solid phase carrier via an oxygen atom and a linker bonded thereto, to which a protected amino acid can be condensed, thereby elongating the amino acid on the N-terminal side. For the condensation reaction, the condensation reagent and base used in the linking step described above can be used. For example, a variety of methods are possible as the activator of the carboxyl group, such as the combination of DIC and Oxyma, the combination of DIC and HOAt, the combination of HATU and DIPEA, or via a mixed acid anhydride or an acid halide. Subsequently, by sequentially performing deprotection of the protecting group for the amino group and condensation of a protected amino acid, a fragment composed of 5 amino acids can be synthesized. Next, by cutting out from the solid phase, the peptide compound (6) with the C-terminal unprotected can be produced.

In the production methods for the peptide compounds represented by formulae (4) to (6) shown above, chemical reactions may occur with functional groups other than the target functional group. In such cases, only the desired reaction can proceed by introducing a protecting group to the non-target functional group. For such a protecting group desorption reaction, the method can be used that is described in, for example, "Greene's, 'Protective Groups in Organic Synthesis' (5th ed., John Wiley & Sons 2014)". For conversion reactions of functional groups of the compounds, Comprehensive Organic Transformations: A Guide to Functional Group Preparations (5th ed.) by Larock and March's Advanced Organic Chemistry: Reactions, Mechanisms, and Structure (8th ed.) by Smith can be referenced.

In an aspect, it is possible to use column chromatography in the isolation and/or purification of the cyclic peptide compound, or a salt thereof, or a solvate thereof produced by the method of the present invention, or it is also possible not to use column chromatography.

The cyclic peptide compound, or a salt thereof, or a solvate thereof produced by the method of the present invention can be isolated and/or purified by crystallizing by crystallization. Specifically, for example, the reaction solution after the condensation reaction may be subjected to a separate operation, the organic layer be concentrated and/or filtered as necessary, then a solvent suitable for crystallization be added to the obtained residue, optionally a seed crystal is added, and stirred as necessary to obtain a crystal of the cyclic peptide compound, or a salt thereof, or a solvate thereof. The solvent added during crystallization is not particularly limited as long as it is a solvent with which the cyclic peptide compound can be crystallized, but preferably a solvent with which an operation to reduce the solubility of the cyclic peptide compound can be performed for a solution in which the cyclic peptide compound is dissolved. For example, when a cyclic peptide compound can be crystallized by reducing the solubility of the cyclic peptide compound by addition of a poor solvent or the cooling of a solution, a solvent capable of such operation can be used. In addition, when a crystal of the cyclic peptide compound can be obtained by maintaining a crude crystal of the cyclic peptide compound in a suspension state for any period of time, a solvent capable of such manipulation can be used for crystallization. Specific examples of the solvent added during crystallization include acetone, water, DMSO, acetonitrile, ethanol, and a mixed solvent thereof.

The following is description of each symbol used in the structural formula of the cyclic peptide compound represented by formula (1) and the structural formulae of the peptide compounds represented by formulae (2) to (7).

$R_1$ is a $C_1$-$C_6$ alkyl. $R_1$ is preferably a $C_3$-$C_4$ alkyl, and more preferably n-propyl or 2-methylpropyl.

$P_1$ is a $C_1$-$C_6$ alkyl. $P_1$ is preferably a $C_1$-$C_4$ alkyl, and more preferably methyl.

$R_2$ is a $C_1$-$C_6$ alkyl. $R_2$ is preferably a $C_3$-$C_4$ alkyl, and more preferably 1-methylpropyl.

$R_3$ is hydrogen, or $R_3$ forms a 4- to 7-membered saturated heterocyclic ring together with $P_3$, a carbon atom to which $R_3$ is bonded, and a nitrogen atom to which $P_3$ is bonded. Preferably, $R_3$ is hydrogen, or $R_3$ forms a 5-membered saturated heterocyclic ring together with $P_3$, a carbon atom to which $R_3$ is bonded, and a nitrogen atom to which $P_3$ is bonded. In an aspect, $R_3$ is preferably hydrogen. In an aspect, $R_3$ preferably forms a 5-membered saturated heterocyclic ring together with $P_3$, a carbon atom to which $R_3$ is bonded, and a nitrogen atom to which $P_3$ is bonded.

$P_3$ is a $C_1$-$C_6$ alkyl or a $C_3$-$C_8$ cycloalkyl, or $P_3$ forms a 4- to 7-membered saturated heterocyclic ring together with $R_3$, a carbon atom to which $R_3$ is bonded, and a nitrogen atom to which $P_3$ is bonded. Preferably, $P_3$ is a $C_1$-$C_4$ alkyl, or $P_3$ forms a 5-membered saturated heterocyclic ring together with $R_3$, a carbon atom to which $R_3$ is bonded, and a nitrogen atom to which $P_3$ is bonded. In an aspect, $P_3$ is preferably methyl. In an aspect, $P_3$ preferably forms a 5-membered saturated heterocyclic ring together with $R_3$, a carbon atom to which $R_3$ is bonded, and a nitrogen atom to which $P_3$ is bonded.

$P_4$ is a $C_1$-$C_6$ alkyl. $P_4$ is preferably a $C_1$-$C_4$ alkyl, and more preferably methyl.

$R_5$ is benzyl optionally substituted with one or more groups selected from the group consisting of a $C_1$-$C_6$ alkyl, a $C_1$-$C_6$ haloalkyl, and a $C_3$-$C_8$ cycloalkyl. $R_5$ is preferably benzyl optionally substituted with a $C_1$-$C_4$ haloalkyl, and more preferably 4-trifluoromethylbenzyl.

$P_6$ is a $C_1$-$C_6$ alkyl. $P_6$ is preferably a $C_1$-$C_4$ alkyl, and more preferably methyl.

$R_7$ is phenethyl optionally substituted with one or more groups selected from the group consisting of a halogen, a $C_1$-$C_6$ haloalkyl, and a $C_1$-$C_6$ alkoxy. $R_7$ is preferably phenethyl optionally substituted with one or more groups selected from the group consisting of a halogen, trifluoromethyl, and methoxy, and more preferably 3-methoxy-4-trifluoromethylphenethyl or 3,5-difluoro-4-trifluoromethylphenethyl.

$R_8$ forms a 4- to 7-membered saturated heterocyclic ring together with $P_8$, a carbon atom to which $R_8$ is bonded, and a nitrogen atom to which $P_8$ is bonded, the 4- to 7-membered saturated heterocyclic ring optionally substituted with a $C_1$-$C_6$ alkoxy. $R_8$ preferably forms a 5-membered saturated heterocyclic ring together with $P_8$, a carbon atom to which $R_8$ is bonded, and a nitrogen atom to which $P_8$ is bonded, the 5-membered saturated heterocyclic ring substituted with a $C_1$-$C_4$ alkyl, and more preferably forms a 5-membered saturated heterocyclic ring together with $P_8$, a carbon atom to which $R_8$ is bonded, and a nitrogen atom to which $P_8$ is bonded, the 5-membered saturated heterocyclic ring substituted with ethoxy.

$R_9$ forms a 3- to 8-membered alicyclic ring together with $Q_9$ and a carbon atom to which $R_9$ and $Q_9$ are bonded, the 3- to 8-membered alicyclic ring optionally substituted with one or more $C_1$-$C_6$ alkyls. $R_9$ preferably forms a 4- to 6-membered alicyclic ring together with $Q_9$ and a carbon atom to which $R_9$ and $Q_9$ are bonded. In an aspect, $R_9$ preferably forms a 4-membered alicyclic ring together with $Q_9$ and a carbon atom to which $R_9$ and $Q_9$ are bonded. In an aspect, $R_9$ preferably forms a 5-membered alicyclic ring together with $Q_9$ and a carbon atom to which $R_9$ and $Q_9$ are bonded.

$P_9$ is hydrogen or a $C_1$-$C_6$ alkyl. $P_9$ is preferably hydrogen or a $C_1$-$C_4$ alkyl. In an aspect, $P_9$ is preferably hydrogen. In an aspect, $P_9$ is preferably methyl.

$R_{10}$ is a $C_1$-$C_6$ alkyl or a $C_3$-$C_8$ cycloalkyl. $R_{10}$ is preferably a $C_4$-$C_6$ cycloalkyl, and more preferably cyclopentyl.

$P_{10}$ is a $C_1$-$C_6$ alkyl. $P_{10}$ is preferably a $C_1$-$C_4$ alkyl, and more preferably methyl.

$R_{11}$ is a di-$C_1$-$C_6$ alkylaminocarbonyl or a 4- to 8-membered cyclic aminocarbonyl. $R_{11}$ is preferably a di-$C_1$-$C_4$ alkylaminocarbonyl or a 5- to 6-membered cyclic aminocarbonyl, and more preferably dimethylaminocarbonyl.

$P_{11}$ is a $C_1$-$C_6$ alkyl. $P_{11}$ is preferably a $C_1$-$C_4$ alkyl, and more preferably methyl.

$X_1$, $X_3$, and $X_5$ are each independently hydrogen or a protecting group for an amino group. $X_1$, $X_3$, and $X_5$ are preferably each independently one selected from the group consisting of hydrogen, a carbamate-based protecting group, an acyl-based protecting group, a sulfonamide-based protecting group, and a silyl-based protecting group. In an aspect, the carbamate-based protecting group is one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group. In an aspect, the acyl-based protecting group is one selected from the group consisting of a trifluoroacetyl group, an acetyl group, and a benzoyl group. In an aspect, the sulfonamide-based protecting group is one selected from the group consisting of a 2-nitrobenzenesulfonyl group, a 4-nitrobenzenesulfonyl group, and a 2,4-dinitrobenzenesulfonyl group. In an aspect, the silyl-based protecting group is one selected from the group consisting of a TMS group, a TBDMS group, a TES group, a TIPS group, and a TBDPS group.

In an aspect, $X_1$ is hydrogen or a carbamate-based protecting group. In an aspect, $X_1$ is preferably hydrogen. In an aspect, $X_1$ is preferably a Fmoc group.

In an aspect, $X_3$ is hydrogen or a carbamate-based protecting group. In an aspect, $X_3$ is preferably hydrogen. In an aspect, $X_3$ is preferably a Cbz group.

In an aspect, $X_5$ is hydrogen or a carbamate-based protecting group. In an aspect, $X_5$ is preferably hydrogen. In an aspect, $X_5$ is preferably a Cbz group.

$X_2$, $X_4$, and $X_6$ are each independently a halogen, a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl. $X_2$, $X_4$, and $X_6$ are preferably each independently a halogen, a hydroxy group, an optionally substituted $C_1$-$C_6$ alkoxy, an optionally substituted $C_6$-$C_{10}$ aryloxy, an optionally substituted $C_7$-$C_{14}$ aralkoxy, an optionally substituted 4-to 8-membered cyclic aminooxy, or a group represented by $-OSiR_xR_yR_z$, wherein $R_x$, $R_y$, and $R_z$ are each independently a $C_1$-$C_6$ alkyl or a $C_6$-$C_{10}$ aryl. In an aspect, the halogen is chlorine or bromine. In an aspect, the optionally substituted alkoxy is t-butoxy, methoxy, ethoxy, or isopropoxy. In an aspect, the optionally substituted aryloxy is pentafluorophenyloxy or nitrophenyloxy. In an aspect, the optionally substituted aralkoxy is optionally substituted benzyloxy. In an aspect, the optionally substituted cyclic aminooxy is N-hydroxysucciniminooxy. In an aspect, the group represented by $-OSiR_xR_yR_z$ is trimethylsilyloxy, triethylsilyloxy, triisopropylsilyloxy, triphenylsilyloxy, tri-t-butylsilyloxy, dit-butylisobutylsilyloxy, or tris(triethylsilyl)silyloxy.

In an aspect, $X_2$ is a hydroxy group, t-butoxy, or benzyloxy. In an aspect, $X_2$ is preferably a hydroxy group. In an aspect, $X_2$ is preferably t-butoxy.

In an aspect, $X_4$ is a hydroxy group, t-butoxy, or benzyloxy. In an aspect, $X_4$ is preferably a hydroxy group. In an aspect, $X_4$ is preferably t-butoxy.

In an aspect, $X_6$ is a hydroxy group, t-butoxy, or benzyloxy. In an aspect, $X_6$ is preferably a hydroxy group. In an aspect, $X_6$ is preferably t-butoxy.

In an aspect of the structural formula of the cyclic peptide compound represented by formula (1) and the structural formulae of the peptide compounds represented by formulae (2) and (3),

$R_1$ is a $C_1$-$C_6$ alkyl;
$P_1$ is a $C_1$-$C_6$ alkyl;
$R_2$ is a $C_1$-$C_6$ alkyl;
$R_3$ is hydrogen, or $R_3$ forms a 4- to 7-membered saturated heterocyclic ring together with $P_3$, a carbon atom to which

$R_3$ is bonded, and a nitrogen atom to which $P_3$ is bonded;
$P_3$ is a $C_1$-$C_6$ alkyl or a $C_3$-$C_8$ cycloalkyl, or $P_3$ forms a 4- to 7-membered saturated heterocyclic ring together with $R_3$, a carbon atom to which $R_3$ is bonded, and a nitrogen atom to which $P_3$ is bonded;
$P_4$ is a $C_1$-$C_6$ alkyl;
$R_5$ is benzyl optionally substituted with one or more groups selected from the group consisting of a $C_1$-$C_6$ alkyl, a $C_1$-$C_6$ haloalkyl, and a $C_3$-$C_8$ cycloalkyl;
$P_6$ is a $C_1$-$C_6$ alkyl;
$R_7$ is phenethyl optionally substituted with one or more groups selected from the group consisting of a halogen, a $C_1$-$C_6$ haloalkyl, and a $C_1$-$C_6$ alkoxy;
$R_8$ forms a 4- to 7-membered saturated heterocyclic ring together with $P_8$, a carbon atom to which $R_8$ is bonded, and a nitrogen atom to which $P_8$ is bonded, the 4- to 7-membered saturated heterocyclic ring optionally substituted with a $C_1$-$C_6$ alkoxy;
$R_9$ forms a 3- to 8-membered alicyclic ring together with $Q_9$ and a carbon atom to which $R_9$ and $Q_9$ are bonded, the 3- to 8-membered alicyclic ring optionally substituted with one or more $C_1$-$C_6$ alkyls;
$P_9$ is hydrogen or a $C_1$-$C_6$ alkyl;
$R_{10}$ is a $C_1$-$C_6$ alkyl or a $C_3$-$C_8$ cycloalkyl;
$P_{10}$ is a $C_1$-$C_6$ alkyl;
$R_{11}$ is a di-$C_1$-$C_6$ alkylaminocarbonyl or a 4- to 8-membered cyclic aminocarbonyl;
$P_{11}$ is a $C_1$-$C_6$ alkyl;
$X_1$ and $X_5$ are each independently hydrogen or a protecting group for an amino group; and
$X_2$ and $X_4$ are each independently a halogen, a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

In an aspect of the structural formula of the cyclic peptide compound represented by formula (1) and the structural formulae of the peptide compounds represented by formulae (2) and (3), preferably,

$R_1$ is a $C_3$-$C_4$ alkyl;
$P_1$ is a $C_1$-$C_4$ alkyl;
$R_2$ is a $C_3$-$C_4$ alkyl;
$R_3$ is hydrogen, or $R_3$ forms a 5-membered saturated heterocyclic ring together with $P_3$, a carbon atom to which $R_3$ is bonded, and a nitrogen atom to which $P_3$ is bonded;
$P_3$ is a $C_1$-$C_4$ alkyl, or $P_3$ forms a 5-membered saturated heterocyclic ring together with $R_3$, a carbon atom to which $R_3$ is bonded, and a nitrogen atom to which $P_3$ is bonded;
$P_4$ is a $C_1$-$C_4$ alkyl;
$R_5$ is benzyl optionally substituted with a $C_1$-$C_4$ haloalkyl;
$P_6$ is a $C_1$-$C_4$ alkyl;
$R_7$ is phenethyl optionally substituted with one or more groups selected from the group consisting of a halogen, trifluoromethyl, and methoxy;
$R_8$ forms a 5-membered saturated heterocyclic ring together with $P_8$, a carbon atom to which $R_8$ is bonded, and a nitrogen atom to which $P_8$ is bonded, the 5-membered saturated heterocyclic ring substituted with a $C_1$-$C_4$ alkyl;
$R_9$ forms a 4- to 6-membered alicyclic ring together with $Q_9$ and a carbon atom to which $R_9$ and $Q_9$ are bonded;
$P_9$ is hydrogen or a $C_1$-$C_4$ alkyl;
$R_{10}$ is a $C_4$-$C_6$ cycloalkyl;
$P_{10}$ is a $C_1$-$C_4$ alkyl;
$R_{11}$ is a di-$C_1$-$C_4$ alkylaminocarbonyl or a 5- to 6-membered cyclic aminocarbonyl;
$P_{11}$ is a $C_1$-$C_4$ alkyl;
$X_1$ and $X_5$ are each independently one selected from the group consisting of hydrogen, a carbamate-based protecting group, an acyl-based protecting group, a sulfonamide-based protecting group, and a silyl-based protecting group; and
$X_2$ and $X_4$ are each independently a halogen, a hydroxy group, an optionally substituted $C_1$-$C_6$ alkoxy, an optionally substituted $C_6$-$C_{10}$ aryloxy, an optionally substituted $C_7$-$C_{14}$ aralkoxy, an optionally substituted 4- to 8-membered cyclic aminooxy, or a group represented by $-OSiR_xR_yR_z$, wherein $R_x$, $R_y$, and $R_z$ are each independently a $C_1$-$C_6$ alkyl or a $C_6$-$C_{10}$ aryl.

In an aspect of the structural formula of the cyclic peptide compound represented by formula (1) and the structural formulae of the peptide compounds represented by formulae (2) and (3), more preferably,

$R_1$ is n-propyl or 2-methylpropyl;

$P_1$ is methyl;
$R_2$ is 1-methylpropyl;
$R_3$ is hydrogen, or $R_3$ forms a 5-membered saturated heterocyclic ring together with $P_3$, a carbon atom to which $R_3$ is bonded, and a nitrogen atom to which $P_3$ is bonded;
$P_3$ is methyl, or $P_3$ forms a 5-membered saturated heterocyclic ring together with $R_3$, a carbon atom to which $R_3$ is bonded, and a nitrogen atom to which $P_3$ is bonded;
$P_4$ is methyl;
$R_5$ is 4-trifluoromethylbenzyl;
$P_6$ is methyl;
$R_7$ is 3-methoxy-4-trifluoromethylphenethyl or 3,5-difluoro-4-trifluoromethylphenethyl;
$R_8$ forms a 5-membered saturated heterocyclic ring together with $P_8$, a carbon atom to which $R_8$ is bonded, and a nitrogen atom to which $P_8$ is bonded, the 5-membered saturated heterocyclic ring optionally substituted with ethoxy;
$R_9$ forms a 4-membered alicyclic ring or 5-membered alicyclic ring together with $Q_9$ and a carbon atom to which $R_9$ and $Q_9$ are bonded;
$P_9$ is hydrogen or methyl;
$R_{10}$ is cyclopentyl;
$P_{10}$ is methyl;
$R_{11}$ is dimethylaminocarbonyl;
$P_{11}$ is methyl;
$X_1$ is hydrogen or a carbamate-based protecting group;
$X_5$ is hydrogen or a carbamate-based protecting group;
$X_2$ is a hydroxy group, t-butoxy, or benzyloxy; and
$X_4$ is a hydroxy group, t-butoxy, or benzyloxy.

In an aspect of the structural formulae of the peptide compounds represented by formulae (4) to (6),

$R_1$ is a $C_1$-$C_6$ alkyl;
$P_1$ is a $C_1$-$C_6$ alkyl;
$R_2$ is a $C_1$-$C_6$ alkyl;
$R_3$ is hydrogen, or $R_3$ forms a 4- to 7-membered saturated heterocyclic ring together with $P_3$, a carbon atom to which $R_3$ is bonded, and a nitrogen atom to which $P_3$ is bonded;
$P_3$ is a $C_1$-$C_6$ alkyl or a $C_3$-$C_8$ cycloalkyl, or $P_3$ forms a 4- to 7-membered saturated heterocyclic ring together with $R_3$, a carbon atom to which $R_3$ is bonded, and a nitrogen atom to which $P_3$ is bonded;
$P_4$ is a $C_1$-$C_6$ alkyl;
$R_5$ is benzyl optionally substituted with one or more groups selected from the group consisting of a $C_1$-$C_6$ alkyl, a $C_1$-$C_6$ haloalkyl, and a $C_3$-$C_8$ cycloalkyl;
$P_6$ is a $C_1$-$C_6$ alkyl;
$R_7$ is phenethyl optionally substituted with one or more groups selected from the group consisting of a halogen, a $C_1$-$C_6$ haloalkyl, and a $C_1$-$C_6$ alkoxy;
$R_8$ forms a 4- to 7-membered saturated heterocyclic ring together with $P_8$, a carbon atom to which $R_8$ is bonded, and a nitrogen atom to which $P_8$ is bonded, the 4- to 7-membered saturated heterocyclic ring optionally substituted with a $C_1$-$C_6$ alkoxy;
$R_9$ forms a 3- to 8-membered alicyclic ring together with $Q_9$ and a carbon atom to which $R_9$ and $Q_9$ are bonded, the 3- to 8-membered alicyclic ring optionally substituted with one or more $C_1$-$C_6$ alkyls;
$P_9$ is hydrogen or a $C_1$-$C_6$ alkyl;
$R_{10}$ is a $C_1$-$C_6$ alkyl or a $C_3$-$C_8$ cycloalkyl;
$P_{10}$ is a $C_1$-$C_6$ alkyl;
$R_{11}$ is a di-$C_1$-$C_6$ alkylaminocarbonyl or a 4- to 8-membered cyclic aminocarbonyl;
$P_{11}$ is a $C_1$-$C_6$ alkyl;
$X_1$, $X_3$, and $X_5$ are each independently hydrogen or a protecting group for an amino group; and
$X_2$, $X_4$, and $X_6$ are each independently a halogen, a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

In an aspect of the structural formulae of the peptide compounds represented by formulae (4) to (6), preferably,

$R_1$ is a $C_3$-$C_4$ alkyl;
$P_1$ is a $C_1$-$C_4$ alkyl;

$R_2$ is a $C_3$-$C_4$ alkyl;
$R_3$ is hydrogen, or $R_3$ forms a 5-membered saturated heterocyclic ring together with $P_3$, a carbon atom to which $R_3$ is bonded, and a nitrogen atom to which $P_3$ is bonded;
$P_3$ is a $C_1$-$C_4$ alkyl, or $P_3$ forms a 5-membered saturated heterocyclic ring together with $R_3$, a carbon atom to which $R_3$ is bonded, and a nitrogen atom to which $P_3$ is bonded;
$P_4$ is a $C_1$-$C_4$ alkyl;
$R_5$ is benzyl optionally substituted with a $C_1$-$C_4$ haloalkyl;
$P_6$ is a $C_1$-$C_4$ alkyl;
$R_7$ is phenethyl optionally substituted with one or more groups selected from the group consisting of a halogen, trifluoromethyl, and methoxy;
$R_8$ forms a 5-membered saturated heterocyclic ring together with $P_8$, a carbon atom to which $R_8$ is bonded, and a nitrogen atom to which $P_8$ is bonded, the 5-membered saturated heterocyclic ring substituted with a $C_1$-$C_4$ alkyl;
$R_9$ forms a 4- to 6-membered alicyclic ring together with $Q_9$ and a carbon atom to which $R_9$ and $Q_9$ are bonded;
$P_9$ is hydrogen or a $C_1$-$C_4$ alkyl;
$R_{10}$ is a $C_4$-$C_6$ cycloalkyl;
$P_{10}$ is a $C_1$-$C_4$ alkyl;
$R_{11}$ is a di-$C_1$-$C_4$ alkylaminocarbonyl or a 5- to 6-membered cyclic aminocarbonyl;
$P_{11}$ is a $C_1$-$C_4$ alkyl;
$X_1$, $X_3$, and $X_5$ are each independently one selected from the group consisting of hydrogen, a carbamate-based protecting group, an acyl-based protecting group, a sulfonamide-based protecting group, and a silyl-based protecting group; and
$X_2$, $X_4$, and $X_6$ are each independently a halogen, a hydroxy group, an optionally substituted $C_1$-$C_6$ alkoxy, an optionally substituted $C_6$-$C_{10}$ aryloxy, an optionally substituted $C_7$-$C_{14}$ aralkoxy, an optionally substituted 4- to 8-membered cyclic aminooxy, or a group represented by $-OSiR_xR_yR_z$, wherein $R_x$, $R_y$, and $R_z$ are each independently a $C_1$-$C_6$ alkyl or a $C_6$-$C_{10}$ aryl.

In an aspect of the structural formulae of the peptide compounds represented by formulae (4) to (6), more preferably,

$R_1$ is n-propyl or 2-methylpropyl;
$P_1$ is methyl;
$R_2$ is 1-methylpropyl;
$R_3$ is hydrogen, or $R_3$ forms a 5-membered saturated heterocyclic ring together with $P_3$, a carbon atom to which $R_3$ is bonded, and a nitrogen atom to which $P_3$ is bonded;
$P_3$ is methyl, or $P_3$ forms a 5-membered saturated heterocyclic ring together with $R_3$, a carbon atom to which $R_3$ is bonded, and a nitrogen atom to which $P_3$ is bonded;
$P_4$ is methyl;
$R_5$ is 4-trifluoromethylbenzyl;
$P_6$ is methyl;
$R_7$ is 3-methoxy-4-trifluoromethylphenethyl or 3,5-difluoro-4-trifluoromethylphenethyl;
$R_8$ forms a 5-membered saturated heterocyclic ring together with $P_8$, a carbon atom to which $R_8$ is bonded, and a nitrogen atom to which $P_8$ is bonded, the 5-membered saturated heterocyclic ring optionally substituted with ethoxy;
$R_9$ forms a 4-membered alicyclic ring or 5-membered alicyclic ring together with $Q_9$ and a carbon atom to which $R_9$ and $Q_9$ are bonded;
$P_9$ is hydrogen or methyl;
$R_{10}$ is cyclopentyl;
$P_{19}$ is methyl;
$R_{11}$ is dimethylaminocarbonyl;
$P_{11}$ is methyl;
$X_1$ is hydrogen or a carbamate-based protecting group;
$X_3$ is hydrogen or a carbamate-based protecting group;
$X_5$ is hydrogen or a carbamate-based protecting group;
$X_2$ is a hydroxy group, t-butoxy, or benzyloxy;
$X_4$ is a hydroxy group, t-butoxy, or benzyloxy; and
$X_6$ is a hydroxy group, t-butoxy, or benzyloxy.

In an aspect, the cyclic peptide compound, or a salt thereof, or a solvate thereof produced by the method of the present invention is preferably a solvate, and more preferably a hydrate.

In an aspect, the cyclic peptide compound produced by the method of the present invention is a cyclic peptide compound represented by the following formula (1a):

[Formula 30]

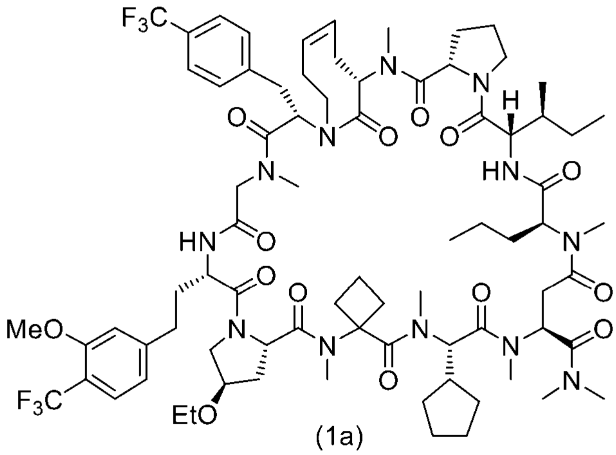

(1a)

or a salt thereof, or a solvate thereof.

In an aspect, the present invention relates to a method for producing a cyclic peptide compound represented by formula (1a), or a salt thereof, or a solvate thereof. The method includes a step of reacting an N-terminal amino acid residue of a peptide compound represented by formula (2a) or (3a) with a C-terminal amino acid residue of the peptide compound in a solvent for cyclization (cyclization step) (hereinafter, also referred to as "aspect 1‴").

[Formula 31]

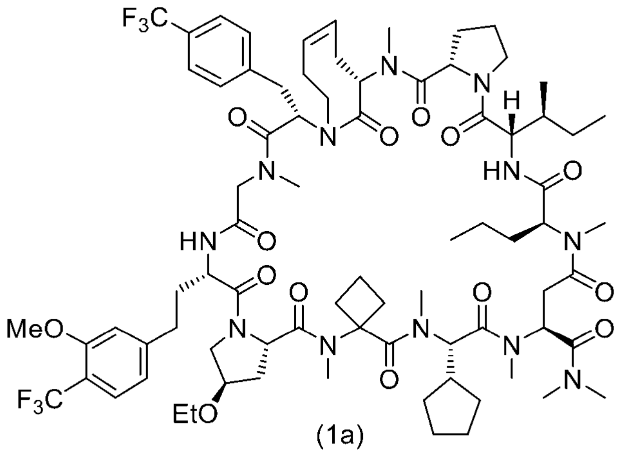

(1a)

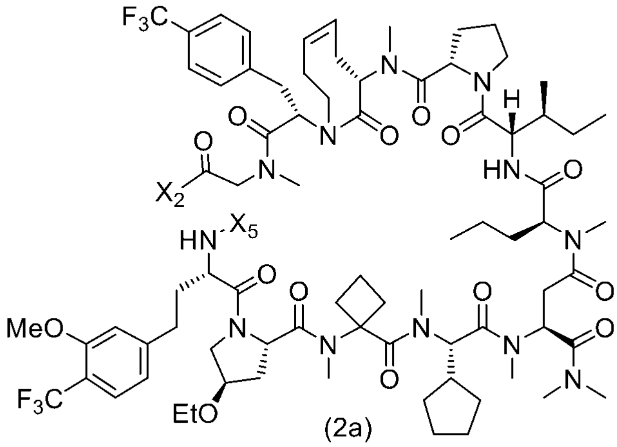

(2a)

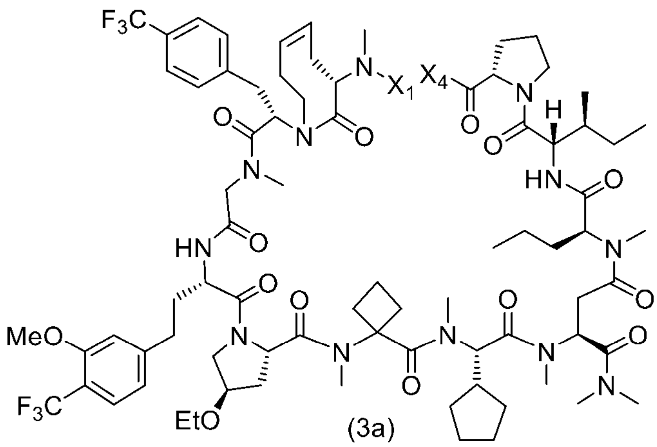

(3a)

In an aspect, the present invention relates to a method for producing a cyclic peptide compound represented by formula (1a), or a salt thereof, or a solvate thereof. The method includes

(a) a step of providing peptide compounds represented by formulae (4a) to (6a), or salts thereof, or solvates of the peptide compounds or salts;

(b) a step of reacting N-terminal amino acid residues of the peptide compounds represented by formulae (4a) to (6a) with C-terminal amino acid residues of the peptide compounds for linkage (linking step); and

(c) a step of reacting an N-terminal amino acid residue of a peptide compound obtained in the step (b) with a C-terminal amino acid residue of the peptide compound for cyclization (cyclization step) (hereinafter, also referred to as "aspect 2‴").

[Formula 32]

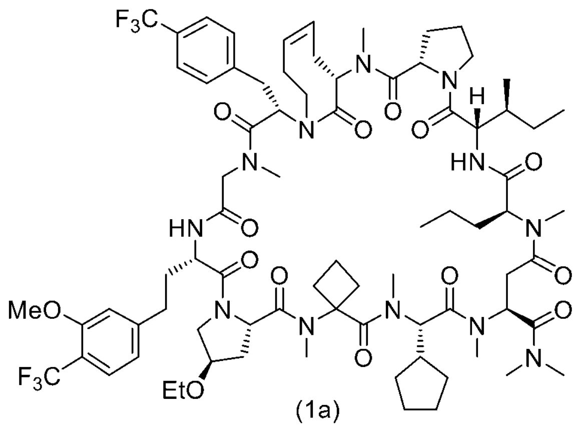

(1a)

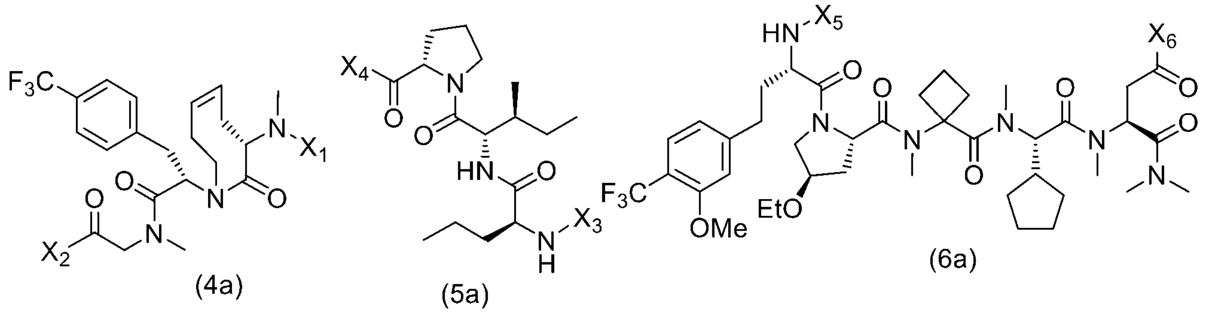

(4a) (5a) (6a)

In aspect 2', the step (b) may include (b-1) a step of linking an N-terminal amino acid residue of the peptide compound represented by formula (5a) with a C-terminal amino acid residue of the peptide compound represented by formula (6a), thereby converting them into a peptide compound represented by formula (7a).

[Formula 33]

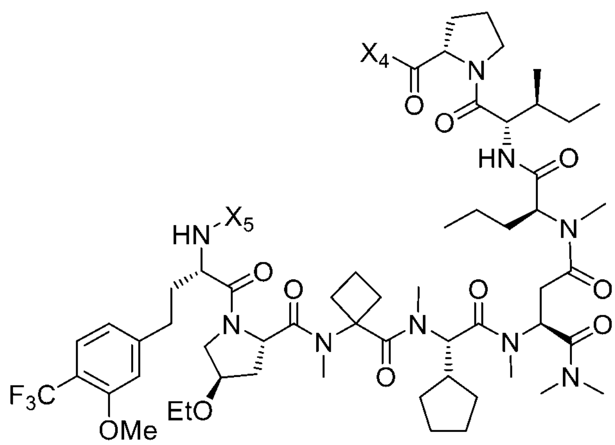

(7a)

In aspect 2', the step (b) may include, in addition to the step (b-1),

(b-2) a step of linking an N-terminal amino acid residue of the peptide compound represented by formula (4a) with a C-terminal amino acid residue of the peptide compound represented by formula (7a), thereby converting them into a peptide compound represented by formula (2a) (linking step), and
the step (c) may include
(c-1) a step of reacting an N-terminal amino acid residue of the peptide compound represented by formula (2a) with a

C-terminal amino acid residue of the peptide compound in a solvent for cyclization (cyclization step).

In aspect 2', the step (b) may include, in addition to the step (b-1),

(b-3) a step of linking an N-terminal amino acid residue of the peptide compound represented by formula (7a) with a C-terminal amino acid residue of the peptide compound represented by formula (4a), thereby converting them into a peptide compound represented by formula (3a) (linking step), and
the step (c) may include
(c-2) a step of reacting an N-terminal amino acid residue of the peptide compound represented by formula (3a) with a C-terminal amino acid residue of the peptide compound in a solvent for cyclization (cyclization step).

In aspects 1' and 2', the linkage of an N-terminal amino acid residue of a peptide compound with a C-terminal amino acid residue of a peptide compound is preferably linkage of an amino group of the N-terminal amino acid residue with a carboxyl group of the C-terminal amino acid residue, and the linkage of an N-terminal amino acid residue of a peptide compound with a C-terminal amino acid residue of a peptide compound is more preferably linkage by means of an amide bond of an amino group of the N-terminal amino acid residue with a carboxyl group of the C-terminal amino acid residue.
The solvent in the cyclization step, the condensation reagent in the cyclization step, the base in the cyclization step, the by-products generated in the cyclization step, the solvent in the linking step, the condensation reagent in the linking step, the base in the linking step, and the like in aspects 1' and 2' are the same as those described in the above aspects 1 and 2.
Each symbol used in the structural formula of the cyclic peptide compound represented by formula (1a) and the structural formulae of the peptide compounds represented by formulae (2a) to (7a) in aspects 1' and 2' is the same as those described in the above aspects 1 and 2.

Peptide compound

In an aspect, the present invention relates to a compound represented by formula (4a).

[Formula 34]

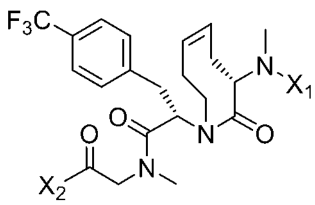

(4a)

In formula (4a), $X_1$ is hydrogen or a protecting group for an amino group. Here, the protecting group in $X_1$ is the same as those described in the above aspects 1 and 2. In an aspect, $X_1$ is preferably hydrogen. In an aspect, $X_1$ is preferably a Fmoc group.
In formula (4a), $X_2$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl. Here, the optionally substituted alkoxy, the optionally substituted aryloxy, the optionally substituted aralkoxy, the optionally substituted cyclic aminooxy, and $-OSiR_xR_yR_z$ in $X_2$ are the same as those described in the above aspects 1 and 2. In an aspect, $X_2$ is preferably t-butoxy.
In an aspect, the compound represented by formula (4a) is preferably tert-butyl 2-[methyl-[(2S)-2-[(4Z,7S)-7-(methylamino)-8-oxo-2,3,6,7-tetrahydroazocin-1-yl]-3-[4-(trifluoromethyl)phenyl]propanoyl]amino]acetate (compound 9).
In an aspect, the compound represented by formula (4a) (peptide compound (4a)) can be produced using the following method.

[Formula 35]

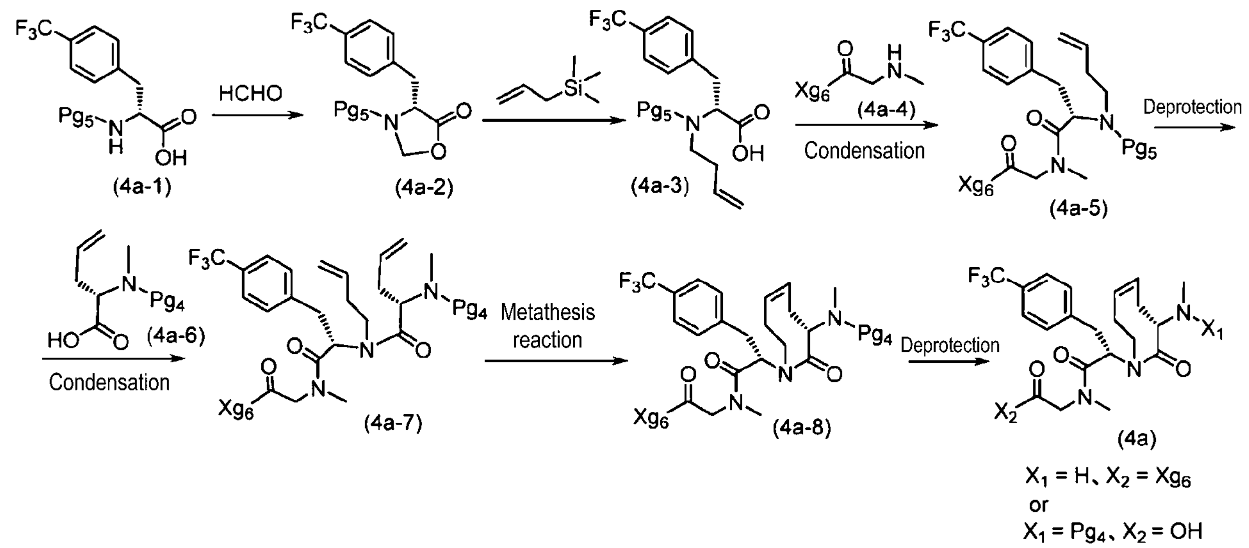

(4a-1) (4a-2) (4a-3) (4a-5) (4a-7) (4a-8) (4a)

By allowing an aldehyde to act on a protected amino acid (4a-1) in accordance with the method of Freidinger et al. (J. Org. Chem., 1983, 48(1), 77-81), an oxazolidinone form with a cyclic protecting group introduced (4a-2) can be obtained. Next, by performing a ring-opening reaction using a silicon compound having an olefin in accordance with the method of Nguyen et al. (Synthesis, 2009, 12, 1991), a compound with an alkyl group having an olefin introduced onto the nitrogen atom (4a-3) can be obtained. Next, by condensing an amino acid whose C-terminus has been protected (4a-4), the amino acid can be elongated on the C-terminus side. For the condensation reaction, the condensation reagent and the base used in the linking step above can be used. For example, a variety of methods are possible as the activator of the carboxyl group, such as the combination of DIC and Oxyma, the combination of DIC and HOAt, the combination of HATU and DIPEA, or via a mixed acid anhydride or an acid halide. Then, after deprotecting the protecting group for the amino group, the protected amino acid having an olefin as a side chain (4a-6) can then be elongated. Next, through a metathesis reaction, the intramolecular olefins can be cyclized. In the metathesis reaction, dichloro(2-isopropoxybenzylidene)(tricyclohexylphosphine)ruthenium(II): CAS No. 203714-71-0, dichloro(3-phenyl-1H-inden-1-ylidene)bis(tricyclohexylphosphine)ruthenium(II): CAS No. 250220-36-1, dichloro(benzylidene)bis(tricyclohexylphosphine)ruthenium(II): CAS No. 172222-30-9, [1,3-bis(2,4,6-trimethylphenyl)-2-imidazolidinylidene]dichloro(3-phenyl-1H-inden-1-ylidene)(tricyclohexylphosphine)ruthenium(II): CAS No. 536724-67-1, [1,3-bis(2,4,6-trimethylphenyl)-2-imidazolidinylidene]dichloro(benzylidene)(tricyclohexylphosphine)ruthenium(II): CAS No. 246047-72-3, [1,3-bis-(2,4,6-trimethylphenyl)-2-imidazolidinylidene]dichloro(2-isopropoxybenzylidene)ruthenium(II): CAS No. 301224-40-8, [1,3-bis-(2-tolyl)-2-imidazolidinylidene]dichloro(2-isopropoxybenzylidene)ruthenium(II): CAS No. 927429-61-6, [1,3-bis(2,4,6-trimethylphenyl)-2-imidazolidinylidene]dichloro[(2-isopropoxy)(5-trifluoroacetamido)benzylidene ruthenium(II): CAS No. 1025728-56-6, [1,3-bis(2,6-diisopropylphenyl)-2-imidazolidinylidene]dichloro[5-(isobutoxycarbonylamido)-2-isopropoxybenzylidene]ruthenium(II): CAS No. 1212009-05-6, or the like can be used as the catalyst. Next, the C-terminal protecting group can be deprotected to produce the peptide compound (4a) with the C-terminus unprotected, or the N-terminal protecting group can be deprotected to produce the peptide compound (4a) with the N-terminus unprotected.

The peptide compound (4a) can also be produced using the following method.

[Formula 36]

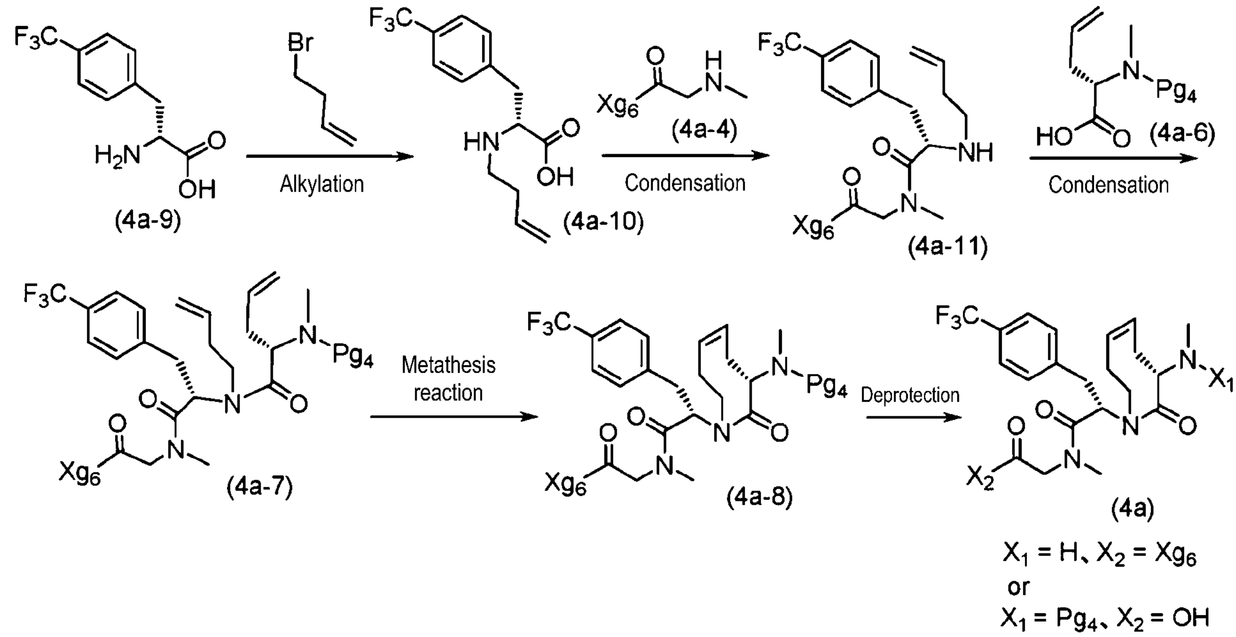


By allowing an alkylating agent having an olefin to act on an amino acid (4a-9) in the presence of a base, an amino acid with an alkyl group having an olefin introduced onto the N atom (4a-10) can be obtained. Then, by condensing an amino acid whose C-terminus has been protected (4a-4), the amino acid can be elongated on the C-terminus side. For the condensation reaction, the condensation reagent and base used in the linking step above can be used. For example, a variety of methods are possible as the activator of the carboxyl group, such as the combination of DIC and Oxyma, the combination of DIC and HOAt, the combination of HATU and DIPEA, or via a mixed acid anhydride or an acid halide. Then, after deprotecting the protecting group for the amino group, the protected amino acid having an olefin as a side chain can then be elongated. Next, through a metathesis reaction, the intramolecular olefins can be cyclized. The catalyst used in the metathesis reaction is the same as described above. Next, the C-terminal protecting group can be deprotected to produce the peptide compound (4a) with the C-terminus unprotected, or the N-terminal protecting group can be deprotected to produce the peptide compound (4a) with the N-terminus unprotected.

In an aspect, the present invention relates to a compound represented by formula (5a).

[Formula 37]

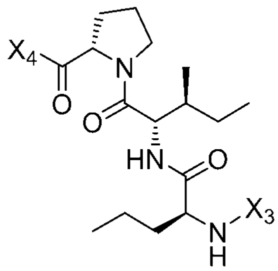

(5a)

In formula (5a), $X_3$ is hydrogen or a protecting group for an amino group. Here, the protecting group for an amino group in $X_3$ is the same as those described in the above aspects 1 and 2. In an aspect, $X_3$ is preferably hydrogen. In an aspect, $X_3$ is preferably a Fmoc group or a Cbz group.

In formula (5a), $X_4$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl. Here, the optionally substituted alkoxy, the optionally substituted aryloxy, the optionally substituted aralkoxy, the optionally substituted cyclic aminooxy, and $-OSiR_xR_yR_z$ in $X_2$ are the same as those described in the above aspects 1 and 2. In an aspect, $X_4$ is preferably t-butoxy.

In an aspect, the compound represented by formula (5a) is preferably tert-butyl (2S)-1-[(2S,3S)-3-methyl-2-[[(2S)-2-(methylamino)pentanoyl]amino]pentanoyl]pyrrolidine-2-acetate (compound 13).

In an aspect, the compound represented by formula (5a) (peptide compound (5a)) can be produced using the following method.

[Formula 38]

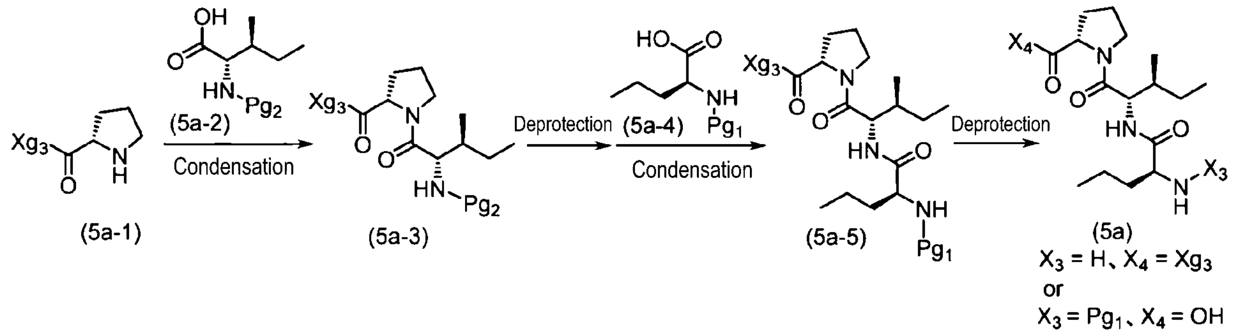


By condensing a protected amino acid (5a-2) to proline whose C-terminus has been protected (5a-1), a compound (5a-3) can be obtained. For the condensation reaction, the condensation reagent and base used in the linking step described above can be used, and for example, a variety of methods are possible as the activator of the carboxyl group, such as the combination of DIC and Oxyma, the combination of DIC and HOAt, the combination of HATU and DIPEA, or via a mixed acid anhydride or an acid halide. Subsequently, after deprotecting the protecting group for the nitrogen atom, a protected amino acid (5a-4) can be condensed, thereby synthesizing a fragment composed of 3 amino acids (5a-5). Next, the C-terminal protecting group can be deprotected to produce the peptide compound (5a) with the C-terminus unprotected, or the N-terminal protecting group can be deprotected to produce the peptide compound (5a) with the N-terminus unprotected.

In an aspect, the present invention relates to a compound represented by formula (6a).

[Formula 39]

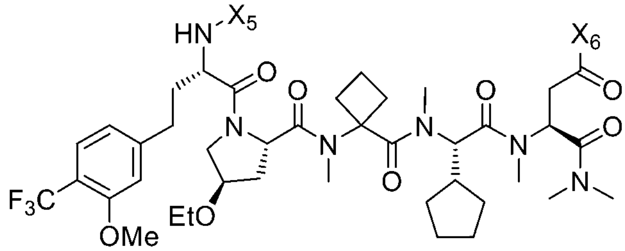

(6a)

In formula (6a), $X_5$ is hydrogen or a protecting group for an amino group. Here, the protecting group for an amino group in $X_5$ is the same as those described in the above aspects 1 and 2. In an aspect, $X_5$ is preferably hydrogen. In an aspect, $X_5$ is preferably a Cbz group.

In formula (6a), $X_6$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl. Here, the optionally substituted alkoxy, the optionally substituted aryloxy, the optionally substituted aralkoxy, the optionally substituted cyclic aminooxy, and $-OSiR_xR_yR_z$ in $X_6$ are the same as those described in the above aspects 1 and 2. In an aspect, $X_6$ is preferably t-butoxy.

In an aspect, the compound represented by formula (6a) is preferably (3S)-3-[[(2S)-2-[[1-[[(2S,4R)-1-[(2S)-2-(benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butyric acid (compound 20).

In an aspect, the compound represented by formula (6a) (peptide compound (6a)) can be produced using the following method.

[Formula 40]

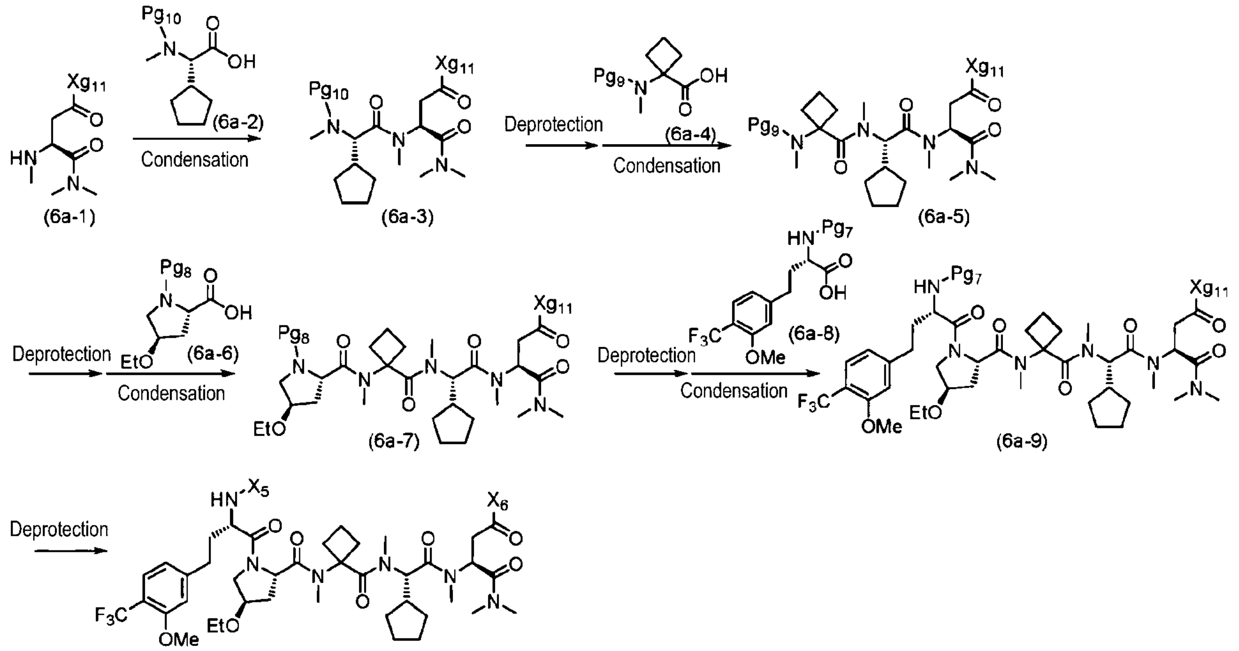


(6a)
$X_5$ = H、$X_6$ = Xg-1
or
$X_5$ = $Pg_7$、$X_6$ = OH

By condensing a protected amino acid (6a-2) to an amino acid having a β-amino acid skeleton with the carboxy group protected (6a-1), a compound (6a-3) can be obtained. For the condensation reaction, the condensation reagent and the base used in the linking step described above can be used, and for example, a variety of methods are possible as the activator of the carboxyl group, such as the combination of DIC and Oxyma, the combination of DIC and HOAt, the combination of HATU and DIPEA, or via a mixed acid anhydride or an acid halide. Subsequently, by sequentially performing deprotection of the protecting group for the amino group and condensation of a protected amino acid, a fragment composed of 5 amino acids (6a-9) can be synthesized. Next, the C-terminal protecting group can be deprotected to produce the peptide compound (6a) with the C-terminus unprotected, or the N-terminal protecting group can be deprotected to produce the peptide compound (6a) with the N-terminus unprotected.

In an aspect, the present invention relates to a compound represented by formula (7a).

[Formula 41]

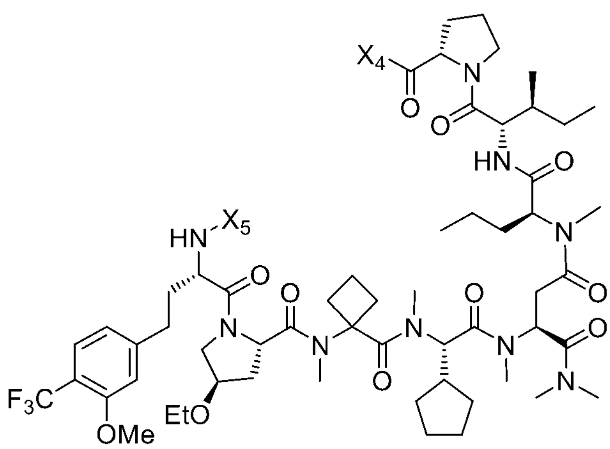

(7a)

In formula (7a), $X_5$ is hydrogen or a protecting group for an amino group. Here, the protecting group for an amino group in $X_5$ is the same as those described in the above aspects 1 and 2. In an aspect, $X_5$ is preferably hydrogen. In an

aspect, $X_5$ is preferably a Cbz group.

In formula (7a), $X_4$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl. Here, the optionally substituted alkoxy, the optionally substituted aryloxy, the optionally substituted aralkoxy, the optionally substituted cyclic aminooxy, and $-OSiR_xR_yR_z$ in $X_4$ are the same as those described in the above aspects 1 and 2. In an aspect, $X_4$ is preferably a hydroxy group or t-butoxy.

In an aspect, the compound represented by formula (7a) is preferably (2S)-1-[(2S,3S)-2-[[(2S)-2-[[(3S)-3-[[(2S)-2-[[1-[[(2S,4R)-1-[(2S)-2-(benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentylacetyl]-methyl-amino]-4-(dimethylamino)-4-oxo-butanoyl]-methyl-amino]pentanoyl]amino]-3-methyl-pentanoyl] pyrrolidine-2-carboxylic acid (compound 22). Also, in an aspect, the compound represented by formula (7a) is preferably tert-butyl (2S)-1-[(2S,3S)-2-[[(2S)-2-[[(3S)-3-[[(2S)-2-[[1-[[(2S,4R)-1-[(2S)-2-amino-4-[3-methoxy-4-(trifluoromethyl) phenyl]butanoyl]-4-ethoxy-pyrrolidine-2-carbonyl]-methyl-amino]cyclobutanecarbonyl]-methyl-amino]-2-cyclopentylacetyl]-methyl-amino]-4-(dimethylamino)-4-oxo-butanoyl]-methyl-amino]pentanoyl]amino]-3-methyl-pentanoyl]pyrrolidine-2-carboxylate (compound 37).

In an aspect, the compound represented by formula (7a) can be produced by reacting an N-terminal amino acid residue of the peptide compound represented by formula (5a) with a C-terminal amino acid residue of the peptide compound represented by formula (6a) in a solvent for linkage (linking step). The solvent in the linking step, the condensation reagent in the linking step, the base in the linking step, and the like are the same as those described in the above aspects 1 and 2.

In an aspect, the present invention relates to a compound represented by formula (2a).

[Formula 42]

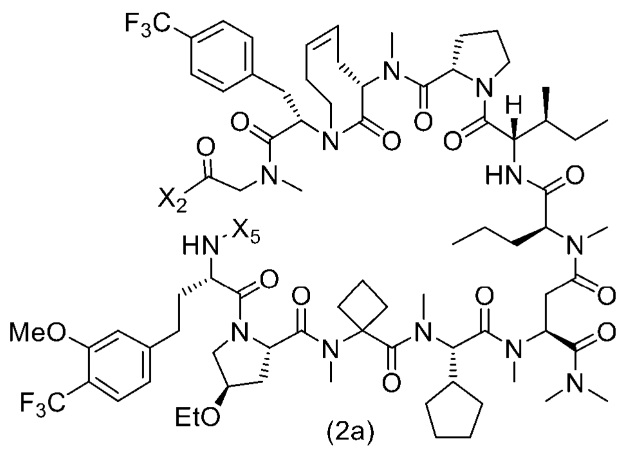

(2a)

In formula (2a), $X_5$ is hydrogen or a protecting group for an amino group. Here, the protecting group for an amino group in $X_5$ is the same as those described in the above aspects 1 and 2. In an aspect, $X_5$ is preferably hydrogen. In an aspect, $X_5$ is preferably a Cbz group.

In formula (2a), $X_2$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl. Here, the optionally substituted alkoxy, the optionally substituted aryloxy, the optionally substituted aralkoxy, the optionally substituted cyclic aminooxy and $-OSiR_xR_yR_z$ in $X_2$ are the same as those described in the above aspects 1 and 2. In an aspect, $X_2$ is preferably a hydroxy group or t-butoxy.

In an aspect, the compound represented by formula (2a) is preferably 2-[[(2S)-2-[(4Z,7S)-7-[[(2S)-1-[(2S,3S)-2-[[(2S)-2-[[(3S)-3-[[(2S)-2-[[1-[[(2S,4R)-1-[(2S)-2-amino-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoyl]-methyl-amino]pentanoyl]amino]-3-methyl-pentanoyl]pyrrolidine-2-carbonyl]-methyl-amino]-8-oxo-2,3,6,7-tetrahydroazocin-1-yl]-3-[4-(trifluoromethyl)phenyl]propanoyl]-methyl-amino]acetic acid (compound 24).

In an aspect, the compound represented by formula (2a) can be produced by reacting an N-terminal amino acid residue of the peptide compound represented by formula (4a) with a C-terminal amino acid residue of the peptide compound represented by formula (7a) in a solvent for linkage (linking step). The solvent in the linking step, the condensation reagent in the linking step, the base in the linking step, and the like are the same as those described in the above aspects 1 and 2.

In an aspect, the present invention relates to a compound represented by formula (3a).

[Formula 43]

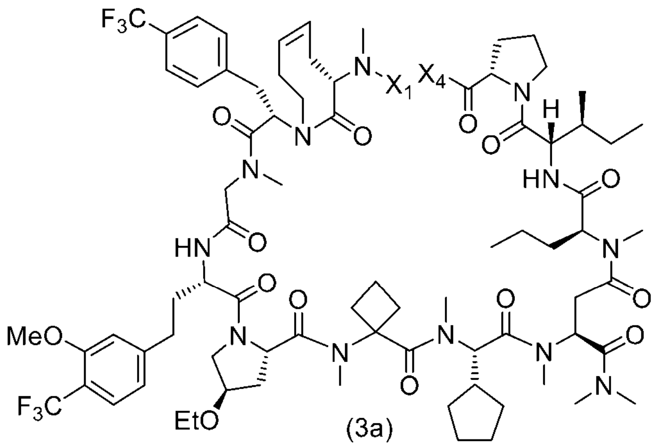

(3a)

In formula (3a), $X_1$ is hydrogen or a protecting group for an amino group. Here, the protecting group for an amino group in $X_1$ is the same as those described in the above aspects 1 and 2. In an aspect, $X_1$ is preferably hydrogen. In an aspect, $X_1$ is preferably a Fmoc group.

In formula (3a), $X_4$ is a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl. Here, the optionally substituted alkoxy, the optionally substituted aryloxy, the optionally substituted aralkoxy, the optionally substituted cyclic aminooxy and $-OSiR_xR_yR_z$ in $X_4$ are the same as those described in the above aspects 1 and 2. In an aspect, $X_4$ is preferably a hydroxy group or t-butoxy.

In an aspect, the compound represented by formula (3a) is preferably (S)-2-[(S)-3-[(S)-2-cyclopentyl-2-[1-[(2S,4R)-4-ethoxy-1-[(S)-4-[3-methoxy-4-(trifluoromethyl)phenyl]-2-[(2-[(S)-N-methyl-2-[(R,Z)-3-(methylamino)-2-oxo-3,4,7,8-tetrahydroazocin-1(2H)-yl]-3-[4-(trifluoromethyl)phenyl]propanamido]acetamido)butanoyl]-N-methylpyrrolidine-2-carboxyamido]-N-methylcyclobutane-1-carboxyamido]-N-methylacetamido]-4-(dimethylamino)-N-methyl-4-oxobutanamido]pentanoyl]-L-isoleucyl-L-proline (compound 40).

In an aspect, the compound represented by formula (3a) can be produced by reacting an N-terminal amino acid residue of the peptide compound represented by formula (7a) with a C-terminal amino acid residue of the peptide compound represented by formula (4a) in a solvent for linkage (linking step). The solvent in the linking step, the condensation reagent in the linking step, the base in the linking step and the like are the same as those described in the above aspects 1 and 2.

In the production methods for the compounds represented by formulae (2a) to (7a), it is preferable not to use solid phase synthesis.

In the production method for the cyclic peptide compound represented by formula (1a), it is preferable not to use solid phase synthesis.

Crystal of cyclic peptide compound

In an aspect, the present invention relates to a crystal of a cyclic peptide compound represented by formula (1a), or a salt thereof, or a solvate thereof. Specific examples of the crystal of this compound include a non-solvate crystal or a solvate crystal of this compound, or a non-solvate crystal or a solvate crystal of a salt of this compound. Among them, a solvate crystal of the cyclic peptide compound represented by formula (1a) is preferred. Preferred examples of the solvate crystal include a hydrate crystal.

The diffraction angle 2θ in powder X-ray diffraction is a diffraction peak measured using CuKα or CuKα1 radiation. A crystal that is further identified with the diffraction angles 2θ in powder X-ray diffraction from these solvate crystals may be referred to as "form A crystal" of a hydrate as shown below, for example, or simply referred to as "form A".

In an aspect, when the crystal of the cyclic peptide compound of formula (1a) is a hydrate crystal, the crystal is a form A crystal having a powder X-ray diffraction pattern including at least 7 peaks of the following as diffraction angles 2θ in powder X-ray diffraction. Note that the diffraction angles (2θ values) described below are preferably diffraction angles (2θ values) of a hydrate crystal stored at a relative humidity of 10% or more for 15 minutes or longer, and more preferably diffraction angles (2θ values) of a hydrate crystal stored at a relative humidity of 10% for 15 minutes.

6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73°

(±0.2°)

In an aspect, when the crystal of the cyclic peptide compound of formula (1a) is a hydrate crystal, the crystal is a form A crystal having a powder X-ray diffraction pattern including at least 8 peaks of the following as diffraction angles 2θ in powder X-ray diffraction. Note that the diffraction angles (2θ values) described below are preferably diffraction angles (2θ values) of a hydrate crystal stored at a relative humidity of 10% or more for 15 minutes or longer, and more preferably diffraction angles (2θ values) of a hydrate crystal stored at a relative humidity of 10% for 15 minutes.

6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°)

In an aspect, when the crystal of the cyclic peptide compound of formula (1a) is a hydrate crystal, the crystal is a form A crystal having a powder X-ray diffraction pattern including the following peaks as diffraction angles 2θ in powder X-ray diffraction. Note that the diffraction angles (2θ values) described below are preferably diffraction angles (2θ values) of a hydrate crystal stored at a relative humidity of 30% or more for 15 minutes or longer, and more preferably diffraction angles (2θ values) of a hydrate crystal stored at a relative humidity of 30% for 15 minutes.

6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°)

In an aspect, when the crystal of the cyclic peptide compound of formula (1a) is a hydrate crystal, the crystal is a form B crystal having a powder X-ray diffraction pattern including at least 7 peaks of the following as diffraction angles 2θ in powder X-ray diffraction. Note that the diffraction angles (2θ values) described below are preferably diffraction angles (2θ values) of a hydrate crystal stored at a relative humidity of 30% or more for 15 minutes or longer, and more preferably diffraction angles (2θ values) of a hydrate crystal stored at a relative humidity of 30% for 15 minutes.

4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°)

In an aspect, when the crystal of the cyclic peptide compound of formula (1a) is a hydrate crystal, the crystal is a form B crystal having a powder X-ray diffraction pattern including at least 8 peaks of the following as diffraction angles 2θ in powder X-ray diffraction. Note that the diffraction angles (2θ values) described below are preferably diffraction angles (2θ values) of a hydrate crystal stored at a relative humidity of 30% or more for 15 minutes or longer, and more preferably diffraction angles (2θ values) of a hydrate crystal stored at a relative humidity of 30% for 15 minutes.

4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°)

In an aspect, when the crystal of the cyclic peptide compound of formula (1a) is a hydrate crystal, the crystal is a form B crystal having a powder X-ray diffraction pattern including the following peaks as diffraction angles 2θ in powder X-ray diffraction. Note that the diffraction angles (2θ values) described below are preferably diffraction angles (2θ values) of a hydrate crystal stored at a relative humidity of 30% or more for 15 minutes or longer, and more preferably diffraction angles (2θ values) of a hydrate crystal stored at a relative humidity of 30% for 15 minutes.

4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°)

In an aspect, when the crystal of the cyclic peptide compound of formula (1a) is a solvate crystal, the crystal is a form F crystal having a powder X-ray diffraction pattern including at least 7 peaks of the following as diffraction angles 2θ in powder X-ray diffraction.

6.99°, 8.49°, 9.49°, 9.88°, 10.21°, 11.81°, 12.32°, 12.75°, 13.17°, 13.94°, 14.92°, 15.20°, 15.64°, 16.78°, 17.01°, and 17.47° (±0.2°)

In an aspect, when the crystal of the cyclic peptide compound of formula (1a) is a solvate crystal, the crystal is a form F crystal having a powder X-ray diffraction pattern including at least 8 peaks of the following as diffraction angles 2θ in powder X-ray diffraction.

6.99°, 8.49°, 9.49°, 9.88°, 10.21°, 11.81°, 12.32°, 12.75°, 13.17°, 13.94°, 14.92°, 15.20°, 15.64°, 16.78°, 17.01°, and 17.47° (±0.2°)

In an aspect, when the crystal of the cyclic peptide compound of formula (1a) is a solvate crystal, the crystal is a form F crystal having a powder X-ray diffraction pattern including the following peaks as diffraction angles 2θ in powder X-ray diffraction.

6.99°, 8.49°, 9.49°, 9.88°, 10.21°, 11.81°, 12.32°, 12.75°, 13.17°, 13.94°, 14.92°, 15.20°, 15.64°, 16.78°, 17.01°, and 17.47° (±0.2°)

In an aspect, when the crystal of the cyclic peptide compound of formula (1a) is a solvate crystal, the crystal is a form J crystal having a powder X-ray diffraction pattern including at least 7 peaks of the following as diffraction angles 2θ in powder X-ray diffraction. Note that the diffraction angles (2θ values) described below are preferably diffraction angles (2θ values) of a solvate crystal stored at a relative humidity of less than 10% for 15 minutes or longer, and more preferably diffraction angles (2θ values) of a solvate crystal stored at a relative humidity of less than 10% for 15 minutes.

6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°)

In an aspect, when the crystal of the cyclic peptide compound of formula (1a) is a solvate crystal, the crystal is a form J crystal having a powder X-ray diffraction pattern including at least 8 peaks of the following as diffraction angles 2θ in powder X-ray diffraction. Note that the diffraction angles (2θ values) described below are preferably diffraction angles (2θ values) of a solvate crystal stored at a relative humidity of less than 10% for 15 minutes or longer, and more preferably diffraction angles (2θ values) of a solvate crystal stored at a relative humidity of less than 10% for 15 minutes.
6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°)

In an aspect, when the crystal of the cyclic peptide compound of formula (1a) is a solvate crystal, the crystal is a form J crystal having a powder X-ray diffraction pattern including the following peaks as diffraction angles 2θ in powder X-ray diffraction. Note that the diffraction angles (2θ values) described below are preferably diffraction angles (2θ values) of a solvate crystal stored at a relative humidity of less than 10% for 15 minutes or longer, and more preferably diffraction angles (2θ values) of a solvate crystal stored at a relative humidity of less than 10% for 15 minutes.
6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°°)

In an aspect, when the crystal of the cyclic peptide compound of formula (1a) is a hydrate crystal, the crystal is a form J crystal having a powder X-ray diffraction pattern including at least 7 peaks of the following as diffraction angles 2θ in powder X-ray diffraction. Note that the diffraction angles (2θ values) described below are preferably diffraction angles (2θ values) of a hydrate crystal stored at a relative humidity of less than 10% for 15 minutes or longer, and more preferably diffraction angles (2θ values) of a hydrate crystal stored at a relative humidity of less than 10% for 15 minutes.
6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°)

In an aspect, when the crystal of the cyclic peptide compound of formula (1a) is a hydrate crystal, the crystal is a form J crystal having a powder X-ray diffraction pattern including at least 8 peaks of the following as diffraction angles 2θ in powder X-ray diffraction. Note that the diffraction angles (2θ values) described below are preferably diffraction angles (2θ values) of a hydrate crystal stored at a relative humidity of less than 10% for 15 minutes or longer, and more preferably diffraction angles (2θ values) of a hydrate crystal stored at a relative humidity of less than 10% for 15 minutes.
6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°)

In an aspect, when the crystal of the cyclic peptide compound of formula (1a) is a hydrate crystal, the crystal is a form J crystal having a powder X-ray diffraction pattern including the following peaks as diffraction angles 2θ in powder X-ray diffraction. Note that the diffraction angles (2θ values) described below are preferably diffraction angles (2θ values) of a hydrate crystal stored at a relative humidity of less than 10% for 15 minutes or longer, and more preferably diffraction angles (2θ values) of a hydrate crystal stored at a relative humidity of less than 10% for 15 minutes.
6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°)

In an aspect, when the crystal of the cyclic peptide compound of formula (1a) is a hydrate crystal, the crystal includes a form J crystal having a powder X-ray diffraction pattern including at least 7 peaks of the following: 6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°) as diffraction angles 2θ in powder X-ray diffraction at a relative humidity of less than 10%, and includes a form A crystal having a powder X-ray diffraction pattern including at least 7 peaks of the following: 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°) at a relative humidity of 10% or more.

In an aspect, when the crystal of the cyclic peptide compound of formula (1a) is a hydrate crystal, the crystal includes a form J crystal having a powder X-ray diffraction pattern including at least 8 peaks of the following: 6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°) as diffraction angles 2θ in powder X-ray diffraction at a relative humidity of less than 10%, and includes a form A crystal having a powder X-ray diffraction pattern including at least 8 peaks of the following: 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°) at a relative humidity of 10% or more.

In an aspect, when the crystal of the cyclic peptide compound of formula (1a) is a hydrate crystal, the crystal includes a form J crystal having a powder X-ray diffraction pattern including peaks of 6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97° (±0.2°) as diffraction angles 2θ in powder X-ray diffraction at a relative humidity of less than 10%, and includes a form A crystal having a powder X-ray diffraction pattern including peaks of 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°) at a relative humidity of 10% or more.

In an aspect, when the crystal of the cyclic peptide compound of formula (1a) is a solvate crystal, the crystal is a form Y crystal having a powder X-ray diffraction pattern including at least 7 peaks of the following as diffraction angles 2θ in powder X-ray diffraction. Note that the diffraction angles (2θ values) described below are preferably diffraction angles (2θ

values) of a solvate crystal stored at a relative humidity of less than 30% for 15 minutes or longer, and more preferably diffraction angles (2θ values) of a solvate crystal stored at a relative humidity of less than 30% for 15 minutes.
5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°)

In an aspect, when the crystal of the cyclic peptide compound of formula (1a) is a solvate crystal, the crystal is a form Y crystal having a powder X-ray diffraction pattern including at least 8 peaks of the following as diffraction angles 2θ in powder X-ray diffraction. Note that the diffraction angles (2θ values) described below are preferably diffraction angles (2θ values) of a solvate crystal stored at a relative humidity of less than 30% for 15 minutes or longer, and more preferably diffraction angles (2θ values) of a solvate crystal stored at a relative humidity of less than 30% for 15 minutes.
5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°)

In an aspect, when the crystal of the cyclic peptide compound of formula (1a) is a solvate crystal, the crystal is a form Y crystal having a powder X-ray diffraction pattern including the following peaks as diffraction angles 2θ in powder X-ray diffraction. Note that the diffraction angles (2θ values) described below are preferably diffraction angles (2θ values) of a solvate crystal stored at a relative humidity of less than 30% for 15 minutes or longer, and more preferably diffraction angles (2θ values) of a solvate crystal stored at a relative humidity of less than 30% for 15 minutes.
5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°)

In an aspect, when the crystal of the cyclic peptide compound of formula (1a) is a hydrate crystal, the crystal is a form Y crystal having a powder X-ray diffraction pattern including at least 7 peaks of the following as diffraction angles 2θ in powder X-ray diffraction. Note that the diffraction angles (2θ values) described below are preferably diffraction angles (2θ values) of a solvate crystal stored at a relative humidity of less than 30% for 15 minutes or longer, and more preferably diffraction angles (2θ values) of a hydrate crystal stored at a relative humidity of less than 30% for 15 minutes.
5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°)

In an aspect, when the crystal of the cyclic peptide compound of formula (1a) is a hydrate crystal, the crystal is a form Y crystal having a powder X-ray diffraction pattern including at least 8 peaks of the following as diffraction angles 2θ in powder X-ray diffraction. Note that the diffraction angles (2θ values) described below are preferably diffraction angles (2θ values) of a solvate crystal stored at a relative humidity of less than 30% for 15 minutes or longer, and more preferably diffraction angles (2θ values) of a hydrate crystal stored at a relative humidity of less than 30% for 15 minutes.
5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°)

In an aspect, when the crystal of the cyclic peptide compound of formula (1a) is a hydrate crystal, the crystal is a form Y crystal having a powder X-ray diffraction pattern including the following peaks as diffraction angles 2θ in powder X-ray diffraction. Note that the diffraction angles (2θ values) described below are preferably diffraction angles (2θ values) of a hydrate crystal stored at a relative humidity of less than 30% for 15 minutes or longer, and more preferably diffraction angles (2θ values) of a hydrate crystal stored at a relative humidity of less than 30% for 15 minutes.
5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°)

In an aspect, when the crystal of the cyclic peptide compound of formula (1a) is a hydrate crystal, the crystal includes a form Y crystal having a powder X-ray diffraction pattern including at least 7 peaks of the following: 5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°) as diffraction angles 2θ in powder X-ray diffraction at a relative humidity of less than 30%, and includes a form B crystal having a powder X-ray diffraction pattern including at least 7 peaks of the following: 4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°) at a relative humidity of 30% or more.

In an aspect, when the crystal of the cyclic peptide compound of formula (1a) is a hydrate crystal, the crystal includes a form Y crystal having a powder X-ray diffraction pattern including at least 8 peaks of the following: 5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°) as diffraction angles 2θ in powder X-ray diffraction at a relative humidity of less than 30%, and includes a form B crystal having a powder X-ray diffraction pattern including at least 8 peaks of the following: 4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°) at a relative humidity of 30% or more.

In an aspect, when the crystal of the cyclic peptide compound of formula (1a) is a hydrate crystal, the crystal includes a form Y crystal having a powder X-ray diffraction pattern including peaks of 5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24° (±0.2°) as diffraction angles 2θ in powder X-ray diffraction at a relative humidity of less than 30%, and includes a form B crystal having a powder X-ray diffraction pattern including peaks of 4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°) at a relative humidity of 30% or more.

In an aspect, when the crystal of the cyclic peptide compound of formula (1a) is a hydrate crystal, the crystal is a

form K crystal having a powder X-ray diffraction pattern including at least 7 peaks of the following as diffraction angles 2θ in powder X-ray diffraction.
7.49°, 7.91°, 8.14°, 9.11°, 9.33°, 11.04°, 11.71°, 12.52°, 13.21°, 13.70°, 14.82°, 15.13°, 15.52°, 15.68°, 17.22°, and 17.51° (±0.2°)

In an aspect, when the crystal of the cyclic peptide compound of formula (1a) is a hydrate crystal, the crystal is a form K crystal having a powder X-ray diffraction pattern including at least 8 peaks of the following as diffraction angles 2θ in powder X-ray diffraction.
7.49°, 7.91°, 8.14°, 9.11°, 9.33°, 11.04°, 11.71°, 12.52°, 13.21°, 13.70°, 14.82°, 15.13°, 15.52°, 15.68°, 17.22°, and 17.51° (±0.2°)

In an aspect, when the crystal of the cyclic peptide compound of formula (1a) is a hydrate crystal, the crystal is a form K crystal having a powder X-ray diffraction pattern including the following peaks as diffraction angles 2θ in powder X-ray diffraction.
7.49°, 7.91°, 8.14°, 9.11°, 9.33°, 11.04°, 11.71°, 12.52°, 13.21°, 13.70°, 14.82°, 15.13°, 15.52°, 15.68°, 17.22°, and 17.51° (±0.2°)

In an aspect, the present invention relates to a method for producing the crystal of a cyclic peptide compound represented by formula (1a), or a salt thereof, or a solvate thereof. The method comprises: a step of dissolving the cyclic peptide compound in a polar organic solvent in an amount that allows the cyclic peptide compound to dissolve therein to obtain a solution; and a step of adding a hydrocarbon-based solvent or water to the solution to obtain a crystal of the cyclic peptide compound (hereinafter, also referred to as "aspect 3"). In aspect 3, the properties of the cyclic peptide compound to be dissolved are not restricted, and for example, the cyclic peptide compound in a solid state, amorphous state, or crystalline state can be used.

In an aspect, the present invention relates to a method for producing the crystal of a cyclic peptide compound represented by formula (1a), or a salt thereof, or a solvate thereof. The method comprises: a step of adding a mixed solution of a hydrocarbon-based solvent and a polar organic solvent or a mixed solution of water and a polar organic solvent to the cyclic peptide compound in an amorphous state or crystalline state to obtain a crystal of the cyclic peptide compound (hereinafter, also referred to as "aspect 4").

As the polar organic solvent used in aspects 3 and 4, specifically, DMSO, acetone, 2-butanone, methanol, ethanol, 1-propanol, 2-propanol, ethyl acetate, propylene glycol, and a mixed solvent thereof are preferably exemplified, and acetone is more preferably exemplified. As the "amount that allows the cyclic peptide compound to dissolve therein" in aspect 3, the polar organic solvent can be used in the range of 3 to 10 v/w, preferably in the range of 3 to 7 v/w, with respect to the cyclic peptide compound of formula (1a).

As the hydrocarbon-based solvent used in aspects 3 and 4, specifically, heptane, hexane, pentane, toluene, xylene, and a mixed solvent thereof are preferably exemplified, and heptane is more preferably exemplified.

In aspect 4, as the mixing ratio between the hydrocarbon-based solvent and the polar organic solvent in the mixed solution of the hydrocarbon-based solvent and the polar organic solvent, 0.5 to 10 parts by weight of the hydrocarbon-based solvent can be used with respect to 1 part by weight of the polar organic solvent, and it is preferable to use preferably 1 to 7 parts by weight of the hydrocarbon-based solvent, and still more preferably 1 to 5 parts by weight of the hydrocarbon-based solvent. Also, in aspect 4, as the mixing ratio between water and the polar organic solvent in the mixed solution of water and the polar organic solvent, 0.5 to 10 parts by weight of water can be used with respect to 1 part by weight of the polar organic solvent, and it is preferable to use preferably 1 to 7 parts by weight of water, and still more preferably 1 to 5 parts by weight of water.

Furthermore, in a certain aspect of aspect 4, in the operation of adding a mixed solution of a hydrocarbon-based solvent and a polar organic solvent or a mixed solution of water and a polar organic solvent to the cyclic peptide compound in an amorphous state or crystalline state, glass beads (for example, 1 to 5 grains) may be added to the crystal and shaken.

In an aspect, the present invention relates to a method for producing the crystal of a cyclic peptide compound represented by formula (1a), or a salt thereof, or a solvate thereof. The method comprises: a step of dissolving the cyclic peptide compound in an amorphous state in DMSO to obtain a solution; a step of freeze-drying the solution to obtain a freeze-dried product of the cyclic peptide compound; and a step of adding a mixed solution of water and a polar organic solvent to the freeze-dried product to obtain a crystal of the cyclic peptide compound (hereinafter, also referred to as "aspect 5").

As the polar organic solvent used in aspect 5, specifically, DMSO, acetone, 2-butanone, methanol, ethanol, 1-propanol, 2-propanol, propylene glycol, and a mixed solvent thereof are preferably exemplified, and acetone is more preferably exemplified.

In aspect 5, as the mixing ratio between water and the polar organic solvent in the mixed solution of water and the polar organic solvent, 0.5 to 10 parts by weight of water can be used with respect to 1 part by weight of the polar organic solvent, and it is preferable to use preferably 1 to 7 parts by weight of water, and still more preferably 1 to 5 parts by weight of water.

In an aspect, the present invention relates to a method for producing the crystal of a cyclic peptide compound

represented by formula (1a), or a salt thereof, or a solvate thereof. The method comprises a step of heating the crystal of the cyclic peptide compound to obtain another crystalline polymorph of the cyclic peptide compound (hereinafter, also referred to as "aspect 6"). The heating temperature is, for example, 30 to 350°C, preferably 30 to 120°C.

In aspects 3 to 6, the methods may further comprise, after the step of obtaining a crystal of the cyclic peptide compound, a step of filtering the crystal.

In aspects 3 to 6, the methods may further comprise, after the step of obtaining a crystal of the cyclic peptide compound, a step of drying the crystal.

In an aspect, the crystal of the cyclic peptide compound produced by the method of the present invention is preferably a solvate crystal, and more preferably a hydrate crystal.

In an aspect, the crystal of the cyclic peptide compound produced by the method of the present invention is formed as a solvate crystal in a solvent and obtained as a hydrate crystal after the filtrating step and/or the drying step. Also, in an aspect, the crystal of the cyclic peptide compound produced by the method of the present invention is obtained as a hydrate crystal by incorporating atmospheric moisture after the filtering step and/or the drying step.

Composition containing cyclic peptide compound

In an aspect, the present invention relates to a composition comprising a cyclic peptide compound represented by formula (1a), or a salt thereof, or a solvate thereof. The compound contains a cyclic dimer of formula (1a), which is an impurity, at a proportion of 1.5 w/w% or less.

In an aspect, the present invention relates to a composition comprising a cyclic peptide compound represented by formula (1a), or a salt thereof, or a solvate thereof. The compound contains a cyclic dimer of formula (1a), which is an impurity, at a proportion of 0.001 w/w% or more.

In an aspect, the present invention relates to a composition comprising a cyclic peptide compound represented by formula (1a), or a salt thereof, or a solvate thereof. The compound contains acetone at a proportion of 2.0 w/w% or less.

In an aspect, the present invention relates to a composition comprising a cyclic peptide compound represented by formula (1a), or a salt thereof, or a solvate thereof. The compound contains acetone at a proportion of 0.001 w/w% or more.

[Examples]

The contents of the present invention will be further described by the following Examples, but the present invention is not limited to their contents. Except those as specifically described, raw starting substances, starting raw materials, solvents, and reagents were obtained from commercial suppliers, or synthesized using known methods. Compound 25 used in Example 1-26 described below was synthesized by the method described in International Publication No. WO 2022/234853.

The analysis conditions by LCMS are shown below.

LCMS analysis conditions method 1

Apparatus: Shimadzu LCMS 2020
Column: CORTECS C18 Column, 3.0 mm ID × 50 mm, 2.7 μm
Mobile phase: 0.05% TFA/water (A), 0.05% TFA/MeCN (B)
Elution method: B) 5% (0 min) → 95% (2.0 min) → 95% (2.8 min) → 5% (2.81 min) → 5% (3 min)
Flow rate: 1.5 mL/min
Column temperature: 40°C
Detection wavelength: 190 to 800 nm (PDA)

LCMS analysis conditions method 2

Apparatus: Waters UPLC/SQD
Column: Ascentis Express RP 90A amide, 2.1 mm ID × 50 mm, 2.7 μm (PDA)m
Mobile phase: 0.1% FA/water (A), 0.1% FA/MeCN (B)
Elution method: B) 5% (0 min) → 100% (4.5 min) → 100% (5.0 min) → 5% (5.01 min) → 5% (7 min)
Flow rate: 0.5 mL/min

Column temperature: 40°C
Detection wavelength: 210 to 400 nm (PDA)

LCMS analysis conditions method 3

Apparatus: Waters UPLC/SQD
Column: Ascentis Express 90A C18, 2.1 mm ID × 50 mm, 2.7 μm
Mobile phase: 0.1% FA/water (A), 0.1% FA/MeCN (B)
Elution method: B) 5% (0 min) → 100% (5 min) → 5% (5.01 min) → 5% (7 min)
Flow rate: 0.5 mL/min
Column temperature: Off
Detection wavelength: 210 to 400 nm (PDA)

LCMS analysis conditions method 4

Apparatus: Waters UPLC/SQD
Column: ACQUITY UPLC BEH C18 Column, 2.1 mm ID × 50 mm, 1.7 μm
Mobile phase: 0.1% FA/water (A), 0.1% FA/MeCN (B)
Elution method: B) 5% (0 min) → 98% (8 min) → 98% (10 min) → 5% (10.01 min) → 5% (12 min)
Flow rate: 0.5 mL/min
Column temperature: 60°C
Detection wavelength: 210 to 400 nm (PDA)

LCMS analysis conditions method 5

Apparatus: Waters UPLC/SQD
Column: Ascentis Express C18 2.1 × 50 mm, 5 μm
Mobile phase: 10 mM ammonium acetate in water (A), MeOH (B)
Elution method: B) 50% (0 min) → 100% (1 min) → 100% (1.99 min) → 50% (2.01 min) → 50% (2.5 min)
Flow rate: 1.0 mL/min
Column temperature: 35°C
Detection wavelength: 210 to 400 nm (PDA)

LCMS analysis conditions method M

Apparatus: Waters Acquity UPLC/QDa
Column: Ascentis Express 90A C18, 2.1 mm ID × 50 mm, 2.7 μm
Mobile phase: 0.05% TFA/water (A), 0.05% TFA/MeCN (B)
Elution method: B) 5% (0 min) → 100% (5 min) → 5% (5.01 min) → 5% (7 min)
Flow rate: 0.5 mL/min
Column temperature: 35°C
Detection wavelength: 210 nm (PDA)

LCMS analysis conditions method K-1

Apparatus: Waters Acquity UPLC/QDa
Column: CAPCELL CORE ADME, 2.1 mm × 50 mm, 2.7 μm (Osaka Soda)
Mobile phase: 0.05% TFA/water (A), 0.05% TFA/MeCN (B)

Elution method: B) 5% (0 min) → 100% (10 min) → 5% (10.1 min) → 5% (12 min)
Flow rate: 0.5 mL/min
Column temperature: 35°C
Detection wavelength: 210 nm (PDA)

LCMS analysis conditions method K-2

Apparatus: Waters Acquity UPLC/QDa
Column: CAPCELL CORE ADME, 2.1 mm × 50 mm, 2.7 μm (Osaka Soda)
Mobile phase: 0.05% TFA/water (A), 0.05% TFA/MeCN (B)
Elution method: B) 5% (0 min) → 100% (5 min) → 5% (5.1 min) → 5% (7 min)
Flow rate: 0.5 mL/min
Column temperature: 35°C
Detection wavelength: 210 nm (PDA)

LCMS analysis conditions method P3-4

Apparatus: Waters Acquity UPLC/QDa
Column: Ascentis Express 90A C18, 2.1 mm ID × 50 mm, 2.7 μm
Mobile phase: 0.05% TFA/water (A), 0.05% TFA/MeCN (B)
Elution method: B) 5% (0 min) → 100% (5 min) → 5% (5.01 min) → 5% (7 min)
Flow rate: 0.5 mL/min
Column temperature: 35°C
Detection wavelength: 210 nm (PDA)

LCMS analysis conditions method FC 2

Apparatus: Waters UPLC
Column: ACQUITY UPLC CSH C18, 2.1 mm ID × 100 mm, 1.7 μm
Mobile phase: 0.05% TFA/water (A), 0.05% TFA/MeCN (B)
Elution method: B) 20% (0 min) → 100% (10 min) → 100% (13.5 min) → 20% (13.6 min) → 20% (15.5 min)
Flow rate: 0.3 mL/min
Column temperature: 50°C
Detection wavelength: 210 nm (PDA)

LC analysis conditions method cyc

Apparatus: Waters UPLC
Column: ACQUITY UPLC CSH Phenyl-Hexyl, 2.1 mm ID × 150 mm, 1.7 μm
Mobile phase: 0.05% TFA/water (A), 0.05% TFA/MeCN (B)
Elution method: B) 20% (0 min) → 100% (24 min) → 100% (29 min) → 20% (29.1 min) → 20% (34 min)
Flow rate: 0.3 mL/min
Column temperature: 50°C
Detection wavelength: 220 nm (PDA)

LC analysis conditions method H

Apparatus: Waters UPLC

Column: Ascentis Express RP-Amide, 3.0 mm ID × 50 mm ×, 2.7 μm
Mobile phase: 0.05% TFA/water (A), 0.05% TFA/MeCN (B)
Elution method: B) 5% (0 min) → 95% (10.0 min) → 95% (12.0 min) → 5% (12.1 min) → 5% (15.0 min)
Flow rate: 0.7 mL/min
Column temperature: 30°C
Detection wavelength: 210 nm (PDA)

The analysis conditions by HPLC are shown below.

HPLC analysis conditions method 1

Apparatus: Waters H-Class
Column: ACQUITY UPLC BEH C18 Column, 2.1 mm ID × 50 mm, 1.7 μm
Mobile phase: 0.1% FA/water (A), 0.1% FA/MeCN (B)
Elution method: B) 5% (0 min) → 98% (4.0 min) → 98% (6.0 min) → 5% (6.01 min) → 5% (8 min)
Flow rate: 0.5 mL/min
Column temperature: 60°C
Detection wavelength: 210 nm (PDA)

The measuring method by qNMR was performed by dissolving a residue containing a target compound and an internal standard substance in $CDCl_3$ or DMSO-$d_6$ and subjecting to the following analysis conditions. The yield was calculated by the following expression using the value of the content of the target compound in the residue calculated by qNMR or HPLC.

[Expression 1]

$$\text{yield (\%)} = \frac{\text{weight of residue (g)} \times \text{content (\%)}}{\text{theoretical yield (g)}} \times 100$$

Measurement apparatus: Bruker Avance III 400
Internal standard substance: 3,5-bis(trifluoromethyl)benzoic acid
Measurement conditions ($^{19}$F-NMR): $CDCl_3$ or DMSO-$d_6$, 24.8°C, pulse angle 90°, digital resolution 0.24 Hz, relaxation time 15 seconds, no spin, cumulative number 64 times
Measurement apparatus: JEOL JNM-ECZ500R/S1
Measurement conditions ($^1$H-NMR): methanol-$d_4$, 25.3°C, pulse angle 45°, digital resolution 0.76 Hz, relaxation time 5 seconds, with spin, cumulative number 8 times

The measuring methods by LCMS, LC, and HPLC were performed by preparing a mixed solution containing a target compound as a sample according to any of the following methods and subjecting it to the analysis conditions described above.

Sample preparation method 1: a mixed solution containing a target compound was diluted with acetonitrile.
Sample preparation method 2: a mixed solution containing a target compound was diluted with a mixed solution of acetonitrile and water in a ratio of 9 : 1.
Sample preparation method K: a mixed solution containing a target compound was diluted with a mixed solution of acetonitrile and n-propylamine in a ratio of 100 : 1.

The reaction conversion rate was calculated by any of the following expressions using the area value of raw material and the area value of target material, or the area value of raw material, the area value of raw material, and the area value of target material, or the area value of raw material before reaction and the area value of raw material after reaction calculated by HPLC analysis.

reaction conversion rate (%) = area value of target material/(area value of raw material + area value of target material) × 100 Expression 1:

reaction conversion rate (%) = 100 - (area value of raw material after reaction/area value of raw material before reaction $\times$ 100) Expression 2:

The selectivity of cyclization (compound 1/cyclic dimer) was calculated by the following expressions using the area value of target material and the area value of cyclic dimer calculated by HPLC analysis.

compound 1 ratio (%) = area value of compound 1/(area value of compound 1 + area value of cyclic dimer) $\times$ 100 Expression 1:

cyclic dimer ratio (%) = area value of cyclic dimer/(area value of compound 1 + area value of cyclic dimer) $\times$ 100 Expression 2:

Example 1-1

Synthesis of compound 2: 9H-fluoren-9-ylmethyl (4S)-5-oxo-4-[[4-(trifluoromethyl)phenyl]methyl]oxazolidine-3-carboxylate

[Formula 44]

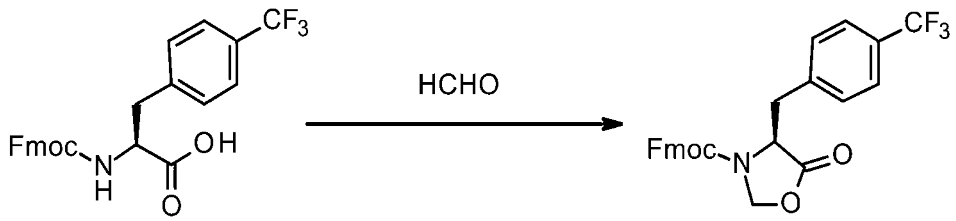


To a reaction vessel after nitrogen replacement, DCM (45 L) and (2S)-2-{[(9H-fluoren-9-ylmethoxy)carbonyl]amino}-3-[4-(trifluoromethyl)phenyl]propionic acid (3.05 kg) were added at room temperature, and the mixture was stirred. Subsequently, paraformaldehyde (0.90 kg) and $MgSO_4$ (2.02 kg) were added, and the mixture was stirred at 20°C for 10 minutes. The external temperature of the reaction vessel was cooled to 15°C, and $BF_3OEt_2$ (0.95 kg) was slowly added dropwise at an internal temperature of 15 to 20°C. The reaction mixture was stirred at 20 to 25°C for 12 hours, then filtered through a filter lined with silica gel (3.05 kg), and washed with DCM (15.3 L $\times$ 2). The filtrate was concentrated under reduced pressure at an external temperature of 30°C, and the crude product containing compound 2 was subjected to column purification (petroleum ether/ethyl acetate = 4/1) and concentrated under reduced pressure to obtain compound 2 (2.76 kg).

Example 1-2

Synthesis of compound 3: (2S)-2-[but-3-enyl(9H-fluoren-9-ylmethoxycarbonyl)amino]-3-[4-(trifluoromethyl)phenyl]propionic acid

[Formula 45]

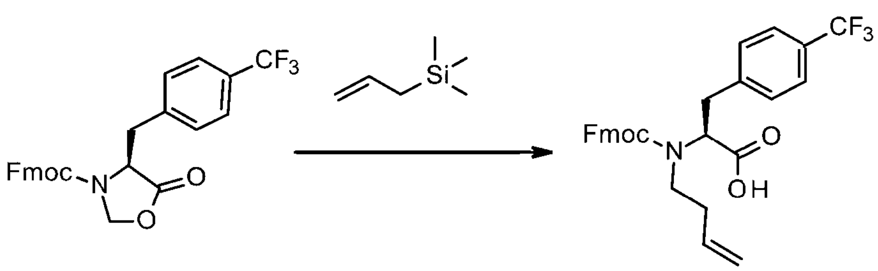

To a reaction vessel after nitrogen replacement, toluene (22.4 L) and compound 2 (2.80 kg) were added at room temperature, and the mixture was stirred. Subsequently, allyltrimethylsilane (1.37 kg) and $ZnBr_2$ (1.35 kg) were added, and the mixture was stirred at 20°C for 10 minutes. The internal temperature of the reaction vessel was heated to 40 to 45°C, and the reaction mixture was stirred for 10 hours. The reaction mixture was added to ice water (28.0 L) at an internal temperature of 10 ± 5°C and stirred. The aqueous layer was discharged, and the organic layer was washed with 5% saline (28.0 L). The resulting organic layer was concentrated under reduced pressure at an external temperature of 45 to 50°C to obtain compound 3 (2.44 kg).

Example 1-3

Synthesis of compound 4: tert-butyl 2-[[(2S)-2-[but-3-enyl(9H-fluoren-9-ylmethoxycarbonyl)amino]-3-[4-(trifluoromethyl)phenyl]propanoyl]-methyl-amino]acetate

[Formula 46]

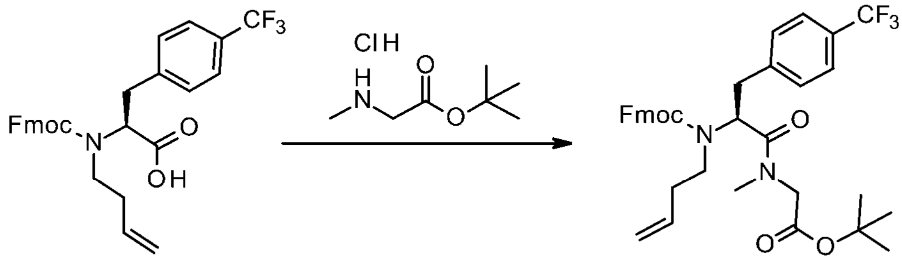

To a reaction vessel after nitrogen replacement, N-methyl-2-pyrrolidone (17.0 L) and compound 3 (2.44 kg) were added at room temperature, and the mixture was stirred. Subsequently, sarcosine tert-butyl ester hydrochloride (0.87 kg) and HATU (2.18 kg) were added at 20°C, and the mixture was stirred for 30 minutes. DIPEA (1.85 kg) was added dropwise over 60 minutes at an internal temperature of 15 to 20°C. The reaction mixture was stirred at 20 to 25°C for 3 hours, and then diluted with methyl tert-butyl ether (48.8 L). The organic layer was washed with water (48.8 L × 2) and 5% saline (24.4 L), and then concentrated under reduced pressure to obtain a crude product containing compound 4. The resulting crude product was subjected to column purification (petroleum ether/ethyl acetate = 4/1) and concentrated under reduced pressure at 35°C to obtain compound 4 (2.79 kg) as a dark yellow oil.

Example 1-4

Synthesis of compound 5: tert-butyl 2-[[(2S)-2-(but-3-enylamino)-3-[4-(trifluoromethyl)phenyl]propanoyl]-methyl-amino]acetate

[Formula 47]

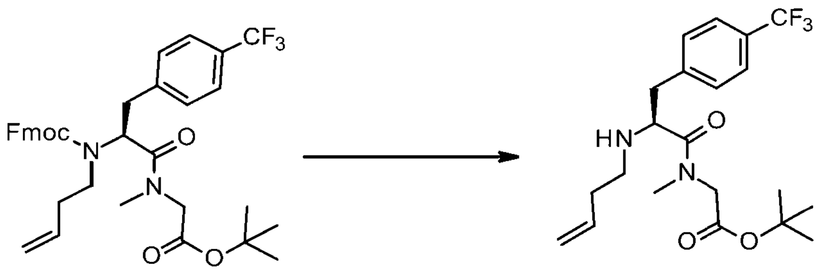

To a reaction vessel after nitrogen replacement, toluene (28.0 L) and compound 4 (2.79 kg) were added at room temperature, and the mixture was stirred. Subsequently, DBU (0.67 kg) was added at an internal temperature of 20°C, and the mixture was stirred for 2 hours. The reaction mixture was added to ice water (27.9 L) at an internal temperature of 20 ± 5°C and stirred. After stirring with the addition of ethyl acetate (14.0 L), the aqueous layer was discharged. The organic

layer was washed with 5% saline (28.0 L), and the organic layer was concentrated under reduced pressure at 45 ± 5°C. The crude product containing compound 5 was subjected to column purification (petroleum ether/ethyl acetate = 4/1) and then concentrated under reduced pressure to obtain compound 5 (1.49 kg) as a pale yellow oil.

Example 1-5

Synthesis of compound 6: 9H-fluoren-9-ylmethyl N-[(1S)-1-chlorocarbonylbut-3-enyl]-N-methyl-carbamate

[Formula 48]

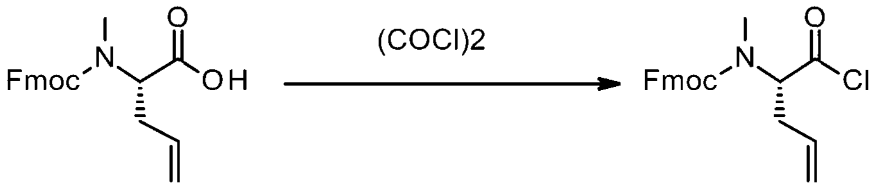


To a reaction vessel after nitrogen replacement, DCM (10 L), (2S)-2-[9H-fluoren-9-ylmethoxycarbonyl(methyl) amino]pent-4-enoic acid (1.7 kg), and DMF (0.02 kg) were added at an internal temperature of 20°C, and the mixture was stirred. Oxalyl chloride (1.84 kg) was added at an internal temperature of 10°C over 2 hours, and the mixture was stirred for 2 hours keeping the internal temperature at 10°C. The resulting solution was concentrated under reduced pressure at 30 to 35°C. Addition of DCM (3.4 L) to the concentrated product and concentration under reduced pressure were performed twice to obtain compound 6 (1.65 kg) as a yellow oil.

Example 1-6

Synthesis of compound 7: tert-butyl 2-[[(2S)-2-[but-3-enyl-[(2S)-2-[9H-fluoren-9-ylmethoxycarbonyl(methyl)amino] pent-4-enoyl]amino]-3-[4-(trifluoromethyl)phenyl]propanoyl]-methyl-amino]acetate

[Formula 49]

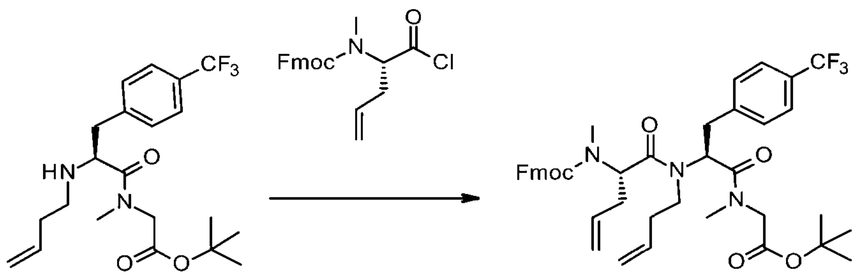

To a reaction vessel after nitrogen replacement, DCM (15.0 L) and compound 5 (1.49 kg) were added at room temperature, and the mixture was stirred. Subsequently, compound 6 (1.72 kg) dissolved in DCM (2.96 L) was added dropwise over 1 hour at an internal temperature of 0 to 10°C. The mixture was stirred at an internal temperature of 10°C for 30 minutes, followed by dropwise addition of DIPEA (0.926 kg) at an internal temperature of 0 to 10°C over 1 hour. After stirring at 20°C for 2 hours, the reaction solution was added to ice water (14.8 L) at an internal temperature of 20°C and stirred. An organic layer 1 was set aside by liquid-liquid separation. The aqueous layer was extracted with DCM (7.4 L), which was combined with the organic layer 1 that had been set aside. The combined organic layer was washed with 5% saline (14.9 L × 2), and the organic layer was concentrated under reduced pressure at 30 \± 5°C. The concentrated product containing compound 7 was subjected to column purification (petroleum ether/ethyl acetate = 5/1) and concentrated under reduced pressure to obtain compound 7 (1.97 kg) as a colorless powder.

Example 1-7

Synthesis of compound 8: tert-butyl 2-[[(2S)-2-[(4Z,7S)-7-[9H-fluoren-9-ylmethoxycarbonyl(methyl)amino]-8-oxo-2,3,6,7-tetrahydroazocin-1-yl]-3-[4-(trifluoromethyl)phenyl]propanoyl]-methyl-amino]acetate

[Formula 50]

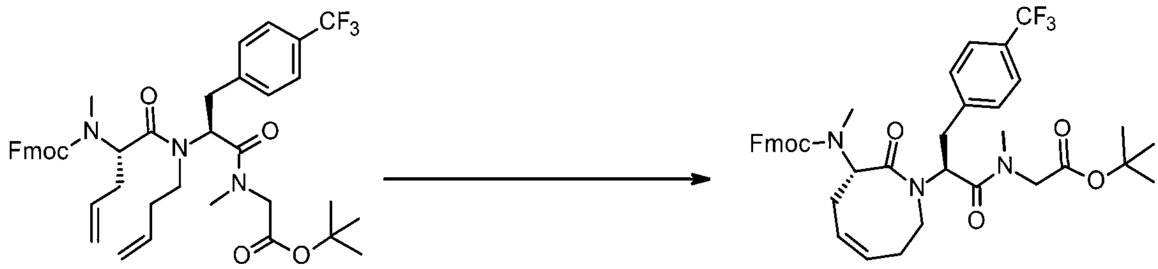

To a reaction vessel after nitrogen replacement, toluene (59.0 L) and compound 7 (0.655 kg) were added at room temperature, and the mixture was stirred. Subsequently, p-benzoquinone (28.4 g) was added, and the internal temperature was increased to 100°C. To the mixed solution that had been heated to an internal temperature of 100°C, a solution of first generation HOVEYDA-GRUBBS catalyst (42.0 g) in toluene (100 mL) was added dropwise over 20 minutes. The reaction described above was repeated in three batches. The resulting solutions from the three batches were mixed and concentrated under reduced pressure at 45°C, and the crude product containing compound 8 was subjected to column purification (petroleum ether/ethyl acetate = 4/1) and concentrated under reduced pressure to obtain compound 8 (1.05 kg) as a yellow oil.

Example 1-8

Synthesis of compound 9: tert-butyl 2-[methyl-[(2S)-2-[(4Z,7S)-7-(methylamino)-8-oxo-2,3,6,7-tetrahydroazocin-1-yl]-3-r4-(trifluoromethyl)phenyl]propanoyl]amino]acetate

[Formula 51]

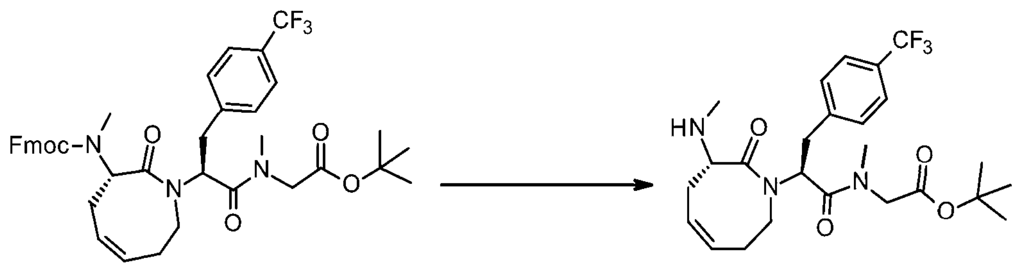

To a reaction vessel after nitrogen replacement, toluene (11.0 L) and compound 8 (1.05 kg) were added at room temperature, and the mixture was stirred. Subsequently, DBU (223 g) was added to the mixed solution at 10°C, and the mixture was stirred at an internal temperature of 20°C for 2 hours. The resulting solution was added to ice water (11.0 L) at 10 ± 5°C and stirred. The mixture was extracted with ethyl acetate (5.25 L). The organic layer was washed with 5% saline (5.25 L), and the organic layer was concentrated under reduced pressure at 40°C to about 1.1 L. Toluene (9.5 L) and a 1M aqueous potassium dihydrogen phosphate solution (6.3 L) were added to the concentrated solution, and the mixture was stirred at 20°C for 3 hours. The resulting solution was filtered, and the filtered product was washed with toluene/n-heptane = 1/1 (11.0 L). Ethanol (31.5 L) was added to the resulting filtered product, and heated to 40°C to dissolve it. D(-)-Tartaric acid (215 g) was added, and the mixture was stirred at 20°C for 3 hours to obtain a precipitate. The resulting precipitate was filtered and washed with n-heptane (3.15 L × 2). The washed precipitate was transferred to a reaction vessel, to which a 1M aqueous tripotassium phosphate solution was added, and the pH was adjusted to 7 to 8. The mixture was extracted with DCM (10.5 L). The organic layer was washed with 5% saline (5.25 L), and the resulting solution was concentrated under reduced pressure at 40°C to about 0.5 L. n-Heptane (10.5 L) was added to the concentrated solution to obtain a precipitate.

The resulting precipitate was filtered and washed with n-heptane (3.15 L). The resulting residue was dried under reduced pressure to obtain compound 9 (449 g), which is a colorless solid.
LCMS (ESI) of compound 9: retention time: 1.24 minutes, m/z = 498 $[M+H]^+$ (LCMS analysis conditions method 1)

Example 1-9

Synthesis of compound 10: tert-butyl (2S)-1-[(2S,3S)-2-(benzyloxycarbonylamino)-3-methyl-pentanoyl]pyrrolidine-2-carboxylate

[Formula 52]

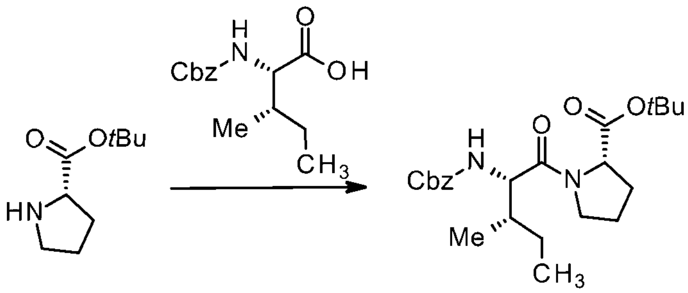

To a reaction vessel after nitrogen replacement, tert-butyl (2S)-pyrrolidine-2-carboxylate (18.8 g), (2S,3S)-2-(benzyloxycarbonylamino)-3-methyl-pentanoic acid (20.0 g), and DMF (140 mL) were added at room temperature, and the mixture was stirred. The resulting solution was cooled to 0°C and DIPEA (52.7 mL) was added. A propylphosphonic anhydride solution, 50 wt.% in ethyl acetate (58.3 mL) was added to the mixed solution at 0°C over 20 minutes, and the mixture was stirred at 0°C for 1.5 hours. To the mixture, water (100 mL) and ethyl acetate (200 mL) were added in this order. An aqueous layer 1 and an organic layer 1 were separated by liquid-liquid separation, and the aqueous layer 1 was taken apart. The organic layer 1 was washed with a 5% aqueous potassium hydrogen sulfate solution (100 mL), a 5% aqueous sodium carbonate solution (100 mL), and 10% saline (100 mL). To the aqueous layer 1 that had been taken apart, water (100 mL) and ethyl acetate (200 mL) were added, and after stirring, an organic layer 2 was obtained by liquid-liquid separation. The organic layer 1 and the organic layer 2 were combined and concentrated under reduced pressure to obtain compound 10 (33.8 g). LCMS (ESI) of compound 10: retention time: 2.75 minutes, m/z = 419 $[M+H]^+$ (LCMS analysis conditions method 2)

Example 1-10

Synthesis of compound 11: tert-butyl (2S)-1-[(2S,3S)-2-amino-3-methyl-pentanoyl]pyrrolidine-2-carboxylate

[Formula 53]

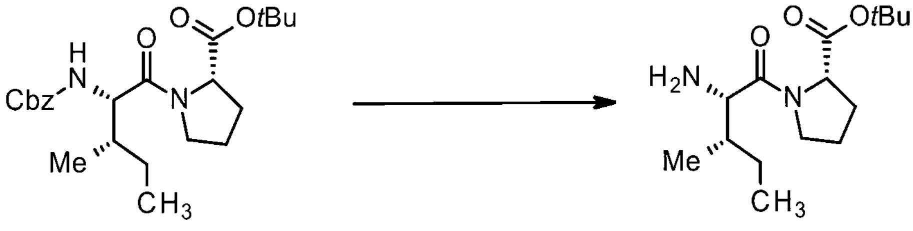

To a reaction vessel after nitrogen replacement, a solution of compound 10 (31.6 g) obtained in Example 1-9 in 2-MeTHF (221 mL) was cooled to 10°C. To the solution was added 5% Pd/C (6.32 g, 50% aqueous content), then triethylsilane (60.3 mL) was added over 20 minutes. The resulting mixture was stirred at an internal temperature of 15°C for 6 hours, and then further stirred at room temperature for 17 hours. The mixed solution was filtered, and the filtrate was concentrated under reduced pressure to obtain compound 11. Compound 11 was used in Example 1-11 without further

purification.
LCMS (ESI) of compound 11: retention time: 1.14 minutes, m/z = 285 $[M+H]^+$ (LCMS analysis conditions method 2)

Example 1-11

Synthesis of compound 12: tert-butyl (2S)-1-[(2S,3S)-2-[[(2S)-2-[9H-fluoren-9-ylmethoxycarbonyl(methyl)amino]pentanoyl]amino]-3-methyl-pentanoyl]pyrrolidine-2-carboxylate

[Formula 54]

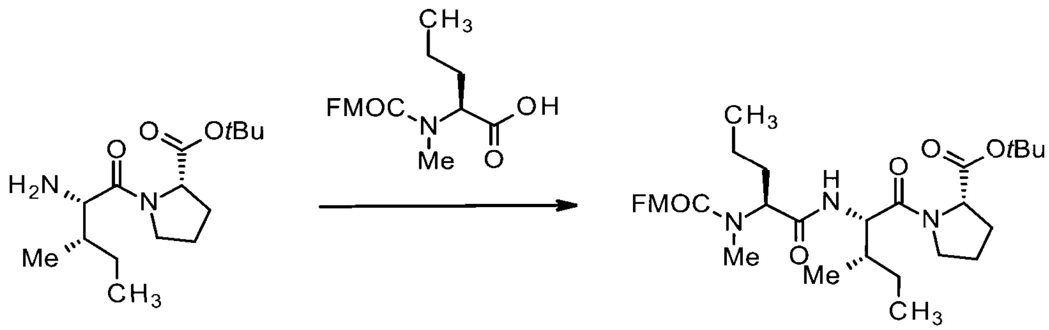

To a solution of compound 11 obtained in Example 1-10 in acetonitrile (221 mL) was added (2S)-2-[9H-fluoren-9-ylmethoxycarbonyl(methyl)amino]pentanoic acid (28.0 g) and DIPEA (39.6 mL) in this order at 5°C. To the mixture, HATU (34.5 g) was slowly added at 2.5°C, and the resulting solution was stirred at 2.5°C for 1 hour, and then stirred at room temperature for 2.5 hours. To the mixture, 5% aqueous sodium carbonate solution (189 mL) and water (150 mL) were added in this order. The resulting solution was extracted with toluene (80 mL) and 2-MeTHF (140 mL). The organic layer was washed with a 5% aqueous potassium hydrogen sulfate solution (190 mL × 2) and 10% saline (190 mL × 2). The resulting organic layer was concentrated under reduced pressure to obtain compound 12. Compound 12 was used in Example 1-12 without further purification.
LCMS (ESI) of compound 12: retention time: 3.43 minutes, m/z = 620 $[M+H]^+$ (LCMS analysis conditions method 2)

Example 1-12

Synthesis of compound 13: tert-butyl (2S)-1-[(2S,3S)-3-methyl-2-[[(2S)-2-(methylamino)pentanoyl]amino]pentanoyl] pyrrolidine-2-acetate

[Formula 55]

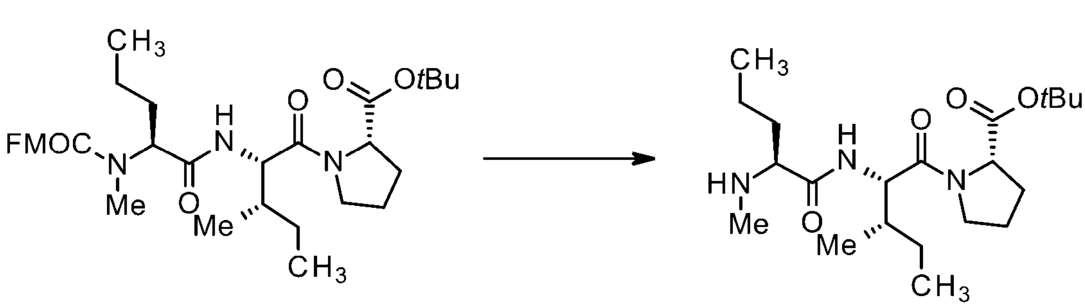

To a reaction vessel after nitrogen replacement, compound 12 (296 mg) synthesized by the same method as in Example 1-11 and toluene (2.07 mL) were added, and the mixture was stirred. DBU (0.072 mL) was added to the mixed solution, and the mixture was stirred for 30 minutes. Acetonitrile (1.00 mL) was added to the mixed solution, and the mixture was then stirred for 30 minutes. DBU (0.072 mL) was added, and the mixture was further stirred for 30 minutes. After addition of 1N hydrochloric acid (2.00 mL) and n-heptane (1.00 mL), an aqueous layer 1 and an organic layer 1 were separated. The organic layer 1 was extracted with 1N hydrochloric acid (1.00 mL) to obtain an aqueous layer 2 containing

compound 13. The aqueous layer 1 and the aqueous layer 2 were combined, extracted with a 5% aqueous potassium carbonate solution (2.00 mL) and toluene (4.00 mL), and the organic layer containing compound 13 was separated. The resulting organic layer was washed with 10% saline (2.00 mL) and then concentrated under reduced pressure to obtain compound 13 (166 mg).
LCMS (ESI) of compound 13: retention time: 1.36 minutes, m/z = 398 $[M+H]^+$ (LCMS analysis conditions method 2)

Example 1-13

Synthesis of compound 14-resin Compound 14: (3S)-4-(dimethylamino)-3-[9H-fluoren-9-ylmethoxycarbonyl(methyl) amino]-4-oxo-butyric acid

[Formula 56]

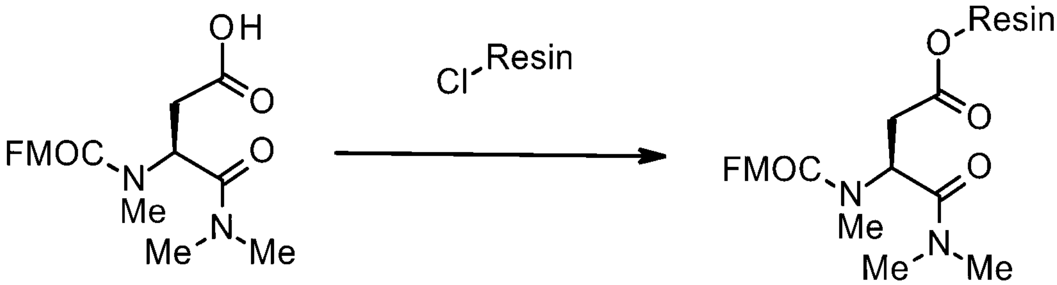

A reaction vessel with a filter (1 L) was charged with 2-chlorotrityl chloride resin (1.12 mmol/g, 70 g, 78.4 mmol) and DCM (560 mL), and let the mixture stands at room temperature for 30 minutes. After filtration of DCM under reduced pressure, a solution of (3S)-4-(dimethylamino)-3-[9H-fluoren-9-ylmethoxycarbonyl(methyl)amino]-4-oxy-butanoic acid (23.4 g, 56.0 mmol) in DCM (140 mL) was added, and another DCM (140 mL) was used for washing. DIPEA (27.4 mL) was added to the reaction vessel, and after stirring for 45 minutes, DCM (140 mL) was added and the mixture was stirred at room temperature for 105 minutes. The reaction solution was filtered under reduced pressure, and followed by washing with DCM (280 mL × 2). A solution of methanol (28.0 mL) and DIPEA (14.0 mL) in DMF (238 mL) was added to the reaction vessel, and the mixture was stirred at room temperature for 120 minutes. After filtration of the reaction solution under reduced pressure, IPA (280 mL) was charged and the mixture was stirred. After 15 minutes, the reaction solution was filtered under reduced pressure, DMF (280 mL) was added, and the mixture was stirred for 15 minutes. After filtration of the reaction solution under reduced pressure, the entire amount of compound 14-resin, excluding the portion for measuring the amount of support, was used to proceed to Example 1-14.
The amount of support of amino acids on the resin was calculated as follows. The obtained compound 14-resin (4.76 mg) was put in a reaction vessel, a 20% Pip/DMF solution (50 mL) was added, and the mixture was shaken at room temperature for 1 hour. The absorbance of the solution (301 nm) was measured (measured using Shimadzu, UV-1600 PC (cell length: 1.0 cm)), and the amount of support on compound 14-resin was calculated to be 0.632 mmol/g.

Example 1-14

Synthesis of compound 15-resin Compound 15: (3S)-3-[[(2S)-2-cyclopentyl-2-[9H-fluoren-9-ylmethoxycarbonyl(methyl)amino]acetyl]-methyl-amino]-4-(dimethylamino)-4-oxo-butyric acid

[Formula 57]

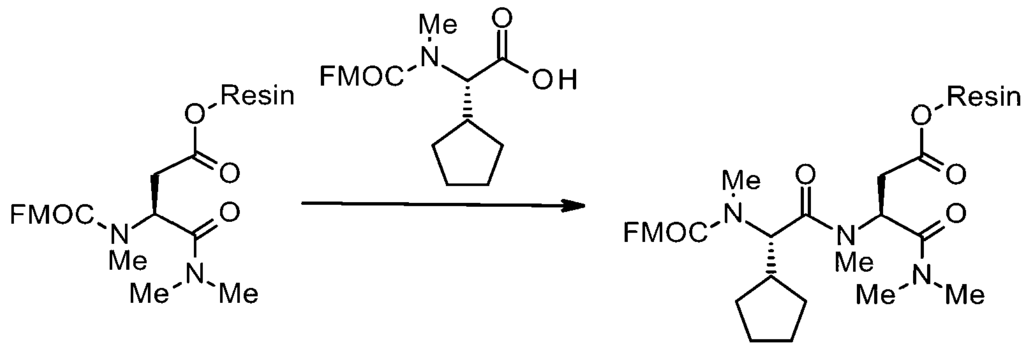

A reaction vessel with a filter (1 L) was charged with compound 14-resin (0.632 mmol/g), to which a 20% Pip/DMF solution (280 mL) was added, and the mixture was stirred at room temperature for 10 minutes to perform the de-Fmoc reaction. The reaction solution was filtered under reduced pressure, a 20% Pip/DMF solution (280 mL) was added again, and the mixture was stirred at room temperature for 10 minutes to perform the de-Fmoc reaction. The reaction mixture was filtered under reduced pressure, and the resin was washed 10 times with DMF (280 mL). The condensation reaction of Fmoc-MeGly(cPent)-OH (Cas No. 187475-29-2) was performed on the resulting resin. The condensation reaction was performed by adding a solution of Fmoc-MeGly(cPent)-OH (Cas No. 187475-29-2, 42.5 g), oxyma (7.96 g), and DIC (34.9 mL) in DMF (280 mL) to the resin, stirring the mixture for 5 minutes, and allowing it to stand still at room temperature for 16 hours. The solution of condensation reaction was filtered under reduced pressure, IPA (280 mL) was charged, and the mixture was stirred. After 10 minutes, the reaction solution was filtered under reduced pressure, DMF (280 mL) was added, and the mixture was stirred for 10 minutes. After filtration of the reaction solution under reduced pressure, the entire amount of compound 15-resin, excluding the portion for measuring the amount of support, was used to proceed to Example 1-15.

The amount of support of amino acids on the resin was calculated as follows. The obtained compound 15-resin (4.91 mg) was put in a reaction vessel, a 20% Pip/DMF solution (50 mL) was added, and the mixture was shaken at room temperature for 1 hour. The absorbance of the solution (301 nm) was measured (measured using Shimadzu, UV-1600 PC (cell length: 1.0 cm)), and the amount of support on compound 15-resin was calculated to be 0.635 mmol/g. Using a small amount of compound 15 supported on the resin, the compound was cut out from the resin with TFE/DCM (1/1), and the structure was confirmed by LC/MS.

LCMS (ESI) of compound 15: retention time: 2.53 minutes, m/z = 536 $[M+H]^+$ (LCMS analysis conditions method 2)

Example 1-15

Synthesis of compound 16-resin Compound 16: (3S)-3-[[(2S)-2-cyclopentyl-2-[[1-[9H-fluoren-9-ylmethoxycarbonyl(methyl)amino]cyclobutanecarbonyl]-methyl-amino]acetyl]-methyl-amino]-4-(dimethylamino)-4-oxo-butyric acid

[Formula 58]

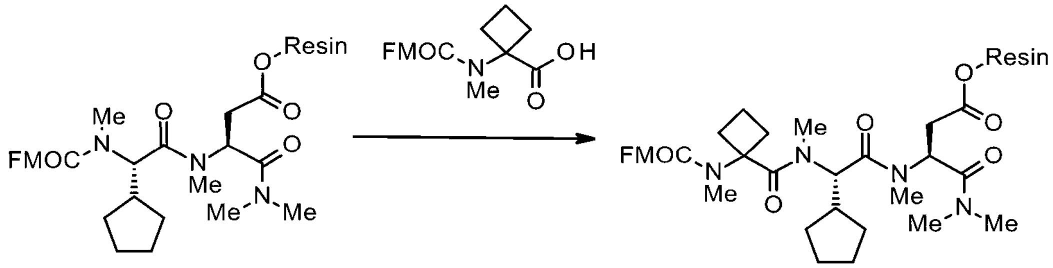

A reaction vessel with a filter (1 L) was charged with compound 15-resin (0.635 mmol/g), to which a 20% Pip/DMF solution (280 mL) was added, and the mixture was stirred at room temperature for 5 minutes to perform the de-Fmoc reaction. After filtration of the reaction solution under reduced pressure, a 20% Pip/DMF solution (280 mL) was added again, and the mixture was stirred at room temperature for 5 minutes to perform the de-Fmoc reaction. The reaction solution was filtered under reduced pressure, and the resin was then washed 10 times with DMF (280 mL). The condensation reaction of Fmoc-MecVal-OH (Cas No. 1700368-07-5) was performed on the resulting resin. The condensation reaction was performed by adding a solution of Fmoc-MecVal-OH (Cas No. 1700368-07-5, 39.4 g) and oxyma

(7.96 g) in DMF (280 mL), then adding DIC (34.9 mL), stirring the mixture for 5 minutes, and then allowing it to stand still for 94 hours. After filtration of the solution of condensation reaction under reduced pressure, the resin was washed with each of DMF (280 mL), IPA (280 mL), and DMF (280 mL). After filtration of the washing solution under reduced pressure, the entire amount of the resulting compound 16-resin, excluding the portion for measuring the amount of support, was used to proceed to Example 1-17.

The amount of support of amino acids on the resin was calculated as follows. The obtained compound 16-resin (5.24 mg) was put in a reaction vessel, a 20% Pip/DMF solution (50 mL) was added, and the mixture was shaken at room temperature for 1 hour. The absorbance of the solution (301 nm) was measured (measured using Shimadzu, UV-1600 PC (cell length: 1.0 cm)), and the amount of support on compound 16-resin was calculated to be 0.513 mmol/g. Using a small amount of compound 16 supported on the resin, the compound was cut out from the resin with TFE/DCM (1/1), and the structure was confirmed by LC/MS.

LCMS (ESI) of compound 16: retention time: 2.75 minutes, m/z = 647 [M+H]+ (LCMS analysis conditions method 2)

Example 1-16

Synthesis of compound 17: 9H-fluoren-9-ylmethyl (2S,4R)-2-chlorocarbonyl-4-ethoxy-pyrrolidine-1-carboxylate

[Formula 59]

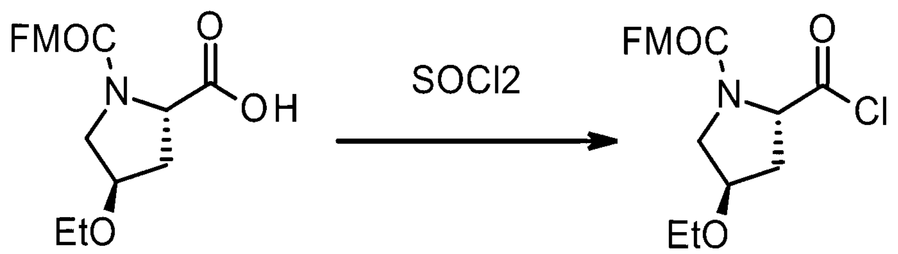


To a reaction vessel after nitrogen replacement, (2S,4R)-4-ethoxy-1-(9H-fluoren-9-ylmethoxycarbonyl)pyrrolidine-2-carboxylic acid (Cas No. 1446478-31-4, 42.7 g), toluene (128 mL), and thionyl chloride (12.3 mL) were sequentially added at room temperature. After stirring at an external temperature of 60°C for 0.5 hours, the mixture was stirred at an external temperature of 55°C for 3 hours. Part of the reaction mixture was sampled and diluted in MeOH, allowed to stand still for 5 minutes to be converted to the corresponding compound 18, and the reaction conversion rate to compound 17 was thus confirmed to proceed at 99.9% by HPLC analysis (calculation expression 1 for the reaction conversion rate). The external temperature of the reaction vessel was set to 40°C, and the reaction solution was concentrated under reduced pressure. After the concentration under reduced pressure, the operation of adding DCM (214 mL) and concentrating under reduced pressure was repeated twice. The resulting concentrated solution was dried under reduced pressure overnight to obtain a crude product containing compound 17 (45.5 g, 95% yield).

Compound 18: O1-(9H-fluoren-9-ylmethyl) O2-methyl (2S,4R)-4-ethoxypyrrolidine-1,2-dicarboxylate

[Formula 60]

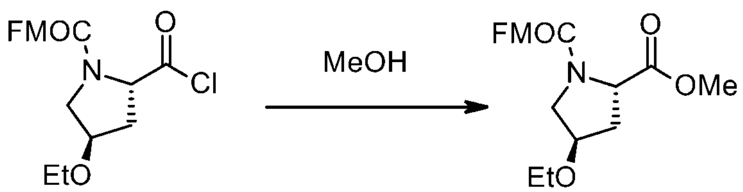


LCMS (ESI) of compound 18: retention time: 2.68 minutes, m/z = 396 [M+H]+ (LCMS analysis conditions method 2)

Example 1-17

Synthesis of compound 19-resin Compound 19: (3S)-3-[[(2S)-2-cyclopentyl-2-[[1-[[(2S,4R)-4-ethoxy-1-(9H-fluoren-9-ylmethoxycarbonyl)pyrrolidine-2-carbonyl]-methyl-amino]cyclobutanecarbonyl]-methyl-amino]acetyl]-methyl-amino]-4-(dimethylamino)-4-oxo-butyric acid

[Formula 61]

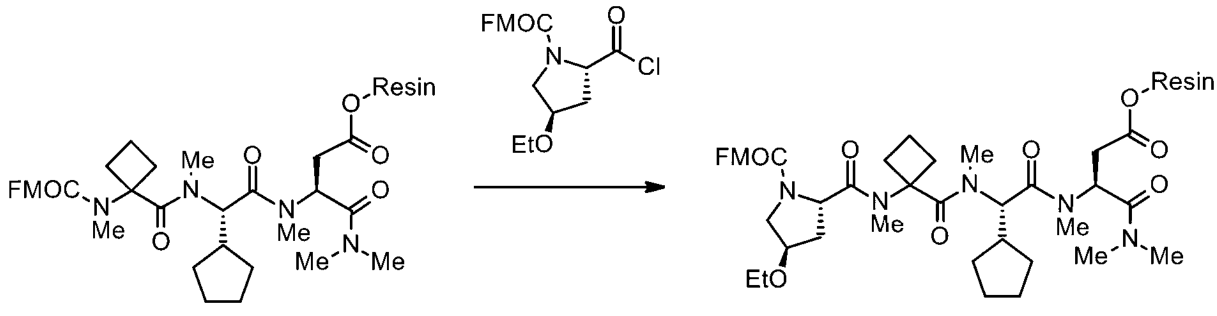

A reaction vessel with a filter (1 L) was charged with compound 16-resin (0.513 mmol/g), and swelling with DMF (280 mL) for 15 minutes and solution discharging were performed twice. To the swollen resin, a 20% Pip/DMF solution (280 mL) was added, and the mixture was stirred at room temperature for 15 minutes to perform the de-Fmoc reaction. After filtration of the reaction solution under reduced pressure, a 20% Pip/DMF solution (280 mL) was added again, and the mixture was stirred at room temperature for 15 minutes to perform the de-Fmoc reaction. The reaction solution was filtered under reduced pressure, and the resin was then washed 13 times with DCM (280 mL). The condensation reaction of compound 17 was performed on the resulting resin. The condensation reaction was performed by sequentially adding a DCM solution (280 mL) of compound 17 (46.0 g) obtained in Example 1-16 and collidine (74.0 mL), stirring the mixture for 5 minutes, and allowing it to stand still at room temperature for 4 hours. After filtration of the solution of condensation reaction under reduced pressure, the resin was washed with DCM (280 mL), and the entire amount of the resulting compound 19-resin, excluding the portion for measuring the amount of support, was used to proceed to Example 1-18.

The amount of support of amino acids on the resin was calculated as follows. The obtained compound 17-resin (7.27 mg) was put in a reaction vessel, a 20% Pip/DMF solution (50 mL) was added, and the mixture was shaken at room temperature for 1 hour. The absorbance of the solution (301 nm) was measured (measured using Shimadzu, UV-1600 PC (cell length: 1.0 cm)), and the amount of support on compound 19-resin was calculated to be 0.473 mmol/g. Using a small amount of compound 19 supported on the resin, the compound was cut out from the resin with TFE/DCM (1/1), and the structure was confirmed by LC/MS.

LCMS (ESI) of compound 17: retention time: 2.68 minutes, m/z = 788 $[M+H]^+$ (LCMS analysis conditions method 2)

Example 1-18

Synthesis of compound 20-resin Compound 20: (3S)-3-[[(2S)-2-[[1-[[(2S,4R)-1-[(2S)-2-(benzyloxycarbonylamino)-4-[3-methoxy-4-(trifluoromethyl)phenyl]butanoyl]-4-ethoxy-pyrrolidine-2-carbonyll-methyl-amino]cyclobutanecarbonyl]-methyl-amino]-2-cyclopentyl-acetyl]-methyl-amino]-4-(dimethylamino)-4-oxo-butyric acid

[Formula 62]

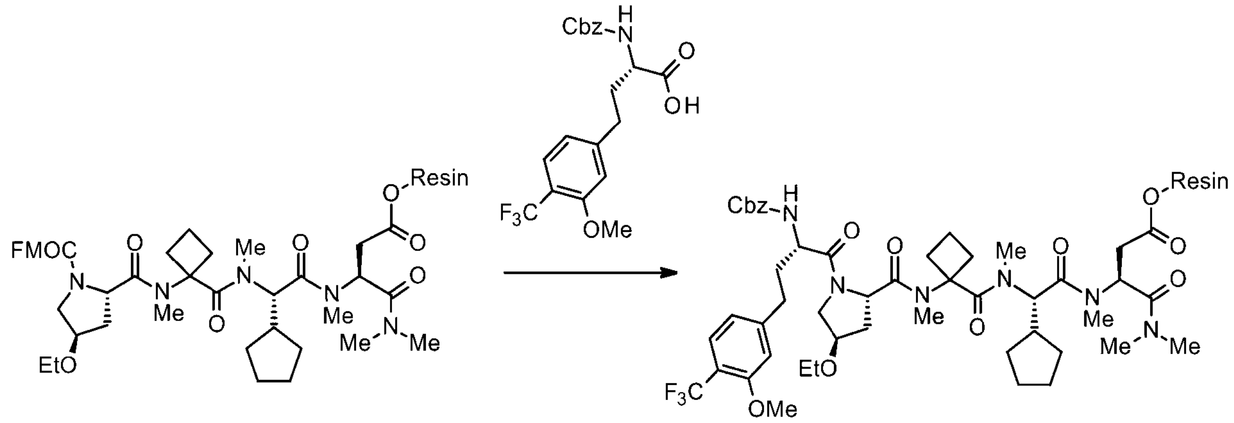

A reaction vessel with a filter (1 L) was charged with compound 19-resin (0.473 mmol/g), and swelling with DMF (280 mL) for 15 minutes and solution discharging were performed twice. To the swollen resin, a 20% Pip/DMF solution (280 mL) was added, and the mixture was shaken at room temperature for 15 minutes to perform the de-Fmoc reaction. After filtration of the reaction solution under reduced pressure, a 20% Pip/DMF solution (280 mL) was added again, and the mixture was shaken at room temperature for 15 minutes to perform the de-Fmoc reaction. The reaction solution was filtered under reduced pressure, and the resin was then washed 13 times with DMF (280 mL). The condensation reaction of Cbz-Hph(4-CF3-3-OMe)-OH was performed on the resulting resin. The condensation reaction was performed by adding a solution of Cbz-Hph(4-CF3-3-OMe)-OH (46.1 g) and oxyma (7.96 g) in DMF (280 mL) and DIC (34.9 mL) to the resin, stirring the mixture for 5 minutes, and then allowing it to stand still for 3.5 hours. The solution of condensation reaction was filtered under reduced pressure, and the resin was then washed twice with DMF (280 mL). IPA (280 mL) was added, and the mixture was then stirred for 15 minutes. After discharging the solution, DCM (280 mL) was added and the mixture was stirred for 15 minutes. Washing with IPA (280 mL) and DCM (280 mL) and solution discharging were repeated again. After washing with IPA (280 mL × 3), the resin was dried under reduced pressure. After drying under reduced pressure, the resulting compound 20-resin was 122 g.

Using a small amount of compound 20 supported on the resin, the compound was cut out from the resin with TFE/DCM (1/1), and the structure was confirmed by LC/MS.

LCMS (ESI) of compound 20: retention time: 2.91 minutes, m/z = 960 $[M+H]^+$ (LCMS analysis conditions method 2)

Example 1-19

Synthesis of compound 20

[Formula 63]

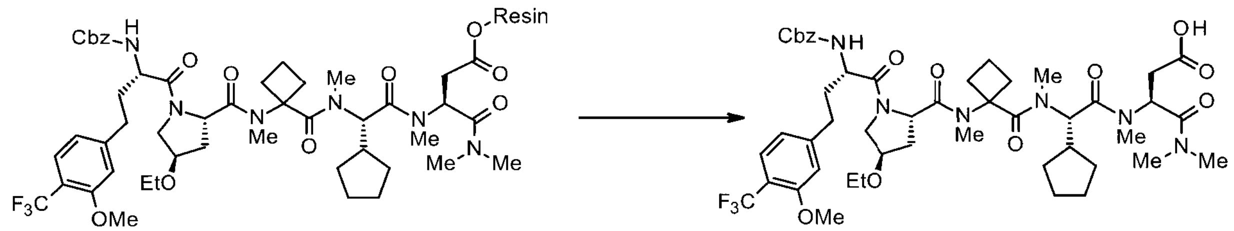

To a reaction vessel after nitrogen replacement, compound 20-resin (60.0 g) obtained by the same method for compound 20-resin (122 g) obtained in Example 1-18, and 2-MeTHF (1.10 L) were sequentially added at room temperature. The external temperature of the reaction vessel was set to 0°C, and hexamethyldisilazane (77.3 mL) was added. After stirring for 20 minutes, trimethylsilyl trifluoromethanesulfonate (50.0 mL) was added such that the internal temperature did not exceed 7°C. 20 minutes after the completion of addition, the reaction mixture was filtered under reduced pressure, and the residue was washed with 2-MeTHF (364 mL × 3). The filtrate was washed with a 5% aqueous disodium hydrogen phosphate solution (1.10 L), a 5% aqueous potassium hydrogen sulfate solution (1.10 L), and a 5% aqueous sodium chloride solution (1.10 L). The resulting organic layer was concentrated under reduced pressure to

obtain 77.6 g of a crude product. Part of the obtained crude product and 3,5-bis(trifluoromethyl)benzoic acid were dissolved in $CDCl_3$ and subjected to qNMR analysis. After content correction, compound 20 was calculated to be contained in 69.0 g.

LCMS (ESI) of compound 20: retention time: 3.90 minutes, m/z = 960 $[M+H]^+$ (LCMS analysis conditions method 3)

Example 1-20

Synthesis of compound 21: tert-butyl (2S)-1-[(2S,3S)-2-[[(2S)-2-[[(3S)-3-[[(2S)-2-[[1-[[(2S,4R)-1-[(2S)-2-benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoyl]-methyl-amino] pentanoyl]amino]-3-methyl-pentanoyl]pyrrolidine-2-carboxylate

[Formula 64]

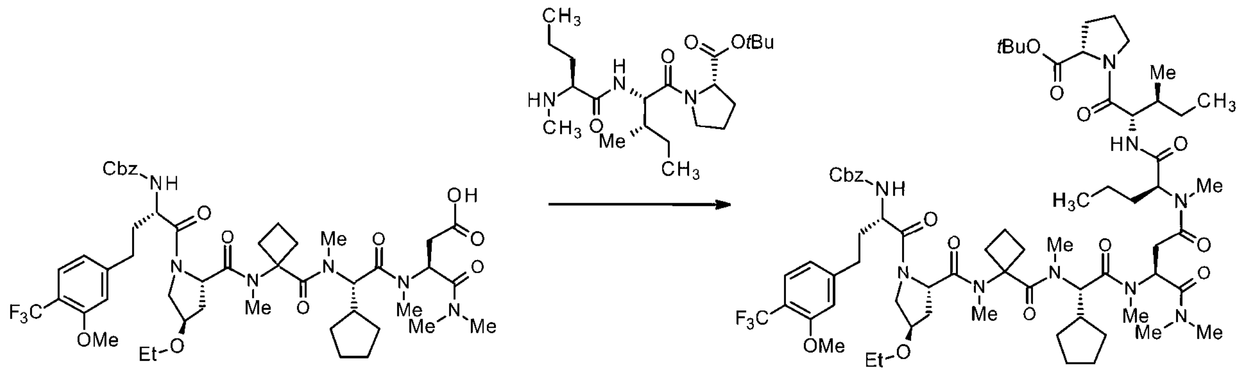

The crude product containing compound 20 (80.7 g) and compound 13 (45.8 g) were added to a reaction vessel. After nitrogen replacement, 2-MeTHF (484 mL), DIPEA (64.5 mL), and DMF (121 mL) were added at room temperature, and the mixture was stirred. To the resulting solution was added HATU (48.0 g) at room temperature. After 1 hour, the reaction mixture was sampled for sample preparation (sample preparation method 2), and the reaction conversion rate was confirmed to be 98% or more by HPLC analysis (calculation expression 1 for the reaction conversion rate). The external temperature of the reaction vessel was set to 5°C, and a 2.5% aqueous ammonia solution (484 mL) was added to the reaction mixture. After discharging the aqueous layer by a liquid separation operation, the organic layer was washed with a 10% aqueous sodium hydrogen sulfate solution (484 mL), 5% aqueous sodium carbonate solution (484 mL) and 5% saline (484 mL). The resulting organic layer was concentrated under reduced pressure with the external temperature set to 40°C. To the resulting concentrated mixture, 2-MeTHF (161 mL $\times$ 2) was added, and concentration under reduced pressure was repeated to obtain 134 g of a crude product. The yield of the crude product was not calculated, and it was used for Example 1-21.

LCMS (ESI) of compound 21: retention time: 5.02 minutes, m/z = 1340 $[M+H]^+$ (LCMS analysis conditions method 3)

Example 1-21

Synthesis of compound 22: (2S)-1-[(2S,3S)-2-[[(2S)-2-[[(3S)-3-[[(2S)-2-[[1-[[(2S,4R)-1-[(2S)-2-(benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentylacetyl]-methyl-amino]-4-(dimethylamino)-4-oxo-butanoyl]-methyl-amino]pentanoyl] amino]-3-methyl-pentanoyl]pyrrolidine-2-carboxylic acid

[Formula 65]

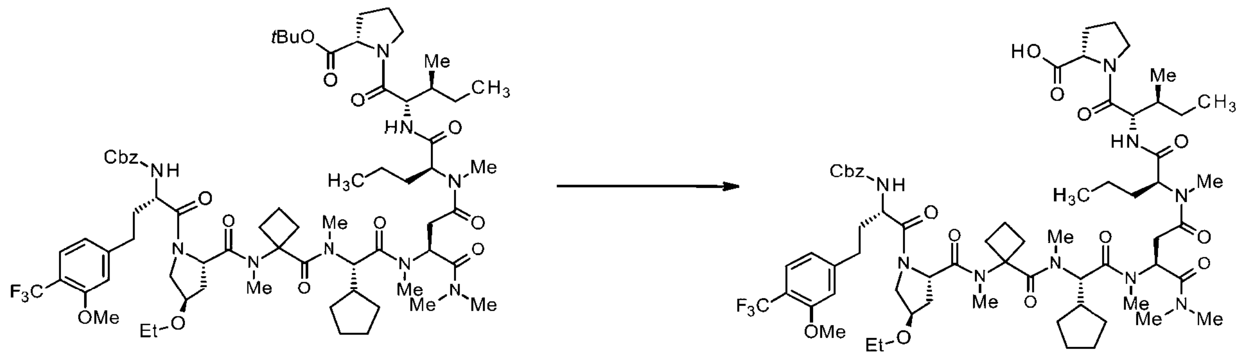

To a reaction vessel after nitrogen replacement, a solution of the crude product containing compound 19 of Example 1-20 (111 g) in 2-MeTHF (666 mL) was added hexamethyldisilazane (69.5 mL) and then trimethylsilyl trifluoromethanesulfonate (44.9 mL) in this order such that the internal temperature did not exceed 15°C. After the completion of addition, the external temperature was raised to 10°C and the mixture was stirred for 1 hour and 20 minutes. The reaction mixture was sampled for sample preparation (sample preparation method 2), and the reaction conversion rate to compound 22 was confirmed to be 99% by HPLC analysis (calculation expression 1 for the reaction conversion rate). At an external temperature of 0°C, a mixed solution of a 5% aqueous sodium carbonate solution (555 mL) and 5% saline (333 mL) was added such that the internal temperature did not exceed 25°C. After the completion of addition, the aqueous layer was discharged by a liquid separation operation. The resulting organic layer was washed with a 10% aqueous sodium hydrogen sulfate solution (555 mL), a 5% aqueous sodium carbonate solution (555 mL), and 5% saline (555 mL). The resulting organic layer was concentrated under reduced pressure at an external temperature of 40°C. To the resulting concentrated product, 2-MeTHF (222 mL × 2) was added, and concentration under reduced pressure was repeated to obtain 128 g of crude product. The obtained crude product and 3,5-bis(trifluoromethyl)benzoic acid were dissolved in $CDCl_3$ and subjected to qNMR analysis, and as a result, compound 22 was calculated to be contained in 96.1 g.

LCMS (ESI) of compound 22: retention time: 4.24 minutes, m/z = 1283 $[M+H]^+$ (LCMS analysis conditions method 3)

Example 1-22

Compound 23: tert-butyl 2-[[(2S)-2-[(4Z,7S)-7-[[(2S)-1-[(2S,3S)-2-[[(2S)-2-[[(3S)-3-[[(2S)-2-[[1-[[(2S,4R)-1-[(2S)-2-(benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoyl]-methyl-amino]pentanoyl]amino]-3-methyl-pentanoyl]pyrrolidine-2-carbonyl]-methyl-amino]-8-oxo-2,3,6,7-tetrahydroazocin-1-yl]-3-[4-(trifluoromethyl)phenyl]propanoyl]-methyl-amino]acetate

[Formula 66]

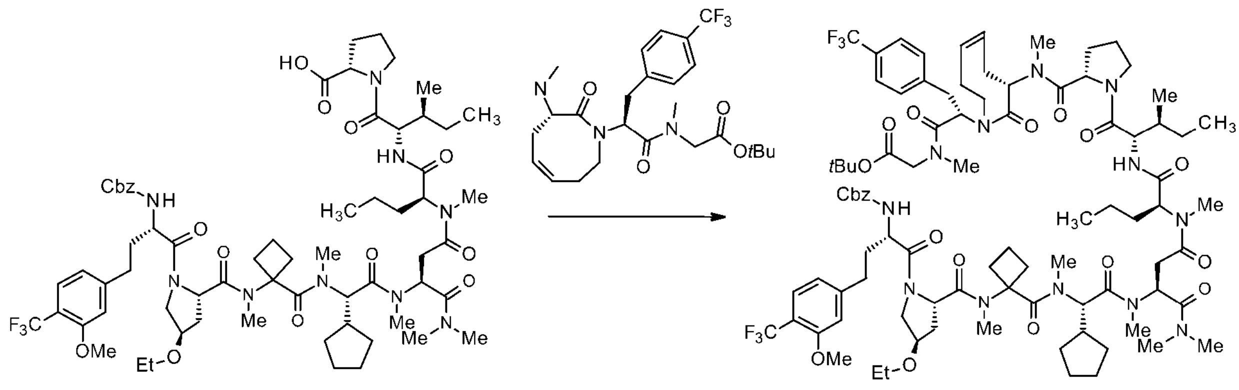

To a reaction vessel, the crude product containing compound 22 (94.0 g) of Example 1-21 and compound 9 (40.1

g) were added, and after nitrogen replacement, 2-MeTHF (564 mL) and DMF (141 mL) were added and the mixture was stirred at room temperature. DIPEA (56.2 mL) was added and the mixture was stirred at an external temperature of 43°C for 1 hour. After cooling to an external temperature of 33°C, HATU (41.8 g) was added to the resulting solution. After 1 hour, the mixture was cooled to room temperature and stirred for 6 hours. The reaction mixture was sampled for sample preparation (sample preparation method 2), and the reaction conversion rate was confirmed to be 98% or more by HPLC analysis (calculation expression 1 for the reaction conversion rate). The solution was washed with a 2.8% aqueous ammonia solution (564 mL) at room temperature. The organic layer was washed with a 10% aqueous sodium hydrogen sulfate solution (564 mL), a 5% aqueous sodium carbonate solution (564 mL) and 5% saline (564 mL). The resulting solution was concentrated under reduced pressure at an external temperature of 40°C to obtain 157 g of a crude product. The obtained crude product containing compound 23 and 3,5-bis(trifluoromethyl)benzoic acid were dissolved in $CDCl_3$ and subjected to qNMR analysis, and as a result, compound 23 was calculated to be contained in 127.8 g (98.9% yield).
LCMS (ESI) of compound 23: retention time: 5.34 minutes, m/z = 1763 $[M+H]^+$ (LCMS analysis conditions method 3)

Example 1-23

Synthesis of compound 24: 2-[[(2S)-2-[(4Z,7S)-7-[[(2S)-1-[(2S,3S)-2-[[(2S)-2-[[(3S)-3-[[(2S)-2-[[1-[[(2S,4R)-1-[(2S)-2-amino-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoyl]-methyl-amino]pentanoyl]amino]-3-methyl-pentanoyl]pyrrolidine-2-carbonyl]-methyl-amino]-8-oxo-2,3,6,7-tetrahydroazocin-1-yl]-3-[4-(trifluoromethyl)phenyl]propanoyl]-methyl-amino]acetic acid

[Formula 67]

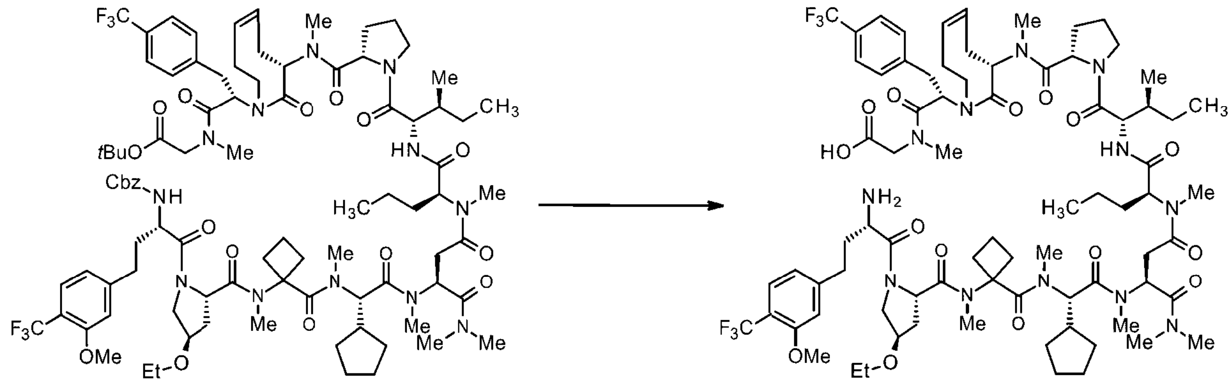

To a reaction vessel, the crude product containing compound 23 (90.8 g), L-cysteine (6.3 g), and 2-MeTHF (363 mL) were sequentially added. After nitrogen replacement in the reaction vessel, the external temperature was set to 20°C. Hexamethyldisilazane (119 mL) was added while stirring. Subsequently, trimethylsilyl trifluoromethanesulfonate (93.5 mL) was added over 20 minutes. After 25 minutes, the mixture was heated to an internal temperature of 50°C. After stirring for 8 hours at that temperature, the reaction mixture was cooled to room temperature and stored overnight. The mixture was heated to the internal temperature to 50°C again, and the reaction solution was stirred at 50°C for 4 hours. After cooling to an external temperature of 0°C, a 5% aqueous sodium carbonate solution (272 mL) was slowly added dropwise such that the internal temperature did not exceed 40°C. Thereafter, the aqueous layer containing compound 24 was set aside by a liquid separation operation. To the aqueous layer that had been set aside, 2-MeTHF (727 mL), MeCN (182 mL), and 10% sodium hydrogen sulfate (545 mL) were added, and a liquid separation operation was performed. After discharging the aqueous layer, the organic layer was washed with a 5% aqueous disodium hydrogen phosphate solution (545 mL × 2) and a 10% aqueous sodium chloride solution (273 mL × 2). The obtained organic layer was concentrated under reduced pressure with the external temperature set to 40°C. To the resulting concentrated product, 2-MeTHF (182 mL × 2) was added, and concentration under reduced pressure was repeated to obtain 115 g of crude product. The crude product containing compound 24 and 3,5-bis(trifluoromethyl)benzoic acid were dissolved in DMSO-$d_6$ and subjected to qNMR analysis, and as a result, compound 24 was calculated to be contained in 68.2 g (83.9% yield).
LCMS (ESI) of compound 24: retention time: 4.37 minutes, m/z = 1572 $[M+H]^+$ (LCMS analysis conditions method 4)
The deprotection reaction for a Cbz group is generally performed under catalytic hydrogen reduction conditions in the presence of a metal catalyst, exemplified by palladium/carbon. However, when applying the conditions of the

conventional method of catalytic hydrogen reduction to the deprotection reaction for the Cbz group in compound 23, which has an olefin in the molecule, there is a problem that the olefin in the molecule is reduced. To address this problem, the present inventors have found a method by which deprotection for the Cbz group can be achieved without reducing the intramolecular olefin under the conditions described in Example 1-23, that is, TMSOTf/HMDS conditions.

Example 1-24-1

Synthesis of compound 1: (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.0$^{4,8}$.0$^{26,30}$]pentatetracont-42-ene-23,1'-cyclobutane]-17-carboxamide (cyclization position B)

[Formula 68]

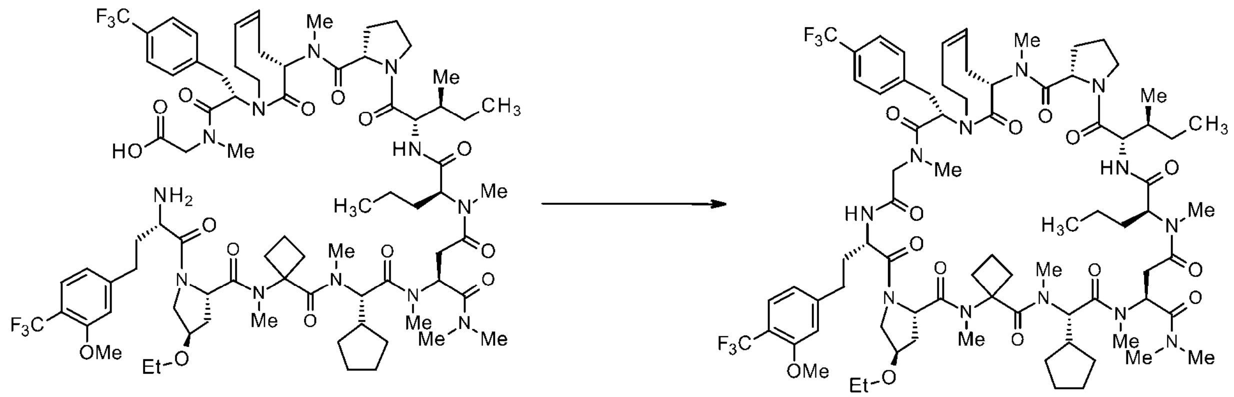

To a reaction vessel after nitrogen replacement, a solution of COMU (65.0 g) in acetonitrile (1.9 L) was cooled to 0°C. To the resulting solution was slowly added a solution of compound 24 (62.7 g) and Lutidine (21.3 mL) in acetonitrile (0.941 L) dropwise manner (4.4 mL/min). Immediately after the completion of addition, the reaction mixture was sampled for sample preparation (sample preparation method 2), and it was confirmed that the reaction conversion rate was 99% (calculation expression 1 for the reaction conversion rate) and compound 1/cyclic dimer = 98/2 (calculation expressions 1 and 2 for cyclization selectivity) by HPLC analysis. The external temperature of the reaction vessel was heated to 40°C and the reaction solution was concentrated. The external temperature of the reaction vessel was cooled to 25°C, and to the resulting concentrated product, isopropyl acetate (627 mL) and a 2.5% aqueous ammonia solution (627 mL) were added and the mixture was stirred. After discharging the aqueous layer by liquid-liquid separation, the resulting organic layer was washed with a 10% aqueous sodium hydrogen sulfate solution (627 mL), a 5% aqueous disodium hydrogen phosphate solution (627 mL × 2) and a 5% aqueous sodium chloride solution (627 mL). The resulting organic layer was washed with a 0.5% aqueous sodium chloride solution (627 mL × 2). The external temperature was set to 40°C, and the resulting organic layer was concentrated under reduced pressure to obtain 92.23 g of a crude product containing compound 1. The obtained crude product containing compound 1 was used in Example 1-25.

Cyclic dimer:

[Formula 69]

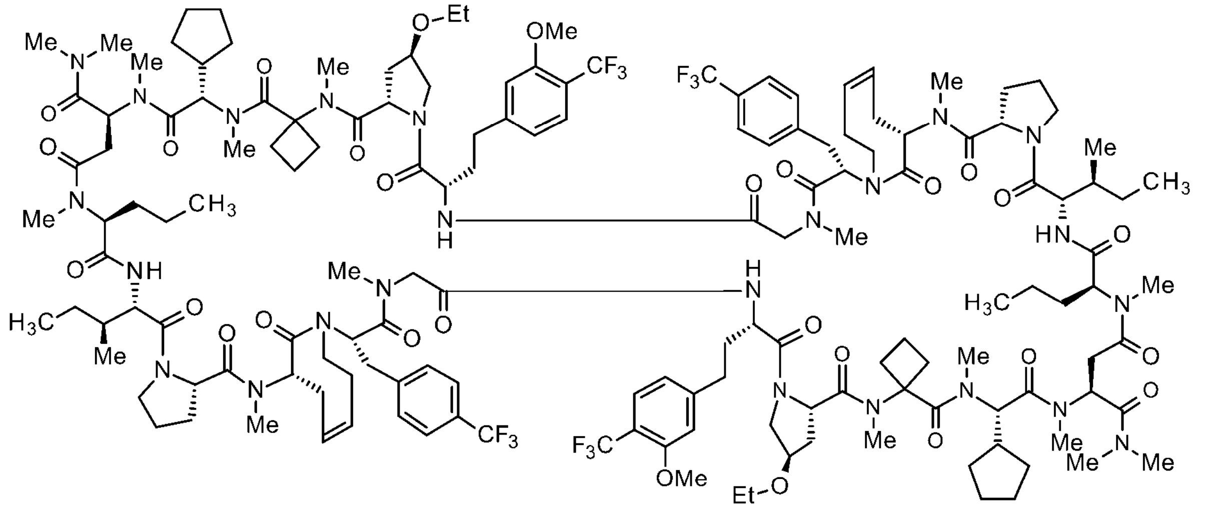

LCMS (ESI) of compound 1: retention time: 6.07 minutes, m/z = 1555 $[M+H]^+$ (LCMS analysis conditions method 4)
LCMS (ESI) of cyclic dimer: retention time: 7.63 minutes, m/z = 1555 $[M+H]^{2+}$ (LCMS analysis conditions method 4)
HPLC of compound 1: retention time: 4.09 minutes (HPLC analysis conditions method 1)
HPLC of cyclic dimer: retention time: 4.91 minutes (HPLC analysis conditions method 1)

Example 1-24-2

Synthesis of compound 1: (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.$0^{4,8}$.$0^{26,30}$]pentatetracont-42-ene-23,1'-cyclobutane]-17-carboxamide (cyclization position B)

[Formula 70]

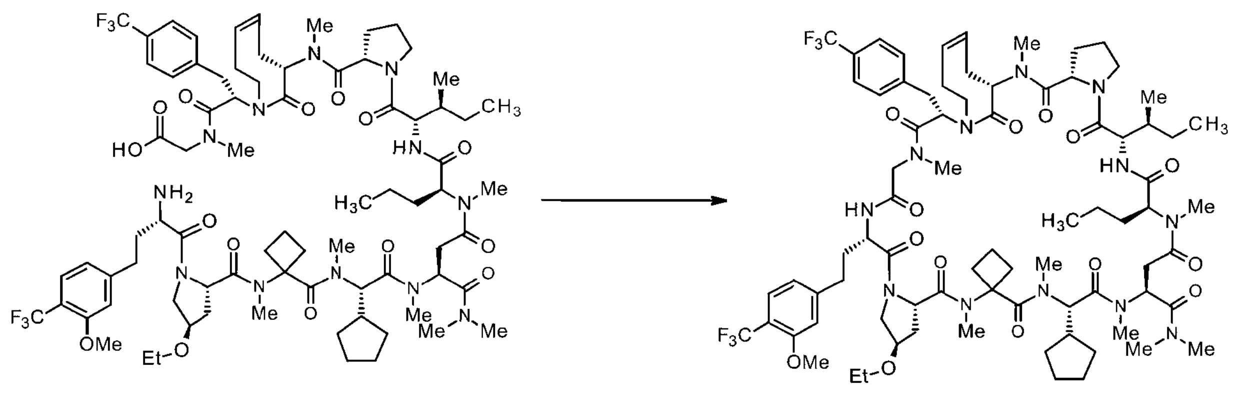

To a reaction vessel, HATU (72.6 mg) and acetonitrile (1.80 mL) were added. A solution of the crude product containing compound 24 (100 mg) of Example 1-23 and DIPEA (40 μL) in acetonitrile (5.5 mL) was added dropwise over 5 hours and 42 minutes. Immediately after the completion of addition, the reaction mixture was sampled for sample preparation (sample preparation method 2), and it was confirmed that the reaction conversion rate was 99% (calculation expression 1 for the reaction conversion rate) and compound 1/cyclic dimer = 98/2 (calculation expressions 1 and 2 for cyclization selectivity) by HPLC analysis. HPLC of compound 1: retention time: 3.99 minutes (HPLC analysis conditions

method 1) HPLC of cyclic dimer: retention time: 4.80 minutes (HPLC analysis conditions method 1)

Example 1-25

Crystallization of compound 1: synthesis of hydrate crystal (form A) of (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo [37.5.1.0$^{4,8}$.0$^{26,30}$]pentatetracont-42-ene-23,1'-cyclobutane]-17-carboxamide

24 g of the concentrated dry product containing compound 1 obtained in Example 1-24-1 was loaded into Biotage Sfar C18 Duo 100 Å 30 μm 240 g, developed with 0.1% formic acid-water/0.1% formic acid-acetonitrile = 90/10 → 18/82), and purified. To the resulting compound 1 (9.70 g), acetone (29.0 mL) and heptane (29.0 mL) were added at an external temperature of 35°C. After confirming dissolution, an acetone/heptane/water solvate crystal of compound 1 (form F) (about 1.00 mg), obtained by the same operation as in Example 3-8, was added to the reaction vessel, and the mixture was stirred at 35°C for 23 hours. The mixture was cooled to 25°C and further stirred for 6 hours. Heptane (4.90 mL) was added over 1 hour, and the mixture was stirred at 25°C for 14 hours. Heptane (4.90 mL) was further added over 1 hour, and the mixture was stirred for 3 hours. Finally, heptane (4.90 mL) was added over 2 hours, and the mixture was stirred for 3 hours. The reaction mixture was filtered under reduced pressure, and the resulting crystal was washed with a mixed solution of acetone (7.76 mL) and heptane (11.6 mL). The resulting crystal was dried for 16 hours with the external temperature set to 40°C. The dried powder was collected to obtain a white powder (6.8 g, form A).

Example 1-26

Synthesis of compound 1: (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro [2,8,11,14,18,21,24,30,33,36,39-undecazatetracyclo[37.5.1.0$^{4,8}$.0$^{26,30}$]pentatetracont-42-ene-23,1'-cyclobutane]-17-carboxamide (cyclization position A)

[Formula 71]

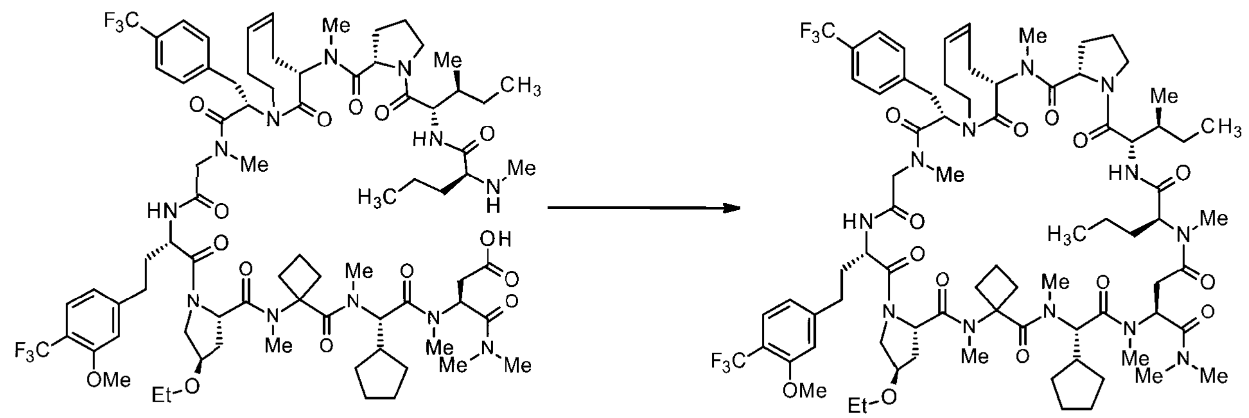

To a reaction vessel after nitrogen replacement, HATU (6.94 g) and acetonitrile (180 mL) were added. After confirming the dissolution of HATU, a solution containing compound 25 (9.56 g) and DIPEA (3.82 mL) in acetonitrile (540 mL) was added dropwise over 6 hours. Immediately after the completion of addition, 10 μL of the reaction mixture was sampled and diluted in 0.1 mL of acetonitrile containing 2 μL of ethanolamine. LCMS analysis confirmed that compound 1/cyclic dimer = 75/25 (calculation expressions 1 and 2 for cyclization selectivity).

LCMS (ESI) of compound 1: retention time: 0.78 minutes, m/z = 1553 $[M-H]^-$ (LCMS analysis conditions method 5)
LCMS (ESI) of cyclic dimer: retention time: 0.94 minutes, m/z = 1553 $[M-H]^{2-}$ (LCMS analysis conditions method 5)

Chemical name and structural formula of compound 25:

(3S)-3-[[(2S)-2-cyclopentyl-2[[1-[[(2S,4R)-4-ethoxy-1-[(2S)-4-[3-methoxy-4-(trifluoromethyl)phenyl]-2-[[2-[methyl-[(2S)-2-[(4Z,7S)-7-[methyl-[(2S)-1-[(2S,3S)-3-methyl-2-[[(2S)-2-(methylamino)pentanoyl]amino]pentanoyl]pyrrolidine-2-carbonyl]amino]-8-oxo-2,3,6,7-tetrahydroazocin-1-yl]-3-[4-(trifluoromethyl)phenyl]propanoyl]amino]acetyl]amino]butanoyl]pyrrolidine-2-carbonyl]-methyl-amino]cyclobutanecarbonyl]-methyl-amino]acetyl]-methyl-amino]-4-(dimethylamino)-4-oxo-butyric acid

[Formula 72]

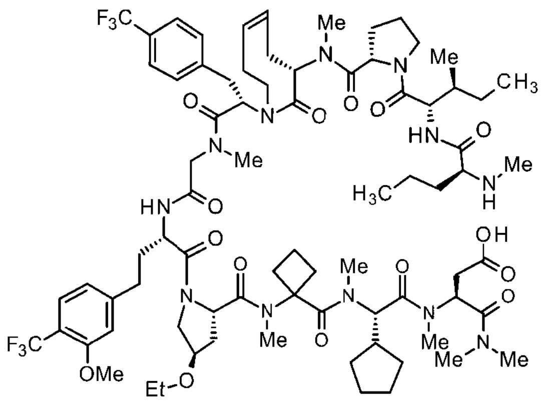

Example 2-1

Compound 26: (S)-2-(but-3-en-1-ylamino)-3-(4-(trifluoromethyl)phenyl)propionic acid

[Formula 73]

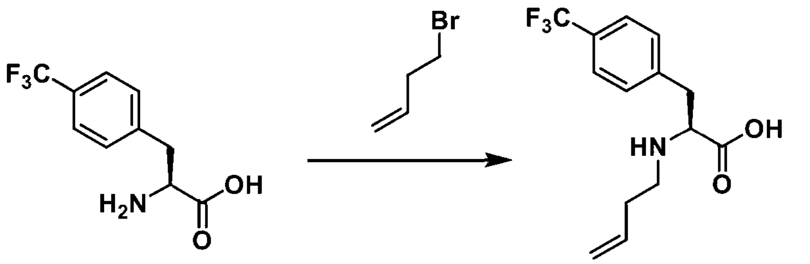

To a reaction vessel after nitrogen replacement, acetonitrile (822 mL), 4-bromo-1-butene (235.07 g), and triethylamine (17.66 g) were added at an external temperature of 25°C, and the mixture was stirred for 1 hour. Subsequently, (S)-2-amino-3-(4-(trifluoromethyl)phenyl)propionic acid (135.46 g), water (676 mL), an d triethylamine (158.19 g) were added, the external temperature was raised to 70°C, and the mixture was stirred for 3.5 hours. Cooled to 25°C, the precipitated solid was filtered through Kiriyama funnel and washed with a mixture of acetonitrile and water (1:1, 676 mL). Subsequently, the solid was further washed with acetonitrile (676 mL). The resulting wet powder was dried with the external temperature set to 40°C. The dried powder was collected to obtain a white solid (124.73 g).
LCMS (ESI) of compound 26: retention time: 2.34 minutes, m/z = 288 $[M+H]^+$ (LCMS analysis conditions method P3-4)

Example 2-2

Compound 27: tert-butyl (S)-N-(2-(but-3-en-1-ylamino)-3-(4-(trifluoromethyl)phenyl)propanoyl)-N-methylglycinate hydrochloride

[Formula 74]

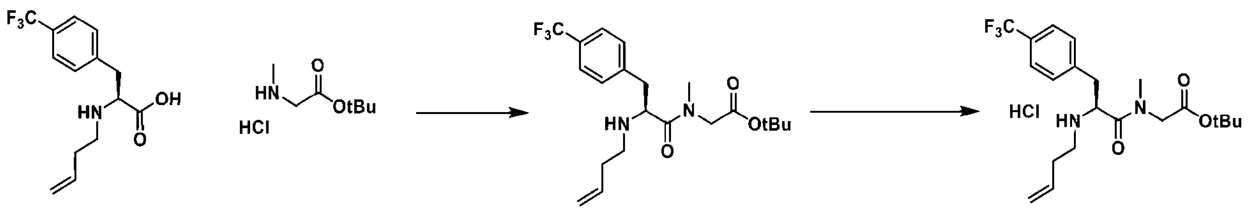

To a reaction vessel after nitrogen replacement, (S)-2-(but-3-en-1-ylamino)-3-(4-(trifluoromethyl)phenyl)propionic acid (107.26 g), sarcosine tert-butyl ester hydrochloride (102.00 g), acetonitrile (751 mL), and diazabicycloundecene (233.03 g) were added at an external temperature of 25°C, and the mixture was stirred for 10 minutes. After confirming that the solution was homogeneous, the external temperature was set to 2°C and a 50% solution of propanephosphonic acid anhydride in 2-methyltetrahydrofuran (309.03 g) was added dropwise over 2 hours and 14 minutes. After confirming that the reaction was completed, toluene (751 mL) and a 1N aqueous NaOH solution (536 mL) were added to the reaction mixture, which was stirred for 30 minutes, and the aqueous layer was discharged by liquid-liquid separation. The organic layer was stored at room temperature overnight. After storage, a 5% aqueous sodium carbonate solution (536 mL) was added to the organic layer, which was stirred for 10 minutes, and the aqueous layer was then discharged by liquid-liquid separation. Subsequently, a 5% aqueous sodium dihydrogen phosphate solution (751 mL) was added to the organic layer, which was stirred for 10 minutes, and the aqueous layer was then discharged by liquid-liquid separation. Furthermore, a 5% aqueous sodium dihydrogen phosphate solution (751 mL) was added to the organic layer once more, the mixture was stirred for 10 minutes, and the aqueous layer was then discharged by liquid-liquid separation. Subsequently, 5% saline (751 mL) was added to the organic layer, which was stirred for 10 minutes, and the aqueous layer was then discharged by liquid-liquid separation. The organic layer was stored at an external temperature of 5°C overnight. After storage, the organic layer was concentrated under reduced pressure at 40°C to about 215 mL. Toluene (215 mL) was added to the concentrated solution, the mixture was concentrated under reduced pressure at 40°C to about 215 mL, and this operation was repeated twice more. The precipitated inorganic salt was filtered, the target material and toluene in the resulting filtrate were quantified, and toluene was added such that it reached 296 mL. In another reaction vessel, pyridine hydrochloride (43.28 g) and acetonitrile (148 mL) were added, and the prepared solution was added dropwise over 45 minutes to the solution of the target material in toluene at an external temperature of 25°C. Precipitation of a crystal was confirmed during the dropwise addition. Furthermore, rinsing with acetonitrile (74 mL) was performed and the mixture was stirred for 1 hour. Thereafter, toluene (1.7 L) was added, the mixture was stirred for 1 hour, and then the external temperature was lowered to 0°C and the mixture was further stirred for 2 hours. The resulting crystal was filtered through Kiriyama funnel and washed twice with toluene (296 mL), which was cooled to 0°C. The resulting wet powder was stored at an external temperature of 5°C for a weekend. After storage, the wet powder was dried with the external temperature set to 40°C. The dried powder was collected to obtain a white solid (98.66 g).
LCMS (ESI) of compound 27: retention time: 3.03 minutes, m/z = 415 $[M+H]^+$ (LCMS analysis conditions method P3-4)

Example 2-3

Compound 28: 2-[[(2S)-2-[(4Z,7S)-7-[9H-fluoren-9-ylmethoxycarbonyl(methyl)amino]-8-oxo-2,3,6,7-tetrahydroazocin-1-yl]-3-[4-(trifluoromethyl)phenyl]propanyl]- methyl-amino]acetic acid

[Formula 75]

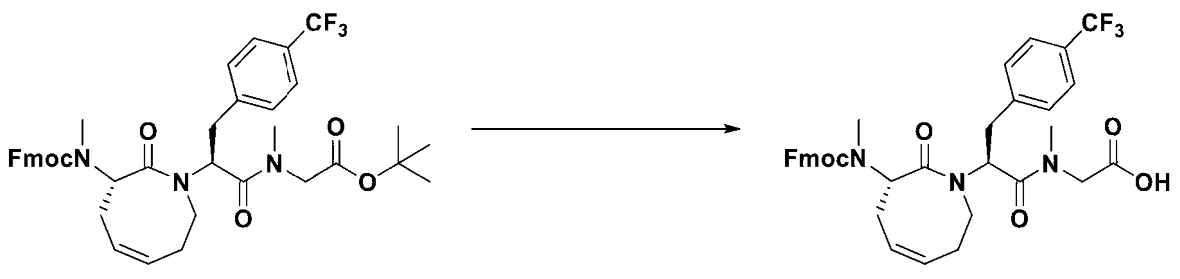

To a reaction vessel, 2-MeTHF (1.35 mL), compound 8 (145 mg), and HMDS (98 μL) were added at room temperature, and the mixture was stirred. Subsequently, TMSOTf (51 μL) was added to the mixed solution at room temperature, and the mixture was stirred for 4 hours. After measuring by HPLC and confirming that the reaction conversion rate was 99% or more, the external temperature was cooled to 5°C, a 5% aqueous dipotassium phosphate solution (1.35 mL) was slowly added at an internal temperature of 15°C or lower, and the mixture was stirred at room temperature. The aqueous layer was discharged by liquid-liquid separation. The organic layer was washed with a 5% aqueous sodium dihydrogen phosphate solution (1.35 mL × 2) and 5% saline (1.35 mL), and the organic layer was concentrated under reduced pressure at an external temperature of 40°C to obtain compound 28 (133 mg).
LCMS (ESI) of compound 28: retention time: 4.34 minutes, m/z = 664 $[M+H]^+$ (LCMS analysis conditions method P3-4)

To a reaction vessel, a solution of compound 8 (3.89 kg) in 2-MeTHF (20.5 kg) was added at room temperature, then cooled to an external temperature of 15°C, HMDS (2.19 kg) was added, and the mixture was stirred. Subsequently, TMSOTf (1.80 kg) was slowly added to the mixed solvent at an internal temperature of 25°C or lower, and the mixture was stirred at an internal temperature of 25°C for 2 hours. 2-MeTHF (16.6 kg) and acetonitrile (4.60 kg) were added, and after cooling to an internal temperature of 15°C or lower, a 5% aqueous sodium hydrogen carbonate solution (27.3 kg) was slowly added at an internal temperature of 30°C, the mixture was stirred at room temperature, and the aqueous layer was discharged by liquid-liquid separation. The organic layer was washed with a 5% aqueous sodium hydrogen sulfate solution (27.2 kg) and 5% saline (27.2 kg × 3), and concentrated at an external temperature of 40°C to 10 L. Toluene (56.0 kg) was added to the concentrated solution, which was concentrated to 55 L at an external temperature of 40°C, and then addition of toluene (15.6 kg) and concentration at an external temperature of 40°C to 54 L were repeated twice. After confirming precipitation of a crystal, cyclohexane (14.0 kg) was added and the mixture was stirred overnight, filtered, and the crystal was washed with a toluene/cyclohexane = 3:1 mixed solution (15.2 kg). The resulting wet crystal was dried under reduced pressure at an external temperature of 50°C to obtain compound 28-toluene monosolvate (3.21 kg) as a white solid.

Example 2-4

Synthesis of compound 10: tert-butyl N-[(benzyloxy)carbonyl]-L-isoleucyl-L-prolinate

[Formula 76]

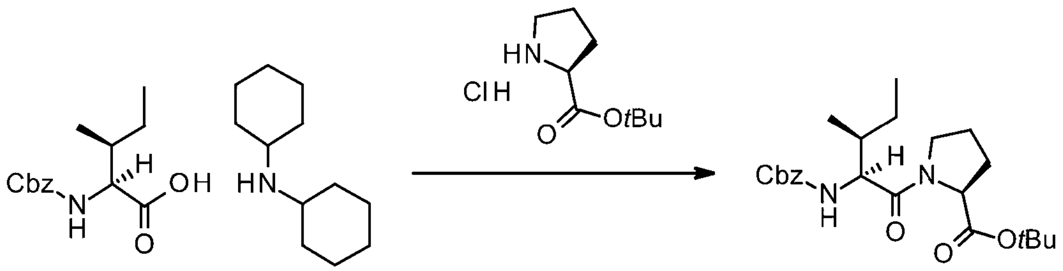

To N-cyclohexylcyclohexanaminium (2S,3S)-2-{[(benzyloxy)carbonyl]amino }-3-methylpentanoate (135 g), toluene (583 g) and 5% aqueous sodium hydrogen sulfate (2066 g) were added, and after stirring at room temperature for 10 minutes, the organic layer was separated. The obtained organic layer was washed with 5% aqueous sodium hydrogen sulfate (2066 g), followed by 5% saline (1397 g), and the solvent was distilled off under reduced pressure. To the resulting residue, toluene (55 mL), 1,3-dimethyl-2-imidazolidinone (270 mL), (2S)-2-(tert-butoxycarbonyl)pyrrolidin-1-ium chloride (75.3 g), 2-methyltetrahydrofuran (540 mL), and 4-methylmorpholine (133 mL) were added, and the mixture was stirred at an external temperature of 15°C. To this mixture, a 50% propylphosphonic anhydride solution in 2-methyltetrahydrofuran (370 mL) was added dropwise over about 1 hour, and the resulting reaction mixture was stirred at an external temperature of 20°C for 1 hour. To this reaction mixture, 5% aqueous sodium hydrogen carbonate (993 g) and 1-methylimidazole (24 mL) were added at an internal temperature of 20°C or lower, and after stirring at an external temperature of 20°C for about 30 minutes, the organic layer was separated. The obtained organic layer was washed with 10% aqueous sodium hydrogen sulfate (709 g), 10% aqueous sodium hydrogen sulfate (639 g), and 10% aqueous sodium hydrogen carbonate (710 g) in this order, and the solvent was distilled off under reduced pressure to obtain a solution (137 g) containing the title compound.
LCMS (ESI) of compound 10: retention time: 4.21 minutes, m/z = 419 $[M+H]^+$ (LCMS analysis conditions method M)

Example 2-5

Synthesis of compound 11: tert-butyl L-isoleucyl-L-prolinate

[Formula 77]

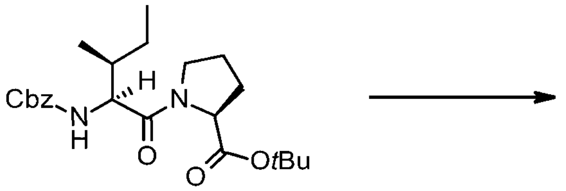

Pd/C (50% wet, 36.7 g) and 2-methyltetrahydrofuran (253 mL) were stirred at an external temperature of 25°C under hydrogen pressure (0.4 MPa) for 2 hours. To the resulting mixture, the solution (137 g) containing compound 10 obtained in Example 2-4 and 2-methyltetrahydrofuran (495 mL) were added, and the mixture was stirred at an external temperature of 25°C under hydrogen pressure (0.2 MPaG) for 2 hours. The reaction mixture was filtered, the solid was washed three times with 2-methyltetrahydrofuran (127 mL), all filtrates were combined, and the solvent was distilled off under reduced pressure to obtain a solution (160 g) containing the title compound.
LCMS (ESI) of compound 11: retention time: 2.40 minutes, m/z = 285 $[M+H]^+$ (LCMS analysis conditions method M)

Example 2-6

Synthesis of compound 29: tert-butyl N-[(benzyloxy)carbonyl]-N-methyl-L-norvalyl-L-isoleucyl-L-prolinate

[Formula 78]

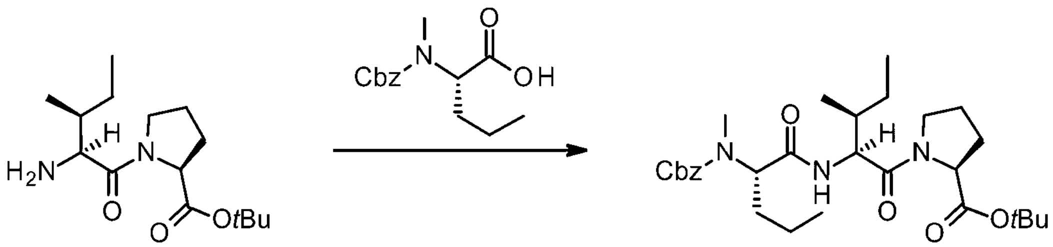

A mixture of the solution (120 g) containing compound 11 obtained in Example 2-5, N-[(benzyloxy)carbonyl]-N-methyl-L-norvaline (72.12 g), 2-methyltetrahydrofuran (257 mL), and 4-methylmorpholine (100 mL) was stirred at an external temperature of 15°C. To this mixture, a 50% propylphosphonic anhydride solution in 2-methyltetrahydrofuran (277 mL) was added dropwise over about 40 minutes, and the resulting reaction mixture was stirred at an external temperature of 20°C for 1 hour. To this reaction mixture, 5% aqueous sodium hydrogen carbonate (472 g) and 1-methylimidazole (18 mL) were added at an internal temperature of 30°C or lower, and after stirring at an external temperature of 15°C for about 30 minutes, the organic layer was separated. The obtained organic layer was washed with 10% aqueous sodium hydrogen sulfate (338 g), 10% aqueous sodium hydrogen sulfate (338 g), and 10% aqueous sodium hydrogen carbonate (340 g) in this order at an external temperature of 20°C, and the solvent was distilled off under reduced pressure to obtain a solution (219 g) containing the title compound.
LCMS (ESI) of compound 29: retention time: 4.58 minutes, m/z = 532 $[M+H]^+$ (LCMS analysis conditions method M)

Example 2-7

Synthesis of compound 13: tert-butyl N-methyl-L-norvalyl-L-isoleucyl-L-prolinate

[Formula 79]

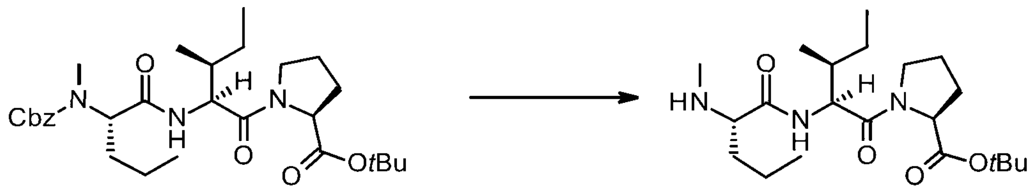

Pd/C (50% wet, 22.9 g) and 2-methyltetrahydrofuran (400 mL) were stirred at an external temperature of 25°C under hydrogen pressure (0.4 MPaG) for 2 hours. To the resulting mixture, the solution (182 g) containing compound 29 obtained in Example 2-6 and 2-methyltetrahydrofuran (50 mL) were added, and the mixture was stirred at an external temperature of 25°C under hydrogen pressure (0.4 MPaG) for 2 hours. The reaction mixture was filtered, the solid was washed three times with 2-methyltetrahydrofuran (100 mL), and all filtrates were combined and concentrated under reduced pressure to obtain a solution (109 g) containing the title compound. For 9.5346 g of this solution, the solvent was distilled off under reduced pressure, and heptane (100 mL) was added to the resulting residue. The mixture was dissolved at an external temperature of 50°C, and the seed crystal (11.0 mg) obtained in Example 2-7-1 was added at an internal temperature of 40°C. After stirring the mixture at an external temperature of 42°C for 15 minutes, at an external temperature of 43°C for 13 minutes, and at an external temperature of 44°C for 17 minutes, the external temperature was cooled to 0°C at a rate of 12°C per hour, and the mixture was further stirred at an external temperature of 0°C for 1.5 hours. The resulting solid was filtered, washed with cold heptane (25 mL), and dried under reduced pressure at an external temperature of 30°C to 40°C to obtain the title compound (4.8474 g).
LCMS (ESI) of compound 13: retention time: 2.56 minutes, m/z = 398 $[M+H]^+$ (LCMS analysis conditions method M)

Example 2-7-1

Synthesis of seed crystal of compound 13: tert-butyl N-methyl-L-norvalyl-L-isoleucyl-L-prolinate

Part of the solution containing the title compound obtained in the reaction of Example 2-7 was concentrated under reduced pressure, and to the resulting residue (0.3811 g), heptane (7622 μL) was added. After dissolving the produced solid at an external temperature of 50°C, it was cooled to room temperature while stirring, and to the resulting slurry, heptane (3811 μL) was added and stirring was continued. The solid was filtered, washed with heptane (1906 μL), and dried under reduced pressure at room temperature to obtain the title compound (0.2187 g). LCMS (ESI) of seed crystal of compound 13: retention time: 2.60 minutes, m/z = 398 $[M+H]^+$ (LCMS analysis conditions method M)

Example 2-8-1

Synthesis of compound A11: (tert-butyl (3S)-3-[benzyloxycarbonyl(methyl)amino]-4-(dimethylamino)-4-oxo-butanoate)

[Formula 80]

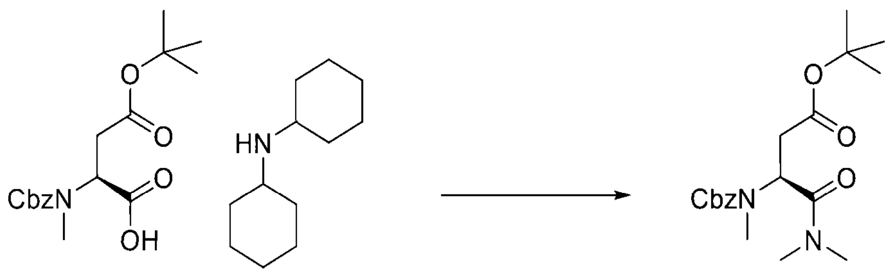

To a reaction vessel, (2S)-2-(((benzyloxy)carbonyl)(methyl)amino)-4-tert-butoxy-4-oxobutanoic acid dicyclohexylamine salt (Cas No. 42417-70-9, 25.00 g, 48.2 mmol) and 2-methyltetrahydrofuran (126 g) were added at 25°C. Separatory washing with a 10% aqueous sodium hydrogen sulfate monohydrate solution (150 g) was repeated twice, followed by washing with a 5% aqueous sodium chloride solution (150 g). The obtained organic layer was concentrated under reduced pressure conditions. The operation of adding 2-methyltetrahydrofuran (95 g) to the resulting residue and

concentrating under reduced pressure conditions was repeated twice. To the resulting residue (47.91 g), 2-methyltetrahydrofuran (95 g), acetonitrile (75 g), DIPEA (35.46 g, 274 mmol), and dimethylamine hydrochloride (7.88 g, 96.6 mmol) were added at 25°C. A propylphosphonic anhydride solution in 2-methyltetrahydrofuran (50.4 wt%, 61.33 g, 97.1 mmol) was added dropwise over 1 hour and 30 minutes. Sampling was performed 1 hour after the completion of the dropwise addition, and the completion of the reaction was confirmed by HPLC analysis. A 2M aqueous sodium hydroxide solution (150 g) was added. The mixture was stirred for 10 minutes, then allowed to stand still, and then the aqueous layer was removed. The resulting organic layer was washed with a 2M aqueous sodium hydroxide solution (150 g), a 13% aqueous sulfuric acid solution (150 g), a 10% aqueous sodium hydrogen sulfate monohydrate solution (150 g), and a 5% aqueous sodium carbonate solution (150 g), and then concentrated under reduced pressure conditions. The operation of adding 2-methyltetrahydrofuran (125 g) and concentrating under reduced pressure conditions was repeated twice to obtain a solution (42.39 g) containing compound A11.
LCMS (ESI) of compound A11: retention time: 3.37 minutes, m/z = 387 $[M+Na]^+$ (LCMS analysis conditions method K-2)

Example 2-8-2

Synthesis of compound A12: (tert-butyl (3S)-4-(dimethylamino)-3-(methylamino)-4-oxo-butanoate)

[Formula 81]

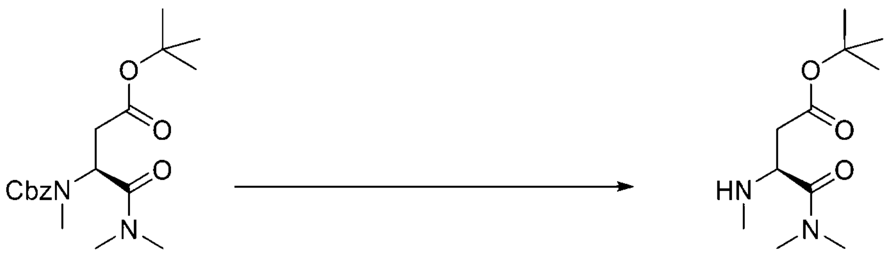

To a reaction vessel, 10% palladium carbon (54.33% wet, 3.39 g, 1.45 mmol, 3 mol% on a Pd metal basis) and 2-methyltetrahydrofuran (75 g) were added. Nitrogen replacement was performed at 25°C, followed by hydrogen replacement, and the mixture was stirred under a hydrogen atmosphere (0.40 MPaG) for 2 hours. The solution (42.39 g) of compound A11 obtained in Example 2-8-1 and 2-methyltetrahydrofuran (22 g) were added. Sampling was performed after stirring under a hydrogen atmosphere (0.20 MPaG) for 1 hour and 30 minutes, and the completion of the reaction was confirmed by HPLC analysis. After filtration of the reaction mixture, the cake was washed twice with 2-methyltetrahydrofuran (75 g). A mixed solution of the filtrate and the washing solution was concentrated under reduced pressure conditions to obtain a solution (30.76 g) containing compound A12.
LCMS (ESI) of compound A12: retention time: 1.44 minutes, m/z = 231 $[M+H]^+$ (LCMS analysis conditions method K-2)

Example 2-8-3

Synthesis of compound A14: (tert-butyl (3S)-3-[[(2S)-2-[benzyloxycarbonyl(methyl)amino]-2-cyclopentyl-acetyl]-methyl-amino]-4-(dimethylamino)-4-oxo-butanoate)

[Formula 82]

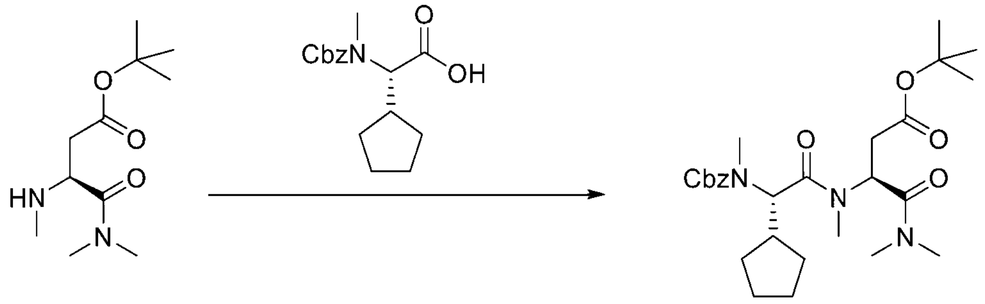

To a reaction vessel, the solution (30.76 g) of compound A12 obtained in Example 2-8-2, (S)-2-(((benzyloxy) carbonyl)(methyl)amino)-2-cyclopentylacetic acid (Cas No. 2411591-78-9, 16.96 g, 58.2 mmol), 2-methyltetrahydrofuran (40 g), acetonitrile (17 g), and DIPEA (27.74 g, 215 mmol) were added at 25°C. HATU (27.49 g, 72.3 mmol) was added over 10 minutes. Sampling was performed 3 hours after the completion of addition, and the completion of the reaction was confirmed by HPLC analysis. Toluene (30 g), a 5% aqueous potassium carbonate solution (23 g), and 1-methylimidazole (3.97 g, 48.4 mmol) were added, and the mixture was stirred for 30 minutes. A 2.5% aqueous ammonia solution (88 g) and 2-methyltetrahydrofuran (25 g) were added. The mixture was stirred for 10 minutes, then allowed to stand still, and then the aqueous layer was removed. The resulting organic layer was washed with a 2.5% aqueous ammonia solution (113 g), a 10% aqueous sodium hydrogen sulfate monohydrate solution (113 g, twice), and a 5% aqueous potassium carbonate solution (113 g), and then concentrated under reduced pressure conditions. 2-methyltetrahydrofuran (42 g) was added, and the mixture was concentrated under reduced pressure conditions to obtain a solution (52.78 g) containing compound A14.
LCMS (ESI) of compound A14: retention time: 4.10 minutes, m/z = 526 $[M+Na]^+$ (LCMS analysis conditions method K-2)

Example 2-8-4

Synthesis of compound A15: (tert-butyl (3S)-3-[[(2S)-2-cyclopentyl-2-(methylamino)acetyl]-methyl-amino]-4-(dimethylamino)-4-oxo-butanoate)

[Formula 83]

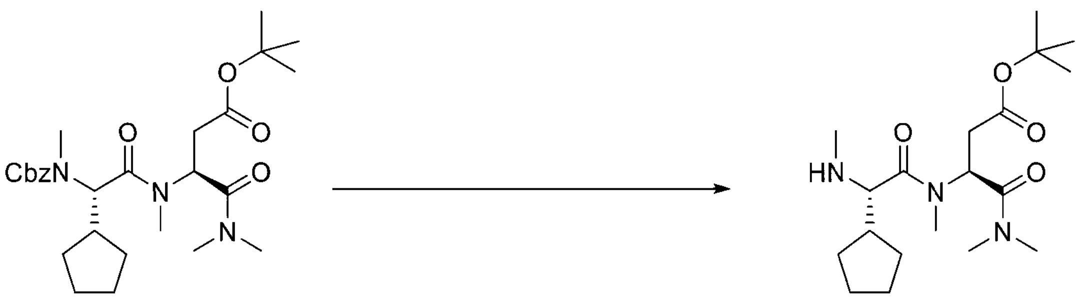

To a reaction vessel, 10% palladium carbon (54.33% wet, 3.39 g, 1.45 mmol, 3 mol% on Pd metal basis) and 2-methyltetrahydrofuran (75 g) were added. Nitrogen replacement was performed at 25°C, followed by hydrogen replacement, and the mixture was stirred in a hydrogen atmosphere (0.40 MPaG) for 2 hours. The solution (52.78 g) of compound A14 obtained in Example 2-8-3 and 2-methyltetrahydrofuran (15 g) were added, and the temperature was then raised to 30°C. Sampling was performed after stirring under a hydrogen atmosphere (0.20 MPaG) for 2 hours, and the completion of the reaction was confirmed by HPLC analysis. After filtering the reaction mixture, the cake was washed twice with 2-methyltetrahydrofuran (75 g). After concentrating a mixed solution of the filtrate and the washing solution under reduced pressure conditions, the operation of adding acetonitrile (75 g) and concentrating under reduced pressure conditions was repeated twice to obtain a solution (42.5 mL) containing compound A15.
LCMS (ESI) of compound A15: retention time: 2.96 minutes, m/z = 370 $[M+H]^+$ (LCMS analysis conditions method K-1)

Example 2-8-5

Synthesis of compound A16: (tert-butyl (3S)-3-[[(2S)-2-cyclopentyl-2-(methylamino)acetyl]-methyl-amino]-4-(dimethylamino)-4-oxo-butanoate) hydrochloride

[Formula 84]

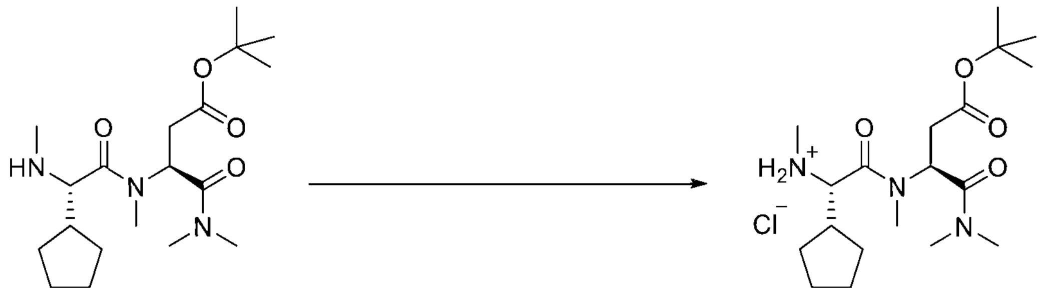

To a reaction vessel, the solution (42.5 mL) of compound A15 obtained in Example 2-8-4 and acetonitrile (8.0 g) were added. After adding MTBE (65 g) at 40°C, a solution of pyridine hydrochloride in acetonitrile (16.94 w/w%, 4.50 g) was added dropwise over 30 minutes. After stirring for 1 hour, a solution of pyridine hydrochloride in acetonitrile (16.94 w/w%, 31.34 g) was added dropwise over 3 hours and 30 minutes, followed by addition of acetonitrile (14 g). After stirring for 1 hour, the mixture was cooled to 10°C over 6 hours. Stirring was further performed at 10°C for 11 hours, and then the slurry was filtered. The resulting solid was washed with MTBE (38 g) twice and dried under reduced pressure conditions to obtain compound A16 (15.68 g).
LCMS (ESI) of compound A16: retention time: 2.92 minutes, m/z = 370 $[M+H]^+$ (LCMS analysis conditions method K-1)

Example 2-8-6

Synthesis of compound 30: (tert-butyl (S)-3-((S)-2-(1-(((benzyloxy)carbonyl)(methyl)amino)-N-methylcyclobutane-1-carboxamido)-2-cyclopentyl-N-methylacetamido)-4-(dimethylamino)-3-oxo-butanoate)

[Formula 85]

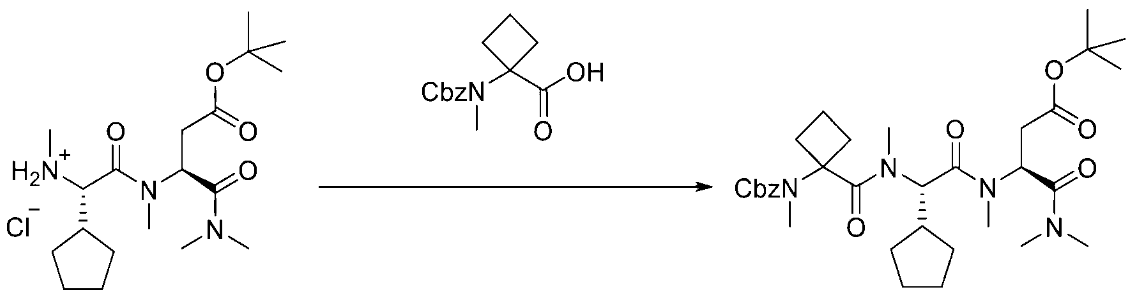

A reaction vessel (2 L) after nitrogen replacement was charged with acetonitrile (224 mL), DIPEA (150 g), compound A16 (74.56 g) obtained in Example 2-8-5, and 1-(benzyloxycarbonyl(methyl)amino)cyclobutanoic acid (Cas No. 1408729-60-1, 131 g), and the mixture was stirred at room temperature for 10 minutes. After confirming complete dissolution, a 50 wt.% propylphosphonic anhydride solution in 2-methyltetrahydrofuran (339 g) was added to the mixed solution over 15 minutes. After the completion of addition, the internal temperature was raised to 60°C and the mixture was stirred for 2 hours. The reaction mixture was sampled for sample preparation (sample preparation method K), and the reaction conversion rate was confirmed to be 99% or more by HPLC analysis (calculation expression 1 for the reaction conversion rate). A 5% aqueous potassium carbonate solution (447 mL) and N,N-dimethyl-4-aminopyridine (90 g) were added, and the mixture was further stirred for 1 hour and 30 minutes. The internal temperature was cooled to 25°C, toluene (373 mL) was added, and the aqueous layer was discharged by a liquid separation operation. The organic layer was washed with a 4% aqueous sulfuric acid solution (447 mL × 2) and a 5% aqueous sodium carbonate solution (447 mL × 2).

The resulting organic layer was concentrated under reduced pressure at an external temperature of 60°C to 226 mL. After the concentration under reduced pressure, addition of THF (447 mL) and concentration under reduced pressure to 226 mL were repeated twice to afford a solution of compound 30 in THF (167.76 g). The yield of the obtained solution was not calculated, and it was used for Example 2-9.
LCMS (ESI) of compound 30: retention time: 7.02 minutes, m/z = 637.43 $[M+Na]^+$ (LCMS analysis conditions method K-1)

Example 2-9

Synthesis of compound 31: (tert-butyl (S)-3-((S)-2-cyclopentyl-N-methyl-2-(N-methyl-1-(methylamino)cyclobutane-1-carboxamido)acetamido)-4-(dimethylamino)-4-oxo-butanoate)

[Formula 86]

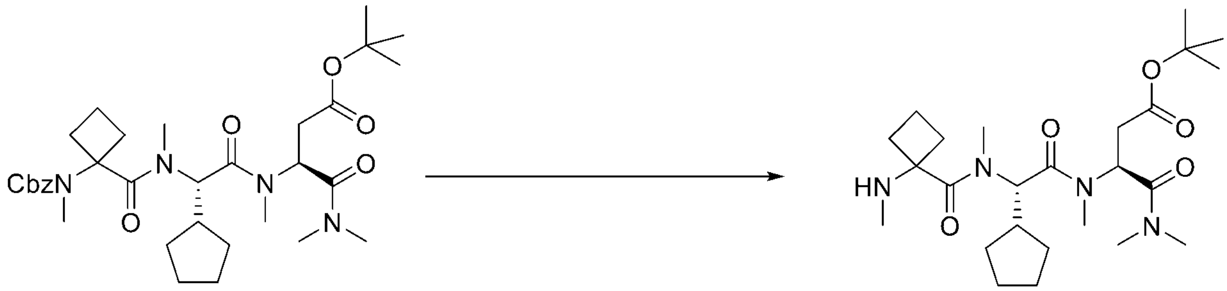

A pressure-resistant reaction vessel (5 L) was charged with the solution of compound 26 in THF (163.97 g) obtained in Example 2-8, THF (665 mL), and 4.57 wt.% Pd/C (14.71 g), the external temperature was set to 25°C, and the mixture was stirred. Nitrogen replacement at 0.20 MPa was performed three times, hydrogen replacement at 0.20 MPa was further performed three times, and the mixture was stirred under a hydrogen atmosphere at 0.20 MPa for 1 hour. The reaction mixture was sampled for sample preparation (sample preparation method 1), and the reaction conversion rate was confirmed to be 99% or more by HPLC analysis (calculation expression 1 for the reaction conversion rate). The inside of the reaction vessel was replaced with nitrogen at 0.20 MPa three times, and Pd/C was removed by filtration. The removed Pd/C was washed three times with THF (444 mL). After the filtrate and the washing solution were combined, the resulting solution was concentrated under reduced pressure at an external temperature of 50°C to 174 mL. Addition of acetonitrile (444 mL) and concentration under reduced pressure to 174 mL were repeated twice to obtain a solution of compound 31 in acetonitrile (100.91 g). The yield of the obtained solution was not calculated, and it was used for Example 2-10.
LCMS (ESI) of compound 31: retention time: 2.63 minutes, m/z = 481.43 $[M+H]^+$ (LCMS analysis conditions method K-2)

Example 2-10

Synthesis of compound 32: (benzyl (2S,4R)-2-((1-(((S)-2-(((S)-4-(tert-butoxy)-1-(dimethylamino)-1,4-dioxabutan-2-yl)(methyl)amino)-1-cyclopentyl-2-oxoethyl)(methyl)carbamoyl)cyclobutyl)(methyl)carbamoyl)-4-ethoxypyrrolidine-1-carboxylate)

[Formula 87]

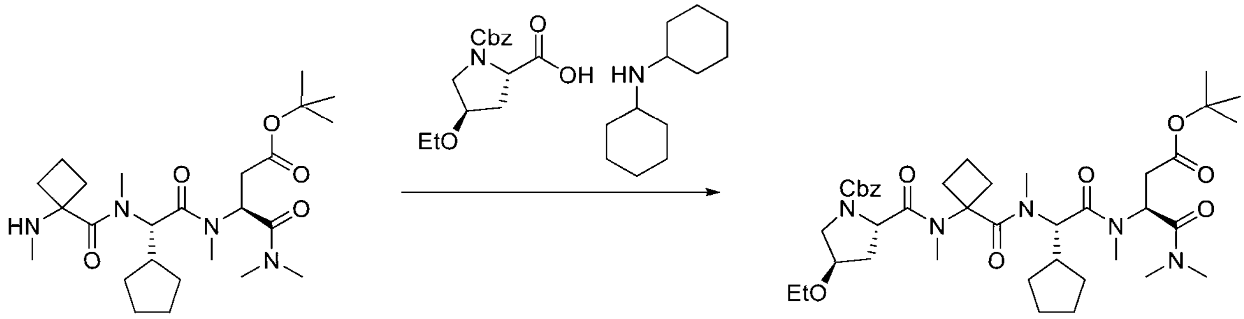

To a reaction vessel (2 L), (2S,4R)-1-((benzyloxy)carbonyl)-4-ethoxypyrrolidine-2-carboxylic acid dicyclohexylamine salt (227 g) and 2-methyltetrahydrofuran (767 mL) were added at room temperature, and the mixture was stirred. A 4% aqueous sulfuric acid solution (1151 mL) was added, the aqueous layer was discharged by a liquid separation operation, and the organic layer was washed with each of a 4% aqueous sulfuric acid solution (1151 mL) and a 5% aqueous sodium chloride solution (1151 mL). The resulting organic layer was concentrated under reduced pressure with the external temperature set to 50°C to 153 mL. Acetonitrile (384 mL) was added to the resulting concentrated solution, and the mixture was concentrated under reduced pressure to 153 mL. To the reaction vessel, compound 31 (89.31 g) obtained in Example 2-9, acetonitrile (384 mL), and DIPEA (103 g) were added, and the mixture was stirred at room temperature. TCFH (121 g) was added, the external temperature of the reaction vessel was raised to 50°C, and the mixture was stirred for 5 hours. The reaction mixture was sampled for sample preparation (sample preparation method K), and the reaction conversion rate was confirmed to be 99% or more by HPLC analysis (calculation expression 1 for the reaction conversion rate). Water (307 mL) and NMI (52.4 g) were added, the mixture was stirred for 30 minutes, stirring was stopped, and the mixture was stored at room temperature overnight. Toluene (767 mL) was added at room temperature, and the aqueous layer was discharged by a liquid separation operation. The organic layer was washed with 10% aqueous ammonia (767 mL × 2), a 4% aqueous sulfuric acid solution (767 mL × 2), and a 5% aqueous sodium carbonate solution (767 mL). The resulting organic layer was concentrated under reduced pressure at an external temperature of 50°C to 242 mL. After the concentration under reduced pressure, addition of THF (537 mL) and concentration under reduced pressure to 242 mL were repeated twice to afford a solution of compound 32 in THF (195.54 g). The yield of the obtained solution was not calculated, and it was used for Example 2-11.
LCMS (ESI) of compound 32: retention time: 4.30 minutes, m/z = 778.59 $[M+Na]^+$ (LCMS analysis conditions method K-2)

Example 2-11

Synthesis of compound 33: (tert-butyl (S)-3-((S)-2-cyclopentyl-2-(1-((2S,4R)-4-ethoxy-N-methylpyrrolidine-2-carboxamido)-N-methylcyclobutane-1-carboxamido)-N-methylacetamido)-4-(dimethylamino)-4-oxo-butanoate)

[Formula 88]

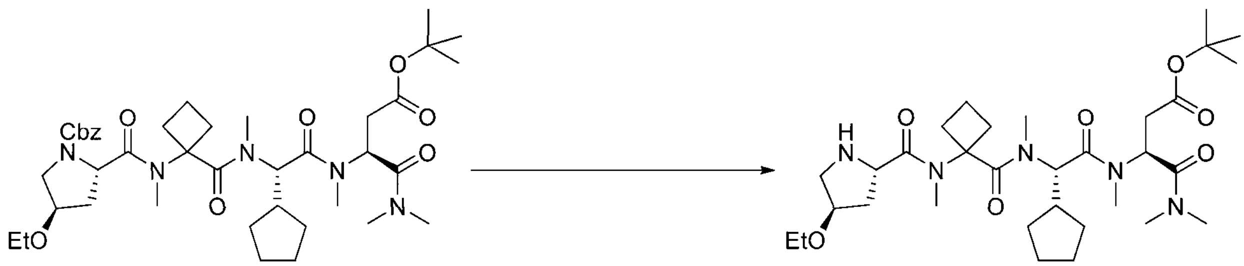

A pressure-resistant reaction vessel (5 L) was charged with 4.57 wt.% Pd/C (8.53 g) and THF (388.5 mL), the external temperature was set to 25°C, and the mixture was stirred. Nitrogen replacement at 0.20 MPa was performed three times, hydrogen replacement at 0.20 MPa was further performed three times, and the mixture was stirred under a hydrogen atmosphere at 0.40 MPa for 3 hours. The solution of compound 32 in THF (179.08 g) obtained in Example 2-10 and THF (531 mL) were added. Nitrogen replacement at 0.20 MPa was performed three times, hydrogen replacement at 0.20 MPa was further performed three times, and the mixture was stirred under a hydrogen atmosphere at 0.20 MPa for 6

hours. The reaction mixture was sampled for sample preparation (sample preparation method 1), and the reaction conversion rate was confirmed to be 99% or more by HPLC analysis (calculation expression 1 for the reaction conversion rate). The inside of the reaction vessel was replaced with nitrogen at 0.20 MPa three times, and the Pd/C was removed by filtration. The removed Pd/C was washed with 2-methyltetrahydrofuran (232 mL) three times. The filtrate and the washing solution were mixed, which was concentrated under reduced pressure at an external temperature of 50°C to 182 mL. 2-Methyltetrahydrofuran (774 mL) was added, and the mixture was concentrated under reduced pressure to 182 mL. To the resulting concentrated solution, 2-methyltetrahydrofuran (55.2 mL) was added, the external temperature was raised to 54°C, and the mixture was stirred. 158 mL of heptane was added, and a crystal of compound 33 (0.396 g), obtained by the same operation as in Example 2-11, was suspended in heptane (15.85 mL), which was added to the reaction vessel. The mixture was stirred at an external temperature of 54°C for 3 hours, the external temperature was cooled to 22°C over 6.5 hours, and the mixture was further stirred for 10 hours. Heptane (143 mL) was added over 1 hour, and the mixture was stirred for 1 hour. Heptane (634 mL) was further added over 4 hours, and the mixture was stirred for 1 hour. The external temperature was cooled to 10°C over 2.5 hour, and the mixture was stirred for 14 hours. The reaction mixture was filtered through Kiriyama funnel, and the resulting residue was washed with heptane (396 mL) twice. The resulting crystal was dried under reduced pressure conditions with the external temperature set to 40°C for 2.5 hours. The dried powder was collected to obtain a white powder (72.9 g).
LCMS (ESI) of compound 33: retention time: 4.50 minutes, m/z = 622.53 $[M+H]^+$ (LCMS analysis conditions method K-1)

A pressure-resistant reaction vessel (100 mL) was charged with 4.57 wt.% Pd/C (324 mg) and THF (9.00 mL), the external temperature was set to 25°C, and the mixture was stirred. Nitrogen replacement at 0.20 MPa was performed three times, hydrogen replacement at 0.20 MPa was further performed three times, and the mixture was stirred under a hydrogen atmosphere at 0.40 MPa for 2 hours. The solution of compound 32 in THF (6.28 g) obtained in Example 2-10 and THF (24.0 mL) were added. Nitrogen replacement at 0.20 MPa was performed three times, hydrogen replacement at 0.20 MPa was further performed three times, and the internal temperature of the reaction vessel was raised to 40°C and the mixture was stirred under a hydrogen atmosphere at 0.20 MPa for 2 hours. The reaction mixture was sampled for sample preparation (sample preparation method 1), and the reaction conversion rate was confirmed to be 99% or more by HPLC analysis (calculation expression 1 for the reaction conversion rate). The inside of the reaction vessel was replaced with nitrogen at 0.20 MPa three times and the Pd/C was removed by filtration. The removed Pd/C was washed three times with tetrahydrofuran (6.30 mL). The filtrate and the washing solution were mixed, which was concentrated under reduced pressure at an external temperature of 50°C to 6.3 mL. Addition of 2-methyltetrahydrofuran (21.0 mL) and concentration under reduced pressure to 6.3 mL were repeated three times. Part of the resulting concentrated solution (5.5 g) was placed in a reaction vessel (50 mL), and 2-methyltetrahydrofuran (1.34 mL) was added. The resulting solution was stirred with the internal temperature raised to 67.5°C. Heptane (35.6 mL) was added, and a crystal of compound 33 (322 mg), obtained by the same operation as in Example 2-11, was suspended in heptane (0.40 mL), which was added to the reaction vessel. The mixture was stirred at an internal temperature of 67.5°C for 1 hour or longer, the internal temperature was cooled to 57.5°C over 2 hours or longer, and the mixture was further stirred for 1 hour or longer. The internal temperature was further cooled to 47.5°C over 1 hour or longer, and the mixture was further stirred for 1 hour or longer. The internal temperature was further cooled to 10°C or lower over 2 hours or longer, and the mixture was further stirred for 1 hour or longer. The reaction mixture was filtered through Kiriyama funnel, and the resulting residue was washed with heptane (10.0 mL) twice and then with a heptane/2-methyltetrahydrofuran (6/1) solution (10.0 mL) that had been cooled to 10°C. The resulting crystal was dried under reduced pressure conditions with the external temperature set to 40°C for 2 hours. The dried powder was collected to obtain a white powder (1.39 g).

Example 2-12

Synthesis of compound 34: ((S)-2-(((benzyloxy)carbonyl)amino)-4-(3-methoxy-4-(trifluoromethyl)phenyl)butanoic acid dicyclohexylamine salt)

[Formula 89]

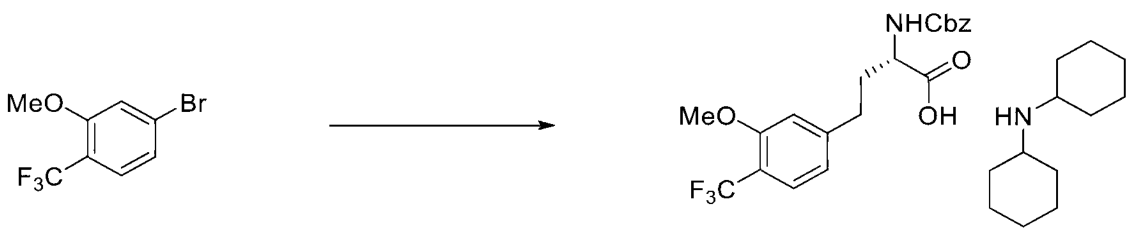

Following the method described in International Publication No. WO 2020/189540, compound 34 (206.8 g) was obtained from 4-bromo-2-methoxy-1-(trifluoromethyl)benzene (190 g).
NMR of compound 34: $^1H$ NMR (500 MHz, methanol-$d_4$) 7.38-7.30 (m, 5H), 7.27-7.26 (m, 1H), 6.97 (s, 1H), 6.84-6.82 (m, 1H), 5.07 (d, J = 13.0 Hz, 1H), 5.04 (d, J = 12.5 Hz, 1H), 4.06-4.03 (m, 1H), 3.83 (s, 3H), 3.14-3.11 (m, 2H), 2.69 (t, J = 8.0 Hz, 2H), 2.15-2.05 (m, 1H), 2.04-2.01 (m, 4H), 2.00-1.90 (m, 1H), 1.84-1.82 (m, 4H), 1.70-1.64 (m, 2H), 1.36-1.23 (m, 8H), 1.20-1.12 (m, 2H).

Example 2-12-1

Synthesis of compound 34-α1: benzyl (S)-2-((tert-butoxycarbonyl)amino)-4-(3-methoxy-4-(trifluoromethyl)phenyl)butanoate

[Formula 90]

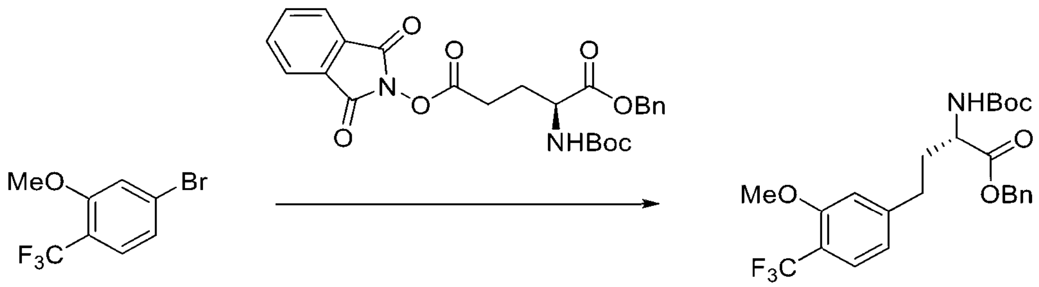

In a reaction vessel (1000 L), nickel bromide trihydrate (1.23 kg) and 1,3-dimethyl-2-imidazolidinone (98.8 kg) were mixed and charged as a slurry, then 4,4'-di-tert-butyl-2,2-dipyridyl (1.21 kg) was charged, and the mixture was stirred at 20.8 to 22.7°C for 28 minutes. After charging 1-benzyl 5-(1,3-dioxoisoindolin-2-yl)(tert-butoxycarbonyl)-L-glutamate (31.0 kg) synthesized in accordance with International Publication No. WO 2020/189540, 4-bromo-2-methoxy-1-(trifluoromethyl)benzene (24.57 kg), 1,3-dimethyl-2-imidazolidinone (230 kg), and N-methylmorpholine (3.25 kg), the internal temperature was cooled to 8.7°C, and zinc (12.6 kg) was charged. The mixture was stirred, and after the temperature was stabilized, chlorotrimethylsilane (21.0 kg) was added dropwise in the internal temperature range of 8.7 to 11.9°C over 3 hours. The reaction mixture was sampled for sample preparation (sample preparation method 1), and the reaction conversion rate was confirmed to be 99% or more by HPLC analysis (calculation expression 1 for the reaction conversion rate). After cooling the internal temperature to 2.6°C, a 15% aqueous ammonium chloride solution (332 kg) was added dropwise. Toluene (135 kg) was added to the resulting mixed solution, which was stirred for 30 minutes or longer and then filtered using Celite. After washing Celite with toluene (135 kg), the aqueous layer was discarded. The organic layer was washed with a mixed solution (465 kg) of disodium ethylenediaminetetraacetate (24.3 kg)-2.5% aqueous ammonia, followed by a 10% aqueous sodium chloride solution (332 kg). The resulting organic layer was concentrated with the external temperature set to 40°C to 93 L, and then 87.3 kg of a solution containing the title compound was obtained.
LCMS (ESI) of compound 34-α1: retention time: 7.23 minutes, m/z = 368.38 $[M-Boc+H]^+$ (LCMS analysis conditions method H)

Example 2-12-2

Synthesis of compound 34-α2: (S)-2-((tert-butoxycarbonyl)amino)-4-(3-methoxy-4-(trifluoromethyl)phenyl)butanoic acid

[Formula 91]

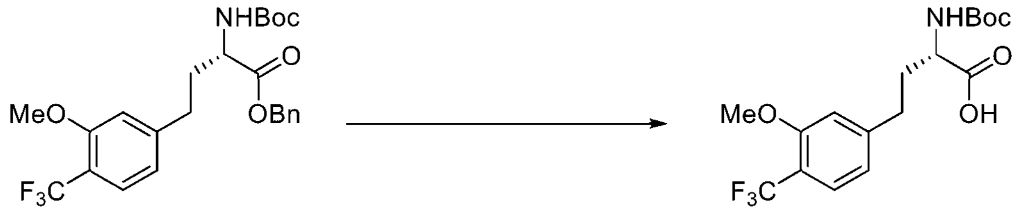

A pressure-resistant reaction vessel (150 L) was charged with the solution (43.7 kg) containing benzyl (S)-2-((tert-butoxycarbonyl)amino)-4-(3-methoxy-4-(trifluoromethyl)phenyl)butanoate while washing with toluene (40.2 kg), 10% Pd/C (8.37 kg) was added, and stirring was started. The pressure was increased to 0.15 MPaG with hydrogen. After the gas phase was replaced with hydrogen, the mixture was then stirred for 2 hours and 2 minutes at an internal temperature of 22.6 to 29.2°C. The reaction mixture was sampled for sample preparation (sample preparation method 1) and the reaction conversion rate was confirmed to be 99% or more by HPLC analysis (calculation expression 1 for the reaction conversion rate). The mixed solution was filtered, and the Pd/C residue was washed using toluene (26.7 kg). The filtrate was acquired again by the same operation, then combined, and concentrated with the external temperature set to 40°C to 155 L, thereby obtaining 155 kg of a solution containing the titled compound.
LCMS (ESI) of compound 34-α2: retention time: 5.72 minutes, m/z = 278.37 [M-Boc+H]+ (LCMS analysis conditions method H)

Example 2-12-3

Synthesis of compound 34-α2: (S)-2-((tert-butoxycarbonyl)amino)-4-(3-methoxy-4-(trifluoromethyl)phenyl)butanoic acid

[Formula 92]

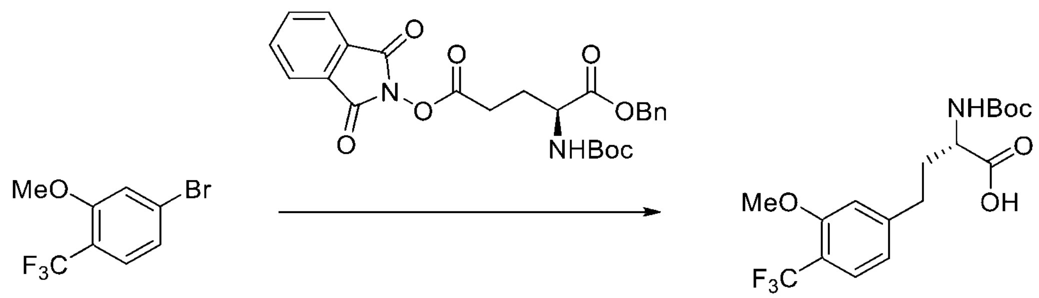

In a reaction vessel (100 mL), nickel bromide trihydrate (0.198 g) and 1,3-dimethyl-2-imidazolidinone (15 mL) were mixed and charged as a slurry, then 4,4'-di-tert-butyl-2,2-dipyridyl (0.195 g) was charged, and the mixture was stirred at 25°C for 30 minutes or longer. After charging 1-benzyl 5-(1,3-dioxoisoindolin-2-yl)(tert-butoxycarbonyl)-L-glutamate (5.00 g), 4-bromo-2-methoxy-1-(trifluoromethyl)benzene (5.29 g), 1,3-dimethyl-2-imidazolidinone (35 mL), and N-methylmorpholine (0.524 g), zinc (2.03 g) was charged in the internal temperature range of 5 to 25°C. The mixture was stirred, and after the temperature was stabilized, chlorotrimethylsilane (3.38 g) was added dropwise in the internal temperature range of 5 to 25°C over 2 hours or longer. The reaction mixture was sampled for sample preparation (sample preparation method 1), and the reaction conversion rate was confirmed to be 99% or more by HPLC analysis (calculation expression 1 for the reaction conversion rate). After confirming the reaction rate, a 10% aqueous potassium hydroxide solution (35 mL) was added dropwise in the internal temperature range of 0 to 25°C. The reaction mixture was sampled for sample preparation (sample preparation method H), and the reaction conversion rate was confirmed to be 99% or more by HPLC analysis (calculation expression H for the reaction conversion rate). Toluene (15 mL) was added to the resulting mixed solution, which was stirred, then filtered using Celite, and the Celite residue was then washed with a mixed solution of water (5 mL)-1,3-dimethyl-2-imidazolidinone (5 mL), thereby obtaining the filtrate. To the obtained filtrate, toluene (15 mL) and a 35% aqueous citric acid solution (25 g) were added, and after confirming that the pH was 4.5 or less, the aqueous layer was discarded. The resulting organic layer was washed with a mixed solution of disodium ethylenediaminetetraacetate (3.93 g)-water (74.2 g) to obtain 25.6 g of a solution containing the title compound.
LCMS (ESI) of compound 34-α2: retention time: 5.72 minutes, m/z = 278.37 [M-Boc+H]+ (LCMS analysis conditions method H)

Example 2-12-4

Synthesis of compound 35: ((S)-2-(((benzyloxy)carbonyl)amino)-4-(3-methoxy-4-(trifluoromethyl)phenyl)butanoate)

[Formula 93]

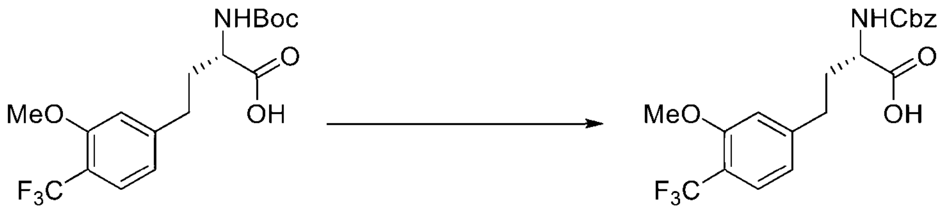

To a reaction vessel (1000 L), the solution (155 kg) containing (S)-2-((tert-butoxycarbonyl)amino)-4-(3-methoxy-4-(trifluoromethyl)phenyl)butanoic acid was added, and methanesulfonic acid (24.7 kg) was added dropwise in the internal temperature range 20.4°C to 27.6°C over 32 minutes. Thereafter, the temperature was raised, and the reaction was initiated at the point when the internal temperature reached 59.5°C. The reaction mixture was sampled for sample preparation (sample preparation method H), and the reaction conversion rate was confirmed to be 99% or more by HPLC analysis (calculation expression 1 for the reaction conversion rate). The reaction mixture was cooled, and water (186 kg) was added dropwise in the internal temperature range of 25.9°C to 32.0°C over 80 minutes. After removing the organic layer, washing with toluene (124 kg) was performed three times, and the pH was adjusted to 6.52 with a 40% aqueous tripotassium phosphate solution (95.7 kg). Thereafter, acetonitrile (61.2 kg) was added, and the pH was again adjusted to 7.83 using a 40% aqueous tripotassium phosphate solution (25.4 kg). To the reaction mixed solution after pH adjustment, N-(benzyloxycarbonyloxy)succinimide (16.0 kg) was added to initiate the reaction. The reaction mixture was sampled for sample preparation (sample preparation method 1), and the reaction conversion rate was confirmed to be 99% or more by HPLC analysis (calculation expression H for the reaction conversion rate). For the obtained reaction mixture, the pH was again adjusted to 7.73 using a 40% aqueous tripotassium phosphate solution (44.7 kg), and then methyl tert-butyl ether (46.3 kg) and heptane (42.4 kg) were added and separation into three layers was confirmed. Only the upper layer was discarded, methyl tert-butyl ether (115 kg) was added again to the lower two layers, followed by addition of a mixed solution of a 24% aqueous sodium hydroxide solution (2.1 kg)-sodium chloride (12.4 kg)-water (110.0 kg), and the aqueous layer was discarded. The resulting organic layer was washed with a mixed solution of a 24% aqueous sodium hydroxide solution (2.1 kg)-sodium chloride (12.4 kg)-water (110.0 kg) four times. Then, the organic layer was sampled for sample preparation (sample preparation method 1), and the amount of residual acetonitrile was confirmed to be 0.44 v/w or less by NMR analysis (calculation formula H for the residual acetonitrile). The resulting organic layer was washed with a 0.2M aqueous sodium hydroxide solution (62.1 kg) and then washed with a mixed solution of a 24% aqueous sodium hydroxide solution (2.1 kg)-sodium chloride (3.1 kg)-water (72.3 kg). The organic layer after washing was sampled for sample preparation (sample preparation method 1), and the residual impurities were confirmed to be 0.10% or less by HPLC analysis. After washing the resulting organic layer with 1M hydrochloric acid (310 kg), the organic layer after washing was sampled for sample preparation (sample preparation method H), and the residual impurities were confirmed to be 0.10% or less by HPLC analysis. The resulting organic layer was washed with 10% saline (166 kg) and concentrated to 62 L. Then, toluene (80.9 kg) was added, and the mixture was concentrated again to 62 L, thereby performing solvent replacement to toluene. By adding toluene (204 kg) to the resulting concentrated residue, a solution (260 kg) containing the title compound was obtained.

LCMS (ESI) of compound 35: retention time: 5.913 minutes, m/z = 368.39 $[M-C02+H]^+$ (LCMS analysis conditions method H)

Example 2-12-5

Synthesis of compound 34: ((S)-2-(((benzyloxy)carbonyl)amino)-4-(3-methoxy-4-(trifluoromethyl)phenyl)butanoic acid dicyclohexylamine salt)

[Formula 94]

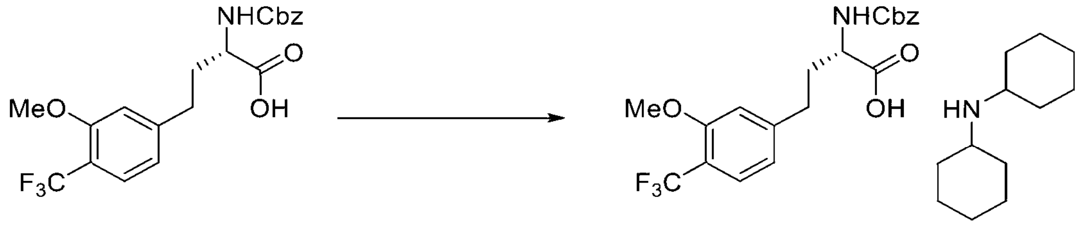

To a reaction vessel (1000 L), the solution (260 kg) containing ((S)-2-(((benzyloxy)carbonyl)amino)-4-(3-methoxy-4-(trifluoromethyl)phenyl)butanoate) was added, the internal temperature was raised to 59.5°C, and then dicyclohexylamine (16.1 kg) was added. The reaction mixture was heated to 66.3°C, and then heptane (49.9 kg) was added dropwise over 1 hour and 7 minutes. ((S)-2-(((benzyloxy)carbonyl)amino)-4-(3-methoxy-4-(trifluoromethyl)phenyl) butanoic acid dicyclohexylamine salt) (91.0 g) was suspended in a mixed solution of toluene (2.51 kg)-heptane (511 g) as a seed crystal suspension, and then the suspension was charged to the reaction vessel. After cooling the internal temperature from 67.0°C to 52.1°C over 1 hour and 38 minutes, stirring was continued for 1 hour or longer, and then the internal temperature was cooled from 50.1°C to 23.0°C over 1 hour and 55 minutes. After cooling, the temperature was kept for 14 hours and stirring was performed, the supernatant solution was sampled for sample preparation (sample preparation method H), and the supernatant concentration was confirmed to be 2 mg/mL or less by HPLC analysis. The resulting slurry was filtered and washed with a mixed solution of toluene (47.4 kg)-heptane (37.2 kg) to obtain a wet powder. The obtained wet powder was dried at an external temperature of 38°C for 50 hours, thereby obtaining ((S)-2-(((benzyloxy) carbonyl)amino)-4-(3-methoxy-4-(trifluoromethyl)phenyl)butanoic acid dicyclohexylamine salt) (25.6 kg).
LCMS (ESI) of compound 34: retention time: 5.89 minutes, m/z = 368.41 [M-CO2-DCHA+H]+ (LCMS analysis conditions method H)

Example 2-13

Synthesis of compound 35: ((S)-2-(((benzyloxy)carbonyl)amino)-4-(3-methoxy-4-(trifluoromethyl)phenyl)butanoate)

[Formula 95]

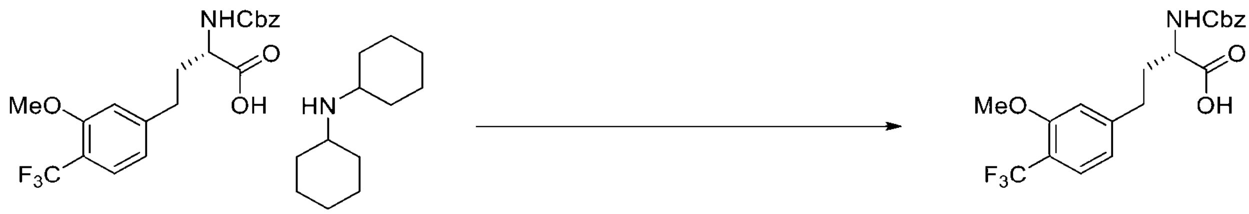

To a reaction vessel (2 L), (S)-2-(((benzyloxy)carbonyl)amino)-4-(3-methoxy-4-(trifluoromethyl)phenyl)butanoic acid dicyclohexylamine salt (57.8 g) and 2-methyltetrahydrofuran (253 mL) were added at room temperature, and the mixture was stirred. A 4% aqueous sulfuric acid solution (253 mL) was added, the aqueous layer was discharged by a liquid separation operation, and the organic layer was washed with each of a 4% aqueous sulfuric acid solution (253 mL) and a 5% aqueous sodium chloride solution (253 mL). The resulting organic layer was concentrated under reduced pressure with the external temperature set to 40°C to 100.8 mL. For the resulting concentrated solution, addition of 2-methyltetrahydrofuran (253 mL) and concentration under reduced pressure to 100.8 mL were repeated twice to obtain a solution (94.29 g) containing the title compound.
LCMS (ESI) of compound 35: retention time: 5.92 minutes, m/z = 368.18 $[M-CO_2+H]^+$ (LCMS analysis conditions LCMS analysis conditions method K-1)

Example 2-14

Synthesis of compound 36: (tert-butyl-(S)-3-((S)-2-(1-((2S,4R)-1-((S)-2-(((benzyloxy)carbonyl)amino)-4-(3-methoxy-4-(trifluoromethyl)phenyl)butanoyl)-4-ethoxy-N-methylpyrrolidine-2-carboxamido)-N-methylcyclobutane-1-carboxamido)-2-cyclopentyl-N-methylacetamido)-4-(dimethylamino)-4-oxo-butanoate)

[Formula 96]

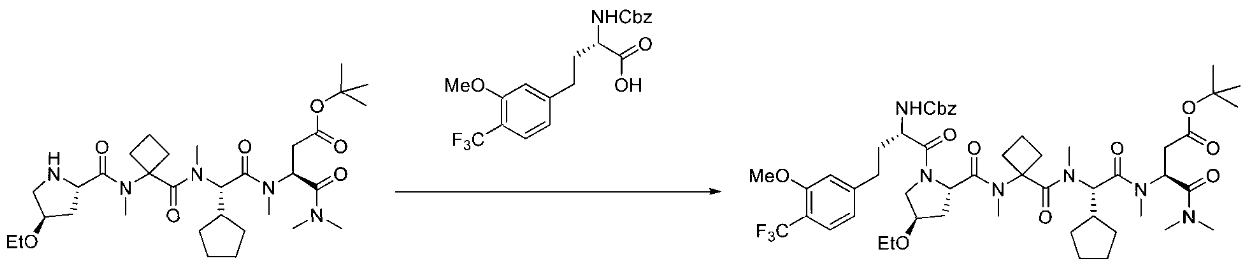

To a reaction vessel (2 L), compound 33 (94.29 g) obtained in Example 2-11, 2-methyltetrahydrofuran (253 mL), DIPEA (46.2 g), and compound 35 (50.5 g) obtained in Example 2-13 were added, and the mixture was stirred with the external temperature cooled to 10°C. A 50 wt.% propylphosphonic anhydride solution in 2-methyltetrahydrofuran (124 g) was added to the mixed solution over 15 minutes. The external temperature of the reaction vessel was raised to 25°C, and the mixture was stirred for 1 hour. The reaction mixture was sampled for sample preparation (sample preparation method K), and the reaction conversion rate was confirmed to be 99% or more by HPLC analysis (calculation expression 1 for the reaction conversion rate). A 5% aqueous potassium carbonate solution (303 mL) and NMI (6.67 g) were added, the mixture was stirred for 30 minutes, stirring was stopped, and the mixture was stored at room temperature overnight. The aqueous layer was discharged by a liquid separation operation, and the organic layer was washed with a 4% aqueous sulfuric acid solution (303 mL × 2). To the organic layer, heptane (182 mL), MTBE (121 mL), acetonitrile (116 mL), and a 2.5% aqueous potassium carbonate solution (288 mL) were added, and the aqueous layer was discharged by a liquid separation operation. To the resulting organic layer, acetonitrile (172 mL), 2-methyltetrahydrofuran (101 mL), and a 2.5% aqueous potassium carbonate solution (434 mL) were added, and the aqueous layer was discharged by a liquid separation operation. Furthermore, to the resulting organic layer, acetonitrile (172 mL), 2-methyltetrahydrofuran (101 mL), and a 2.5% aqueous potassium carbonate solution (434 mL) were added, and the aqueous layer was discharged by a liquid separation operation. The organic layer was concentrated under reduced pressure at an external temperature of 50°C to 167 mL. After the concentration under reduced pressure, addition of IPAc (354 mL) and concentration under reduced pressure to 167 mL were repeated twice to obtain a solution (170.11 g) containing the title compound. LCMS (ESI) of compound 36: retention time: 8.10 minutes, m/z = 1037.62 $[M+H]^+$ (LCMS analysis conditions method K-1)

Example 2-15

Compound 20: (3S)-3-[[(2S)-2-[[1-[[(2S,4R)-1-[(2S)-2-(benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoic acid

[Formula 97]

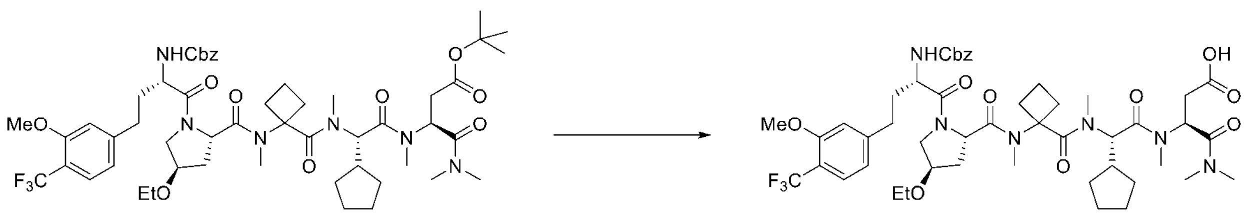

To a reaction vessel after nitrogen replacement, the solution of (tert-butyl-(S)-3-((S)-2-(1-((2S,4R)-1-((S)-2-(((benzyloxy)carbonyl)amino)-4-(3-methoxy-4-(trifluoromethyl)phenyl)butanoyl)-4-ethoxy-N-methylpyrrolidine-2-carboxamido)-N-methylcyclobutane-1-carboxamido)-2-cyclopentyl-N-methylacetamido)-4-(dimethylamino)-4-oxo-butanoate) (47.8 g) in isopropyl acetate (53.1 g) and isopropyl acetate (186 mL) were added at room temperature, and the mixture was stirred. Subsequently, HMDS (19.0 g) was added, and the mixture was stirred with the external temperature of the reaction vessel set to 0°C for 15 minutes. TMSOTf (15.4 g) was added dropwise slowly at the internal temperature not exceeding 20°C. The reaction mixture was stirred with the external temperature of the reaction vessel set to 20°C for 1 hour, then diluted with 2-methyltetrahydrofuran (244 mL), and the external temperature of the reaction vessel was set to 10°C. After slowly adding a 5% aqueous dipotassium hydrogen phosphate solution (478 mL) dropwise, stirring was stopped and the aqueous layer was discharged. The resulting organic layer was washed with 5% sodium dihydrogen phosphate (478 mL), diisopropylethylamine (9.0 mL) was then added, and the mixture was concentrated under reduced pressure to obtain a solution (120.1 g) of compound 20 in 2-methyltetrahydrofuran.
LCMS (ESI) of compound 20: retention time: 4.30 minutes, m/z = 981.66 $[M+Na]^+$ (LCMS analysis conditions method K-2)

Example 2-16

Synthesis of compound 21: tert-butyl (2S)-1-[(2s,3s)-2-[[(2S)-2-[[(3S)-3-[[(2S)-2-[[1-[[(2S,4R)-1-[(2S)-2-benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoyl]-methyl-amino]pentanoyl]amino]-3-methyl-pentanoyl]pyrrolidine-2-carboxylate

[Formula 98]

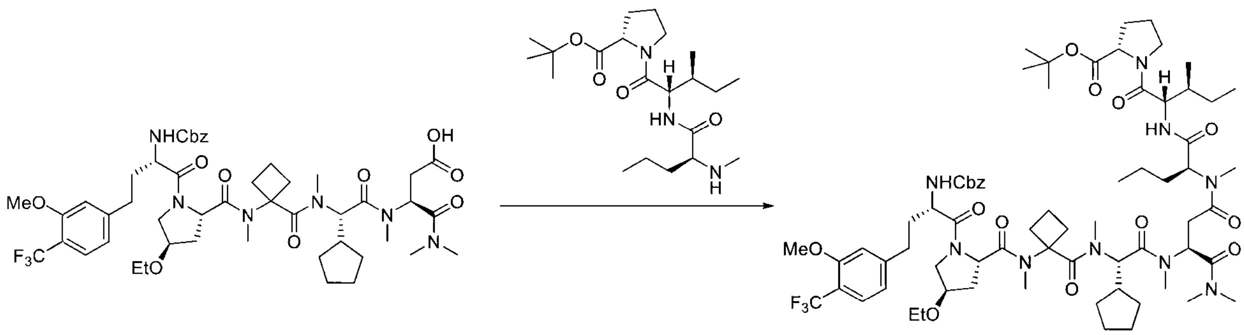

To a reaction vessel after nitrogen replacement, 2-methyltetrahydrofuran (70 mL) and the solution (113.85 g) of (3S)-3-[[(2S)-2-[[1-[[(2S,4R)-1-[(2S)-2-(benzyloxycarbonylamino)-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoic acid (40.6 g) in 2-methyltetrahydrofuran were added, and the mixture was stirred. Subsequently, diisopropylethylamine (25.3 mL) and compound 13 (21.9 g) dissolved in 2-methyltetrahydrofuran (180 mL) were added, followed by addition of acetonitrile (40 mL), and the mixture was stirred. HATU (24.2 g) was suspended in acetonitrile (53 mL), which was added to the reaction vessel, and the mixture was stirred for 1 hour. Acetic acid (1.2 mL) was added, the mixture was stirred for 20 minutes, and then stored overnight. Stirring was resumed, the mixture was then diluted with 2-MeTHF (122 mL), and 10% aqueous ammonia (282 mL) was added. Stirring was stopped, the aqueous layer was discharged, and the resulting organic layer was washed with 10% aqueous ammonia (280 mL), 4% sulfuric acid (280 mL), 4% sulfuric acid (280 mL), and a 5% aqueous sodium carbonate solution (280 mL) in this order. The resulting organic layer was concentrated under reduced pressure to obtain a solution (132.34 g) of compound 21 in 2-methyltetrahydrofuran.

Example 2-17

Synthesis of compound 37: tert-butyl (2S)-1-[(2S,3S)-2-[[(2S)-2-[[(3S)-3-[[(2S)-2-[[1-[[(2S,4R)-1-[(2S)-2-amino-4-[3-methoxy-4-(trifluoromethyl)phenyl]butanoyl]-4-ethoxy-pyrrolidine-2-carbonyl]-methyl-amino]cyclobutanecarbonyl]-methyl-amino]-2-cyclopentyl-acetyl]-methyl-amino]-4-(dimethylamino)-4-oxo-butanoyl]-methyl-amino]pentanoyl]amino]-3-methyl-pentanoyl]pyrrolidine-2-carboxylate

[Formula 99]

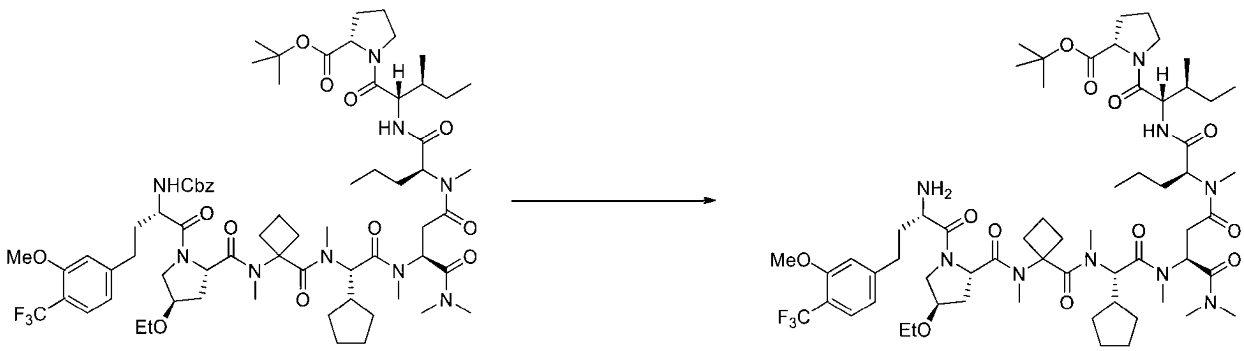

To a reaction vessel after nitrogen replacement, 2-methyltetrahydrofuran (1.0 mL) and 10% activated carbon-supported palladium (90.3 mg) were added at room temperature. Hydrogen replacement of the reaction vessel was performed, and the mixture was stirred under hydrogen pressure (0.40 MPa) for 1 hour. Hydrogen was discharged and replaced with nitrogen, then compound 21 (514 mg) dissolved in 2-methyltetrahydrofuran (1.5 mL) was added, and the mixture was stirred. Nitrogen was discharged and replaced with hydrogen, and the mixture was stirred under hydrogen pressure (0.20 MPa) for a total of 8 hours and 30 minutes. Hydrogen was discharged and replaced with nitrogen, and the activated carbon-supported palladium was then removed by filtration. The cake was washed three times with 2-methyltetrahydrofuran (500 μL), and concentrated to dryness under reduced pressure together with the filtrate to obtain compound 37 (503 mg).
LCMS (ESI) of compound 37: retention time: 8.04 minutes, m/z = 1205.0 $[M+H]^+$ (LCMS analysis conditions method FC2)

Example 2-18

Synthesis of compound 38: tert-butyl [(S)-2-[(S)-3-[(S)-2-(1-[(2S,4R)-1-[(S)-2-amino-[3-methoxy-4-(trifluoromethyl) phenyl]butanoyl]-4-ethoxy-N-methylpyrrolidine-2-carboxyamido]-N-methylcyclobutane-1-carboxyamido)-2-cyclopentyl-N- methylacetamido]-4-[dimethylamino]-N-methyl-4-oxobutanamido]pentanoyl]-L-isoleucyl-L-prolinate tert-butyl

[Formula 100]

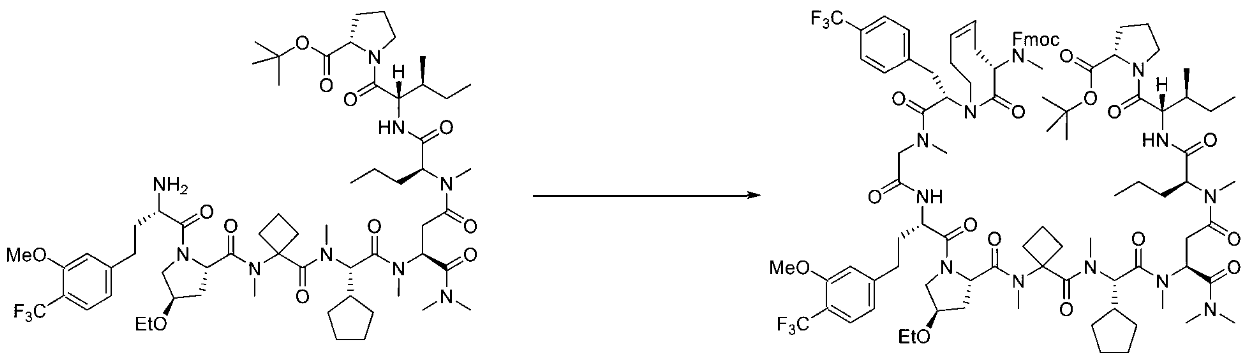

To a reaction vessel, compound 28 (122 mg) obtained in Example 2-3, 2-MeTHF (1.82 mL), acetonitrile (460 μL), and compound 37 (200 mg) were added, and the mixture was stirred at room temperature. NMM (80 μL) and HATU (95.4 mg) were added, and the mixture was stirred at an external temperature of 25°C for 2 hours. After 2 hours, the reaction mixture was sampled for sample preparation (sample preparation method 2), and the reaction conversion rate was confirmed to be 99% or more by HPLC analysis (calculation expression 1 for the reaction conversion rate). A 5% aqueous sodium carbonate solution (1.40 mL) and 1-methylimidazole (13.2 μL) were added, and the mixture was stirred for 30 minutes. After 30 minutes, 10% aqueous ammonia (1.4 mL) was added at room temperature, and the mixture was stirred. After discharging the aqueous layer by a liquid separation operation, a 5% aqueous sodium hydrogen sulfate solution (1.4 mL) was added to the organic layer and the mixture was stirred. After a liquid separation operation, the aqueous layer was discharged. The resulting organic layer was washed with a 5% aqueous sodium carbonate solution (1.4 mL). The resulting organic layer was concentrated under reduced pressure at an external temperature of 40°C to obtain 290 mg of crude

compound 38 (apparent yield 94.0%).
LCMS (ESI) of compound 38: retention time: 12.0 minutes, (LCMS analysis conditions method FC2)

To a reaction vessel, the solution (17.6 kg) of compound 37 (3.41 kg) in 2-MeTHF was added at room temperature, followed by sequential addition of 2-MeTHF (12.3 kg), compound 28-toluene monosolvate (2.38 kg), and acetonitrile (6.20 kg). The external temperature was set to 5°C, NMM (1.26 kg) and HATU (1.65 kg) were added at an internal temperature of 30°C or lower, and the mixture was stirred at an internal temperature of 25°C for 1 hour. After confirming that the reaction conversion rate was 99% or more, a 5% aqueous potassium carbonate solution (23.9 kg) and 1-methylimidazole (234 g) were added, the mixture was stirred for 30 minutes at room temperature, and the aqueous layer was discharged by liquid-liquid separation. The resulting organic layer was washed with 10% aqueous ammonia (23.0 kg), a 5% aqueous sodium hydrogen sulfate solution (23.9 kg), and a 5% aqueous sodium carbonate solution (23.9 kg), and then concentrated at an external temperature of 40°C to 10 L. For the concentrated solution, addition of acetonitrile (16.2 kg) and concentration to 10 L were repeated twice to obtain a solution (19.4 kg) of compound 38 in acetonitrile.

Example 2-19

Synthesis of compound 39:tert-butyl (S)-2-[(S)-3-[(S)-2-cyclopentyl-2-[1-[(2S,4R)-4-ethoxy-1-[(S)-4-[3-methoxy-4-(trifluoromethyl)phenyl]-2-[(2-[(S)-N-methyl-2-[(R,Z)-3-(methylamino)-2-oxo-3,4,7,8-tetrahydroazocin-1(2H)-yl]-3-[4-(trifluoromethyl)phenyl]propanamidolacetamido)butanoyl]-N-methylpyrrolidine-2-carboxyamido]-N-methylcyclobutane-1-carboxyamido]-N-methylacetamido]-4-(dimethylamino)-N-methyl-4-oxobutanamido]pentanoyl]-L-isoleucyl-L-prolinate

[Formula 101]

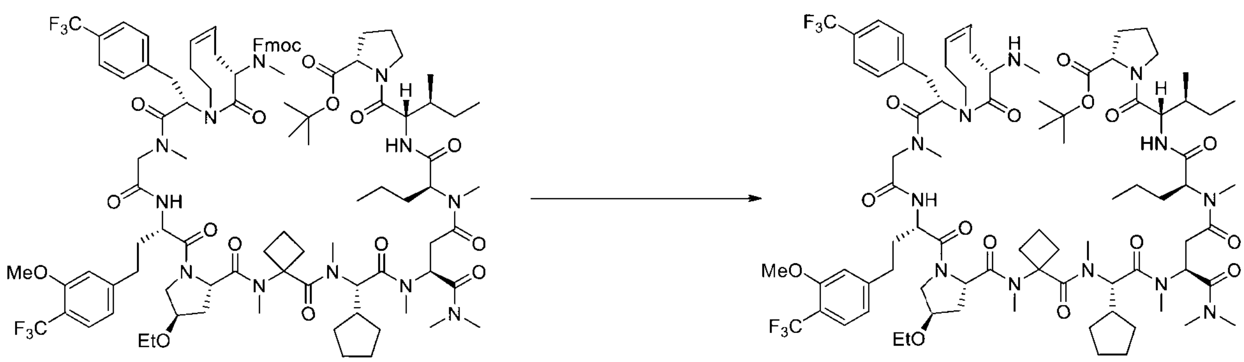

To a reaction vessel, compound 38 (113 mg), acetonitrile (453 μL), and DBU (27.5 μL) were sequentially added, and the mixture was stirred at an external temperature of 25°C for 1 hour. After 1 hour, the reaction mixture was sampled for sample preparation (sample preparation method 2), and the reaction conversion rate was confirmed to be 99% or more by HPLC analysis (calculation expression 1 for the reaction conversion rate). To the reaction vessel, sodium hydrogen sulfite (23.1 mg), triethylamine (34.1 μL), and water (27.6 μL) were added, the mixture was stirred for 2 hours, and the reaction conversion rate of dibenzofulvene was confirmed to be 99% or more by HPLC analysis. To the reaction vessel, toluene (565 μL) and 10% aqueous ammonia (1.13 mL) were added, the mixture was stirred, and the aqueous layer was discharged by a liquid separation operation. The organic layer was further washed twice with 10% aqueous ammonia (1.13 mL). The resulting organic layer was concentrated under reduced pressure with the external temperature set to 40°C to obtain 94.1 mg of crude compound 39 (apparent yield 94.0%). LCMS of compound 39: retention time: 8.14 minutes, (LCMS analysis conditions method FC2)

To a reaction vessel, the solution of compound 38 (4.67 kg) in acetonitrile (14.9 kg) was added, acetonitrile (4.53 kg) was further added, the internal temperature was cooled to 15°C or lower, and DBU (1.65 kg) was added at an internal temperature of 30°C or lower. After stirring at 25°C for 1 hour and confirming that the reaction conversion rate was 99% or more, the internal temperature was cooled again to 15°C or lower. Triethylamine (1.02 kg), water (1.14 kg), and sodium hydrogen sulfite (925 g) were sequentially added at the internal temperature not exceeding 30°C, and the mixture was stirred for 1 hour. After stirring, the reaction solution was stirred with the addition of toluene (20.4 kg) and 10% aqueous ammonia (46.7 kg), and the aqueous layer was discharged by liquid-liquid separation. To the organic layer, acetonitrile (18.4 kg) and 10% aqueous ammonia (46.7 kg) were added with stirring, and then discharging of the aqueous layer by liquid-liquid separation were repeated twice. The organic layer was washed by adding 5% saline (23.4 kg). Liquid-liquid separation was performed, and the organic layer was concentrated at an external temperature of 60°C to 9.3 L. For the

concentrated solution, addition of 2-MeTHF (16.0 kg) and concentration at an external temperature of 60°C to 9.3 L were repeated twice to obtain a solution (7.72 kg) of compound 39 in 2-MeTHF.

Example 2-20

Synthesis of compound 40: (S)-2-[(S)-3-[(S)-2-cyclopentyl-2-[1-[(2S,4R)-4-ethoxy-1-[(S)-4-[3-methoxy-4-(trifluoromethyl)phenyl]-2-[(2-[(S)-N-methyl-2-[(R,Z)-3-(methylamino)-2-oxo-3,4,7,8-tetrahydroazocin-1(2H)-yl]-3-[4-(trifluoromethyl)phenyl]propanamido]acetamido)butanoyl]-N-methylpyrrolidine-2-carboxyamido]-N-methylcyclobutane-1-carboxyamido]-N-methylacetamido]-4-(dimethylamino)-N-methyl-4-oxobutanamido]pentanoyl]-L-isoleucyl-L-proline

[Formula 102]

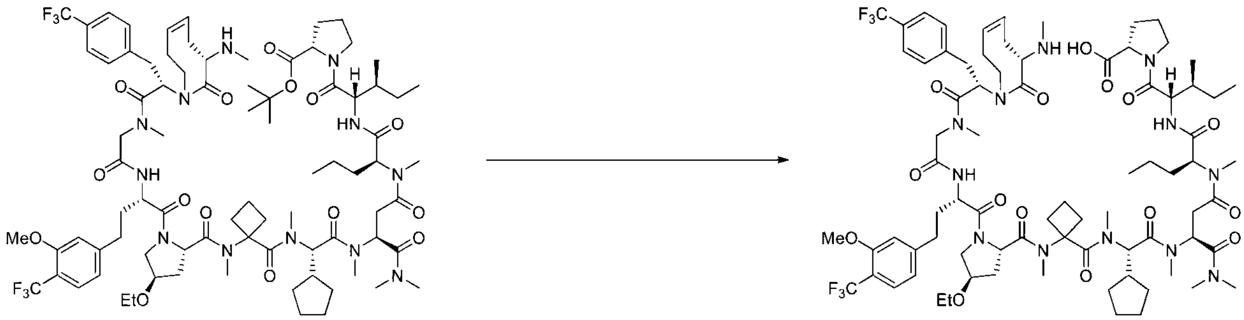

To a reaction vessel, compound 39 (94.0 mg) and 2-MeTHF (470 μL) were added, and the external temperature was cooled to 5°C. HMDS (37.0 μL) and TMSOTf (21.0 μL) were sequentially added, the external temperature was raised to 25°C, and the mixture was stirred for 1 hour. After 1 hour, the reaction mixture was sampled for sample preparation (sample preparation method 2), and the reaction conversion rate was confirmed to be 99% or more by HPLC analysis (calculation expression 1 for the reaction conversion rate). After adding 2-MeTHF (752 μL) to the reaction vessel, the external temperature was again cooled to 5°C. A 5% aqueous dipotassium hydrogen phosphate solution (658 μL) was added at an internal temperature of 10°C, the mixture was stirred, and the aqueous layer was discharged by a liquid separation operation. The organic layer was stirred with the addition of acetonitrile (188 μL) and a 5% aqueous sodium hydrogen sulfate solution (658 μL), and the aqueous layer was discharged by a liquid separation operation. The organic layer was stirred with the addition of a 5% aqueous sodium carbonate solution (658 μL), and the aqueous layer was discharged by a liquid separation operation. The resulting organic layer was washed with 5% saline (658 μL) and concentrated under reduced pressure with the external temperature set to 40°C. As a result of HPLC measurement using a standard sample, the amount of the obtained compound 40 was 72.4 mg (3-step yield: 62.8%). LCMS of compound 40: retention time: 6.86 minutes, (LCMS analysis conditions method FC2)

To a reaction vessel, the solution of compound 39 (3.74 kg) in 2-MeTHF (11.9 kg), 2-MeTHF (7.78 kg) and HMDS (1.12 kg) were added. After cooling the internal temperature to 5°C or lower, TMSOTf (1.03 kg) was added at the internal temperature not exceeding 30°C. After that, the internal temperature was raised to 25°C, and the mixture was stirred for 2 hours. After confirming that the reaction conversion rate was 99% or more, 2-MeTHF (25.6 kg) and acetonitrile (5.88 kg) were added, the internal temperature was cooled to 5°C or lower, and a 5% aqueous dipotassium hydrogen phosphate solution (26.2 kg) was slowly added with keeping the internal temperature below 30°C. After stirring and discharging of the aqueous layer by liquid-liquid separation, the organic layer was sequentially washed with a 5% aqueous sodium hydrogen sulfate solution (26.2 kg), a 5% aqueous sodium carbonate solution (26.2 kg), and a 5% saline (26.2 kg). The organic layer obtained at an external temperature of 50°C was concentrated to 9.4 L, and addition of acetonitrile (7.36 kg) and concentration again to 9.4 L were repeated twice. The concentrated solution was stirred with the addition of acetonitrile (8.82 kg) and heptane (25.6 kg), and the lower layer was collected by liquid-liquid separation to obtain a solution (17.7 kg) of compound 40 in acetonitrile. Based on the quantification by qNMR, the 3-step yield of compound 40 was 93.4%.

Example 2-21

Synthesis of compound 1 (cyclization position C)

[Formula 103]

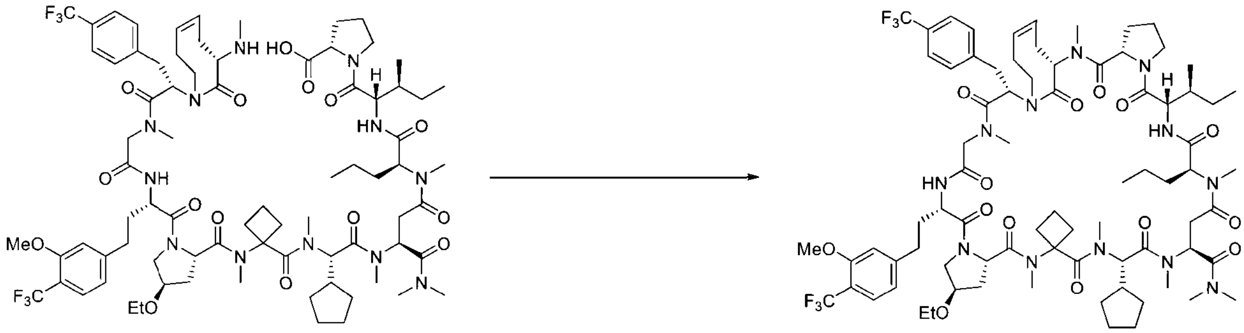

To a reaction vessel, compound 40 (72.4 mg), acetonitrile (17.8 mL), and DIPEA (59 μL) were added. HATU (65.1 mg) was added at an external temperature of 25°C, and the mixture was stirred for 2 hours. The reaction conversion rate was confirmed to be 99% or more by HPLC analysis (LCMS analysis conditions method FC2) (calculation expression 1 for the reaction conversion rate). The reaction mixture was sampled for sample preparation (sample preparation method 1), and it was confirmed that compound 1/cyclic dimer = 97.6/2.4 (calculation expressions 1 and 2 for cyclization selectivity) by HPLC analysis. The external temperature of the reaction vessel was set to 40°C, and the reaction solution was concentrated to 2.14 mL. To the resulting concentrated product, MTBE (1.25 mL), heptane (89 μL), and a 2.5% aqueous ammonia solution (890 μL) were added at room temperature, and the mixture was stirred. After discharging the aqueous layer by liquid-liquid separation, the resulting organic layer was sequentially washed with 4% sulfuric acid (1.25 mL) and a 5% aqueous dipotassium hydrogen phosphate solution (890 μL). The resulting organic layer was washed with a 0.5% aqueous sodium chloride solution (890 μL × 2). As a result of HPLC analysis using a standard sample for the resulting organic layer (analysis conditions: method cyc), the amount of the obtained compound 1 was 61.1 mg (85.4% yield).

LCMS of compound 1: retention time: 6.88 minutes, (LCMS analysis conditions method FC2);
HPLC of compound 1: retention time: 18.40 minutes; HPLC of cyclic dimer: retention time 23.96 minutes (method cyc)

To a reaction vessel, HATU (949 g) and acetonitrile (25.6 kg) were added and dissolved to prepare a solution of HATU in acetonitrile. In another vessel, the solution of compound 40 (1.31 kg) in acetonitrile (5.52 kg), acetonitrile (19.1 kg), and N-methylmorpholine (504 g) were added to prepare a solution of compound 40 in acetonitrile. At an external temperature of 25°C, the prepared solution of compound 40 in acetonitrile was added dropwise to the solution of HATU in acetonitrile over 4 hours and 22 minutes, and washing was performed using acetonitrile (1.04 kg). After the dropwise addition, the mixture was further stirred for 1 hour. The reaction conversion rate was confirmed to be 99% or more by HPLC analysis (LC analysis conditions method cyc) (calculation expression 1 for the reaction conversion rate). The reaction mixture was sampled for sample preparation (sample preparation method 1), and it was confirmed that compound 1/cyclic dimer = 98.5/1.5 (calculation expressions 1 and 2 for cyclization selectivity) by HPLC analysis. The external temperature was set to 40°C, and the reaction mixture was concentrated to about 12.5 L. To the resulting concentrated product, MTBE (14.5 kg) and a 2.5% aqueous ammonia solution (13.3 kg) were added at room temperature, and the mixture was stirred for 34 minutes. After discharging the aqueous layer by liquid-liquid separation, the resulting organic layer was sequentially washed with 5% sulfuric acid (13.1 kg), a 5% aqueous dipotassium hydrogen phosphate solution (13.1 kg), and a 0.5% aqueous sodium chloride solution (13.07 kg). Acetonitrile (2.00 kg) was added to the resulting organic layer, which was further washed with a 0.5% aqueous sodium chloride solution (13.1 kg). After performing solvent replacement of the resulting organic layer with ethanol, as a result of HPLC analysis using a standard sample (analysis conditions: method cyc), the amount of the obtained compound 1 was 1.14 kg (88.3% yield).
HPLC of compound 1: retention time: 18.67 minutes; HPLC of cyclic dimer: retention time: 24.07 minutes (method cyc)

The suppression of cyclic dimer depending on the difference in cyclization position will be discussed.

As a synthesis method for compound 1, the synthesis method according to International Publication No. WO 2022/234853 is known, but as a by-product of the cyclization reaction, a cyclic dimer derived from an intermolecular reaction is produced. In the cyclization reaction at cyclization position A described in International Publication No. WO 2022/234853, although the cyclization was performed under pseudo-dilution conditions by reverse dropwise addition of compound 23 over 6 hours, the cyclic dimer was produced in 25% (Example 1-26 [compound 1/cyclic dimer = 75/25]). The present inventors have made investigations changing the cyclization position and have found that cyclization by cyclization position B can reduce the amount of cyclic dimer despite the short time of dropwise addition (Example 1-24-2 [compound 1/cyclic dimer = 98/2]). Furthermore, in cyclization by cyclization position C, the results showed that the amount of cyclic dimer was reduced even under high concentration conditions without using reverse dropwise addition (Example 2-21 [compound 1/cyclic dimer = 97.6/2.4]). The above facts indicate that the cyclization position B and the

cyclization position C in the present invention are efficient cyclization positions in the production of compound 1, with lower amounts of cyclic dimer, which is an impurity, compared to the conventional cyclization position A.

Example 3 Crystallization

Example 3-1

The amorphous powder of Compound 1 (77.4 mg) was dissolved in dimethyl sulfoxide (0.387 mL), and the solution (0.015 mL) was freeze-dried at -20°C for 3 days. To the resulting product, a mixed solvent of 2-butanone-heptane (1:4 (v/v), 0.015 mL) was added and the mixture was shaken at room temperature for 7 days to obtain a crystal of compound 1. The obtained crystal was confirmed to be a dimethyl sulfoxide/heptane/water solvate crystal (form G) by single crystal X-ray structural analysis (Example 4-7). Figure 1 shows the crystal structure.

Example 3-2

The amorphous powder of Compound 1 (11.4 mg) was dissolved in acetone (34.2 μL), and about 0.1 mg of the crystal obtained in Example 3-1 was added. Thereafter, heptane (22.8 μL) was added, and the mixture was shaken at room temperature for 3 hours. Furthermore, heptane (22.8 μL) was added, and the mixture was shaken at room temperature for 2 hours. Furthermore, heptane (22.8 μL) was added, and the mixture was shaken at room temperature for 18 hours, and then filtered and dried under reduced pressure. The resulting crystal was a hydrate crystal (form A) of compound 1. Figure 2 shows the powder X-ray diffraction pattern of the obtained crystal (Example 4-3).

Example 3-3

The amorphous powder of Compound 1 (10.7 mg) was dissolved in 2-propanol (32.1 μL), and about 0.1 mg of the crystal obtained in Example 3-1 was added. Thereafter, heptane (21.4 μL) was added, and the mixture was shaken at room temperature for 3 hours. Furthermore, heptane (21.4 μL) was added, and after shaking at room temperature for 2 hours, heptane (21.4 μL) was added and the mixture was shaken at room temperature for 3 hours. Heptane (21.4 μL) was further added, and the mixture was shaken at room temperature for 15 hours, thereby obtaining a crystal (form E) of compound 1. Single crystal X-ray structural analysis was performed on the obtained crystal (Example 4-8), and it was confirmed to be a 2-propanol/heptane/water solvate. Figure 3 shows the crystal structure. The obtained crystal (form E) was dried under reduced pressure, and powder X-ray diffraction measurement was performed (Example 4-3). Figure 4 shows the results. The diffraction pattern indicates form A, confirming that the 2-propanol/heptane/water solvate crystal (form E) is transformed into the hydrate crystal (form A) after drying under reduced pressure.

Example 3-4

To the form A crystal (5.9 mg) of compound 1, a mixed solvent of ethanol-heptane (1:1 (v/v), 0.017 mL) and one grain of glass bead were added. Using a shaker (BioShaker M·BR-022UP manufactured by TAITEC CORPORATION), the mixture was shaken at 37°C, 2000 rpm for 7 days, and then filtered and dried under reduced pressure to obtain a crystal (form B) of compound 1. Figure 5 shows the powder X-ray diffraction pattern of the obtained crystal (Example 4-3).

Example 3-5

To the form A crystal (5.8 mg) of compound 1, a mixed solvent of ethyl acetate-heptane (1:1 (v/v), 0.017 mL) and one grain of glass bead were added. Using a shaker (BioShaker M·BR-022UP manufactured by TAITEC CORPORATION), the mixture was shaken at 37°C, 2000 rpm for 7 days, and then filtered and dried under reduced pressure to obtain a crystal (form B) of compound 1. Figure 6 shows the powder X-ray diffraction pattern of the obtained crystal (Example 4-3).

Example 3-6

The amorphous powder of Compound 1 (9.0 mg) was dissolved in ethanol (27.0 μL), heptane (27.0 μL) was added, and the mixture was shaken at room temperature for 4 minutes. About 0.1 mg of the crystal obtained in Example 3-4 was added, and the mixture was shaken at room temperature for 1 minute to obtain a crystal of compound 1. Single crystal X-ray structural analysis was performed (Example 4-9), confirming that the obtained crystal is an ethanol/water solvate crystal (form H). Figure 7 shows the crystal structure. The obtained crystal (form H) was dried under reduced pressure, and powder X-ray diffraction measurement was performed (Example 4-3). Figure 8 shows the results. The diffraction pattern indicates form B, confirming that the ethanol/water solvate crystal (form H) is transformed into the hydrate crystal (form B)

after drying under reduced pressure.

Example 3-7

The amorphous powder of Compound 1 (10.9 mg) was dissolved in ethyl acetate (32.7 μL), heptane (32.7 μL) was added, and the mixture was shaken at room temperature for 4 minutes. About 0.1 mg of the crystal obtained in Example 3-5 was added, and the mixture was shaken at room temperature for 1 minute, thereby obtaining a crystal of compound 1. Single crystal X-ray structural analysis was performed (Example 4-9), confirming that the obtained crystal is an ethyl acetate/water solvate crystal (form C). Figure 9 shows the crystal structure. The obtained crystal (form C) was dried under reduced pressure, and powder X-ray diffraction measurement was performed (Example 4-3). Figure 10 shows the results. The diffraction pattern indicates form B, confirming that the ethyl acetate/water solvate crystal (form C) is transformed into the hydrate crystal (form B) after drying under reduced pressure.

Example 3-8

The amorphous powder of Compound 1 (112.3 mg) was dissolved in acetone (337 μL), and after adding about 0.1 mg of the form A crystal as a seed crystal, heptane (112 μL) was added and the mixture was stirred at room temperature for 2 minutes using a stirrer. Heptane (112 μL) was added again, and the mixture was stirred at room temperature for 10 minutes. Since dissolution of the seed crystal was confirmed, about 0.1 mg of the form A crystal was added again, and the mixture was stirred at room temperature for 1 hour. Thereafter, heptane (225 μL) was added, and the mixture was stirred at room temperature for 20 minutes. Furthermore, about 0.1 mg of the form A crystal was added, and the mixture was stirred at room temperature for 40 minutes. Heptane (337 μL) was added, and the mixture was stirred at room temperature for 16 hours. The resulting product was filtered under reduced pressure and vacuum dried for one day, thereby obtaining 86.3 mg of a form A crystal. For the form A crystal, powder X-ray diffraction measurement was performed (Example 4-1). Figure 11 shows the measurement results. Also, Figure 12 shows the results of thermogravimetry-differential thermal analysis of the form A crystal, and Figure 13 shows the results of $^1$H-NMR measurement. While a weight reduction of 3 wt% was observed in Figure 12, only 1 wt% of acetone was observed in Figure 13, which led to the conclusion that the crystal (form A) was a hydrate crystal and acetone was present as a residual solvent.

Example 3-9

The amorphous powder of Compound 1 (61.7 mg) was dissolved in 2-propanol (185 μL), heptane (123 μL) was added, and the mixture was stirred at room temperature for 3 minutes using a stirrer. About 0.1 mg of the form A crystal was added as a seed crystal, and then the mixture was stirred at room temperature for 30 minutes. Thereafter, heptane (123 μL) was added, and the mixture was stirred at room temperature for 2 hours. Furthermore, heptane (185 μL) was added, and the mixture was stirred at room temperature for 18 hours. The reaction product was filtered under reduced pressure and vacuum dried for one day, thereby obtaining 23.3 mg of a form A crystal. For the form A crystal, powder X-ray diffraction measurement was performed (Example 4-3). Figure 14 shows the measurement results. Also, Figure 15 shows the results of thermogravimetry-differential thermal analysis of the form A crystal, and Figure 16 shows the results of $^1$H-NMR measurement. Since the powder X-ray diffraction pattern in Figure 14 matched the pattern of Example 3-8, a weight reduction of 2 wt% was observed in Figure 15, and only 1 wt% of 2-propanol was observed in Figure 16, it was concluded that the form A crystal was a hydrate crystal and 2-propanol was present as a residual solvent.

Example 3-10

The amorphous powder of Compound 1 (60.1 mg) was dissolved in acetone (180 μL), and the mixture was stirred using a stirrer. Heptane (120 μL) was added, and the mixture was stirred at room temperature for 1 minute using a stirrer. About 0.1 mg of the form A crystal was added, and the mixture was stirred for 30 minutes. Heptane (120 μL) was added again, and the mixture was stirred at room temperature for 1 minute. About 0.1 mg of the form A crystal was added again, and the mixture was stirred at room temperature for 2.5 hours. Furthermore, heptane (180 μL) was added, and the mixture was stirred at room temperature for 18 hours. To the resulting mixture, about 10 grains of zirconia beads (1 mm) were added, and the mixture was stirred at room temperature overnight to obtain an acetone/heptane/water solvate crystal (form F) of compound 1 as a wet powder. The obtained crystal (form F) was sealed in a glass capillary and powder X-ray diffraction measurement was performed (Example 4-2), and the following were observed as major peaks: 6.99°, 8.49°, 9.49°, 9.88°, 10.21°, 11.81°, 12.32°, 12.75°, 13.17°, 13.94°, 14.92°, 15.20°, 15.64°, 16.78°, 17.01°, and 17.47°. Figure 17 shows the measurement results.

Example 3-11

The amorphous powder of Compound 1 (10.0 mg) was dissolved in acetone (30 μL), and the mixture was stirred using a stirrer. Heptane (20 μL) was added, and the mixture was stirred at room temperature for 1 minute using a stirrer. About 0.1 mg of the form A crystal was added, and the mixture was stirred for 5 minutes. Heptane (20 μL) was added again, and the mixture was stirred at room temperature for 2 hours to obtain a single crystal with good quality. The obtained crystal was confirmed to be an acetone/heptane/water solvate crystal (form F) by single crystal structural analysis (Example 4-8). Figure 18 shows the crystal structure.

Example 3-12

Powder X-ray diffraction measurement was performed (Example 4-1) on the crystal (form A) obtained in Example 1-25, and the following were observed as major peaks: 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73°. Figure 19 shows the measurement results. Also, Figure 20 shows the results of thermogravimetry-differential thermal analysis. In order to confirm the influence of humidity on the crystal form of the form A, powder X-ray diffraction measurement was performed at each humidity (Example 4-4). Figure 21 shows the results. It was confirmed that the form A is maintained at a relative humidity of 10% to 90%, but it is transformed into the form J at a relative humidity of 0%. The form J has, as major peaks, 6.95°, 7.33°, 7.93°, 8.84°, 9.45°, 9.97°, 10.44°, 11.19°, 12.43°, 12.93°, 13.46°, 14.36°, 14.74°, 15.21°, 15.87°, 16.76°, 20.87°, and 22.97°.

Example 3-13

The concentrated dry product containing compound 1 (29.4 g) obtained in Example 1-24-1 was dissolved in acetone (90 mL). Dissolved compound 1 was loaded into Biotage Sfar C18 Duo 100 Å 30 μm 240 g, and eluted with 0.1% formic acid-water/0.1% formic acid-acetonitrile = 9/1 → 0/10). The eluent was collected and allowed to stand still for two days, yielding a single crystal with good quality. The obtained crystal was dried under reduced pressure at 40°C for 16 hours to obtain 15 mg of a dry powder of the crystal (form B). Powder X-ray diffraction measurement was performed (Example 4-1) on the obtained crystal (form B), and the following were observed as major peaks: 4.99°, 8.65°, 9.85°, 10.84°, 11.32°, 12.35°, 13.20°, 14.44°, 15.20°, 16.03°, 16.69°, 17.21°, 18.82°, 19.49°, and 20.03°. Figure 22 shows the measurement results. Also, Figure 23 shows the results of thermogravimetry-differential thermal analysis. In order to confirm the influence of humidity on the crystal form of the form B, powder X-ray diffraction measurement was performed at each humidity (Example 4-5). Figure 24 shows the results. It was confirmed that the form B is maintained at a relative humidity of 30% to 90%, but it is transformed into the form Y at a relative humidity of 0%. The form Y has, as major peaks, 5.13°, 8.33°, 8.82°, 9.80°, 10.32°, 11.39°, 12.58°, 13.28°, 14.80°, 15.40°, 15.88°, 17.12°, 17.67°, 19.18°, 19.54°, and 21.24°.

Example 3-14

The crystal obtained in Example 3-13 was confirmed to be a hydrate crystal (form B) by single crystal X-ray structural analysis (Example 4-9). Figure 25 shows the crystal structure.

Example 3-15

The amorphous powder of Compound 1 (77.4 mg) was dissolved in dimethyl sulfoxide (0.387 mL) and the solution (0.015 mL) was freeze-dried at -20°C for 3 days. To the resulting freeze-dried product, a mixed solvent of 1,4-dioxane-heptane (1:4 (v/v), 0.015 mL) was added, and the mixture was shaken at room temperature for 7 days to obtain a crystal of compound 1. The obtained crystal was confirmed to be a 1,4-dioxane/water solvate crystal (form D) by single crystal X-ray structural analysis (Example 4-8). Figure 26 shows the crystal structure.

Example 3-16

The crystal (form A, 6.8 mg) of compound 1 obtained in Example 3-8 was dissolved in dimethyl sulfoxide (0.12 mL), and the mixture was shaken and stirred at room temperature for 13 days. Furthermore, water (1 μL) was added and the mixture was stirred at room temperature for 34 days to obtain a crystal of compound 1. The obtained crystal was confirmed to be a dimethyl sulfoxide/water solvate crystal (form L) by single crystal X-ray structural analysis (Example 4-8). Figure 27 shows the crystal structure. Powder X-ray diffraction measurement (Example 4-3) on the crystal obtained by performing the same procedure confirmed that the form L is a solvate crystal that is amorphized by drying under reduced pressure. Figure 28 shows the powder X-ray diffraction patterns.

Example 3-17

To the crystal (form B, 45.8 mg) of compound 1, propylene glycol (0.12 mL) was added, and the mixture was stirred at room temperature for 5 minutes. Furthermore, propylene glycol (0.020 mL) was added, and the mixture was stirred at 40°C for 5 minutes. Thereafter, propylene glycol (0.070 mL) was added, the mixture was stirred at 40°C for 5 minutes and further allowed to stand still for 5 hours. Propylene glycol (0.070 mL) was further added, and the mixture was stirred at 35°C for overnight. Then, propylene glycol (0.070 mL) was added and the mixture was stirred for about 1 minute to obtain a wet powder of compound 1. The obtained wet powder was confirmed to be a form M crystal by powder X-ray diffraction measurement (Example 4-3). Powder X-ray diffraction measurement confirmed that the form M crystal is transformed into a form N crystal after dried under reduced pressure at room temperature for overnight. Figure 29 shows the powder X-ray diffraction patterns of the crystals (form M and form N). Figure 30 shows the results of thermogravimetry-differential thermal analysis. A weight reduction of about 11% was observed at 157°C, which corresponds to 2.5 molecules of propylene glycol for compound 1. From these results, it was confirmed that the form M crystal and the form N crystal are propylene glycol solvates.

Example 3-18

The crystal (form B, 12.66 mg) of compound 1 was dissolved in propylene glycol (0.10 mL), and the mixture was stirred at room temperature for 20 days. Furthermore, about 0.1 mg of the wet powder obtained in Example 3-17 was added, and the mixture was stirred at room temperature for 2.5 hours. Propylene glycol (0.10 mL) was added, the mixture was heated and stirred on a hot plate at 60°C for 30 minutes, and then stirred at room temperature for 4.5 hours, thereby obtaining a crystal of compound 1. The obtained crystal was confirmed to be a propylene glycol/water solvate crystal (form M) by single crystal X-ray structural analysis (Example 4-8). Figure 31 shows the crystal structure.

Example 3-19

The crystal (form A) obtained in Example 1-25 was weighed in an open aluminum pan in each of 7.773 mg, 7.258 mg, 8.665 mg, and 13.047 mg (total of 36.7 mg), and heated using a thermogravimetry-differential thermal analysis equipment (Example 4-11). Powder X-ray diffraction measurement was performed (Example 4-2) on the obtained crystal, and the following were observed as major peaks: 7.49°, 7.91°, 8.14°, 9.11°, 9.33°, 11.04°, 11.71°, 12.52°, 13.21°, 13.70°, 14.82°, 15.13°, 15.52°, 15.68°, 17.22°, and 17.51°. Figure 32 shows the powder X-ray diffraction pattern of the form K crystal. Also, Figure 33 shows the results of thermogravimetry-differential thermal analysis of the obtained crystal (form K), and Figure 34 shows the results of $^1$H-NMR measurement. While a weight reduction of about 1.8 wt% was observed in Figure 33, the $^1$H-NMR measurement exhibited no peaks other than those corresponding to the measurement solvent DMSO (including tetramethylsilane), compound 1, and water. From these results, the form K crystal was concluded to be a hydrate crystal.

Example 3-20

A crude solution of compound 1 with low purity was concentrated to dryness and dried under reduced pressure at 40°C to prepare a crude amorphous solid. The crude amorphous solid (20 mg) of compound 1 was dissolved in 2-propanol (60 μL), and the mixture was stirred using a stirrer. Heptane (60 μL) was added. About 0.1 mg of the form A seed crystal was added, and the mixture was stirred at room temperature for 1 hour. Since no crystal precipitation was observed, 2-propanol (20 μL) was further added, but oiling-out occurred and no crystal was obtained.

Example 3-21

A crude solution of compound 1 was concentrated to dryness and dried under reduced pressure at 40°C to prepare a crude amorphous solid. The crude amorphous solid (20 mg) of compound 1 was dissolved in ethanol (60 μL), and the mixture was stirred using a stirrer. Heptane (60 μL) was added. Two identical solutions were prepared, and about 0.1 mg of the form A seed crystal was added to one and about 0.1 mg of the form B seed crystal to the other, and the solutions were stirred at room temperature for 1 hour. In both cases, a crystal was precipitated and filtered to obtain a wet powder. Form B crystal was obtained despite the conditions under which the form A seed crystal had been added. Meanwhile, form B crystal was obtained under the conditions where the form B seed crystal had been added.

Example 3-22

A crude solution (17.7 kg) was concentrated at an external temperature of 50°C to 7 L. Part of the concentrated

solution (4.31 kg) was placed in a reactor, and ethanol (1.16 kg) was added. Thereafter, the mixture was stirred at an external temperature of 42°C. Purified water (1.01 kg) and the seed crystal (form B, 5.17 g) were added, and the mixture was stirred for 1 hour. Subsequently, purified water (2.08 kg) was added dropwise over 80 minutes. Furthermore, purified water (1.04 kg) was added dropwise over 40 minutes. The internal temperature was lowered to 25°C or lower over 48 minutes. The mixture was stirred overnight, filtered, and the crystal was washed in two batches with ethanol/purified water (2.50 L/1.66 L mixture). The resulting wet crystal was dried under reduced pressure at an external temperature of 50°C to obtain compound 1 (467 g) as a white solid. Powder X-ray diffraction measurement was performed (Example 4-3) on the obtained crystal (form B) of compound 1. Figure 36 shows the results. The crystal was confirmed to be a form B crystal.

Example 4 Evaluation of crystal

Example 4-1

The powder X-ray diffraction measurement for Example 3-8, Example 3-12, Example 3-13, and Example 3-19 was performed under the following conditions.

Measurement apparatus: SmartLab System, D/Tex Ultra detector (manufactured by Rigaku Corporation)
Radiation source: CuKα1
Tube voltage: 45 kV
Tube current: 200 mA
Scanning range: 4 to 50°
Sampling width: 0.005°

Example 4-2

The powder X-ray diffraction measurement for Example 3-10 was performed under the following conditions to evaluate the crystal form in the suspension.

Measurement apparatus: SmartLab System, D/Tex Ultra detector (manufactured by Rigaku Corporation)
Radiation source: CuKα1
Tube voltage: 45 kV
Tube current: 200 mA
Scanning range: 4 to 50°
Sampling width: 0.005°
Measurement: The sampled suspension was packed in a capillary for X-ray crystallography and measured.

Example 4-3

The powder X-ray diffraction measurement for Example 3-2, Example 3-3, Example 3-4, Example 3-5, Example 3-6, Example 3-7, Example 3-9, Example 3-16, Example 3-17, Example 3-22, Example 5-1, Example 5-2, Example 5-3, Example 5-4, Example 5-5, Example 5-6, Example 5-7, Example 5-8, and Example 5-9 was performed under the following conditions.

Measurement apparatus: D8 Discover, 2D VANTEC-500 solid state detector (manufactured by Bruker)
Radiation source: CuKα
Tube voltage and tube current: 50 kV and 1000 μA
Measurement range: 5 to 31°
Exposure time: 40 seconds

Example 4-4

The powder X-ray diffraction measurement for Example 3-12 was performed under the following conditions to evaluate the crystal form at each relative humidity. The measurement was carried out at the time points between dropping the relative humidity to 0% and raising it to 90%: 0% (Figure 21(A)), 10% (Figure 21(B)), 20% (Figure 21(C)), 50% (Figure 21(D)), and 90% (Figure 21(E)).

Measurement apparatus: SmartLab System, D/Tex Ultra detector, humidity generator HUM-SL (manufactured by Rigaku Corporation)

Radiation source: CuKα1
Tube voltage: 45 kV
Tube current: 200 mA
Scanning range: 4 to 35°
Scanning speed: 5°/min
Sampling width: 0.02°
Humidity change conditions:

[Table 1]

| Set temperature (°C) | Relative humidity (%) | Holding time (min) |
|---|---|---|
| 35.0 | 30 | 10 |
| 35.0 | 20 | 20 |
| 35.0 | 10 | 20 |
| 35.0 | 0 | 40 |
| 35.0 | 10 | 20 |
| 35.0 | 20 | 20 |
| 35.0 | 30 | 20 |
| 35.0 | 40 | 20 |
| 35.0 | 50 | 20 |
| 35.0 | 70 | 20 |
| 35.0 | 90 | 20 |
| 35.0 | 70 | 20 |
| 35.0 | 50 | 20 |
| 35.0 | 40 | 20 |
| 35.0 | 30 | 20 |

Example 4-5

The powder X-ray diffraction measurement for Example 3-13 was performed under the following conditions to evaluate the crystal form at each relative humidity. The measurement was carried out at the time points between dropping the relative humidity to 0% and raising it to 90%: 0% (Figure 24(A)), 30% (Figure 24(B)), 50% (Figure 24(C)), and 90% (Figure 24(D)). Measurement apparatus: SmartLab System, D/Tex Ultra detector, humidity generator HUM-SL (manufactured by Rigaku Corporation)

Radiation source: CuKα1
Tube voltage: 45 kV
Tube current: 200 mA
Scanning range: 4 to 35°
Scanning speed: 5°/min
Sampling width: 0.02°
Humidity change conditions:

[Table 2]

| Set temperature (°C) | Relative humidity (%) | Holding time (min) |
|---|---|---|
| 35.0 | 30 | 10 |
| 35.0 | 0 | 30 |

(continued)

| Set temperature (°C) | Relative humidity (%) | Holding time (min) |
|---|---|---|
| 35.0 | 10 | 20 |
| 35.0 | 20 | 20 |
| 35.0 | 30 | 20 |
| 35.0 | 40 | 20 |
| 35.0 | 50 | 20 |
| 35.0 | 70 | 20 |
| 35.0 | 90 | 20 |
| 35.0 | 70 | 20 |
| 35.0 | 50 | 20 |
| 35.0 | 40 | 20 |
| 35.0 | 30 | 20 |

Example 4-6

The thermogravimetry-differential thermal analysis (TG-DTA) for Example 3-8, Example 3-9, Example 3-12, Example 3-13, Example 3-17, and Example 3-19 was performed under the following conditions.

Measurement apparatus: STA7200RV+AS-3T (manufactured by Hitachi High-Tech Science Corporation)
Measurement range: 30 to 350°C
Heating rate: 10°C/ min
Atmosphere: nitrogen
Measurement: The sample was weighed in an open aluminum pan and covered with a mesh to perform the measurement.

Example 4-7

The single crystal X-ray structural analysis for Examples 3-1 was performed under the following conditions.

Measurement apparatus: Rigaku XtaLAB Synergy Custom with a VariMax Cu Diffractometer (manufactured by Rigaku Corporation)
Counter cathode: Cu
Tube voltage: 40 kV
Tube current: 30 mA
Temperature: -180°C
Measurement: The measurement was made using strategy and exposure time that were believed to yield sufficient diffraction spots for structural analysis.
Structural analysis: The Olex2 program was used, with SIR2008 for initial structure determination and the full-matrix least-squares method (SHELXL-2018/3) for structure refinement.

Example 4-8

The single crystal X-ray structural analysis for Example 3-3, Example 3-11, Example 3-15, Example 3-16, and Example 3-18 was performed under the following conditions. Measurement apparatus: Rigaku XtaLAB Synergy Custom with a VariMax Cu Diffractometer (manufactured by Rigaku Corporation)

Counter cathode: Cu
Tube voltage: 40 kV
Tube current: 30 mA
Temperature: -180°C
Measurement: The measurement was made using strategy and exposure time that were believed to yield sufficient

diffraction spots for structural analysis.
Structural analysis: The Olex2 program was used, with the Dual Space method (SHELXD-2008) for initial structure determination and the full-matrix least-squares method (SHELXL-2018/3) for structure refinement.

Example 4-9

The single crystal X-ray structural analysis for Example 3-6, Example 3-7, and Example 3-14 was performed under the following conditions.

Measurement apparatus: Photon Factory BL-5A, High Energy Accelerator Research Organization (KEK)
Radiation source: synchrotron radiation ($\lambda$ = 0.92 Å)
Temperature: -178°C
Measurement: The measurement was made using strategy and exposure time that were believed to obtain sufficient diffraction spots for structural analysis.
Structural analysis: The Olex2 program was used, with the Dual Space method (SHELXD-2008) for initial structure determination and the full-matrix least-squares method (SHELXL-2018/3) for structure refinement.

Example 4-10

The $^{1}$H-NMR measurement for Example 3-8, Example 3-9, and Example 3-19 was performed under the following conditions.

Measurement apparatus: JNM-ECX500II (manufactured by JEOL Ltd.)
Measurement solvent: DMSO-d6, contains 0.03% (v/v) TMS
Measurement temperature: 295K
Sample preparation: A commercially available deuterated solvent was mixed with the compound to be measured for preparation.
Cumulative number and relaxation waiting time: The measurement was performed at a number of times (8, 16, or 512 times) that was believed to yield a sufficient s/n ratio and at a time that was sufficiently longer than the relaxation time (60 seconds).
Integral value: The residual solvent ratio (% by weight) was calculated based on the integrated intensity ratio of each signal.

Example 4-11

The sample preparation for Examples 3-19 was performed under the following conditions.

Apparatus used: STA7200RV+AS-3T (manufactured by Hitachi High-Tech Science Corporation)
Temperature range: 30 to 120°C
Heating rate: 20°C/min (30 to 120°C), maintained at 120°C for 60 minutes
Atmosphere: nitrogen
Preparation: The sample was weighed in an open aluminum pan and heated.

Example 5 Slurry conversion experiment

Example 5-1

The form A crystal (3.0 mg), form B crystal (3.0 mg), and form K crystal (3.0 mg) of compound 1 were mixed, and a mixed solvent of acetone-heptane (1:1 (v/v), 0.030 mL) and one grain of glass bead were added. Using a shaker (BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(A) shows the powder X-ray diffraction pattern of the obtained crystal (Example 4-3). It was confirmed that the form B crystal is a stable form in the mixed solvent of acetone-heptane (1:1 (v/v)).

Example 5-2

The form A crystal (3.1 mg), form B crystal (3.1 mg), and form K crystal (3.1 mg) of compound 1 were mixed, and a mixed solvent of 2-propanol-heptane (1:1 (v/v), 0.030 mL) and one grain of glass bead were added. Using a shaker

(BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(B) shows the powder X-ray diffraction measurement of the obtained crystal (Example 4-3). It was confirmed that the form B crystal is a stable form in the mixed solvent of 2-propanol-heptane (1:1 (v/v)).

Example 5-3

The form A crystal (3.0 mg), form B crystal (3.0 mg), and form K crystal (3.1 mg) of compound 1 were mixed, and a mixed solvent of ethanol-heptane (1:1 (v/v), 0.030 mL) and one grain of glass bead were added. Using a shaker (BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(C) shows the powder X-ray diffraction measurement of the obtained crystal (Example 4-3). It was confirmed that the form B crystal is a stable form in the mixed solvent of ethanol-heptane (1:1 (v/v)).

Example 5-4

The form A crystal (3.0 mg), form B crystal (3.0 mg), and form K crystal (3.0 mg) of compound 1 were mixed, and a mixed solvent of acetone-water (1:1 (v/v), 0.030 mL) and one grain of glass bead were added. Using a shaker (BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(D) shows the powder X-ray diffraction measurement of the obtained crystal (Example 4-3). It was confirmed that the form B crystal is a stable form in the mixed solvent of acetone-water (1:1 (v/v)).

Example 5-5

The form A crystal (3.0 mg), form B crystal (3.1 mg), and form K crystal (3.1 mg) of compound 1 were mixed, and a mixed solvent of 2-propanol-water (1:1 (v/v), 0.030 mL) and one grain of glass bead were added. Using a shaker (BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(E) shows the powder X-ray diffraction measurement of the obtained crystal (Example 4-3). It was confirmed that the form B crystal is a stable form in the mixed solvent of 2-propanol-water (1:1 (v/v)).

Example 5-6

The form A crystal (3.1 mg), form B crystal (3.0 mg), and form K crystal (3.1 mg) of compound 1 were mixed, and a mixed solvent of ethanol-water (1:1 (v/v), 0.030 mL) and one grain of glass bead were added. Using a shaker (BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(F) shows the powder X-ray diffraction measurement of the obtained crystal (Example 4-3). It was confirmed that the form B crystal is a stable form in the mixed solvent of ethanol-water (1:1 (v/v)).

Example 5-7

The form A crystal (3.0 mg), form B crystal (3.0 mg), and form K crystal (3.1 mg) of compound 1 were mixed, and a mixed solvent of acetone-water (1:4 (v/v), 0.030 mL) and one grain of glass bead were added. Using a shaker (BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(G) shows the powder X-ray diffraction measurement of the obtained crystal (Example 4-3). It was confirmed that the form B crystal is a stable form in the mixed solvent of acetone-water (1:4 (v/v)).

Example 5-8

The form A crystal (3.0 mg), form B crystal (3.0 mg), and form K crystal (3.1 mg) of compound 1 were mixed, and a mixed solvent of 2-propanol-water (1:4 (v/v), 0.030 mL) and one grain of glass bead were added. Using a shaker (BioShaker M·BR-022UP manufactured by TAITEC CORPORATION), the mixture was shaken at 25°C, 2000 rpm for 9 days, and then filtered and dried under reduced pressure to obtain a crystal (form B) of compound 1. Figure 35(H) shows the powder X-ray diffraction measurement of the obtained crystal (Example 4-3). It was confirmed that the form B crystal is a stable form in the mixed solvent of 2-propanol-water (1:4 (v/v)).

Example 5-9

The form A crystal (3.0 mg), form B crystal (3.1 mg), and form K crystal (3.0 mg) of compound 1 were mixed, and a mixed solvent of ethanol-water (1:4 (v/v), 0.030 mL) and one grain of glass bead were added. Using a shaker (BioShaker M·BR-022UP manufactured by TAITEC CORPORATION), the mixture was shaken at 25°C, 2000 rpm for 7 days, and then filtered and dried under reduced pressure to obtain a crystal (form B) of compound 1. Figure 35(I) shows the powder X-ray diffraction measurement of the obtained crystal (Example 4-3). It was confirmed that the form B crystal is a stable form in the mixed solvent of ethanol-water (1:4 (v/v)).

[Industrial Applicability]

According to the present invention, provided are a method for producing a pharmaceutically useful cyclic peptide compound, or a salt thereof, or a solvate thereof, and a method for producing peptide compounds used in the production of the cyclic peptide compound, or a salt thereof, or a solvate thereof.

**Claims**

1. A method for producing a cyclic peptide compound represented by formula (1), or a salt thereof, or a solvate thereof, the method comprising a step of reacting an N-terminal amino acid residue of a peptide compound represented by formula (2) or (3) with a C-terminal amino acid residue of the peptide compound in a solvent for cyclization (cyclization step):

[Formula 1]

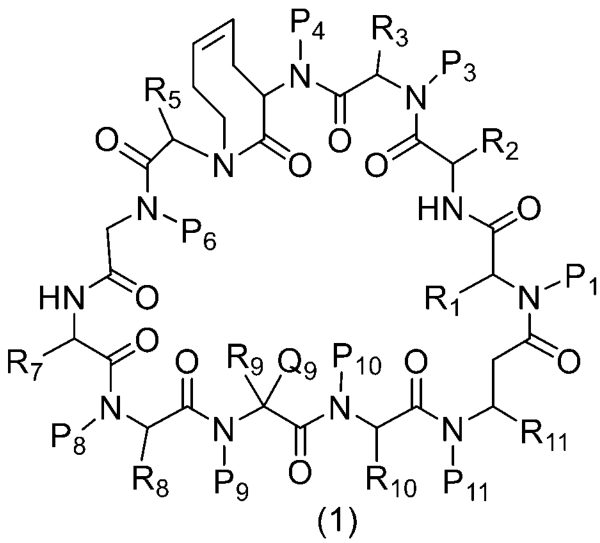

(1)

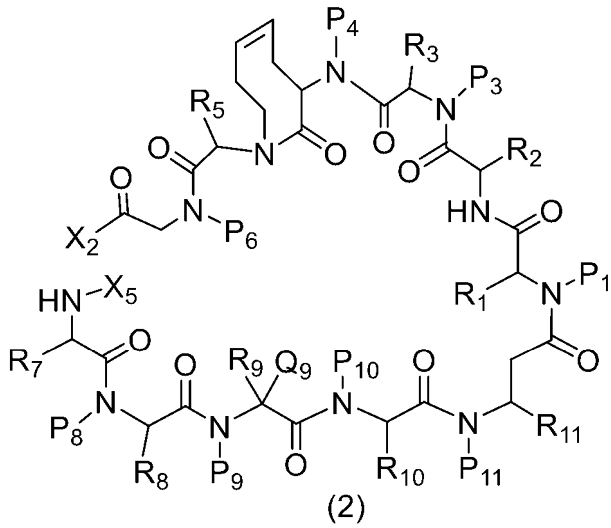

(2)

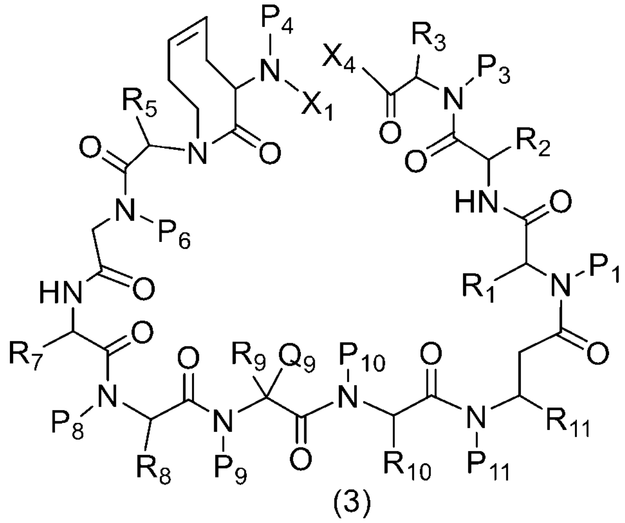

(3)

wherein $R_1$ is a $C_1$-$C_6$ alkyl;

$P_1$ is a $C_1$-$C_6$ alkyl;
$R_2$ is a $C_1$-$C_6$ alkyl;
$R_3$ is hydrogen, or $R_3$ forms a 4- to 7-membered saturated heterocyclic ring together with $P_3$, a carbon atom to which $R_3$ is bonded, and a nitrogen atom to which $P_3$ is bonded;
$P_3$ is a $C_1$-$C_6$ alkyl or a $C_3$-$C_8$ cycloalkyl, or $P_3$ forms a 4- to 7-membered saturated heterocyclic ring together with $R_3$, a carbon atom to which $R_3$ is bonded, and a nitrogen atom to which $P_3$ is bonded;
$P_4$ is a $C_1$-$C_6$ alkyl;
$R_5$ is benzyl optionally substituted with one or more groups selected from the group consisting of a $C_1$-$C_6$ alkyl, a $C_1$-$C_6$ haloalkyl, and a $C_3$-$C_8$ cycloalkyl;
$P_6$ is a $C_1$-$C_6$ alkyl;
$R_7$ is phenethyl optionally substituted with one or more groups selected from the group consisting of a halogen, a $C_1$-$C_6$ haloalkyl, and a $C_1$-$C_6$ alkoxy;
$R_8$ forms a 4- to 7-membered saturated heterocyclic ring together with $P_8$, a carbon atom to which $R_8$ is bonded, and a nitrogen atom to which $P_8$ is bonded, the 4- to 7-membered saturated heterocyclic ring optionally substituted with a $C_1$-$C_6$ alkoxy;
$R_9$ forms a 3- to 8-membered alicyclic ring together with $Q_9$ and a carbon atom to which $R_9$ and $Q_9$ are bonded, the 3- to 8-membered alicyclic ring optionally substituted with one or more $C_1$-$C_6$ alkyls;
$P_9$ is hydrogen or a $C_1$-$C_6$ alkyl;
$R_{10}$ is a $C_1$-$C_6$ alkyl or a $C_3$-$C_8$ cycloalkyl;
$P_{10}$ is a $C_1$-$C_6$ alkyl;
$R_{11}$ is a di-$C_1$-$C_6$ alkylaminocarbonyl or a 4- to 8-membered cyclic aminocarbonyl;

$P_{11}$ is a $C_1$-$C_6$ alkyl;
$X_1$ and $X_5$ are each independently hydrogen or a protecting group for an amino group; and $X_2$ and $X_4$ are each independently a halogen, a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

**2.** A method for producing a cyclic peptide compound represented by formula (1), or a salt thereof, or a solvate thereof, the method comprising:

(a) a step of providing peptide compounds represented by formulae (4) to (6), or salts thereof, or solvates of the peptide compounds or salts;
(b) a step of reacting N-terminal amino acid residues of the peptide compounds represented by formulae (4) to (6) with C-terminal amino acid residues of the peptide compounds in a solvent for linkage (linking step); and
(c) a step of reacting an N-terminal amino acid residue of a peptide compound obtained in the step (b) with a C-terminal amino acid residue of the peptide compound in a solvent for cyclization (cyclization step):

[Formula 2]

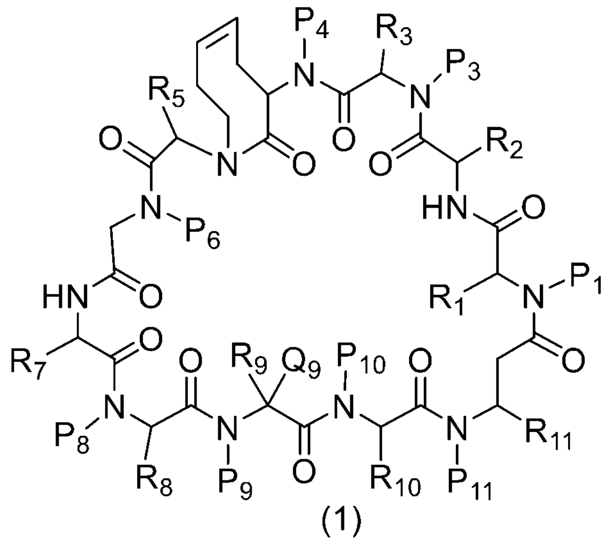

(1)

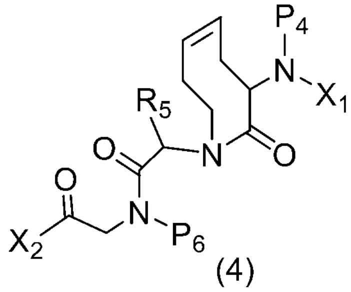

(4)

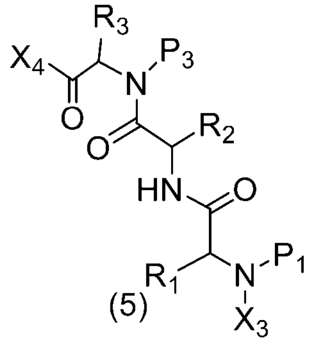

(5)

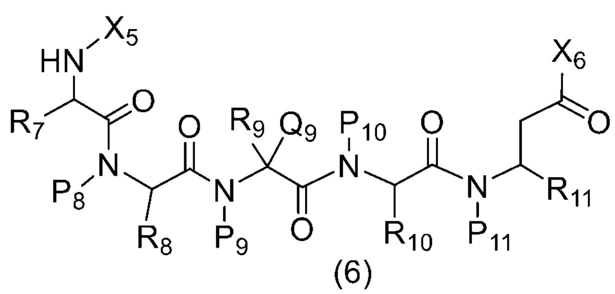

(6)

wherein $R_1$ is a $C_1$-$C_6$ alkyl;

$P_1$ is a $C_1$-$C_6$ alkyl;
$R_2$ is a $C_1$-$C_6$ alkyl;
$R_3$ is hydrogen, or $R_3$ forms a 4- to 7-membered saturated heterocyclic ring together with $P_3$, a carbon atom to which $R_3$ is bonded, and a nitrogen atom to which $P_3$ is bonded;
$P_3$ is a $C_1$-$C_6$ alkyl or a $C_3$-$C_8$ cycloalkyl, or $P_3$ forms a 4- to 7-membered saturated heterocyclic ring together with $R_3$, a carbon atom to which $R_3$ is bonded, and a nitrogen atom to which $P_3$ is bonded;
$P_4$ is a $C_1$-$C_6$ alkyl;
$R_5$ is benzyl optionally substituted with one or more groups selected from the group consisting of a $C_1$-$C_6$ alkyl, a $C_1$-$C_6$ haloalkyl, and a $C_3$-$C_8$ cycloalkyl;
$P_6$ is a $C_1$-$C_6$ alkyl;
$R_7$ is phenethyl optionally substituted with one or more groups selected from the group consisting of a halogen, a $C_1$-$C_6$ haloalkyl, and a $C_1$-$C_6$ alkoxy;
$R_8$ forms a 4- to 7-membered saturated heterocyclic ring together with $P_8$, a carbon atom to which $R_8$ is bonded, and a nitrogen atom to which $P_8$ is bonded, the 4- to 7-membered saturated heterocyclic ring optionally substituted with a $C_1$-$C_6$ alkoxy;
$R_9$ forms a 3- to 8-membered alicyclic ring together with $Q_9$ and a carbon atom to which $R_9$ and $Q_9$ are bonded, the 3- to 8-membered alicyclic ring optionally substituted with one or more $C_1$-$C_6$ alkyls;
$P_9$ is hydrogen or a $C_1$-$C_6$ alkyl;
$R_{10}$ is a $C_1$-$C_6$ alkyl or a $C_3$-$C_8$ cycloalkyl;
$P_{10}$ is a $C_1$-$C_6$ alkyl;
$R_{11}$ is a di-$C_1$-$C_6$ alkylaminocarbonyl or a 4- to 8-membered cyclic aminocarbonyl;
$P_{11}$ is a $C_1$-$C_6$ alkyl;
$X_1$, $X_3$, and $X_5$ are each independently hydrogen or a protecting group for an amino group; and
$X_2$, $X_4$, and $X_6$ are each independently a halogen, a hydroxy group, an optionally substituted alkoxy, an optionally substituted aryloxy, an optionally substituted aralkoxy, an optionally substituted cyclic aminooxy, or a group represented by $-OSiR_xR_yR_z$, wherein $R_x$, $R_y$, and $R_z$ are each independently an alkyl or an aryl.

**3.** The method according to claim 2, comprising, in the step (b),

(b-1) a step of reacting an N-terminal amino acid residue of the peptide compound represented by formula (5) with a C-terminal amino acid residue of the peptide compound represented by formula (6) in a solvent for linkage, thereby converting them into a peptide compound represented by formula (7) (linking step):

[Formula 3]

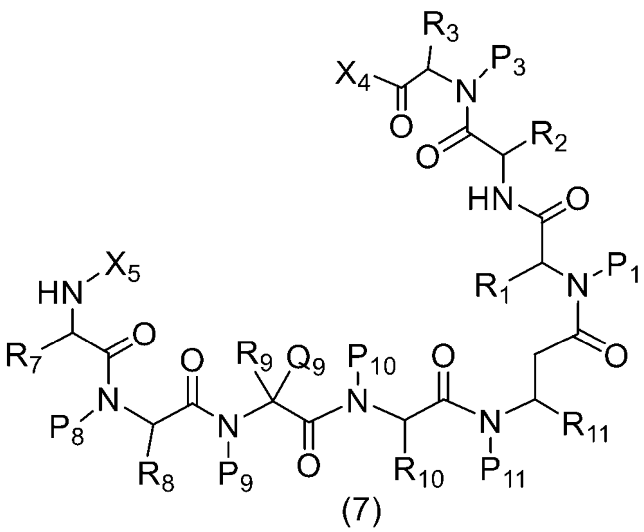

(7)

wherein $R_1$, $R_2$, $R_3$, $R_7$, $R_8$, $R_9$, $R_{10}$, $R_{11}$, $P_1$, $P_3$, $P_8$, $P_9$, $P_{10}$, $P_{11}$, $Q_9$, $X_4$, and $X_5$ are the same as in claim 2.

**4.** The method according to claim 3, further comprising, in the step (b),

(b-2) a step of reacting an N-terminal amino acid residue of the peptide compound represented by formula (4) with a C-terminal amino acid residue of the peptide compound represented by formula (7) in a solvent for linkage, thereby converting them into a peptide compound represented by formula (2) (linking step), and
in the step (c),
(c-1) a step of reacting an N-terminal amino acid residue of the peptide compound represented by formula (2) with a C-terminal amino acid residue of the peptide compound in a solvent for cyclization (cyclization step).

**5.** The method according to claim 3, further comprising, in the step (b),

(b-3) a step of reacting an N-terminal amino acid residue of the peptide compound represented by formula (7) with a C-terminal amino acid residue of the peptide compound represented by formula (4) in a solvent for linkage, thereby converting them into a compound represented by formula (3) (linking step), and
in the step (c),
(c-2) a step of reacting an N-terminal amino acid residue of the peptide compound represented by formula (3) with a C-terminal amino acid residue of the peptide compound in a solvent for cyclization (cyclization step).

**6.** The method according to any one of claims 1 to 5, wherein the linkage of an N-terminal amino acid residue of a peptide compound with a C-terminal amino acid residue of a peptide compound is linkage of an amino group of the N-terminal amino acid residue with a carboxyl group of the C-terminal amino acid residue.

**7.** The method according to any one of claims 1 to 6, wherein the solvent in the cyclization step includes one or more selected from the group consisting of a nitrile-based solvent, a halogen-based solvent, an ether-based solvent, an amide-based solvent, an ester-based solvent, and a carbonate-based solvent.

**8.** The method according to any one of claims 1 to 7, wherein the cyclization step is performed in the presence of a condensation reagent.

**9.** The method according to any one of claims 1 to 8, wherein the cyclization step is performed in the presence of a base.

**10.** The method according to any one of claims 1 to 9, wherein the cyclization step is performed by a liquid phase method.

**11.** The method according to any one of claims 1 to 10, wherein the solvent in the linking step includes one or more selected from the group consisting of a nitrile-based solvent, a halogen-based solvent, an ether-based solvent, an amide-based solvent, an ester-based solvent, and a carbonate-based solvent.

**12.** A crystal of a cyclic peptide compound represented by formula (1a), or a salt thereof, or a solvate thereof:

[Formula 4]

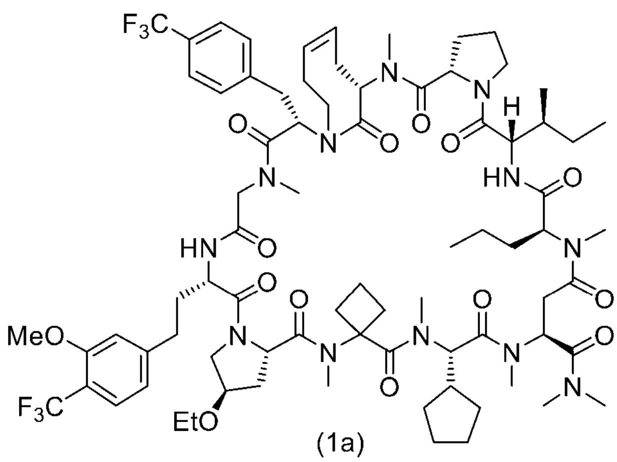

(1a)

**13.** The crystal according to claim 12, wherein the crystal is a solvate crystal, wherein the solvate crystal is a hydrate

crystal, wherein the hydrate crystal is a form A crystal including at least 7 peaks selected from the group consisting of 6.93°, 7.56°, 8.26°, 9.00°, 9.58°, 10.35°, 11.35°, 12.26°, 12.85°, 13.51°, 14.12°, 14.69°, 15.46°, 15.92°, 17.43°, and 17.73° (±0.2°) as diffraction angles (2θ values) by powder X-ray diffraction, and wherein the diffraction angles (2θ values) are diffraction angles (2θ values) of a hydrate crystal stored at a relative humidity of 10% or more for 15 minutes or longer.

**14.** The crystal according to claim 12, wherein the crystal is a solvate crystal, wherein the solvate crystal is a hydrate crystal, wherein the hydrate crystal is a form B crystal including at least 7 peaks selected from the group consisting of 4.99°, 8.65°, 9.85°, 10.84°, 11.32°, 12.35°, 13.20°, 14.44°, 15.20°, 16.03°, 16.69°, 17.21°, 18.82°, 19.49°, and 20.03° (±0.2°) as diffraction angles (2θ values) by powder X-ray diffraction, and wherein the diffraction angles (2θ values) are diffraction angles (2θ values) of a hydrate crystal stored at a relative humidity of 30% or more for 15 minutes or longer.

**15.** A method for producing the crystal of a cyclic peptide compound according to any one of claims 12 to 14, the method comprising: a step of dissolving the cyclic peptide compound in a polar organic solvent in an amount that allows the cyclic peptide compound to dissolve therein to obtain a solution; and a step of adding a hydrocarbon-based solvent or water to the solution to obtain a crystal of the cyclic peptide compound.

**16.** The method according to claim 15, further comprising, after the step of obtaining a crystal of the cyclic peptide compound, a step of filtering the crystal.

*Fig.1*

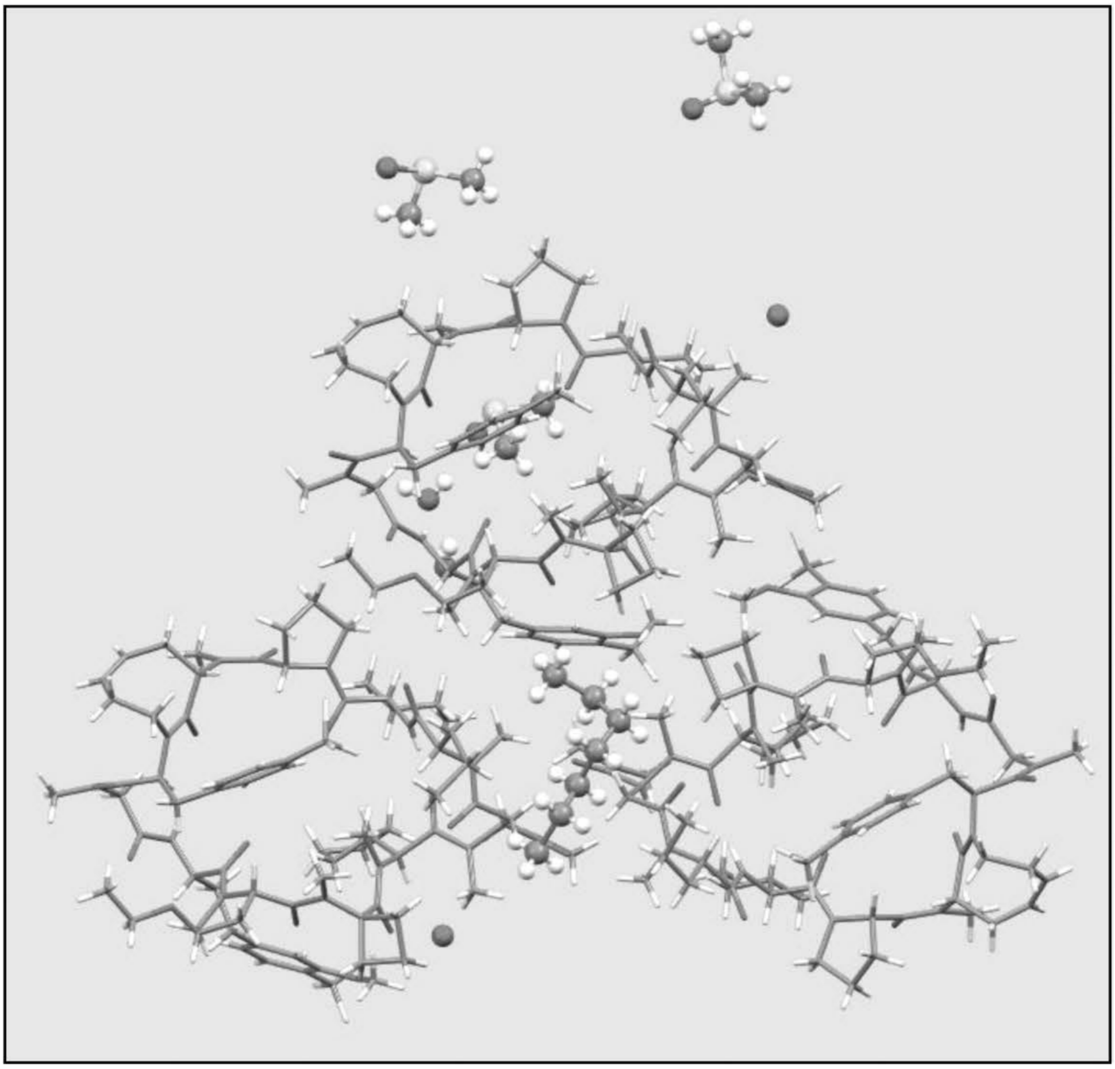

*Fig.2*

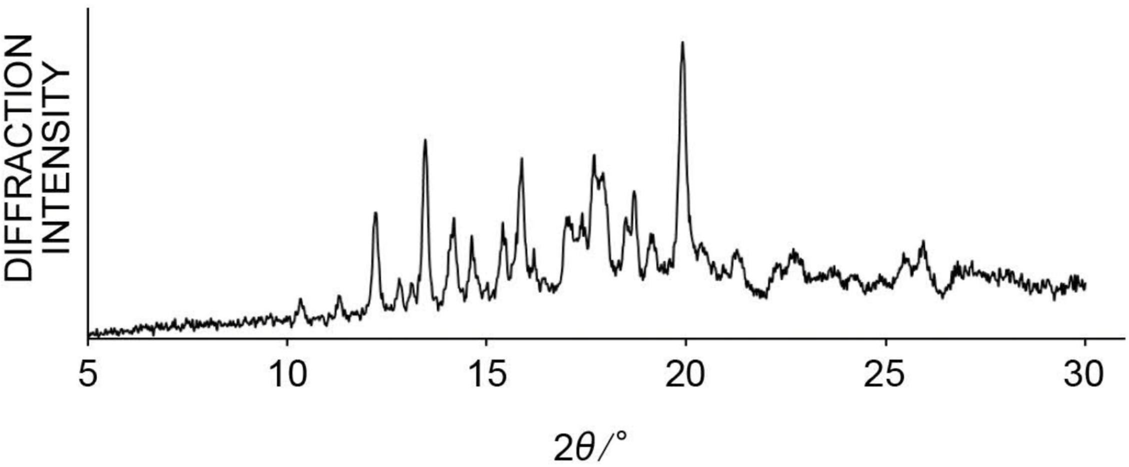

*Fig.3*

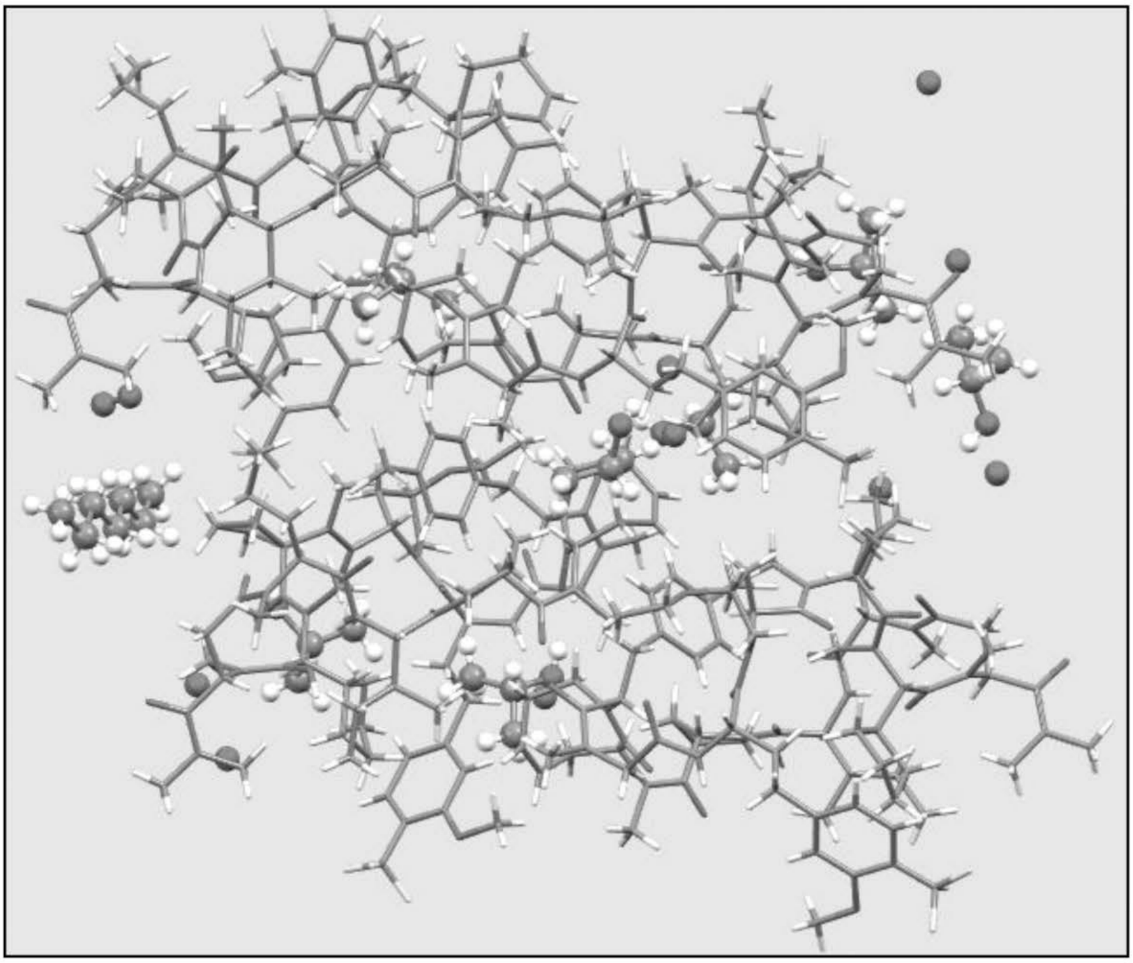

*Fig.4*

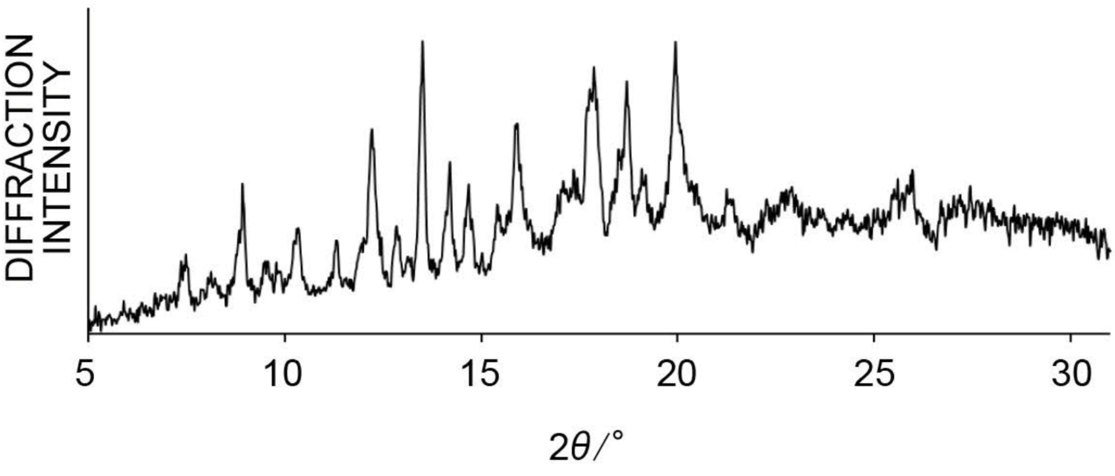

Fig.5

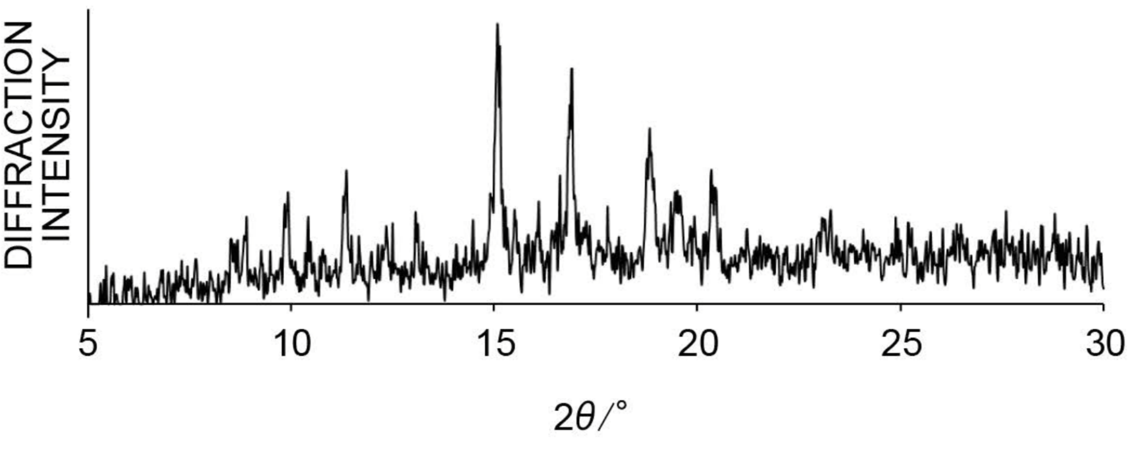

# Fig.6

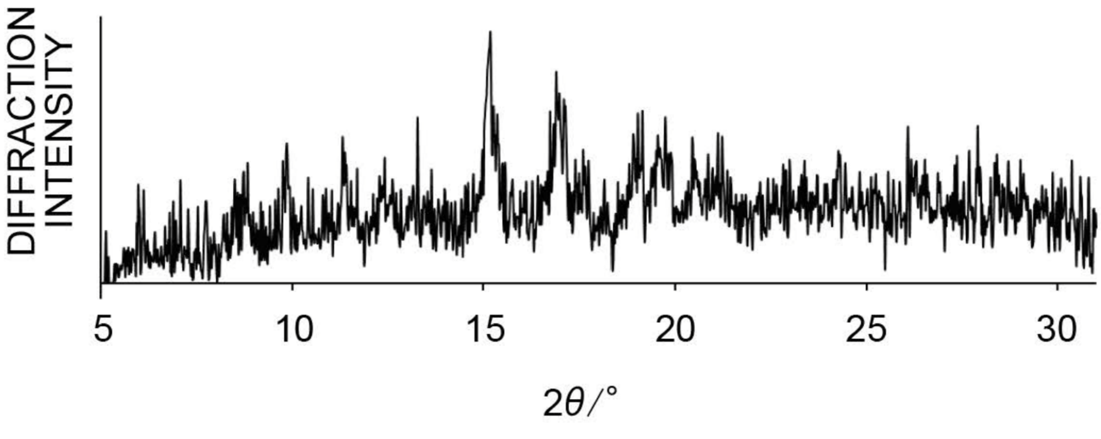

Fig.7

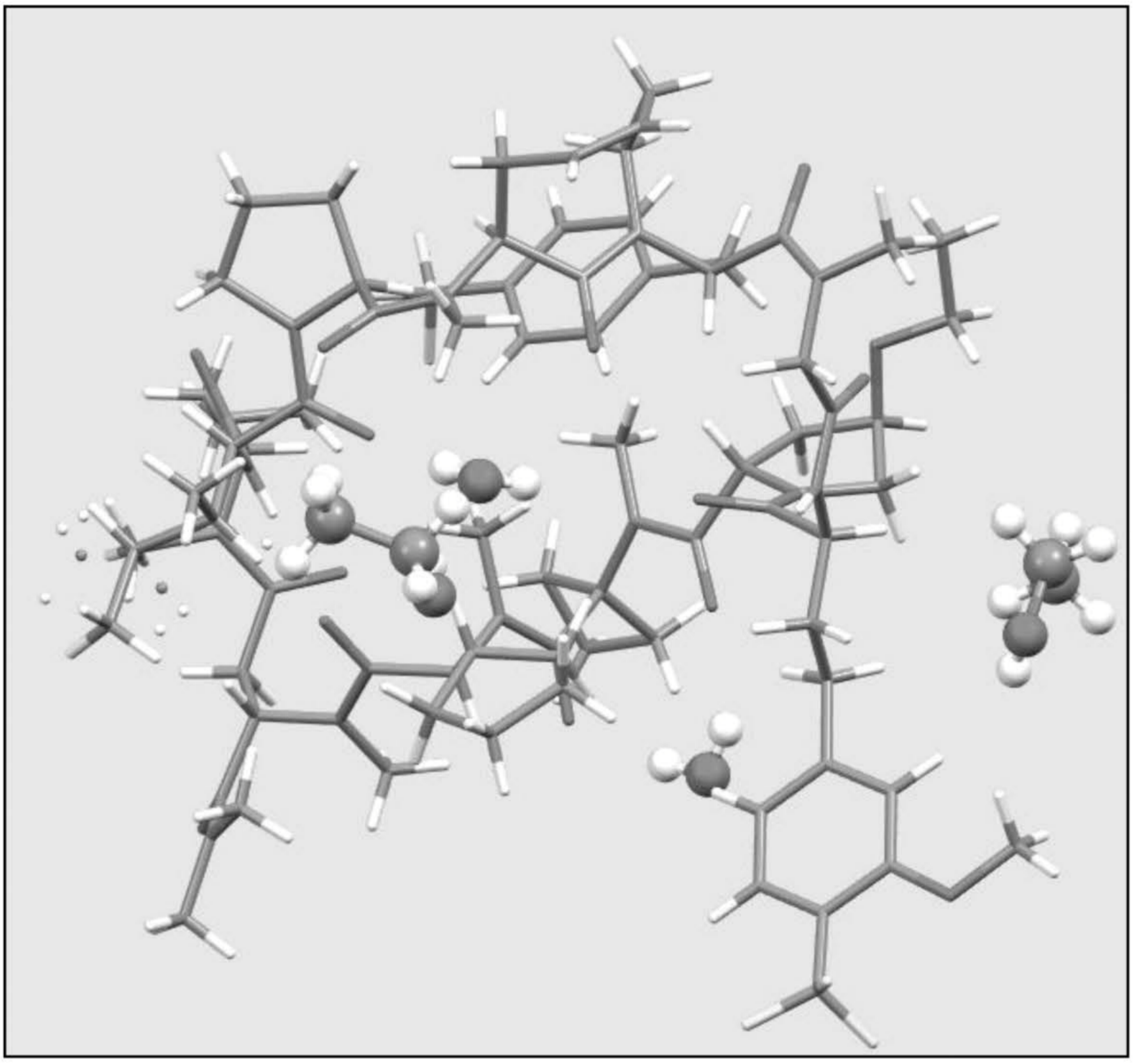

*Fig.8*

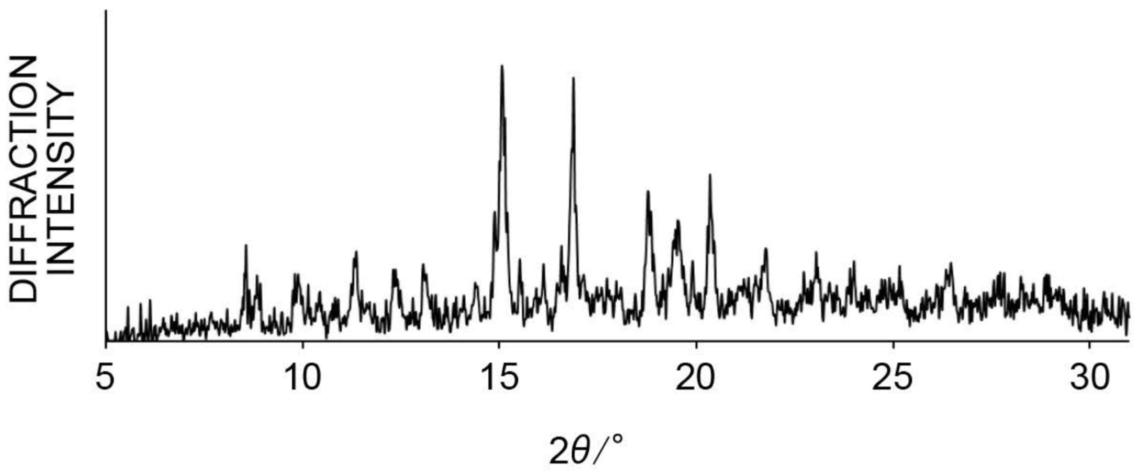

*Fig.9*

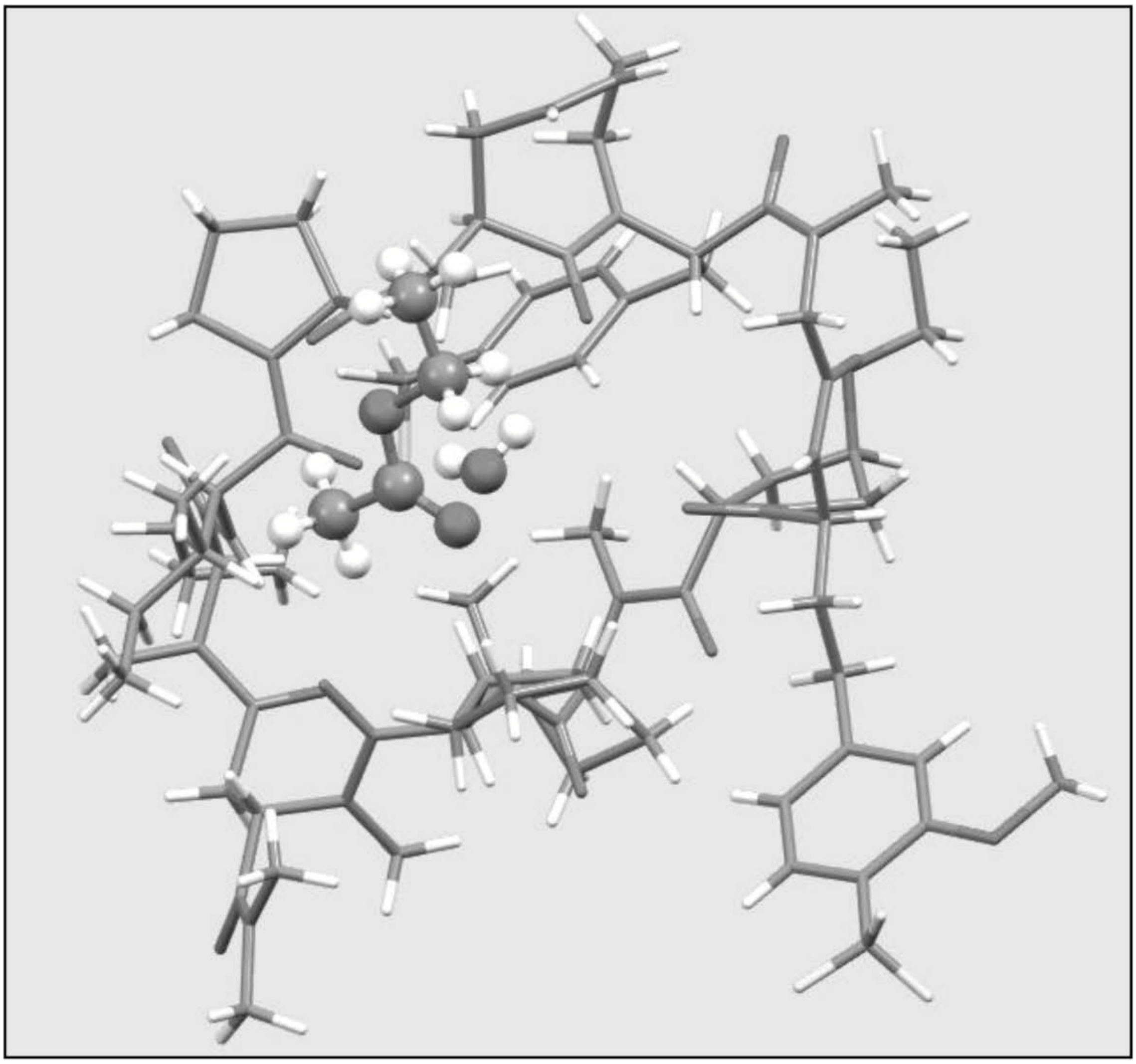

*Fig.10*

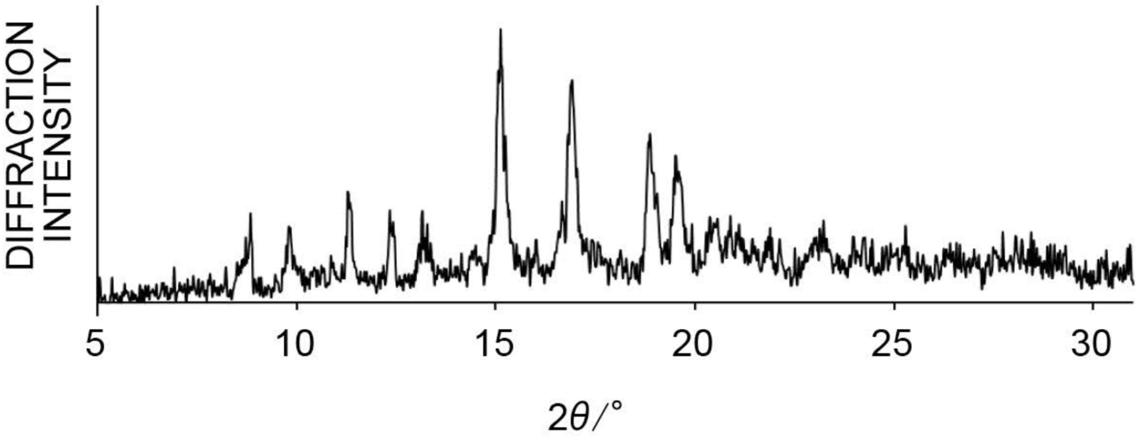

*Fig.11*

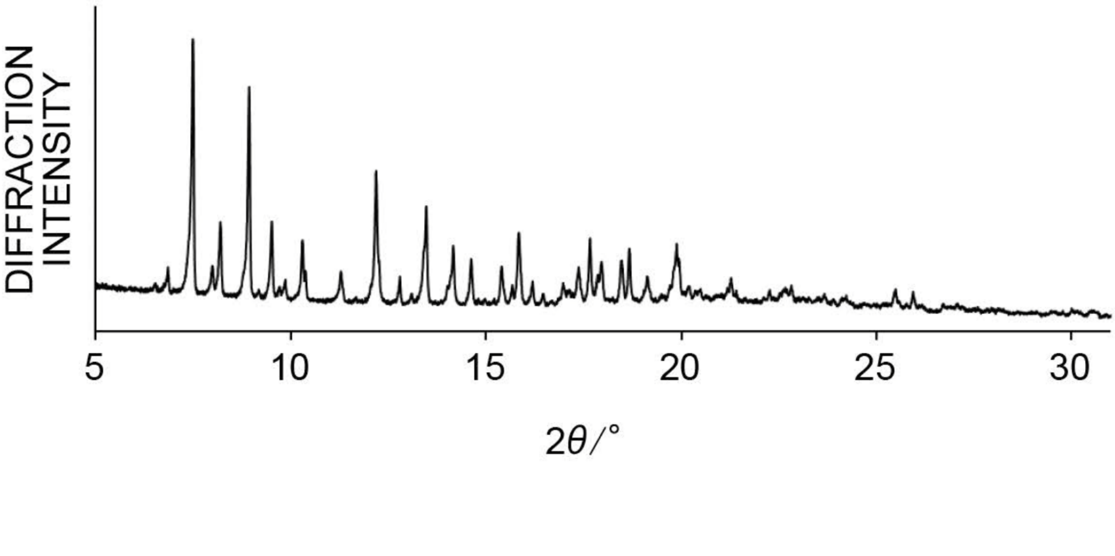

# Fig.12

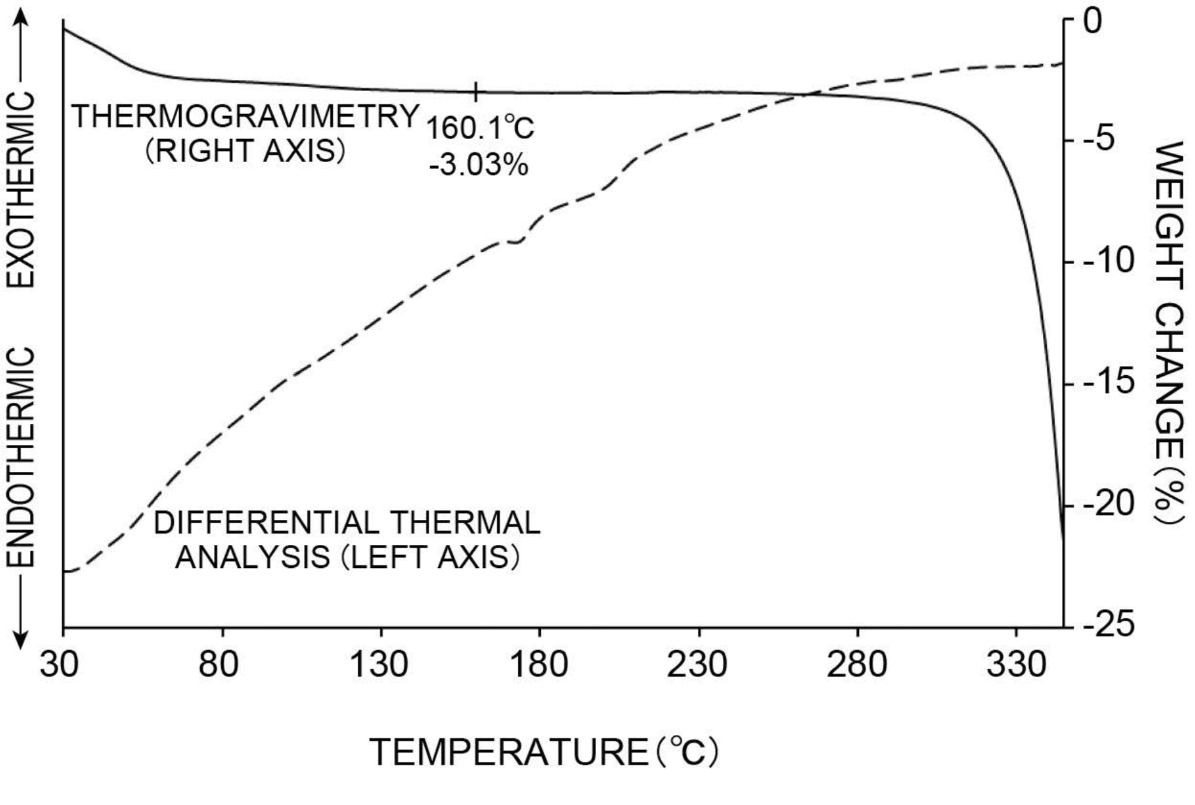

EXOTHERMIC
ENDOTHERMIC
THERMOGRAVIMETRY (RIGHT AXIS)
160.1℃
-3.03%
DIFFERENTIAL THERMAL ANALYSIS (LEFT AXIS)
0
-5
-10
-15
-20
-25
WEIGHT CHANGE (%)
30
80
130
180
230
280
330
TEMPERATURE (℃)

# Fig.13

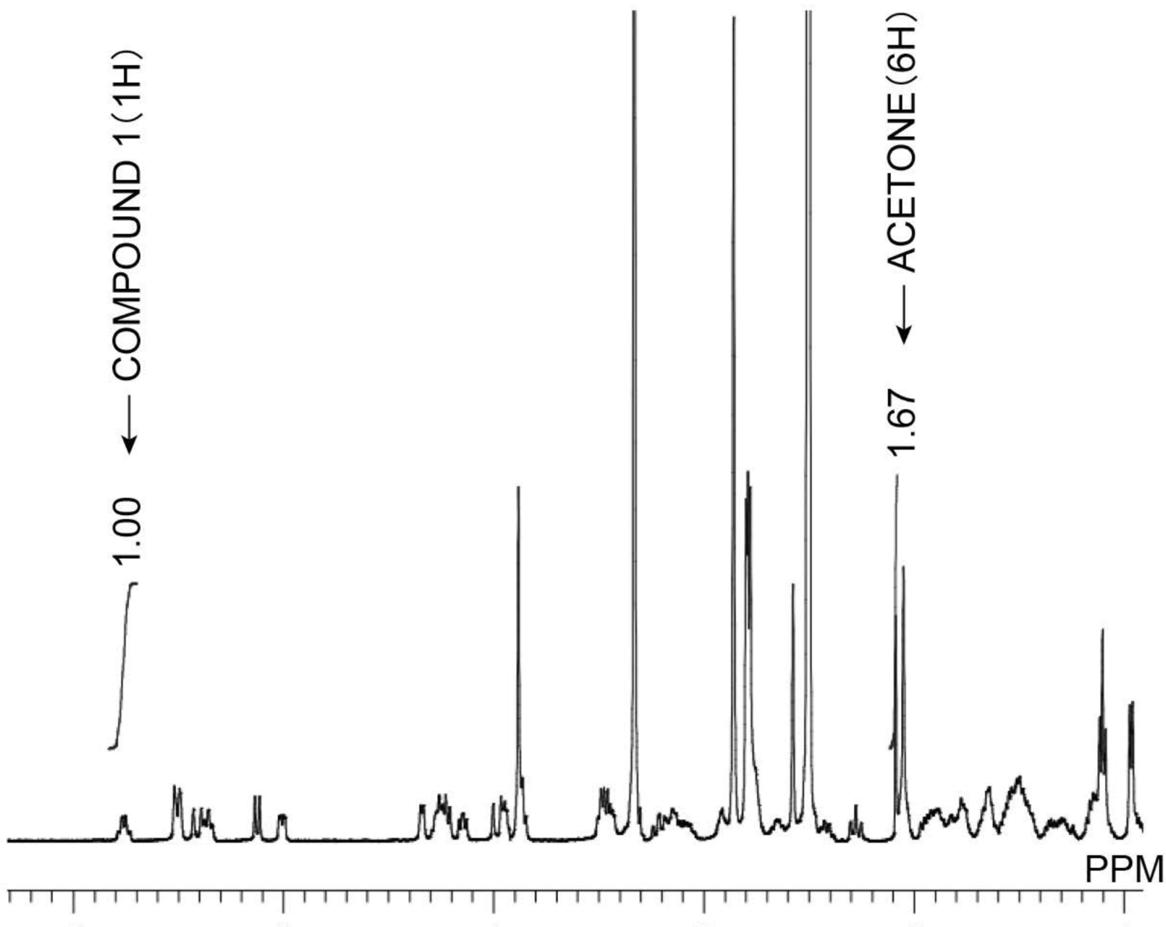

*Fig.14*

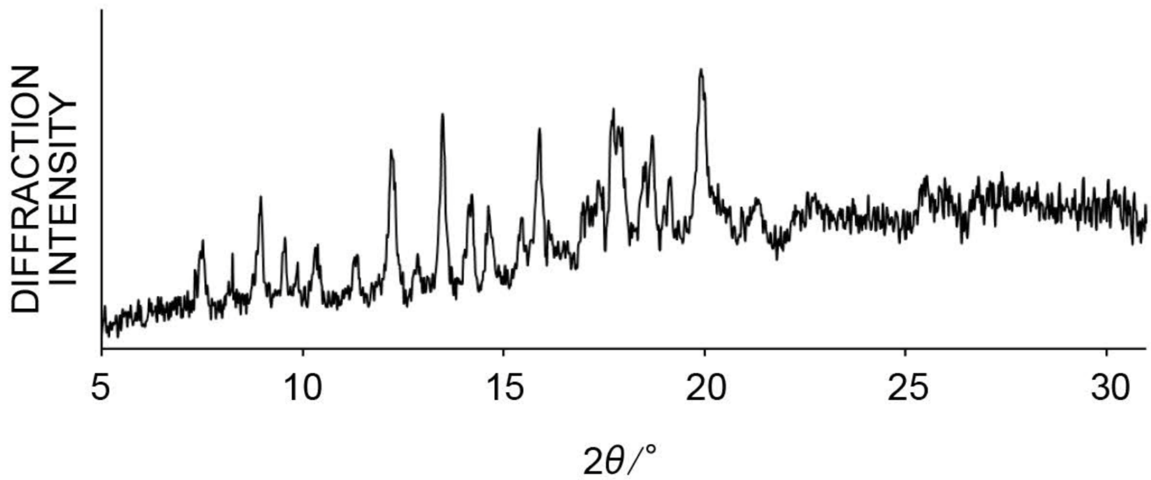

# Fig.15

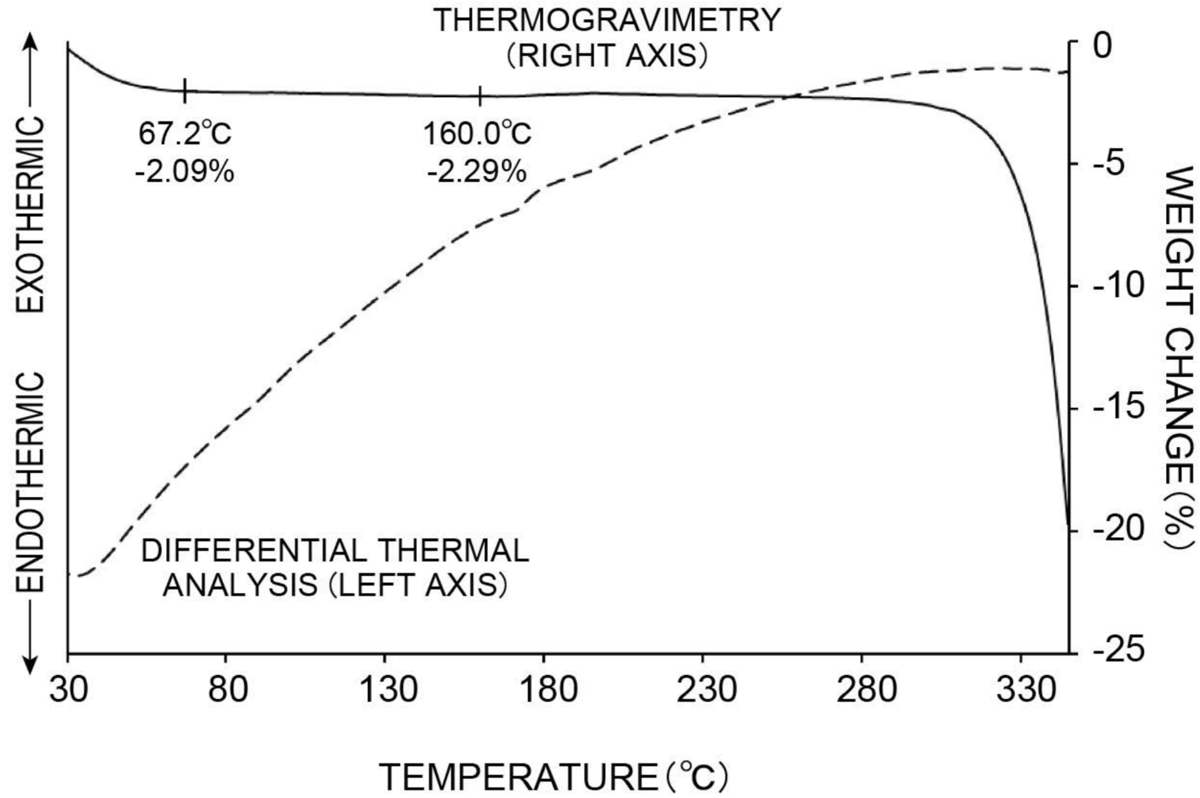
THERMOGRAVIMETRY
(RIGHT AXIS)
67.2°C
-2.09%
160.0°C
-2.29%
DIFFERENTIAL THERMAL
ANALYSIS (LEFT AXIS)
ENDOTHERMIC
EXOTHERMIC
WEIGHT CHANGE (%)
0
-5
-10
-15
-20
-25
30
80
130
180
230
280
330
TEMPERATURE (°C)

*Fig.16*

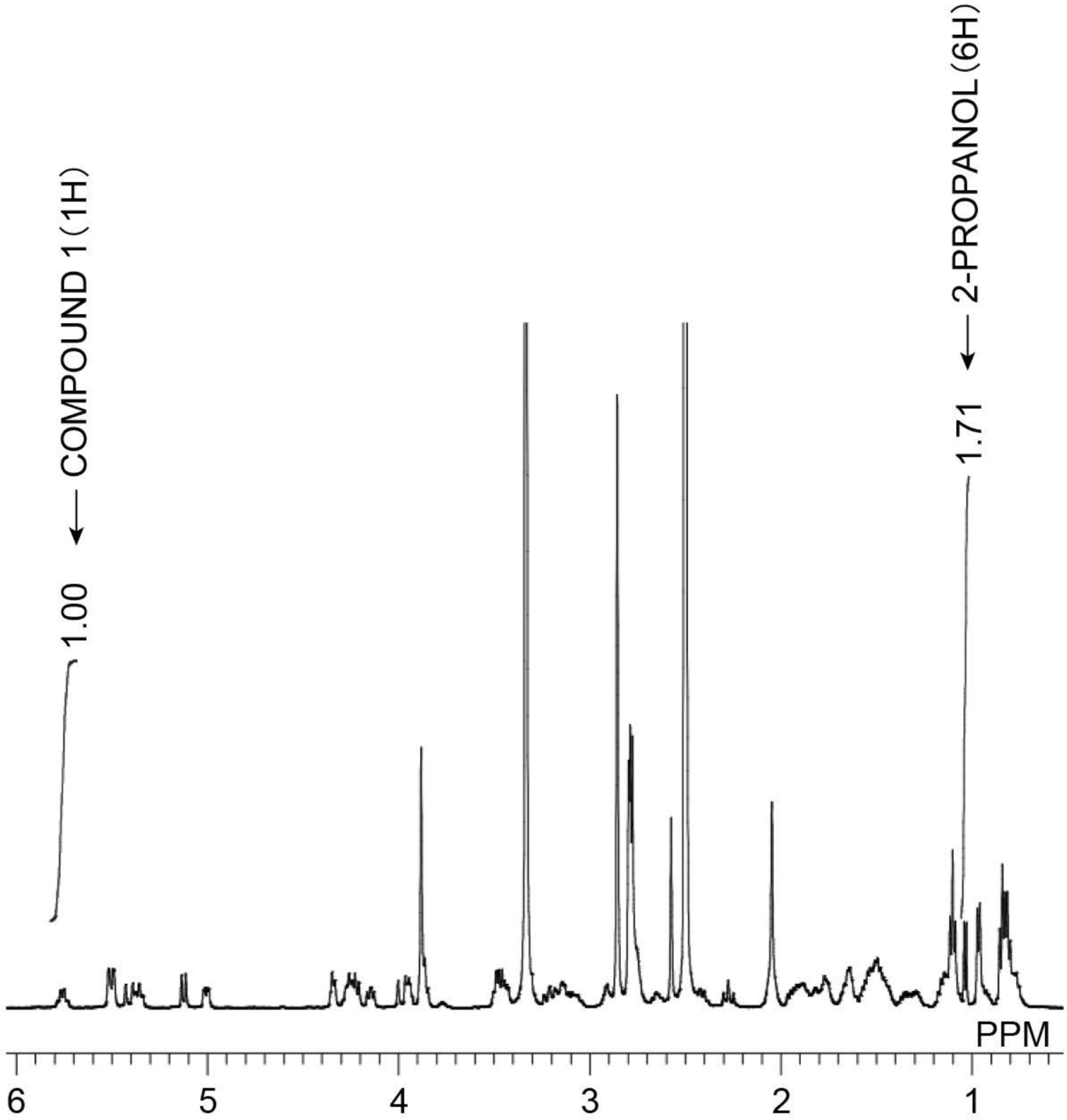

# Fig.17

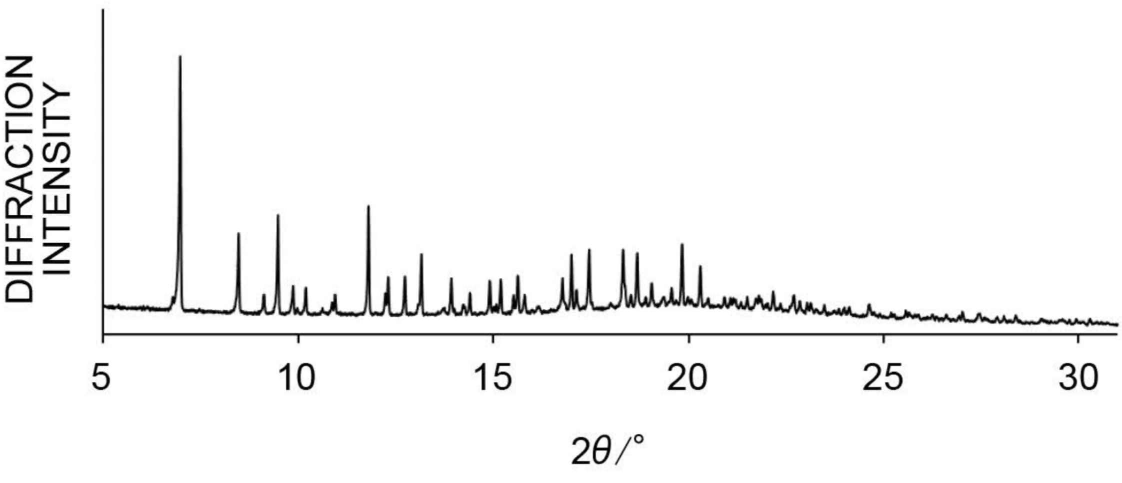

*Fig.18*

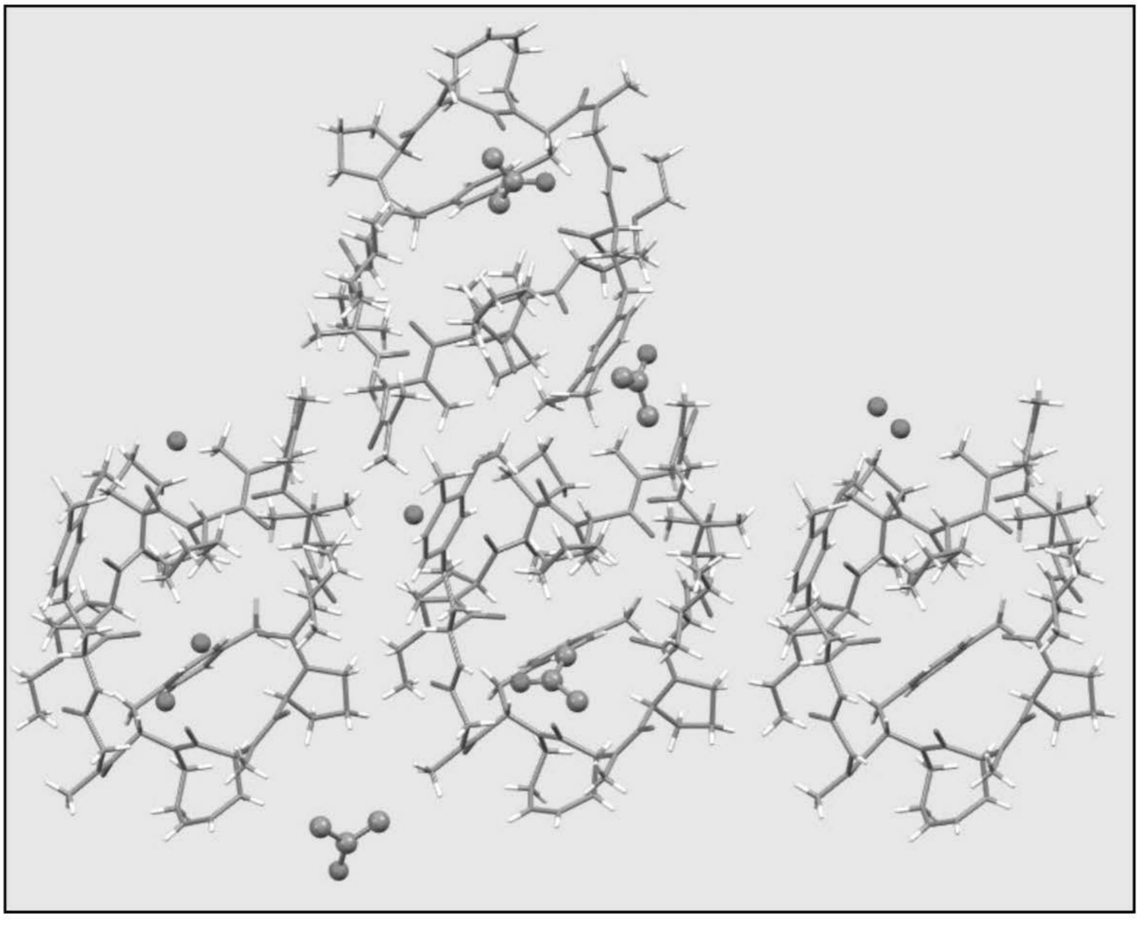

*Fig.19*

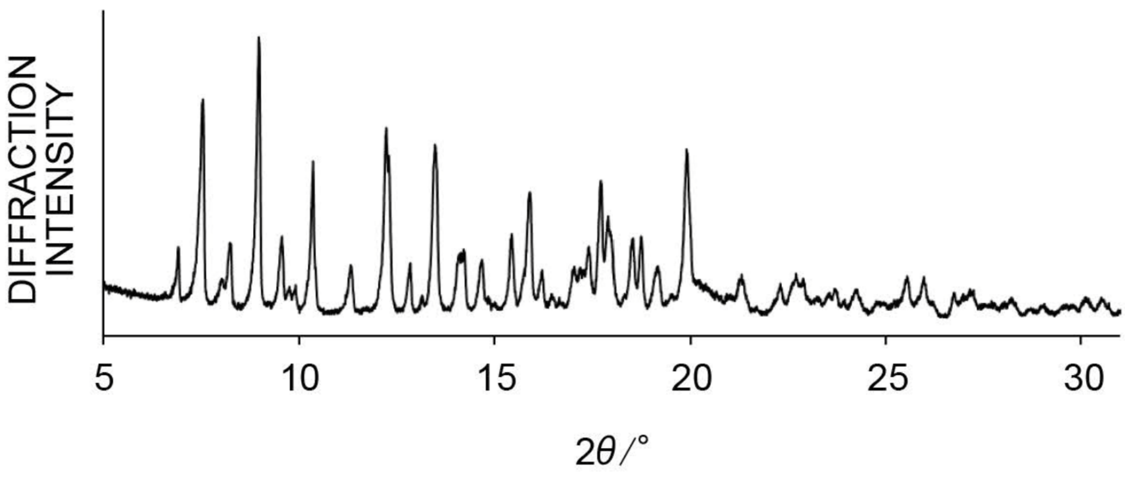

# Fig.20

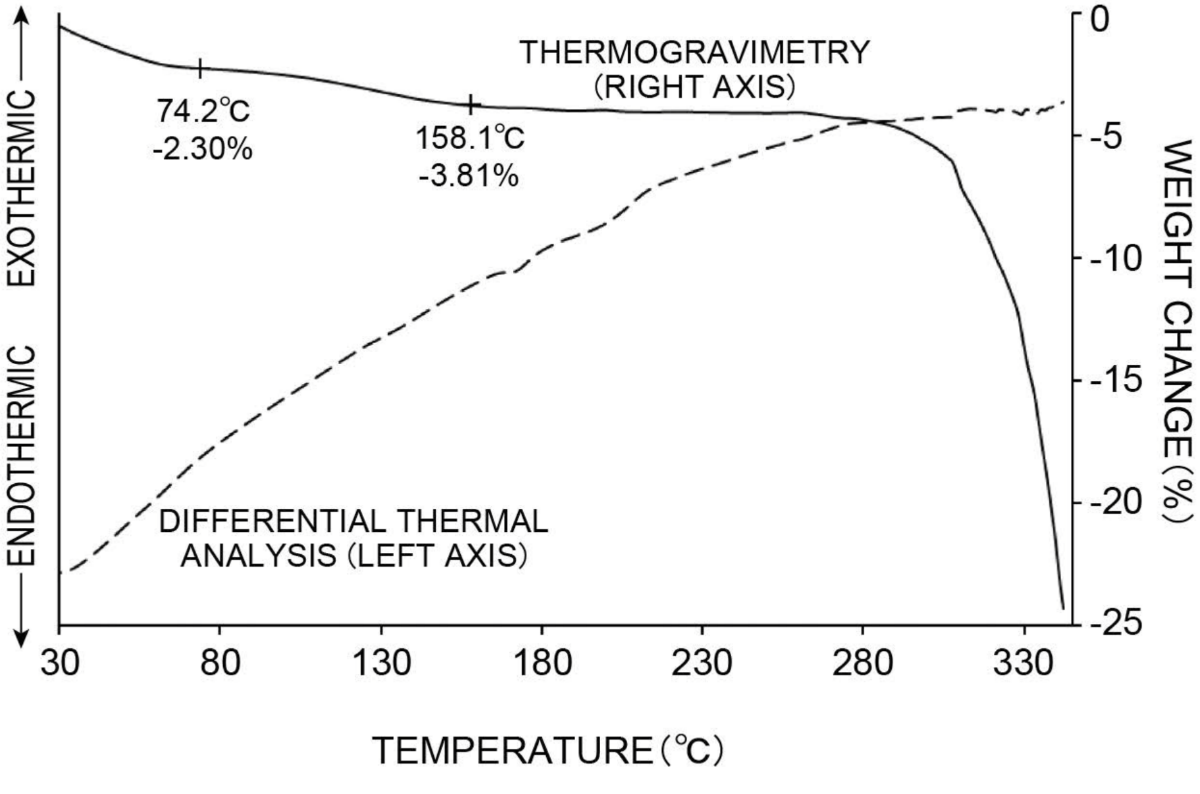
THERMOGRAVIMETRY
(RIGHT AXIS)
74.2°C
-2.30%
158.1°C
-3.81%
DIFFERENTIAL THERMAL
ANALYSIS (LEFT AXIS)
EXOTHERMIC
ENDOTHERMIC
WEIGHT CHANGE (%)
0
-5
-10
-15
-20
-25
30
80
130
180
230
280
330
TEMPERATURE (°C)

# Fig.21

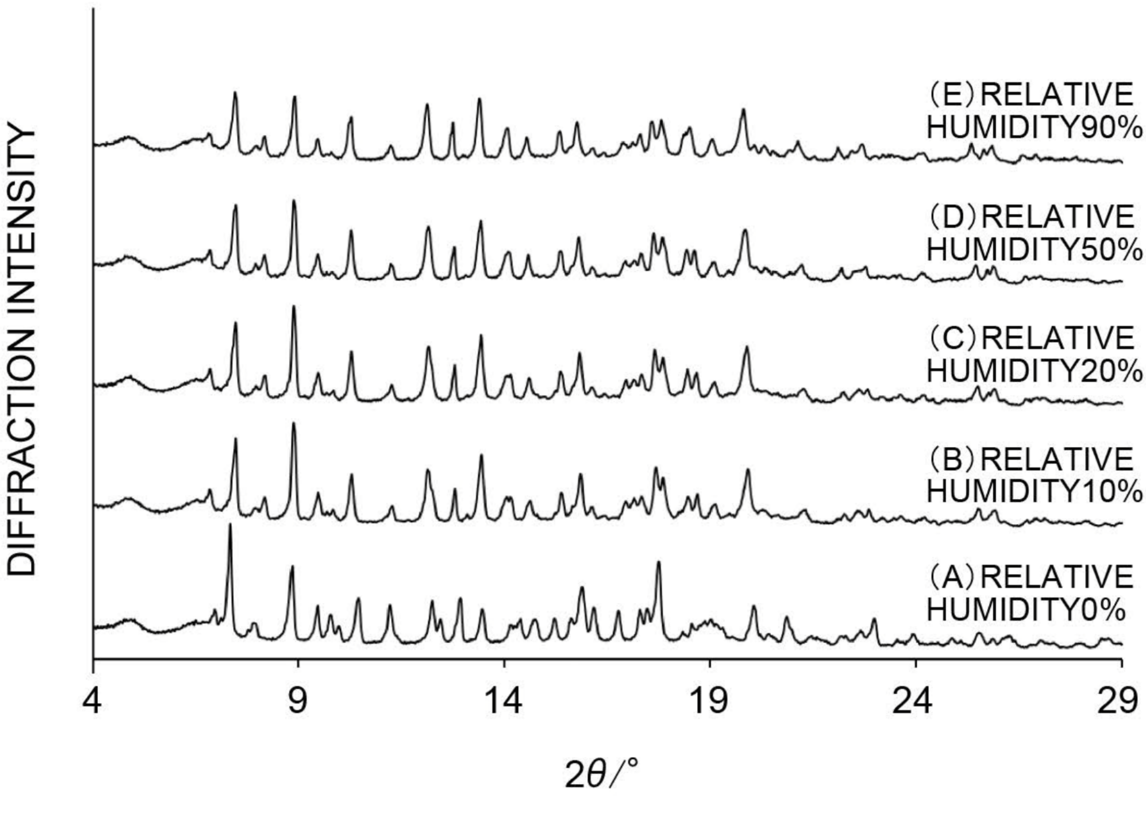
DIFFRACTION INTENSITY
(E) RELATIVE HUMIDITY90%
(D) RELATIVE HUMIDITY50%
(C) RELATIVE HUMIDITY20%
(B) RELATIVE HUMIDITY10%
(A) RELATIVE HUMIDITY0%
4
9
14
19
24
29
2θ/°

# Fig.22

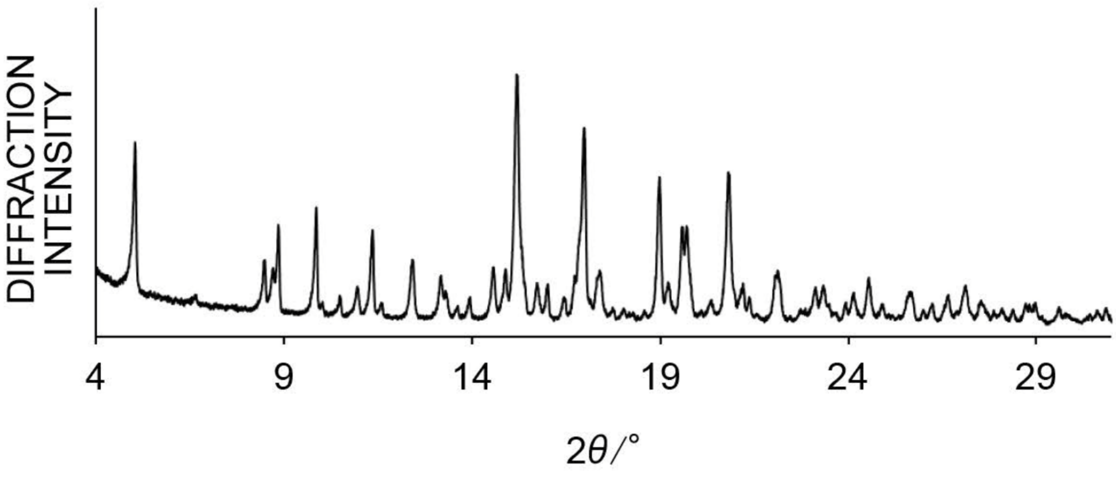

# Fig.23

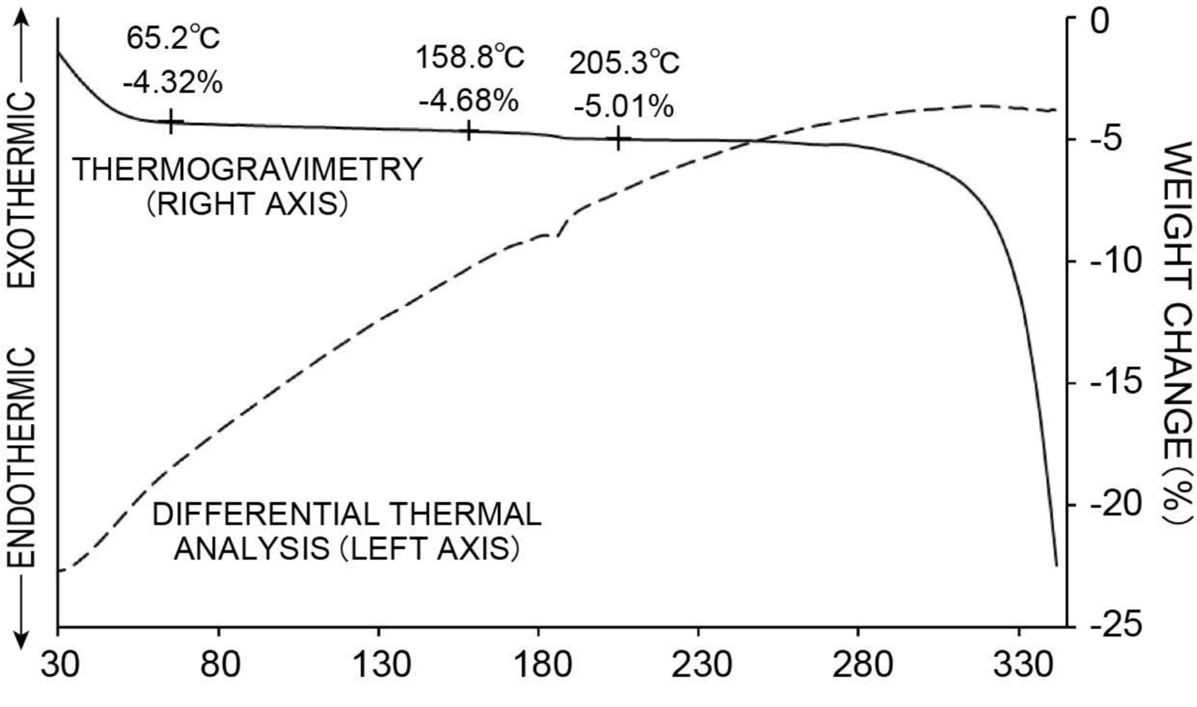
65.2℃
-4.32%
158.8℃
-4.68%
205.3℃
-5.01%
THERMOGRAVIMETRY
(RIGHT AXIS)
DIFFERENTIAL THERMAL
ANALYSIS (LEFT AXIS)
EXOTHERMIC
ENDOTHERMIC
WEIGHT CHANGE (%)
0
-5
-10
-15
-20
-25
30
80
130
180
230
280
330
TEMPERATURE (℃)

# Fig.24

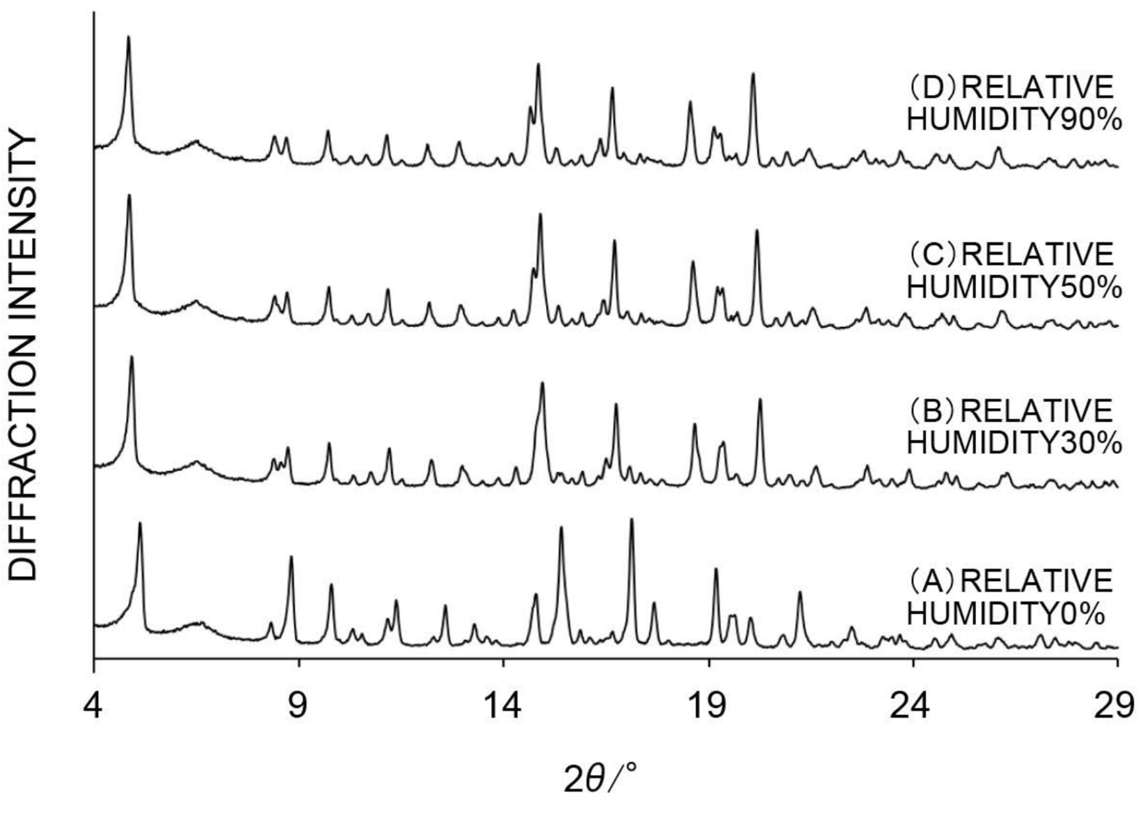
(D) RELATIVE HUMIDITY90%
(C) RELATIVE HUMIDITY50%
(B) RELATIVE HUMIDITY30%
(A) RELATIVE HUMIDITY0%
DIFFRACTION INTENSITY
4
9
14
19
24
29
2θ/°

**Fig.25**

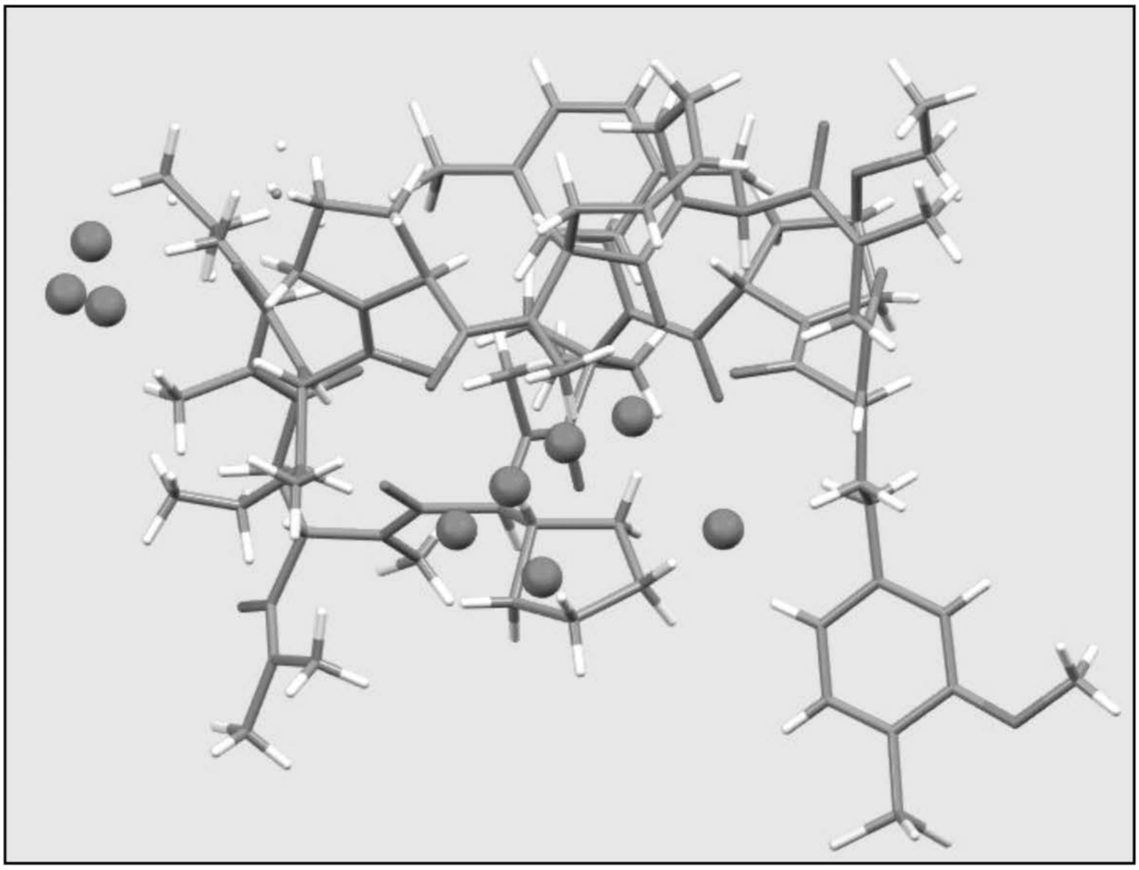

*Fig.26*

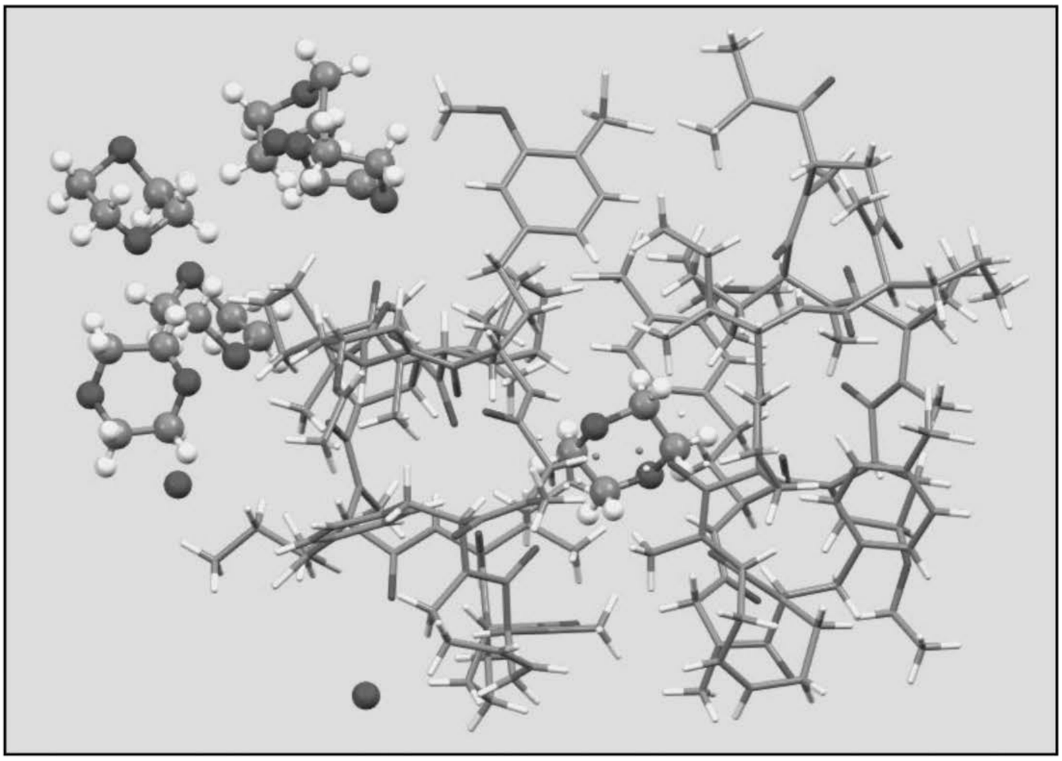

*Fig.27*

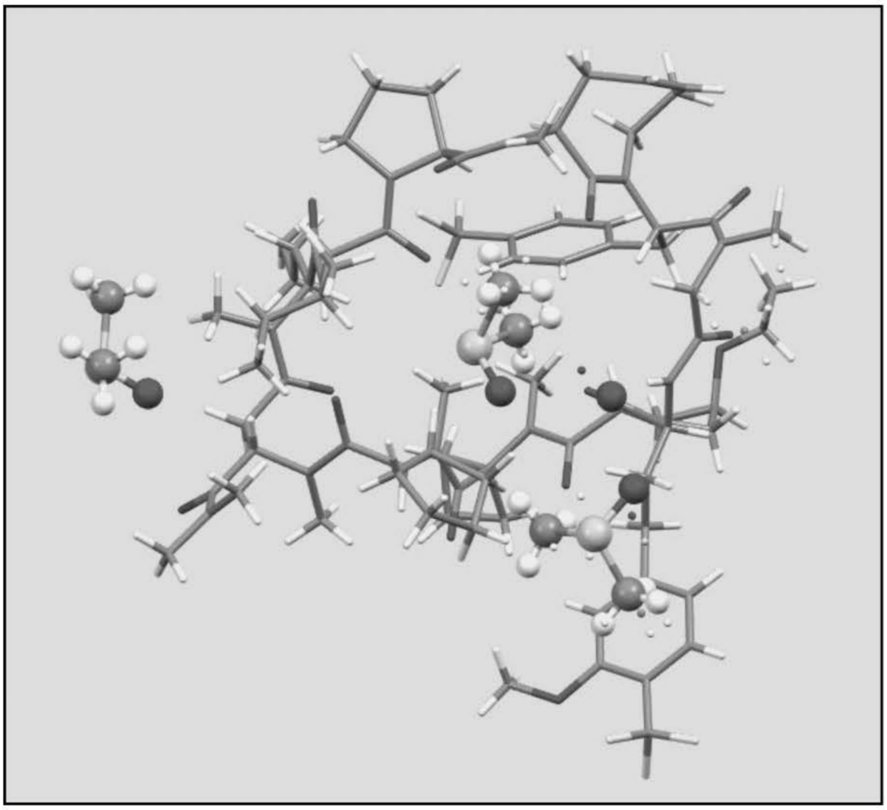

# Fig.28

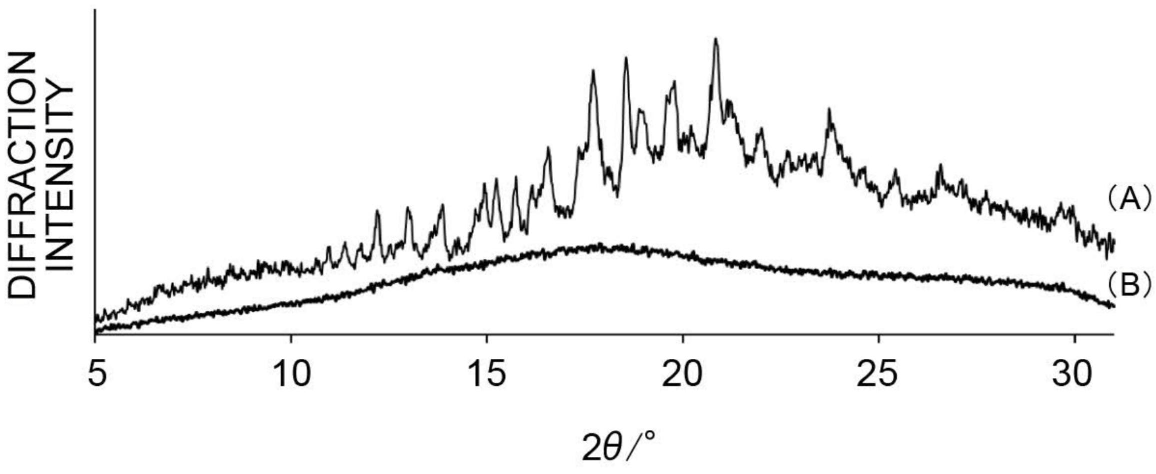

*Fig.29*

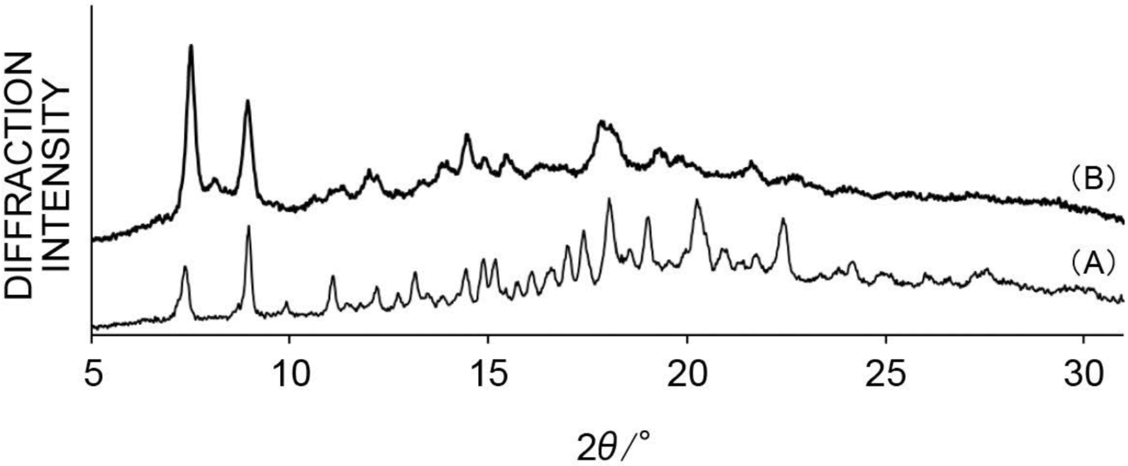

# Fig.30

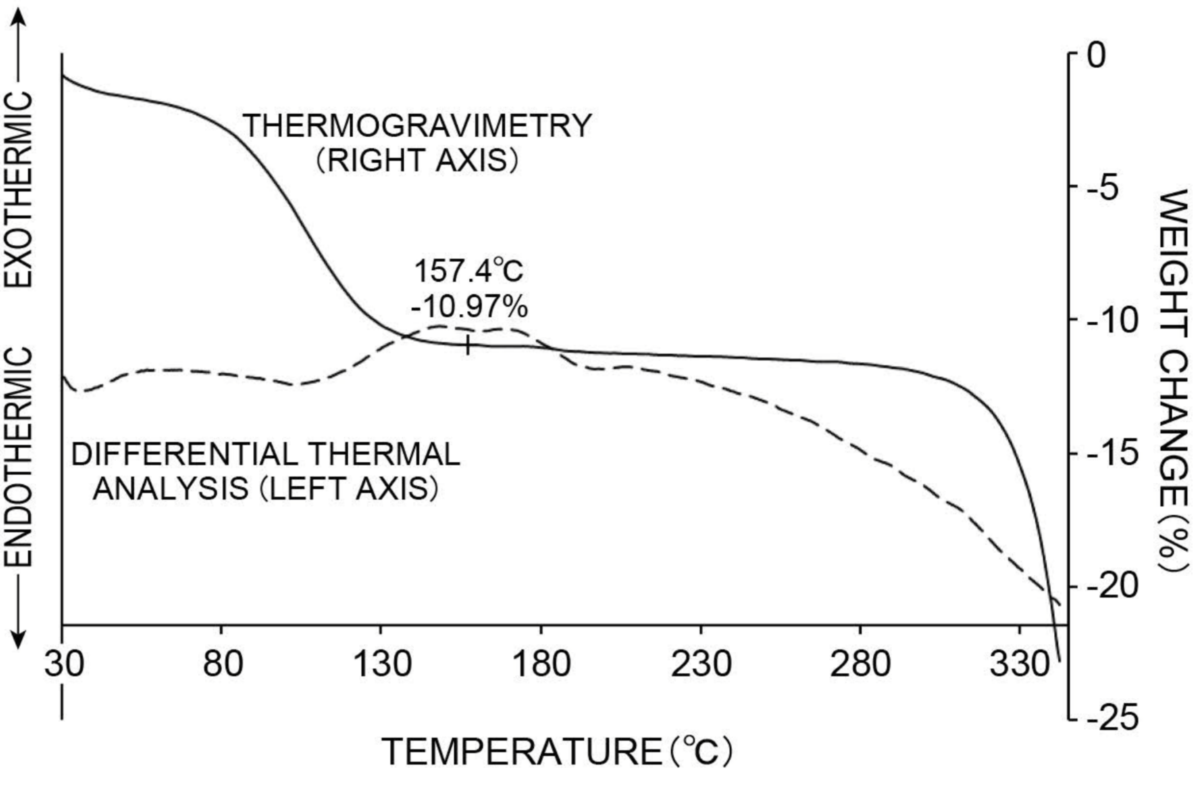
EXOTHERMIC
ENDOTHERMIC
THERMOGRAVIMETRY
(RIGHT AXIS)
157.4℃
-10.97%
DIFFERENTIAL THERMAL
ANALYSIS (LEFT AXIS)
0
-5
-10
-15
-20
-25
WEIGHT CHANGE (%)
30
80
130
180
230
280
330
TEMPERATURE (℃)

*Fig.31*

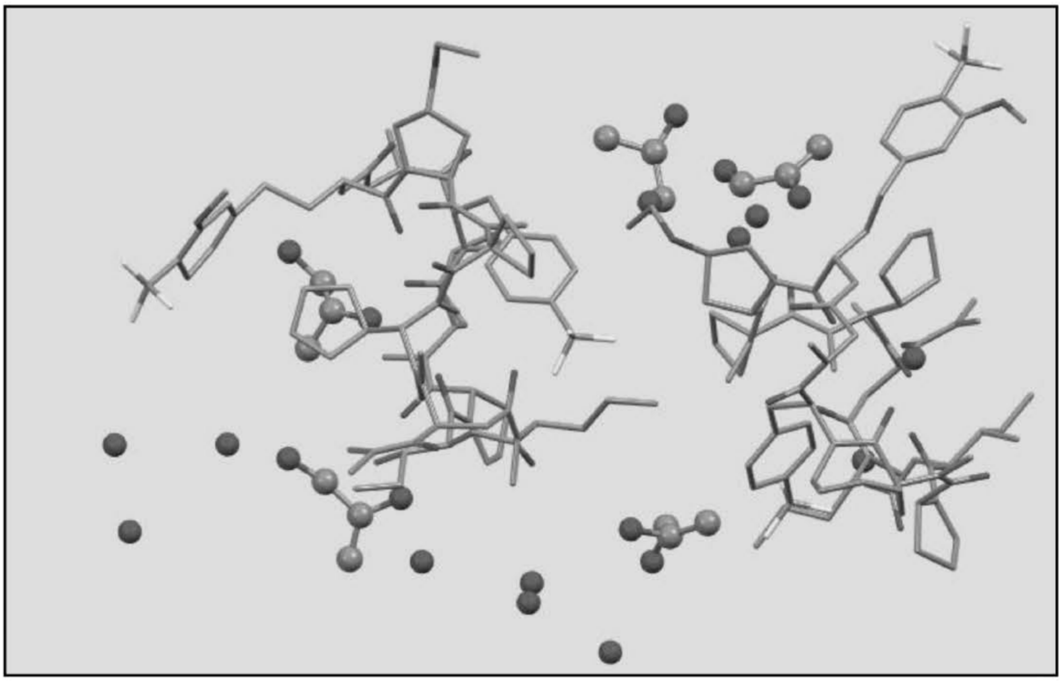

*Fig.32*

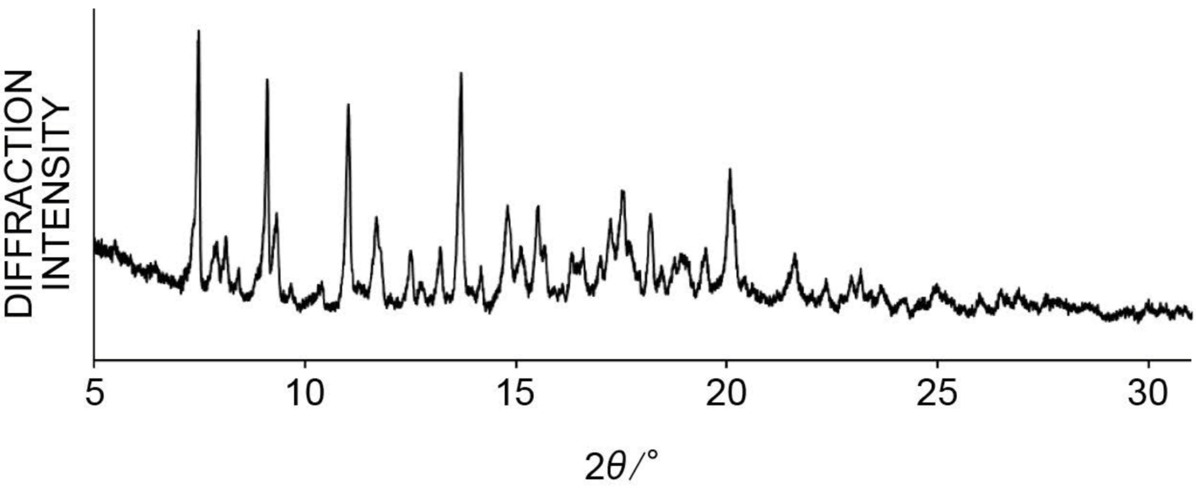

*Fig.33*

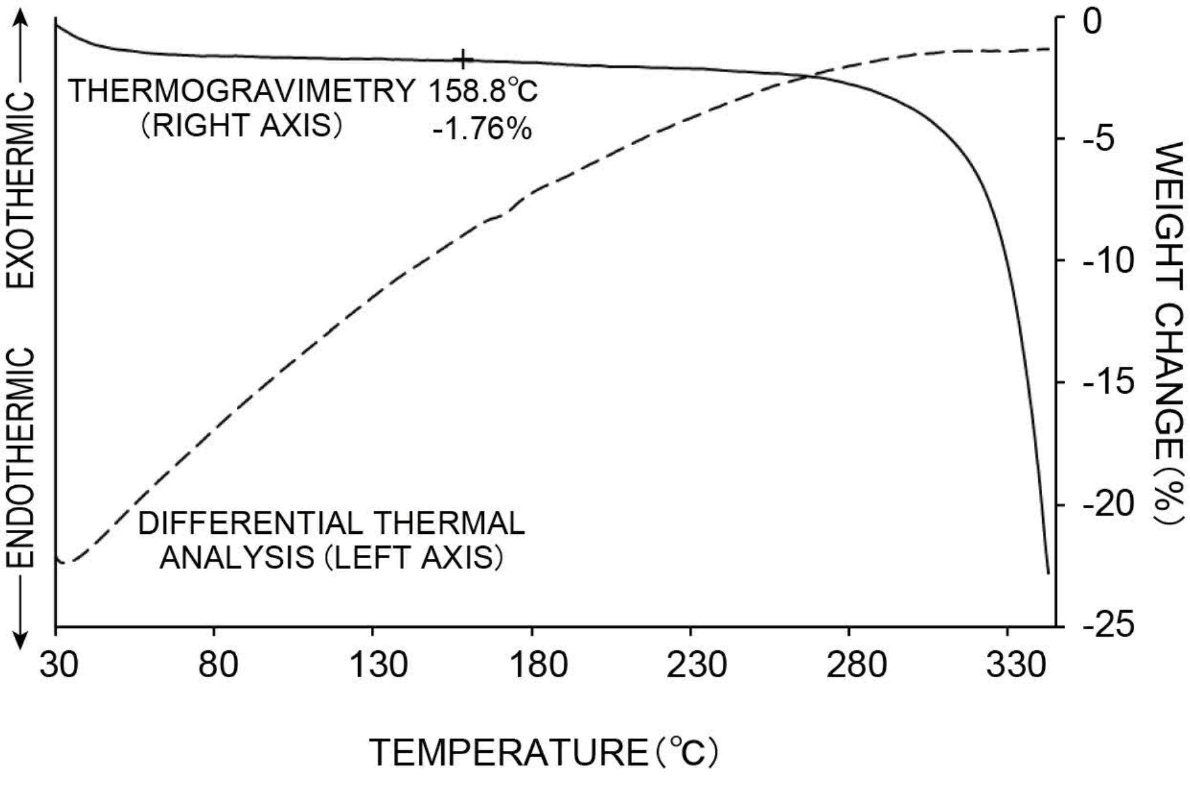
EXOTHERMIC
ENDOTHERMIC
THERMOGRAVIMETRY (RIGHT AXIS)
158.8°C
-1.76%
DIFFERENTIAL THERMAL ANALYSIS (LEFT AXIS)
0
-5
-10
-15
-20
-25
WEIGHT CHANGE (%)
30
80
130
180
230
280
330
TEMPERATURE (°C)

*Fig.34*

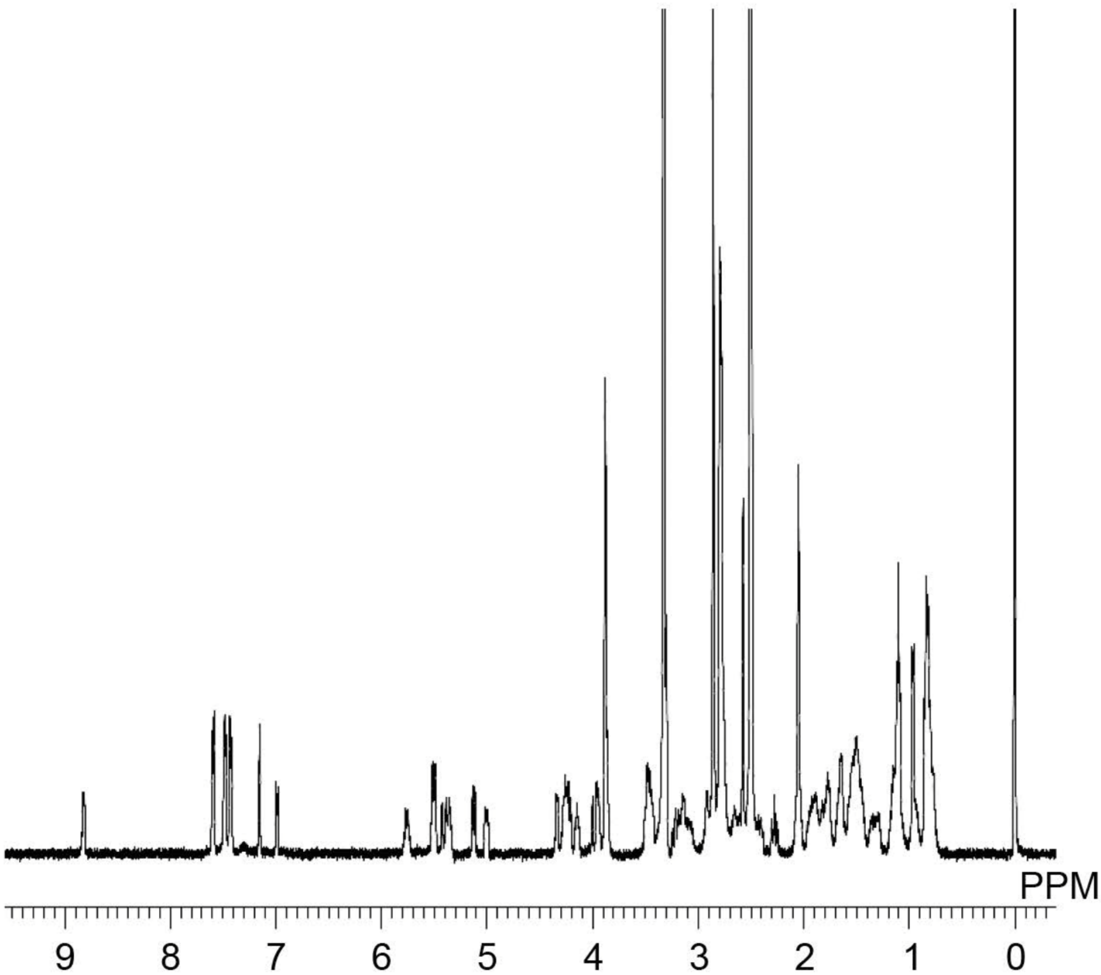

# Fig.35

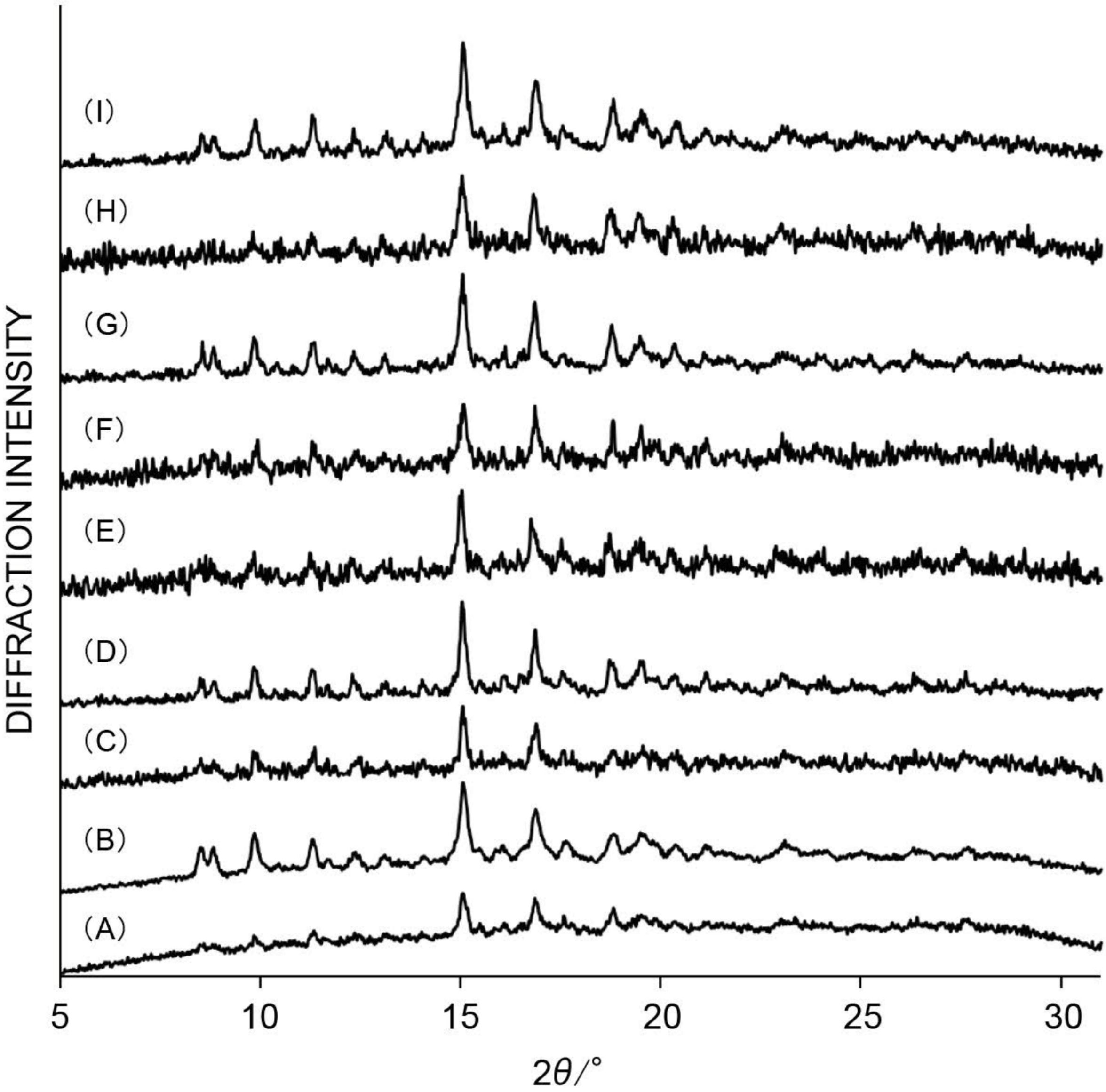

# Fig.36

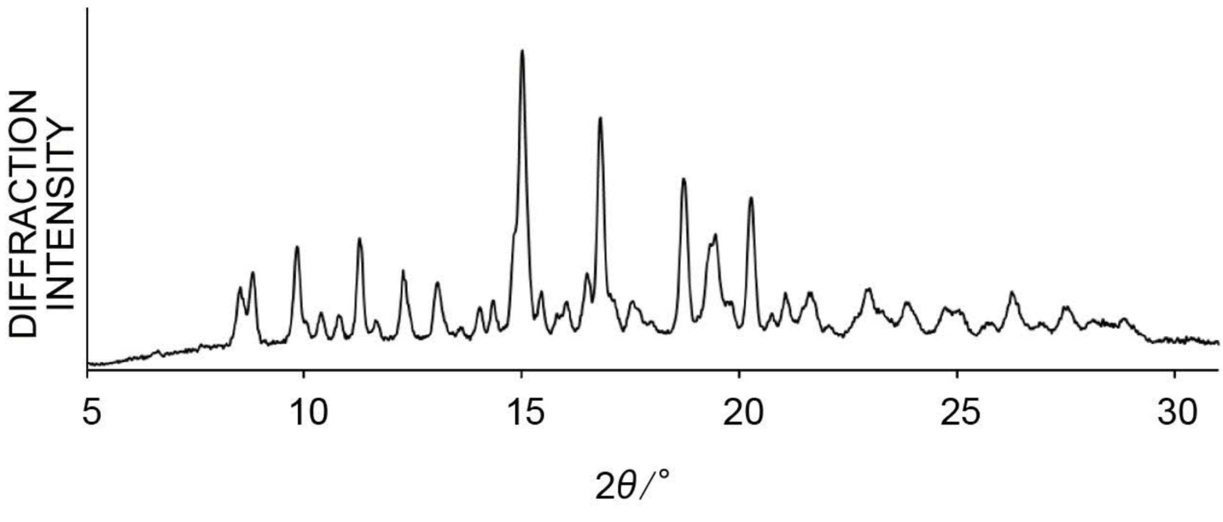

## INTERNATIONAL SEARCH REPORT

International application No.
**PCT/JP2024/015529**

### A. CLASSIFICATION OF SUBJECT MATTER

***C07K 7/52***(2006.01)i; ***C07K 1/02***(2006.01)i; ***C07K 1/30***(2006.01)i; ***C07K 1/34***(2006.01)i; ***C07K 7/06***(2006.01)i; ***C07K 7/64***(2006.01)i
FI: C07K7/52; C07K1/02; C07K1/30; C07K1/34; C07K7/06; C07K7/64

According to International Patent Classification (IPC) or to both national classification and IPC

### B. FIELDS SEARCHED

Minimum documentation searched (classification system followed by classification symbols)

C07K7/52; C07K1/02; C07K1/30; C07K1/34; C07K7/06; C07K7/64

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | WO 2022/234853 A1 (CHUGAI SEIYAKU KABUSHIKI KAISHA) 10 November 2022 (2022-11-10) entire text, all drawings | 1-16 |
| A | WO 2022/234850 A1 (CHUGAI SEIYAKU KABUSHIKI KAISHA) 10 November 2022 (2022-11-10) entire text, all drawings | 1-16 |
| A | WO 2022/138891 A1 (CHUGAI SEIYAKU KABUSHIKI KAISHA) 30 June 2022 (2022-06-30) entire text, all drawings | 1-16 |
| A | WO 2022/234864 A1 (CHUGAI SEIYAKU KABUSHIKI KAISHA) 10 November 2022 (2022-11-10) entire text, all drawings | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/015529

C. DOCUMENTS CONSIDERED TO BE RELEVANT

| Category* | Citation of document, with indication, where appropriate, of the relevant passages | Relevant to claim No. |
|---|---|---|
| A | JP 11-505208 A (FUJISAWA PHARMACEUTICAL CO., LTD.) 18 May 1999 (1999-05-18) entire text, all drawings | 1-16 |

Form PCT/ISA/210 (second sheet) (July 2022)

**INTERNATIONAL SEARCH REPORT**
**Information on patent family members**

International application No.
**PCT/JP2024/015529**

| Patent document cited in search report | Publication date (day/month/year) | Patent family member(s) | Publication date (day/month/year) |
|---|---|---|---|
| WO 2022/234853 A1 | 10 November 2022 | EP 4309741 A1<br>entire text, all drawings<br>CN 117279933 A<br>CA 3218182 A1<br>KR 10-2024-0005777 A | |
| WO 2022/234850 A1 | 10 November 2022 | EP 4299069 A1<br>entire text, all drawings<br>KR 10-2023-0020548 A<br>CN 117241817 A<br>KR 10-2023-0169093 A<br>CA 3219090 A1 | |
| WO 2022/138891 A1 | 30 June 2022 | EP 4269422 A1<br>entire text, all drawings<br>CN 116615411 A<br>KR 10-2023-0124935 A | |
| WO 2022/234864 A1 | 10 November 2022 | US 2022/0411462 A1<br>entire text, all drawings<br>EP 4086272 A1<br>KR 10-2023-0003614 A<br>CN 117279928 A<br>KR 10-2023-0169072 A<br>CA 3219081 A1 | |
| JP 11-505208 A | 18 May 1999 | US 5952299 A<br>entire text, all drawings<br>WO 1996/030399 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022234853 A
- WO 2020189540 A


### Non-patent literature cited in the description


- *Nat. Rev. Drug Discov.*, November 2014, vol. 13 (11), 828-851
- *Nat. Rev. Drug Discov.*, December 2014, vol. 13 (12), 928-942
- *Nat. Rev. Drug Discov.*, November 2016, vol. 15 (11), 771-785
- Comprehensive Organic Transformations, A Guide to Functional Group Preparations. **R. C. LAROCK**. March's Advanced Organic Chemistry: Reactions, Mechanisms
- **M. B. SMITH** ; **J. MARCH**. Structure
- *Chem. Rev.*, 2011, vol. 111, 6557-6602
- **FREIDINGER et al.** *J. Org. Chem.*, 1983, vol. 48 (1), 77-81
- **NGUYEN et al.** *Synthesis*, 2009, vol. 12, 1991
- *CHEMICAL ABSTRACTS*, 203714-71-0
- *CHEMICAL ABSTRACTS*, 250220-36-1
- *CHEMICAL ABSTRACTS*, 172222-30-9
- *CHEMICAL ABSTRACTS*, 536724-67-1
- *CHEMICAL ABSTRACTS*, 246047-72-3
- *CHEMICAL ABSTRACTS*, 301224-40-8
- *CHEMICAL ABSTRACTS*, 927429-61-6
- *CHEMICAL ABSTRACTS*, 1025728-56-6
- *CHEMICAL ABSTRACTS*, 1212009-05-6
- Greene's, 'Protective Groups in Organic Synthesis. John Wiley & Sons, 2014
- Comprehensive Organic Transformations: A Guide to Functional Group Preparations. Larock and March's Advanced Organic Chemistry: Reactions, Mechanisms
- **SMITH**. Structure
- *CHEMICAL ABSTRACTS*, 187475-29-2
- *CHEMICAL ABSTRACTS*, 1700368-07-5
- *CHEMICAL ABSTRACTS*, 1446478-31-4
- *CHEMICAL ABSTRACTS*, 42417-70-9
- *CHEMICAL ABSTRACTS*, 2411591-78-9
- *CHEMICAL ABSTRACTS*, 1408729-60-1